(19) Europäisches Patentamt
European Patent Office
Office européen des brevets

(11) EP 3 402 822 B1

(12) **EUROPEAN PATENT SPECIFICATION**

(45) Date of publication and mention of the grant of the patent:
**12.03.2025 Bulletin 2025/11**

(21) Application number: **17701812.4**

(22) Date of filing: **13.01.2017**

(51) International Patent Classification (IPC):
***C07K 16/28*** *(2006.01)* ***A61P 35/00*** *(2006.01)*

(52) Cooperative Patent Classification (CPC):
**C07K 16/2863; A61K 39/001102; A61K 47/6801; A61K 47/68031; A61K 47/6849; A61K 47/6851; A61K 47/6855; A61K 47/6857; A61K 47/6869; A61P 35/00;** A61K 2039/505; C07K 2317/33; C07K 2317/34; C07K 2317/56; C07K 2317/732;
(Cont.)

(86) International application number:
**PCT/EP2017/050718**

(87) International publication number:
**WO 2017/121877 (20.07.2017 Gazette 2017/29)**

(54) **AXL-SPECIFIC ANTIBODY-DRUG CONJUGATES FOR CANCER TREATMENT**

AXL-SPEZIFISCHE ANTIKÖRPER-WIRKSTOFF-KONJUGATE ZUR KREBSBEHANDLUNG

CONJUGUÉS ANTICORPS-MÉDICAMENT SPÉCIFIQUES D'AXL POUR LE TRAITEMENT DU CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **13.01.2016 US 201662278283 P**
**08.07.2016 PCT/EP2016/066353**

(43) Date of publication of application:
**21.11.2018 Bulletin 2018/47**

(73) Proprietor: **Genmab A/S**
**2500 Valby (DK)**

(72) Inventors:
- **BOSHUIZEN, Julia**
**1058 NC Amsterdam (NL)**
- **BREIJ, Esther**
**3584 CT Utrecht (NL)**
- **KOOPMAN, Louise**
**3584 CT Utrecht (NL)**
- **SATIJN, David**
**3584 CT Utrecht (NL)**
- **VAN DEN BRINK, Edward**
**3584 CT Utrecht (NL)**
- **VERZIJL, Dennis**
**3584 CT Utrecht (NL)**
- **DE JONG, Rob**
**3584 CM Utrecth (NL)**
- **VAN DIJKHUIZEN RADERSMA, Riemke**
**3584 CT Utrecht (NL)**
- **PEEPER, Daniel**
**1183 DN Amstelveen (NL)**
- **PARREN, Paul**
**NL-3984 PR Odijk (NL)**

(74) Representative: **Genmab A/S**
**Carl Jacobsens Vej 30**
**2500 Valby (DK)**

(56) References cited:
**WO-A1-2014/068139 WO-A1-2014/174111**
**WO-A1-2015/193430 WO-A1-2016/005593**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 402 822 B1

- **COIT DANIEL G ET AL: "Cutaneous Melanoma, Version 2.2019, NCCN Clinical Practice Guidelines in Oncology.", JOURNAL OF THE NATIONAL COMPREHENSIVE CANCER NETWORK : JNCCN 01 04 2019, vol. 17, no. 4, 1 April 2019 (2019-04-01), pages 367 - 402, ISSN: 1540-1413**
- **MONTAGUT C ET AL: "Targeting the RAF-MEK-ERK pathway in cancer therapy", CANCER LETTERS, NEW YORK, NY, US, vol. 283, no. 2, 8 October 2009 (2009-10-08), pages 125 - 134, XP026421117, ISSN: 0304-3835, [retrieved on 20090212]**
- **E. J. MORRIS ET AL: "Discovery of a Novel ERK Inhibitor with Activity in Models of Acquired Resistance to BRAF and MEK Inhibitors", CANCER DISCOVERY, vol. 3, no. 7, 1 July 2013 (2013-07-01), US, pages 742 - 750, XP055219076, ISSN: 2159-8274, DOI: 10.1158/2159-8290.CD-13-0070**
- **HATZIVASSILIOU G ET AL: "ERK inhibition overcomes acquired resistance to MEK Inhibitors", MOLECULAR CANCER THERAPEUTICS, AMERICAN ASSOCIATION FOR CANCER RESEARCH, US, vol. 11, no. 5, 11 May 2012 (2012-05-11), pages 1143 - 1154, XP002730627, ISSN: 1535-7163, [retrieved on 20120308], DOI: 10.1158/1535-7163.MCT-11-1010**
- **DAN LI ET AL: "Axl-Targeted Cancer Imaging with Humanized Antibody h173", MOLECULAR IMAGING AND BIOLOGY, vol. 16, no. 4, 15 January 2014 (2014-01-15), pages 511 - 518, XP055210547, ISSN: 1536-1632, DOI: 10.1007/s11307-013-0714-z**
- **ESTHER BREIJ: "Novel antibody-drug conjugates targeting Axl show anti-tumor activity in solid cancer xenograft models", AMERICAN ASSOCIATION OF CANCER RESEARCH, 19 April 2015 (2015-04-19), pages 1 - 2, XP055296816**
- **E BREIJ: "Preclinical efficacy studies using HuMax-Axl-ADC, a novel antibody-drug conjugate targeting Axl-expressing solid cancers.", JOURNAL OF CLINICAL ONCOLOGY, vol. 33, 29 May 2015 (2015-05-29), pages 1 - 2, XP055297040**


Remarks:

•The complete document including Reference Table(s) and the Sequence Listing(s) can be downloaded from the EPO website

•The file contains technical information submitted after the application was filed and not included in this specification

(52) Cooperative Patent Classification (CPC): (Cont.)
C07K 2317/77; C07K 2317/92

## Description

FIELD OF THE INVENTION

The present invention relates to antibody-drug conjugates (ADCs) binding to human AXL for therapeutic use, particularly for treatment of melanoma in combination with at least one therapeutic agent.

BACKGROUND OF THE INVENTION

AXL is a 104-140 kDa transmembrane protein which belongs to the TAM subfamily of mammalian Receptor Tyrosine Kinases (RTKs) and which has transforming abilities (Paccez *et al.,* 2014). Enhanced or *de novo* expression of AXL has been reported in a variety of cancers, including gastric, prostate, ovarian, and lung cancer (Paccez *et al.,* 2014). Notably, several types of cancer with resistance to tyrosine kinase inhibitors, serine/threonine kinase inhibitors and/or chemotherapy have been found to show enhanced or de novo expression of AXL protein (Wilson *et al.,* 2014; Brand *et al.,* 2015; Zhang *et al.,* 2012; Blakely *et al.,* 2012). In particular, melanoma cells with resistance to inhibitors of the serine/threonine kinases B-raf (BRAF), MEK and ERK (MEK also being a tyrosine kinase) showed enhanced or de novo AXL expression (Müller *et al.,* 2014; Konieczkowski *et al.,* 2014). BRAF, MEK and ERK are all part of the Mitogen Activated Protein Kinase (MAPK) pathway. The majority of malignant melanomas harbor oncogenic mutations in BRAF or NRAS, which can result in a constitutively active MAPK pathway (Sullivan *et al.,* 2016).

The AXL extracellular domain is composed of a combination of two membrane-distal N-terminal immunoglobulin (Ig)-like domains (Ig1 and Ig2 domains) and two membrane-proximal fibronectin type III (FNIII) repeats (the FN1- and FN2-domains) (Paccez *et al.,* 2014). AXL can be activated upon binding of its ligand, the vitamin K-dependent growth arrest-specific factor 6 (Gas6). Gas6-binding to AXL leads to AXL dimerization, autophosphorylation and subsequent activation of intracellular signaling pathways, such as the PI3K/AKT, mitogen-activated protein kinase (MAPK), STAT and NF-κB cascades (Leconet *et al.,* 2013). In cancer cells, AXL expression has been associated with tumor cell motility, invasion, migration, and is involved in epithelial-to-mesenchymal transition (EMT) (Linger *et al.,* 2010). Anti-AXL antibodies have been described that attenuate NSCLC and breast cancer xenograft growth *in vivo* by downregulation of receptor expression, reducing tumor cell proliferation and inducing apoptosis (Li *et al.,* 2009; Ye *et al.,* 2010; WO 2011/159980, Genentech). Various other anti-AXL antibodies have also been reported (Leconet *et al.,* 2013; Iida *et al.,* 2014; WO 2012/175691, INSERM; WO 2012/175692, INSERM; WO 2013/064685, Pierre Fabré Medicaments; WO 2013/090776, INSERM; WO 2009/063965, Chugai Pharmaceuticals, WO 2010/131733 and WO 2016/005593), including an ADC based on an anti-AXL antibody and a pyrrolobenzo-diazepine (PBD) dimer (WO 2014/174111, Pierre Fabré Medicament and Spirogen Sarl).

Breij et al., American Association of cancer research, abstract, pages 1-2 2015, describes the use of anti Axl antibody drug conjugates (ADC) to treat lung cancer and epidermoid carcinoma. The anti-Axl antibodies were conjugated to MMAE.

Breij et al., Journal of clinical oncology, vol. 33, pages 1-2, abstract 2015, describes the use of Axl ADC conjugated to MMAE efficiently killing lung cancer cells *in vivo.*

WO 2014/068139 describes anti-Axl antibodies 1003A2 and 1024G11 and their conjugation to saporin for killing various tumor cell lines.

WO 2016/005593 describes inhibitory anti-Axl antibodies inhibiting tumor growth and not competing with the ligand GAS6 for binding to Axl and their use as ADC with MMAE to kill various cancer types *in vivo,* such as breast cancer, lung cancer, cervical carcinoma, esophageal cancer, epidermiod carcinoma, and pancreatic cancer.

WO 2015/193430 describes anti-Axl antibodies and ADC thereof to be used for treating cancers such as melanomas in combination with other chemotherapeutics, such as sorafenib.

However, there remains a need for improved AXL-ADC-based methods of treating melanoma, particularly in view of the resistance to MAPK inhibitors.

SUMMARY OF THE INVENTION

It has been found by the present inventor(s) that ADCs based on anti-AXL antibodies (also referred to as "AXL-ADCs" herein) can be used to efficiently treat melanoma in combination with one or more inhibitors of the MAPK pathway.

So, in one aspect, the invention relates to an ADC comprising an antibody binding to human AXL, for use in treating a melanoma in combination with one or more inhibitors of the MAPK pathway, selected from a BRAF inhibitor, a MEK inhibitor, an ERK inhibitor, or a combination of any two or more thereof. The ADC and the one or more inhibitors may, for example, be administered simultaneously, separately or sequentially.

This aspect of the invention and other aspects and embodiments, including the use of AXL-ADCs based on anti-AXL antibodies characterized by their antigen-binding properties or - sequences, therapeutic moieties suitable for such

ADCs, combinations of such ADCs with certain inhibitors, and related methods of treating melanoma, are described in further detail below. Indeed, each and every aspect or embodiment relating to an AXL-ADC for use in treating melanoma in combination with one or more inhibitors according to the invention is equally applicable as an aspect or embodiment relating to a method of treating melanoma by administering an AXL-ADC and one or more inhibitors, and vice versa. Moreover, any AXL-ADC as defined in any aspect or embodiment herein can be used in combination with one or more inhibitors of the MAPK pathway, e.g., serine/threonine kinase inhibitors, as described herein.

LEGENDS TO THE FIGURES

**Figure 1:** Binding curves of anti-AXL antibodies to HEK293 cells transfected with (A) human AXL-ECD, (B) cynomolgus AXL-ECD, or (C) mouse AXL-ECD. Data shown are mean fluorescence intensities (MFI) of one representative experiment, as described in Example 2.
**Figure 2:** Binding of anti-AXL antibodies to mouse-human AXL chimeras was performed as described in Example 3. The following *Homo sapiens* AXL (hsAXL) and *Mus musculus* AXL (mmAXL) chimeric proteins were tested: (A) hsAXL and mock, (B) hsAXL-mmECD, (C) hsAXL-mmlg1, (D) hsAXL-mmlg2, (E) hsAXL-mmFN1, (F) hsAXL-mmFN2.
**Figure 3:** Anti-AXL antibody-dependent cell-mediated cytotoxicity in A431 cells. Antibody-dependent cell-mediated cytotoxicity by anti-AXL antibodies in A431 cells was determined as described in Example 4.
**Figure 4:** Binding characteristics of AXL antibody-drug conjugates (AXL-ADCs). Binding of AXL-ADCs on HEK293T cells transiently transfected with human AXL was determined as described in Example 5. Data shown are mean fluorescence intensities (MFI) of one representative experiment.
**Figure 5:** *In vitro* cytotoxicity induced by AXL antibody-drug conjugates. Induction of cytotoxicity by AXL antibody-drug conjugates was determined as explained in Example 6.
**Figure 6:** Antibody VH and VL variants that allow binding to AXL. Antibodies with identical VL or VH regions were aligned and differences in VH (Figures A-D) or VL (Figure E) sequences, respectively, were identified and indicated by boxes in the figures. CDR regions are underlined.
**Figure 7:** Induction of cytotoxicity by ADCs in LCLC-103H cells was determined as described in Example 8.
**Figure 8:** Anti-tumor activity by MMAE-conjugated AXL antibodies in a therapeutic LCLC-103H xenograft model as described in Example 9.
**Figure 9:** Immunohistochemical staining of frozen PAXF1657 tumor sections (pancreas cancer PDX model) using a pool of AXL monoclonal antibodies as described in Example 10.
**Figure 10:** (A) Average tumor size after therapeutic treatment with AXL-ADCs the PAXF1657 model. An unconjugated AXL Humab (C) and an untargeted ADC (D) do not show anti-tumor activity, indicating that the therapeutic capacity of AXL-ADCs was dependent on the cytotoxic activity of MMAE and on target binding, error bars represent S.E.M.
**Figure 11:** Binding of anti-AXL antibodies to mouse-human AXL chimeras was performed as described in Example 11. The following Homo sapiens AXL (hsAXL) and Mus musculus AXL (mmAXL) chimeric proteins were tested: (A) hsAXL and mock, (B) hsAXL-mmECD, (C) hsAXL-mmlg1, (D) hsAXL-mmlg2, (E) hsAXL-mmFN1, (F) hsAXL-mmFN2.
**Figure 12:** Binding of human Gas6 (hGas6) on A431 cells that had been pre-incubated with antibodies binding to the Ig1 domain of AXL. Data shown are mean fluorescence intensities (MFI) of one representative experiment.
**Figure 13:** Anti-tumor activity of MMAE-conjugated AXL antibodies in a therapeutic A431 xenograft model, that produces high levels of endogeneous Gas6, as described in Example 13. Panels A and B show results from 2 independent experiments.
**Figure 14:** Anti-tumor activity of MMAE-conjugated AXL antibodies in a therapeutic LCLC-103H xenograft model, that expresses low levels of endogenous Gas6, as described in Example 13. Panels A and B show results from 2 independent experiments.
**Figure 15:** Induction of cytotoxicity by AXL-ADCs in A431 cells (A) and MDA-MB231 cells (B) was determined as described in Example 8.
**Figure 16.** AXL staining in thyroid, esophageal, ovarian, breast, lung, pancreatic, cervical and endometrial cancer. The average AXL staining intensity (OD) of AXL-positive cells is plotted on the X-axis, and the percentage of AXL-positive tumor cells is plotted on the Y-axis. Each dot represents a tumor core, derived from an individual patent.
**Figure 17.** Representative examples of AXL-immunostained tumor cores for different tumor indication.
**Figure 18.** AXL antibodies specifically bind AXL but not to other TAM receptor family members. Binding of HuMab-AXL antibodies to HEK293 cells transfected with human AXL (A), human MER (B), human TYRO3 (C), or untransfected HEK293 cells (D). To confirm proper expression of transfected cells, untransfected HEK293F cells

and cells transfected with AXL (E), MER (F), or TYRO3 (G) were stained with MER- and TYRO3-specific antibodies. Data shown are mean fluorescence intensities (MFI) of one representative experiment, as described in Example 15.

**Figure 19.** Detection of AXL antibodies on the plasma membrane of tumor cell lines that had been incubated with AXL-antibodies for 1 hour at 4°C, followed by an overnight incubation 4°C or 37°C. In both MDA-MB-231 (A and B) and Calu-1 cells (C and D), more antibody was detected on the plasma membrane of cells that had been incubated at 4°C than on cells that had been incubated at 37°C, illustrating internalization of membrane-bound antibody at 37°C.

**Figure 20.** Geomean fluorescence intensity of LCLC-103H cells after incubation with AXL antibodies that had been complexed to Fab-TAMRA/QSY7. IgG1-b12 and Fab-TAMRA/QSY7 alone were included as negative controls.

**Figure 21.** AXL expression in established melanoma cell lines and patient-derived low passage primary melanoma lines (PDX). (A) Variable levels of AXL expression were observed in established melanoma cell lines. Enhanced or de novo AXL expression was observed in PLX4720 resistant cell lines (A375-R, SKMEL28R, SKMEL147). (B) AXL expression was observed in 8/15 patient derived primary melanoma lines. In both established melanoma cell lines and low passage PDX cultures, AXL expression was inversely correlated with MITF expression.

**Figure 22.** AXL protein expression on the cell surface. Examples of AXL expression as determined by quantitative flow cytometry in an Axl-negative and an Axl-positive melanoma cell line. The light gray plots represent staining with AXL-specific antibodies, while the dark grey plots represent staining with isotype control antibody.

**Figure 23.** Sensitivity of established melanoma cell lines to IgG1-AXL-107-vcMMAE. Melanoma cell lines (A-F; CDX) were treated with IgG1-AXL-107-vcM MAE or the isotype control ADC IgG1-b12-vcMMAE for 5 days in triplicate. Cell viability was assessed with a CellTiter-Glo assay and plotted against the ADC concentration.

**Figure 24.** Sensitivity of primary melanoma cell cultures to IgG1-AXL-107-vcMMAE. Low passage primary melanoma cell lines (A-C; PDX) were treated with IgG1-AXL-107-vcMMAE or the isotype control ADC IgG1-b12-vcMMAE for 8 days in triplicate. Cell viability was assessed with a CellTiter-Glo assay and plotted against the ADC concentration.

**Figure 25.** Anti-tumor efficacy of IgG1-AXL-107-vcMMAE in the melanoma model SKMEL147. Average tumor size after therapeutic treatment with IgG1-b12, IgG1-b12-vcMMAE, IgG1-AXL-107, or IgG1-AXL-107-vcMMAE is shown (A). Tumor size in IgG1-AXL-107-vcMMAE mice that were observed (n=2) or retreated with IgG1-AXL-107-vcMMAE (n=4) is shown in (B).

**Figure 26.** SKMEL28 wild-type cells (red) and PLX4720-resistant SKMEL28-R cells (green) were mixed 1:1 and treated with IgG1-AXL-107-vcMMAE (AXL-ADC), IgG1-b12-MMAE (b12-ADC), PLX4720 (PLX), dabrafenib (dabr), trametinib (tram), or combinations as indicated. (A) Total cell numbers relative to untreated cells. (B) GFP/mCherry ratio corresponding to the ratio SKMEL28-R/SKMEL28 cells.

**Figure 27.** Examples of Axl expression detected by immunohistochemistry in primary melanoma samples. (A) Example of melanoma with positive +++ Axl staining intensity (B) Example of melanoma with positive Axl staining intensity between + and ++ (C) Example of Axl expression in melanoma tissues from the same patient pre- and post-treatment with vemurafenib; left = pre-vemurafenib, Axl staining intensity weakly + ; right = post-vemurafenib, Axl staining intensity weakly + to ++ (D) Example of heterogeneous Axl expression with ++ intensity within primary melanoma tissue.

**Figure 28.** Therapeutic effect of IgG1-AXL-107-vcMMAE in the melanoma xenograft model M019R, which is described in Example 18 and 19. (A) Average tumor size after therapeutic treatment with IgG1-AXL-107-vcMMAE, IgG1-b12-vcMMAE, or dabrafenib plus trametinib. (B) Tumor size in individual mice on day 33 after tumor cell inoculation. ****, $p<0.0001$. (C) Kaplan-Meyer graph of groups that were retreated with the combination of dabrafenib plus trametinib (dab/tram), IgG1-AXL-107-vcMMAE, or the triple combination of dab/tram and IgG1-AXL-107-vcMMAE after initial treatment with dab/tram for 30 days as indicated.

**Figure 29.** Therapeutic effect of IgG1-AXL-107-vcMMAE in the melanoma xenograft model M009R, which is described in Example 18 and 20. (A) Average tumor size after therapeutic treatment with IgG1-b12-vcMMAE (control ADC), IgG1-AXL-107-vcMMAE, IgG1-b12-vcMMAE plus dabrafenib plus trametinib, or IgG1-AXL-107-vcMMAE plus dabrafenib plus trametinib. (B) Tumor size in individual mice on day 14 after first treatment. **, $p<0.01$; ***, $p<0.001$.

**Figure 30.** *In vitro* cytotoxicity induced by IgG1-AXL-107-vcMMAE in NRAS-mutant melanoma cell lines. Induction of cytotoxicity by AXL antibody-drug conjugates was determined as explained in Example 21.

**Figure 31.** Expression of Axl in NRAS-mutant melanoma tissues was determined by immunohistochemistry. The H-score in each sample was calculated based on the percentage of Axl-positive tumor cells and staining intensity (1+, 2+, 3+) of Axl-positive tumor cells, as described in Example 22.

## DETAILED DISCLOSURE OF THE INVENTION

The present invention is based, at least in part, on the surprising discovery that in *in vivo* tumor models of melanoma resistant to BRAF inhibitors, a triple combination of AXL-ADC, a BRAF inhibitor (dabrafenib) and a MEK inhibitor (trametinib) was more efficient than, *e.g.,* AXL-ADC alone, the combination of the BRAF and MEK inhibitors alone (Example 19), or a combination of the BRAF and MEK inhibitors with a control ADC (Example 20). This was the case even

when the melanoma model was insensitive to treatment with AXL-ADC as a single agent *in vitro* (at 1μg/mL) or *in vivo* (Example 20). Further, *in vitro* studies of mixtures of BRAF inhibitor sensitive melanoma cells and melanoma cells resistant to a BRAF inhibitor (PLX4720) showed that combinations of AXL-ADC and a BRAF inhibitor (PLX4720 or dabrafenib) or a triple combination of AXL-ADC, BRAF inhibitor (dabrafenib) and MEK inhibitor (trametinib) eradicated both BRAF inhibitor sensitive and BRAF inhibitor resistant cells (Example 17). Finally, in 9 out of 10 tumor samples from advanced, NRAS mutant melanoma patients, AXL expression was detected in at least a subset of the tumor cells (Example 22).

These and other results reported herein indicate that combinations of AXL-ADC and one or more inhibitors of MAPK pathway kinases, e.g., inhibitors of kinases such as BRAF, MEK and ERK are suitable for treating melanoma.

*Therapeutic applications*

The invention provides an AXL-ADC, e.g., HuMax-AXL-ADC, for use in treating melanoma in a subject in combination with one or more inhibitors of the MAPK pathway, selected from a BRAF inhibitor, a MEK inhibitor, and a combination of a BRAF inhibitor and a MEK inhibitor. The AXL-ADC and inhibitor(s) can be administered simultaneously, separately or sequentially. Typically, however, they are administered separately, according to different dosage regimens. Examples of dosage regimens are described herein. Based on the present disclosure and the level of skill in the art, however, other suitable dosage regimens can be envisioned and implemented by the skilled artisan, *e.g.,* a physician.

A "MAP kinase pathway inhibitor", "MAPK pathway inhibitor, "an inhibitor of the MAPK pathway" or "MAPK inhibitor" as used herein refers to a compound, typically a pharmaceutical compound, which inhibits at least one enzyme in the MAPK pathway, resulting in blocking of its serine/threonine/tyrosine kinase activity. The MAPK pathway is a well-known intracellular signalling pathway consisting of a series of proteins that communicates a signal from a tyrosine kinase receptor on the surface of the cell to the DNA in the nucleus of the cell. Activation of the pathway involves subsequent phosphorylation of a number of serine/threonine/tyrosine kinases. These are generally named MAPKKK (*e.g.,* RAF), MAPKK (*e.g.,* MEK) and MAPK (*e.g.,* ERK). The RAF protein kinase family includes the serine/threonine kinases A-RAF, B-RAF (BRAF) and C-RAF, all sharing RAS as a common upstream activator. MEK1 and MEK2 are dual specificity kinases, catalyzing phosphorylation of both tyrosine and threonine on *e.g.* ERK1 and ERK2. ERK1 and ERK2, in turn, catalyze phosphorylation of cytoplasmic and nuclear substrates. Inhibitors of one or more enzymes of the MAPK pathway are known and/or are in clinical development for treatment of melanoma and other malignancies (see, *e.g.,* Table 1 and the references cited therein). Examples of MAPK pathway inhibitors are set forth in Table 1, and include inhibitors of the serine/threonine/tyrosine kinases BRAF, MEK and ERK.

A "serine/threonine kinase inhibitor" or "S/Th KI", as used herein, refers to a compound, typically a pharmaceutical, which inhibits at least the serine/threonine kinase activity of a serine/threonine/tyrosine kinase such as BRAF, MEK or ERK. Serine/threonine kinases are enzymes responsible for the phosphorylation of the hydroxyl-group of a serine or threonine residue, a step that S/Th KIs inhibit, either directly or indirectly. Phosphorylation of serines or threonines results in the activation of intracellular signal transduction cascades. Examples of S/Th KIs useful for cancer therapy, and their targets, are shown in Table 1 below, and include BRAF-inhibitors such as vemurafenib, dabrafenib, encorafenib, sorafenib and analogs or derivatives thereof and MEK inhibitors such as trametinib, cobimetinib, binimetinib, selumetinib and analogs and derivatives thereof. In one embodiment, the term "serine/threonine kinase inhibitor" as used herein refers to a compound which specifically inhibits the protein phosphorylation activity of a serine/threonine kinase, *e.g.,* the serine/threonine kinase activity of MEK, ERK, BRAF and/or mutants thereof (*e.g.*, a BRAF V600 mutant).

A "serine/threonine/tyrosine kinase inhibitor" or "S/Th/T KI" as used herein refers to a compound, typically a pharmaceutical, which inhibits one or both of the serine/threonine and tyrosine kinase activity of a kinase having both types of kinase activity, such as MEK.

As used herein, a "BRAF inhibitor" or "BRAFi" is an inhibitor of the serine/threonine kinase activity of human BRAF (UniProtKB - P15056 (BRAF_HUMAN), optionally also of a mutant thereof and/or an isoform thereof. In one embodiment, the BRAF inhibitor inhibits the serine/threonine kinase activity of one or more mutants of human BRAF, such as those having a mutation in residue V600, L597 or K601, such as V600E. For example, a BRAFi may inhibit the serine/threonine kinase activity of the mutant BRAFi more effectively than they inhibit native human BRAF, thus being selective for the mutant BRAF (also referred to as a "mutBRAFi" herein). In another embodiment, the BRAF inhibitor inhibits the serine/threonine kinase activity of one or both of A-RAF (UniProtKB P10398 (ARAF_HUMAN)) and C-RAF (UniProtKB P04049 (RAF1_HUMAN)) and/or mutants thereof (also referred to herein as a "RAF inhibitor" or "Pan-RAF inhibitor" or "Pan-RAFi" herein). Preferred but non-limiting examples of BRAF inhibitors are listed in Table 1.

As used herein, a "MEK inhibitor" or "MEKi" as used herein is an inhibitor of at least the serine/threonine kinase activity, the tyrosine kinase activity, or both, of MEK1 (UniProtKB Q02750 (MP2K1_HUMAN)), MEK2 (UniProtKB P36507 (MP2K2_HUMAN)) or both, and may also or alternatively inhibit other MEK proteins, such as MEK5 (UniProtKB Q13163 (MP2K5_HUMAN)). Unless contradicted by context, when referring to a serine/threonine kinase inhibitor or S/Th KI of MEK herein, the inhibitor may optionally also inhibit the tyrosine kinase activity of MEK. Preferably, a MEK inhibitor inhibits the serine/threonine kinase activity of MEK1, MEK2 or both. Preferred but non-limiting examples of BRAF inhibitors are

listed in Table 1.

As used herein, an "ERK inhibitor" as used herein is an inhibitor of the serine/threonine kinase activity of ERK1 (UniProtKB P27361 (MK03_HUMAN)), ERK2 (UniProtKB P28482 (MK01_HUMAN)) or both. An ERK inhibitor may specifically inhibit one or more of ERK1 and ERK2, and may also or alternatively specifically inhibit other ERK isoforms. Preferably, an ERKi inhibits the serine/threonine kinase activity of at least one of ERK1 and ERK2. Preferred but non-limiting examples of ERK inhibitors are listed in Table 1.

*Table 1 - Examples of MAPK pathway inhibitors*

| Drug | Primary Target(s) (IC50) |
|---|---|
| Vemurafenib (PLX4032)<br>(N-[3-[[5-(4-chlorophenyl)-1H-pyrrolo[2,3-b]pyridin-3-yl]carbonyl]-2,4-difluorophenyl]-1-propanesulfonamide) | **B-Raf (V600E)** (31 nM)<br>C-Raf (48 nM)<br>MAP4K5 (KHS1) (51 nM)<br>B-Raf (100 nM)<br>(Bollag *et al., 2010*) |
| PLX4720*<br>(N-(3-(5-chloro-1H-pyrrolo[2,3-b]pyridine-3-carbonyl)-2,4-difluorophenyl) propane-1-sulfonamide) | **B-Raf (V600E)** (13 nM)<br>C-Raf-1 (Y340D/Y341D) (6.7 nM)<br>B-Raf (160 nM)<br>(Bollag *et al., 2010*) |
| Dabrafenib (GSK2118436)<br>(N-(3-(5-(2-aminopyrimidin-4-yl)-2-tert-butylthiazol-4-yl)-2-fluorophenyl)-2,6-difluorobenzenesulfonamide) | **B-Raf (V600E)** (0.8 nM)<br>C-Raf (5.0 nM)<br>B-Raf (3.2 nM)<br>(Hong *et al.,* 2012, Laguerre *et al.,* 2009) |
| Encorafenib (LGX818)<br>(Carbamic acid, N-[(15)-2-[[4-[3-[5-chloro-2-fluoro-3-[(methylsulfonyl)amino]phenyl]-1-(1-methylethyl)-1H-pyrazol-4-yl]-2-pyrimidinyl]amino]-1-methylethyl]-, methyl ester) | **B-Raf (V600E)** (EC50 4 nM)<br>(Stuart *et al.,* 2012) |
| Sorafenib (BAY 43-9006)<br>(4-[4-[[4-chloro-3-(trifluoromethyl)phenyl]carbamoylamino]phenoxy]-N-methyl-pyridine-2-carboxamide) | **Raf-1** (6 nM)<br>**B-Raf** (22 nM)<br>B-Raf (V599E) (38 nM)<br>(Wilhelm *et al.,* 2004) |
| GDC-0879<br>((E)-5-(1-(2-hydroxyethyl)-3-(pyridin-4-yl)-1H-pyrazol-4-yl)-2,3-dihydroinden-1-one oxime) | **BRAF** (0.13 nM)<br>C-Raf<br>(Wong *et al.,* 2009) |
| RAF265 (CHIR-265)<br>(1-methyl-5-[2-[5-(trifluoromethyl)-1H-imidazol-2-yl]pyridin-4-yl]oxy-N-[4-(trifluoromethyl)phenyl]benzimidazol-2-amine) | C-Raf/BRAF/BRAF V600E (3-60 nM)<br>(Mordant *et al., 2010*) |
| SB590885<br>((E)-5-(2-(4-(2-(dimethylamino)ethoxy)phenyl)-4-(pyridin-4-yl)-1H-imidazol-5-yl)-2,3-dihydroinden-1-one oxime) | **BRAF** (0.16 nM)<br>C-Raf (1.72 nM)<br>(King *et al.,* 2006) |
| AZ628<br>(3-(2-cyanopropan-2-yl)-N-(4-methyl-3-(3-methyl-4-oxo-3,4-dihydroquinazolin-6-ylamino)phenyl)benzamide) | **C-Raf-1** (29 nM)<br>**BRAF V600E** (34 nM)<br>BRAF (105 nM)<br>(Montagut *et al.,* 2008) |
| AB-024/RXDX-105/CEP-32469<br>Urea, N-[3-[(6,7-dimethoxy-4-quinazolinyl)oxy]phenyl]-N'-[5-(2,2,2-trifluoro-1,1-dimethylethyl)-3-isoxazolyl]-, hydrochloride (1:1) | BRAF, EGFR, RETi |
| TAK-580<br>((R)-2-(1-(6-amino-5-chloropyrimidine-4-carboxamido)ethyl)-N-(5-chloro-4-(trifluoromethyl)pyridin-2-yl)thiazole-5-carboxamide) | Pan-RAF |

(continued)

| Drug | Primary Target(s) (IC50) |
|---|---|
| BAL-3833/CTC3833 | Pan-RAF |
| BGB-283<br>(5-[[(1R,1aS,6bR)-1-[6-(trifluoromethyl)-1H-benzimidazol-2-yl]-1a,6b-dihydro-1H-cyclopropa[b][1]benzofuran-5-yl]oxy]-3,4-dihydro-1H-1,8-naphthyridin-2-one) | B/C-RAF and EGFR |
| GW5074<br>(2H-Indol-2-one, 3-[(3,5-dibromo-4-hydroxyphenyl)methylene]-1,3-dihydro-5-iodo-) | C-RAF (9 nM) |
| Trametinib<br>(N-(3-(3-cyclopropyl-5-(2-fluoro-4-iodophenylamino)-6,8-dimethyl-2,4,7-trioxo-3,4,6,7-tetrahydropyrido[4,3-d]pyrimidin-1(2H)-yl)phenyl)acetamide) | **MEK1/2** (0.92 nM/1.8 nM)<br>(Yamaguchi *et al., 2011*) |
| Cobimetinib<br>[3,4-difluoro-2-[(2-fluoro-4-iodophenyl)amino]phenyl][3-hydroxy-3-(2S)-2-piperidinyl-1-azetidinyl]-methanone | **MEK1** (4.2 nM)<br>(Hoeflich *et al.,* 2012) |
| Selumetinib (AZD6244)<br>(6-(4-bromo-2-chlorophenylamino)-7-fluoro-N-(2-hydroxyethoxy)-3-methyl-3H-benzo[d]imidazole-5-carboxamide) | **MEK1** (14 nM)<br>(Huynh *et al.,* 2007) |
| Binimetinib<br>(1H-Benzimidazole-6-carboxamide, 5-[(4-bromo-2-fluorophenyl)amino]-4-fluoro-N-(2-hydroxyethoxy)-1-methyl-) | **MEK1/2** (12 nM)<br>(Pheneger *et al.,* 2006) |
| Refametinib<br>((S)-N-(3,4-difluoro-2-(2-fluoro-4-iodophenylamino)-6-methoxyphenyl)-1-(2,3-dihydroxypropyl)cyclopropane-1-sulfonamide) | **MEK1** (19 nM)<br>**MEK2** (47 nM)<br>(Iverson *et al.,* 2009) |
| Pimasertib<br>((S)-N-(2,3-dihydroxypropyl)-3-(2-fluoro-4-iodophenylamino)isonicotinamide) | **MEK1/2** (5 nM-2 μM)<br>(Kim *et al., 2010)* |
| U0126-EtOH<br>(2,3-bis(amino(2-aminophenylthio)methylene)succinonitrile,ethanol) | **MEK2** (0.06 μM)<br>**MEK1** (0.07 μM) |
| PD184352<br>(2-(2-chloro-4-iodophenylamino)-N-(cyclopropylmethoxy)-3,4-difluorobenzamide) | **MEK1** (17 nM)<br>**MEK2** (17 nM)<br>(Sebolt-Leopold *et al.,* 1999) |
| BIX 02189<br>((Z)-3-((3-((dimethylamino)methyl)phenylamino) (phenyl)methylene)-N,N-dimethyl-2-oxoindoline-6-carboxamide) | **MEK5** (1.5 nM)<br>ERK5 (59 nM)<br>(Tatake *et al.,* 2008) |
| VTX11E<br>(4-[2-(2-Chloro-4-fluoroanilino)-5-methylpyrimidin-4-yl]-N-[(1S)-1-(3-chlorophenyl)-2-hydroxyethyl]-1H-pyrrole-2-carboxamide) | **ERK2** (Ki:<2 nM)<br>JNK3 (Ki:1.4 μM)<br>(Aronov *et al.,* 2009) |
| LTT-462 | ERK |
| Ulixertinib (BVD-523) | **ERK1/2**<br>ERK2 (<0.3 nM) |
| (4-(5-chloro-2-(isopropylamino)pyridin-4-yl)-N-((S)-1-(3-chlorophenyl)-2-hydroxyethyl)-1H-pyrrole-2-carboxamide) | (Ward *et al.,* 2015) |
| SCH772984 | **ERK2** (1 nM) |

(continued)

| Drug | Primary Target(s) (IC50) |
|---|---|
| ((R)-1-(2-oxo-2-(4-(4-(pyrimidin-2-yl)phenyl)piperazin-1-yl)ethyl)-N-(3-(pyridin-4-yl)-1H-indazol-5-yl)pyrrolidine-3-carboxamide) | **ERK1** (4 nM) |
| * Tool compound for PLX4032 | |

In one aspect, the disclosure provides an AXL-ADC comprising an antibody binding to human AXL, such as HuMax-AXL-ADC, for use in treating melanoma in a subject in combination with one or more inhibitors of the MAPK pathway. In one embodiment of the disclosure, the one or more inhibitors comprise an inhibitor listed in Table 1. Preferably, an inhibitor for use according to the invention is selected from a BRAF inhibitor, a MEK inhibitor and an ERK inhibitor. In a specific embodiment, the one or more inhibitors consist of an inhibitor listed in Table 1, *e.g.,* a BRAF, MEK or ERK inhibitor.

Alternatively, in any aspect or embodiment herein, an AXL-ADC may be used in combination with two or more inhibitors selected from a BRAF inhibitor, a MEK inhibitor and an ERK inhibitor, such as a in combination with a BRAF inhibitor and a MEK inhibitor; a BRAF inhibitor and an ERK inhibitor; a MEK inhibitor and an ERK inhibitor; or a BRAF inhibitor, MEK inhibitor and an ERK inhibitor. In a specific embodiment of the disclosure, at least one of the two or more inhibitors is an inhibitor listed in Table 1. In another specific embodiment of the disclosure, an AXL-ADC is used in combination with two inhibitors selected from a BRAF inhibitor, a MEK inhibitor and an ERK inhibitor, e.g., where both inhibitors are inhibitors listed in Table 1.

In one embodiment, the one or more inhibitors of the MAPK pathway comprise or consist of a BRAF inhibitor.

In a specific embodiment, the BRAF-inhibitor is selected from vemurafenib, dabrafenib, encorafenib, sorafenib, PLX4720, GDC-0879, RAF265, SB590885, AZ628, AB-024, TAK-580, BAL-3833, BGB-283, optionally wherein the melanoma exhibits a mutation in BRAF providing for inhibition of the kinase activity of the mutant BRAF by the BRAF inhibitor. In another embodiment of the disclosure, the BRAF inhibitor is selected from vemurafenib, dabrafenib, encorafenib, sorafenib. The BRAF-inhibitor may be vemurafenib, dabrafenib, or a therapeutically effective analog or derivative of any thereof.

The BRAF-inhibitor may be vemurafenib or a therapeutically effective analog or derivative thereof. In one embodient of the disclosure, the BRAF-inhibitor is vemurafenib. Vemurafenib (PLX4032) is an orally bioavailable, ATP-competitive, small-molecule inhibitor of BRAF kinase, which particularly binds to and inhibits *e.g.* BRAF comprising certain mutations, such as, but not limited to, amino acid substitutions in residue V600 (*e.g.,* V600E), residue L597 (*e.g.,* L597R; Bahadoran *et al.,* 2013); and residue K601 (Dahlman *et al.,* 2012). Vemurafenib may, for example, have an IC50 of about 31 nM for inhibition of BRAF(V600E) kinase activity in a cell-free assay, *e.g.,* in an assay described herein or in Bollag *et al.,* 2010,.

In another preferred embodiment of the disclosure, the BRAF inhibitor is dabrafenib, or a therapeutically effective analog or derivative thereof. In one embodiment of the disclosure, the BRAF-inhibitor is dabrafenib. Dabrafenib is an inhibitor of BRAF kinase, which particularly binds to and inhibits BRAF comprising certain mutations such as, but not limited to, mutations in V600 such as V600E. Dabrafenib may, for example, have an IC50 of about 0.8 nM for inhibition of BRAF(V600E) kinase acvitity in a cell-free assay, *e.g.,* descrbed herein or in Laguerre *et al.,* 2009.

In another preferred embodiment of the disclosure, the BRAF inhibitor is encorafenib, or a therapeutically effective analog or derivative thereof. In one embodiment of the disclosure, the BRAF-inhibitor is encorafenib. Encorafenib is an inhibitor of BRAF kinase, which particularly binds to and inhibits BRAF comprising certain mutations such as, but not limited to, V600E. Encorafenib may, for example, have an IC50 of about 4 nM for inhibition of BRAF(V600E) kinase activity in a cell-free assay, *e.g.,* described herein or in Stuart *et al.,* 2012.

In another preferred embodiment of the disclosure, the BRAF inhibitor is sorafenib, or a therapeutically effective analog or derivative thereof. In one embodiment of the disclosure, the BRAF-inhibitor is sorafenib. Sorafenib is an inhibitor of BRAF kinase, which particularly binds to and inhibits BRAF. Sorafenib may, for example, have an IC50 of about 22 nM for inhibition of BRAF kinase activity in a cell-free assay, *e.g.,* described herein or in Wilhelm *et al.,* 2004.

In one embodiment, the BRAFi is selected from AB-024, TAK-580, BAL-3833 and BGB-283.

In one embodiment, the one or more inhibitors of the MAPK pathway comprise or consist of a MEK inhibitor.

In one embodiment, the MEK inhibitor is trametinib, cobimetinib, binimetinib, selumetinib, refametinib, pimasertib, U0126-EtOH, PD184352, BIX 02189. In one embodiment of the disclosure, the MEK inhibitor is trametinib, cobimetinib, binimetinib, selumetinib, refametinib, pimasertib, U0126-EtOH, PD184352, or a therapeutically effective analog or derivative of any thereof. Preferably, the MEK inhibitor is trametinib, cobimetinib, binimetinib, selumetinib, or a therapeutically effective analog or derivative of any thereof.

Most preferably, the MEK inhibitor is trametinib or a therapeutically effective analog or derivative thereof. In one embodiment, the MEK inhibitor is trametinib. Trametinib is a MEK1/2 inhibitor which may, for example, have an IC50 of about 0.92 nM and 1.8 nM for inhibition of the serine/threonine/tyrosine kinase activities of MEK1 and MEK2, respectively,

in a cell-free assay, e.g., described herein or in Yamaguchi *et al.,* 2011.

In one embodiment of the disclosure, the MEK inhibitor is binimetinib or a therapeutically effective analog or derivative thereof, such as, *e.g.,* binimetinib. Binimetinib is a MEK1/2 inhibitor which may, for example, have an IC50 of about 12 nM for inhibition of the serine/threonine/tyrosine kinase activities of MEK1 and MEK2, in a cell-free assay, *e.g.,* as described herein or in Pheneger *et al.,* 2006.

In one embodiment of the disclosure, the MEK inhibitor is cobinimetinib or a therapeutically effective analog or derivative thereof, such as, *e.g.,* cobinimetinib. Cobimetinib is a MEK1 inhibitor which may, for example, have an IC50 of about 4.2 nM for inhibition of of the serine/threonine/tyrosine kinase activity of MEK1 in a cell-free assay, *e.g.,* described herein or in Hoeflich *et al.,* 2012.

In one embodiment of the disclosure, the MEK inhibitor is selumetinib or a therapeutically effective analog or derivative thereof, such as, *e.g.,* selumetinib. Selumetinib is a MEK1 inhibitor which may, for example, have an IC50 of about 14 nM for inhibition of of the serine/threonine/tyrosine kinase activity of MEK1 in a cell-free assay, *e.g.,* as described herein or in Huynh *et al.,* 2007.

In one embodiment, the one or more inhibitors of the MAPK pathway comprise or consist of an ERK inhibitor.

In one embodiment, the ERK inhibitor is LTT-462, ulixertinib (BVD-523), VTX11E, SCH772984,.

Ulixertinib is an ERK1/2 inhibitor which may, for example, have an IC50 of about <0.3 nM for inhibition of ERK2 kinase activity in a cell-free assay, *e.g.,* described herein or in Ward *et al.,* 2015.

As used herein, a "derivative" of a drug is a compound that is derived or derivable, by a direct chemical reaction, from the reference drug. As used herein, an "analog" or "structural analog" of a reference drug is a compound having a similar structure and/or mechanism of action to the drug but differing in at least one structural element. "Therapeutically active" or "therapeutically effective" analogs or derivatives of a reference drug such as, *e.g.,* vemurafenib, dabrafenib or trametinib have a similar or improved therapeutic efficacy as compared to the drug but may differ in, *e.g.,* one or more of stability, target specificity (i.e., which type of kinase it inhibits), selectivity (i.e., which isoforms or mutants of the kinase it inhibits), inhibitory activity, solubility, toxicity, and the like. Table 1 shows BRAF, MEK, ERK etc. inhibitors which have a similar specificity (i.e., BRAF, MEK, ERK etc. inhibition, respectively), similar selectivity, or other similarities in their mechanism of action.

In a specific embodiment, an analog or derivative of a kinase inhibitor according to thedisclosure, (*e.g.*, a serine/threonine kinase inhibitor), has the same or similar kinase specificity, optionally also selectivity, and a similar or improved IC50 in inhibiting the kinase activity as the reference drug in a suitable assay. For example, the analog or derivative may have an IC50 which is less than about 1000%, such as less than about 300%, such as less than about 200%, such as less than about 120%, such as less than about 100%, such as less than about 80%, such as less than about 50% and, optionally, more than about 1%, such as more than about 10%, about 20% or about 40%, of the IC50 of the reference drug in a suitable assay. Alternatively, an analog or derivative may have an IC50 which is less than about 5μM, such as less than about 1 μM, such as less than about 500 nM, such as less than about 200 nM, such as less than 100 nM, such as less than about 50 nM, such as between 0.01 nM and 1 μM, 0.05 nM and 200 nM, or 0.1 nM to 100 nM in a suitable assay.

Suitable assays for measuring the specificity, selectivity and activity of protein kinase inhibitors are well known in the art (see, *e.g.,* Lynette *et al.* 2009 and Uitdehaag 2012). For example, the BRAF inhibiting activity of an analog or derivative of a BRAF inhibitor as described herein, e.g., of vemurafenib, dabrafenib, encorafenib or sorafenib; the MEK-inhibiting activity of an analog or derivative of a MEK inhibitor as described herein, e.g., of trametinib, conimetinib, binimetinib or selumetinib; or the ERK-inhibiting activity of an analog or derivative of an ERK inhibitor as described herein, *e.g.,* of VTX11E or LTT-462 or ulixertinib can be evaluated in the assay described by Tsai et al. (Proc Natl Acad Sci U S A. 2008 Feb 26; 105(8): 3041-3046). Specifically, the selected kinase(s), kiase variants and/or kinase isoforms may be profiled for inhibition by the analog or derivative as compared to the parent drug using the Z'-LYTE biochemical assay format (SelectScreen; Invitrogen) according to the manufacturer's instructions.

Briefly, the IC50 value for a BRAFi (such as vemurafenib or dabrafenib) for BRAF mutant, *e.g.,* BRAF(V600E), can be determined by RAF kinase activity measurements, *e.g.,* as follows:
The kinase activities of wild-type RAF and mutants are determined by measuring phosphorylation of biotinylated-BAD protein (Bcl2-Associated Agonist Of Cell Death). For each enzyme (0.01 ng), 20 μL reactions are carried out in 20 mM Hepes (pH 7.0), 10 mM MgCl2, 1 mM DTT, 0.01% (v/v) Tween-20, 50 nM biotin-BAD protein, and 1 mM ATP at room temperature. Reactions are stopped at 5 min with 5 μL of a solution containing 20 mM Hepes (pH 7.0), 200 mM NaCl, 80 mM EDTA, 0.3% (w/v) bovine serum albumin (BSA). The stop solution also includes phospho-BAD (Ser112) antibody, streptavidin-coated donor beads, and protein A acceptor beads. The antibody and beads are pre-incubated in stop solution in the dark at room temperature for 30 min. The final dilution of antibody is 1/2000 and the final concentration of each bead is 10 μg/mL. The assay plates are incubated at room temperature for one hour and then are read on a PerkinElmer AlphaQuest reader. Mutant activities are the average of two different batches of purified protein assayed in duplicate in three different experiments. Alternatively, instead of determining an absolute IC50 value, the reference compound (*e.g.,* vemurafenib or dabrafenib) can be used as a control, and the relative inhibitory activity as compared to

that of the reference drug can be calculated, typically in %.

Briefly, the IC50 value for a MEKi (such as trametinib) for a MEK, *e.g.,* MEK1, can be determined by MEK kinase activity measurements, *e.g.,* as follows: Anti-MEK1 antibody is used to immunoprecipitate MEK1 molecules. MEK kinase activity is measured as the ability of immuno-isolated MEK1 to activate recombinant ERK1 in a coupled assay using MBP (Myelin Basic Protein) as the end point of the assay. Phosphorylated MBP is resolved on a 14% SDS-PAGE gel and vacuum-dried before exposure to X-ray film. Alternatively, instead of determining an absolute IC50 value, the reference compound (*e.g.*, trametinib) can be used as a control, and the relative inhibitory activity as compared to that of the reference drug can be calculated, typically in %. More specific substrates than MBP can also be used, e.g., purified, recombinant RSK, MNK, or Elk1 and peptides made according to the phosphorylation sites on this protein.

In one aspect of the disclosure, the invention provides an AXL-ADC for use in a method of treating a melanoma in a subject, the method comprising administering an AXL-ADC in combination with at least one therapeutic agent which is a serine/threonine kinase inhibitor, wherein the ADC and serine/threonine kinase inhibitor(s) are administered simultaneously, separately or sequentially. In one embodiment of the disclosure, the at least one therapeutic agent consists of or comprises a S/Th KI which is a BRAF-inhibitor, MEK-inhibitor or a combination thereof. In one embodiment of the disclosure, the S/Th KI is a BRAF-inhibitor, such as vemurafenib (PLX4032) or a therapeutically effective derivative or analog thereof, *e.g.,* PLX4720 or dabrafenib; or VTXKIIE. In one embodiment of the disclosure, the S/Th KI is a MEK-inhibitor, such as selumetinib (AZD6244) or trametinib.

In one embodiment of the disclosure, the AXL-ADC is for use in a method of treating melanoma in combination with one or more S/Th KIs selected from a BRAF-inhibitor, a MEK-inhibitor, or ERK-inhibitor or a combination of any two or more thereof. In one embodiment of the disclosure, the one or more S/Th KI comprise a BRAF-inhibitor, such as vemurafenib (PLX4032), dabrafenib, encorafenib, sorafenib or a therapeutically effective derivative or analog thereof, *e.g.,* PLX4720. In one embodiment of the disclosure, the one or more S/Th KIs comprise a MEK-inhibitor, such as trametinib, cobimetinib, binimetinib or selumetinib (AZD6244) or a therapeutically effective analog or derivative thereof. In one embodiment of the disclosure, the one or more S/Th KIs comprise an ERK inhibitor, such as, e.g., VTXKIIE, LTT-462, or a therapeutically effective analog or derivative thereof. In one embodiment of the disclosure, the one or more S/Th KIs consist of a BRAF inhibitor, such as vemurafenib, dabrafenib, encorafenib or sorafenib. In one embodiment of the disclosure, the at least one S/Th KIs consist of a MEK inhibitor, such as trametinib, cobimetinib, binimetinib or selumetinib. In one embodiment of the disclosure, the one or more S/Th KIs consist of an ERK inhibitor, such as ulixertinib, VTXIIE, SCH772984 or LTT-462. The following are specific embodiments of the disclosure for treating melanoma according to any aspect or embodiment herein:

In one particular embodiment, the S/Th KI is vemurafenib.
In one particular embodiment, the S/Th KI is dabrafenib.
In one particular embodiment, the S/Th KI is encorafenib.
In one particular embodiment, the S/Th KI is sorafenib.
In one particular embodiment, the S/Th KI is trametinib.
In one particular embodiment, the S/Th KI is cobimetinib.
In one particular embodiment, the S/Th KI is binimetinib.
In one particular embodiment, the S/Th KI is selumetinib.
In one particular embodiment, the S/Th KI is ulixertinib.
In one particular embodiment, the S/Th KI is VTXKIIE.
In one particular embodiment, the S/Th KI is LTT-462.
In one particular embodiment, the S/Th KI is PLX4720.

As described in Examples 19 and 20, a combination of HuMax-AXL-ADC, a BRAF inhibitor and a MEK inhibitor was effective in treating a resistant BRAF mutant melanoma model *in vivo.* Accordingly, in one aspect, the disclosure provides an AXL-ADC comprising an antibody binding to human AXL, such as HuMax-AXL-ADC, for use in treating melanoma in a subject in combination with two inhibitors of the MAPK pathway. In one embodiment of the disclosure, at least one of the two inhibitors (herein referred to as the "first" and the "second" inhibitor), optionally both, are selected from the inhibitors listed in Table 1, or therapeutically effective analogs or derivatives thereof. For example, in separate embodiments of the disclosure, the first inhibitor is selected from vemurafenib, dabrafenib, encorafenib, sorafenib, trametinib, cometinib, binimetinib, selumetinib, LTT-462, ulixertinib, SCH772984, and VTXKIIE, and the second inhibitor is independently selected from the other inhibitors than the first inhibitor in Table 1. More preferably, the first inhibitor and the second inhibitor are both selected from vemurafenib, dabrafenib, encorafenib, sorafenib, trametinib, cometinib, binimetinib, selumetinib, LTT-462, ulixertinib, SCH772984, and VTXKIIE. Preferably, both the first and the second inhibitors for use according to the invention are independently selected from a BRAF, a MEK and an ERK inhibitor. More preferably, the combination of the first and second inhibitors is selected from a BRAF inhibitor and a MEK inhibitor, a BRAF inhibitor and an ERK inhibitor, a MEK inhibitor and an ERK inhibitor. Most preferably, the AXL-ADC is for use in treating melanoma in

combination with a BRAF and a MEK inhibitor.

In one embodiment of the disclosure, the first inhibitor is a BRAF inhibitor and the second inhibitor is a MEK inhibitor selected from trametinib, cobimetinib, binimetinib, selumetinib or an analog or derivative of any thereof.

In one embodiment of the disclosure, the first inhibitor is a BRAF inhibitor and the second inhibitor is an ERK inhibitor selected from VTXKIIE and LTT-462, or an analog or derivative of any thereof.

In one embodiment of the disclosure, the first inhibitor is a MEK inhibitor and the second inhibitor is a BRAF inhibitor selected from vemurafenib, dabrafenib, encorafenib and sorafenib, or an analog or derivative of any thereof.

In one embodiment of the disclosure, the first inhibitor is a MEK inhibitor and the second inhibitor is an ERK inhibitor selected from VTXKIIE and LTT-462, or an analog or derivative of any thereof.

In one embodiment of the disclosure, the first inhibitor is an ERK inhibitor and the second inhibitor is a BRAF inhibitor selected from vemurafenib, dabrafenib, encorafenib sorafenib, or an analog or derivative of any thereof.

In one embodiment of the disclosure, the first inhibitor is an ERK inhibitor and the second inhibitor is a MEK inhibitor selected from trametinib, cobimetinib, binimetinib, selumetinib or an analog or derivative of any thereof.

In one embodiment, the AXL-ADC is for use in treating melanoma in a subject in combination with a combination of a BRAF inhibitor and a MEK inhibitor selected from (a) to (p):

(a) vemurafenib and trametinib;
(b) vemurafenib and cobimetinib;
(c) vemurafenib and binimetinib;
(d) vemurafenib and selumetinib;
(e) dabrafenib and trametinib;
(f) dabrafenib and cobimetinib;
(g) dabrafenib and binimetinib;
(h) dabrafenib and selumetinib;
(i) encorafenib and trametinib;
(j) encorafenib and cobimetinib;
(k) encorafenib and binimetinib;
(l) encorafenib and selumetinib;
(m) sorafenib and trametinib
(n) sorafenib and cobimetinib;
(o) sorafenib and binimetinib; or
(p) sorafenib and selumetinib.

In a specific embodiment of the disclosure, the BRAF inibitor in any one of (a) to (p) is a therapeutically effective analog or derivative of the specified BRAF inhibitor.

In a specific embodiment of the disclosure, the MEK inibitor in any one of (a) to (p) is a therapeutically effective analog or derivative of the specified MEK inhibitor.

In one specific embodiment, the invention provides an AXL-ADC comprising an antibody binding to human AXL, such as HuMax-AXL-ADC, for use in treating melanoma in a subject in combination with vemurafenib and trametinib.

In one specific embodiment of the disclosure, the invention provides an AXL-ADC comprising an antibody binding to human AXL, such as HuMax-AXL-ADC, for use in treating melanoma in a subject in combination with vemurafenib and cobimetinib.

In one specific embodiment of the disclosure, the invention provides an AXL-ADC comprising an antibody binding to human AXL, such as HuMax-AXL-ADC, for use in treating melanoma in a subject in combination with vemurafenib and binimetinib.

In one specific embodiment of the disclosure, the invention provides an AXL-ADC comprising an antibody binding to human AXL, such as HuMax-AXL-ADC, for use in treating melanoma in a subject in combination with vemurafenib and selumetinib.

In one specific embodiment, the invention provides an AXL-ADC comprising an antibody binding to human AXL, such as HuMax-AXL-ADC, for use in treating melanoma in a subject in combination with dabrafenib and trametinib.

In one specific embodiment of the disclosure, the invention provides an AXL-ADC comprising an antibody binding to human AXL, such as HuMax-AXL-ADC, for use in treating melanoma in a subject in combination with dabrafenib and cobimetinib.

In one specific embodiment of the disclosure, the invention provides an AXL-ADC comprising an antibody binding to human AXL, such as HuMax-AXL-ADC, for use in treating melanoma in a subject in combination with dabrafenib and binimetinib.

In one specific embodiment of the disclosure, the invention provides an AXL-ADC comprising an antibody binding to human AXL, such as HuMax-AXL-ADC, for use in treating melanoma in a subject in combination with dabrafenib

and selumetinib.

In one specific embodiment of the disclosure, the invention provides an AXL-ADC comprising an antibody binding to human AXL, such as HuMax-AXL-ADC, for use in treating melanoma in a subject in combination with encorafenib and trametinib.

In one specific embodiment of the disclosure, the invention provides an AXL-ADC comprising an antibody binding to human AXL, such as HuMax-AXL-ADC, for use in treating melanoma in a subject in combination with encorafenib and cobimetinib.

In one embodiment of the disclosure, the invention provides an AXL-ADC comprising an antibody binding to human AXL, such as HuMax-AXL-ADC, for use in treating melanoma in a subject in combination with encorafenib and binimetinib.

In one specific embodiment of the disclosure, the invention provides an AXL-ADC comprising an antibody binding to human AXL, such as HuMax-AXL-ADC, for use in treating melanoma in a subject in combination with encorafenib and selumetinib.

In one specific embodiment of the disclosure, the invention provides an AXL-ADC comprising an antibody binding to human AXL, such as HuMax-AXL-ADC, for use in treating melanoma in a subject in combination with sorafenib and trametinib.

In one specific embodiment of the disclosure, the invention provides an AXL-ADC comprising an antibody binding to human AXL, such as HuMax-AXL-ADC, for use in treating melanoma in a subject in combination with soraafenib and cobimetinib.

In one specific embodiment of the disclosure, the invention provides an AXL-ADC comprising an antibody binding to human AXL, such as HuMax-AXL-ADC, for use in treating melanoma in a subject in combination with sorafenib and binimetinib.

In one specific embodiment of the disclosure, the invention provides an AXL-ADC comprising an antibody binding to human AXL, such as HuMax-AXL-ADC, for use in treating melanoma in a subject in combination with sorafenib and selumetinib.

As used herein, the term "subject" is typically a human to whom the AXL-ADC and one or more MAPK pathway inhibitors are administered, typically a human patient diagnosed as having a melanoma or being at risk for developing a melanoma. In some embodiments of the disclosure, the subject has not earlier undergone treatment of the melanoma with a MAPK pathway inhibitor, such as with a BRAF inhibitor, MEK inhibitor or ERK inhibitor. In other embodiments of the disclosure, the subject is already undergoing or has earlier undergone treatment of the melanoma with one or more MAPK pathway inhibitors, such as those one or more MAPK pathway inhibitors, *e.g.,* serine/threonine kinase inhibitors, for use in combination with an AXL-ADC according to the invention.

As shown herein, the development of resistance in melanoma has been associated with increased or *de novo* expression of AXL (see, *e.g.,* Examples 17 and 21). For example, resistance to one or more of vemurafenib, dabrafenib and trametinib may be associated with *de novo* or enhanced expression of AXL by the tumor cells. Thus, melanomas are eligible for treatment with combinations of these and other MAPK pathway inhibitors and an AXL-specific ADC. The melanoma may be a stage I, stage II, stage III or a stage IV melanoma according to established classification criteria of melanoma, which are well-known in the art. In some embodiments, the melanoma to be treated according to any aspect or embodiment herein is a stage IV melanoma.

In some embodiments, the melanoma harbours a mutation in BRAF providing for inhibition of the kinase activity of the mutant BRAF by one or more BRAF inhibitors. BRAF mutations identified in human cancers such as melanoma are generally located in the glycine-rich P loop of the N lobe and the activation segment and flanking regions within the kinase domain, typically resulting in an over-activated MAPK signaling pathway downstream in the mutant BRAF kinase-expressing tumor cells. In BRAF, specific residues for such mutations include, but are not limited to, V600 (*e.g.,* V600E, V600K, V600D, V600R), residue L597 (*e.g.,* L597R); and residue K601 (K601E). In one embodiment, the mutation is in V600. In one embodiment, the mutation in BRAF is selected from V600E, V600D, V600K, L597R and K601E. In a specific embodiment, the mutation is V600E. Methods of identifying such BRAF mutations are well known in the art, see, *e.g.,* the Examples and Colombino *et al.* (2012). Melanomas harbouring such BRAF mutations are particularly suitable for any aspect or embodiment of the invention which includes a BRAFi, particularly a mutBRAFi such as vemurafenib, dabrafenib, encorafenib, and analogs or derivatives of any thereof.

In some embodiments, the melanoma harbours a mutation in NRAS (UniProtKB - P01111 (RASN_HUMAN)). Such mutations are well-known in the art (see, *e.g.,* Colombino *et al.,* 2012). For example, the mutation may be a mutation which constitutively activates the MAPK pathway (herei referred to as an "activating" mutation), which may be an oncogenic mutation. Non-limiting mutations include amino acid substitutions, deletions or insertions; preferably, the mutation is an amino acid substitution. Specific residues for such mutations include, but are not limited to, Q61 (*e.g.,* Q61R, Q61K and Q61L), G12 (e.g., G12D, G12S, G12C and G12V), and G13 (G13D and G13R). In one embodiment of the disclosure, the melanoma harbours at least one mutation selected from Q61R, Q61K, Q61L G12D, G12S, G12C, G12V, G13D and G13R. Methods of identifying such NRAS mutations are well known in the art, see, *e.g.,* the Examples and

Colombino *et al.* (2012). Melanomas harbouring such NRAS mutations are particularly suitable for any aspect or embodiment of the invention which includes a MEKi, particularly a MEKi such as trametinib, binimetinib, cobimetinib or selumetinib and analogs or derivatives of any thereof. Optionally, the aspect or disclosure does not include administration of a mutBRAFi.

In some embodiments of the disclosure, the melanoma to be treated according to any aspect or embodiment herein is resistant to one or more inhibitors of the MAPK pathway, *e.g.,* to at least one serine/threonine kinase inhibitor. The melanoma may, for example, be resistant to one or more of the BRAF inhibitors vemurafenib, dabrafenib, encorafenib and sorafenib, to one or more of the MEKi's trametinib, cobimetinib, binimetinib and selumetinib, and/or to one or more of the ERK inhibitors ulixertinib, LTT-462, VTX11E and SCH772984.

As used herein, a "resistant", "treatment-resistant" or "refractory" melanoma in a subject with respect to one or more therapeutic agents means that the melanoma does not respond to treatment with the therapeutic agent(s). The melanoma may, for example, have "native resistance" in that it did not respond to a treatment with the therapeutic agent from the onset of the treatment (herein also referred to as "intrinsic resistance"). Alternatively, the melanoma may have "aquired resistance", in that it initially responded to treatment with the therapeutic agent, *e.g.,* by remission or stabilization of the disease, but become non-responsive or less responsive to the therapeutic agent after a certain period of treatment or after a relapse of the melanoma, typically resulting in progressive disease. Other indicators of resistance include relapse or recurrence of the melanoma, increase of tumor burden, newly identified metastases or the like, despite treatment with the therapeutic agent. Whether a melanoma is, or is at risk for becoming, resistant to a therapeutic agent can be determined by a person of skill in the art. For example, the National Comprehensive Cancer Network (NCCN, www.nccn.org) and European Society for Medical Oncology (ESMO, www.esmo.org/Guidelines) provide guidelines for assessing whether a specific cancer responds to treatment.

So, in some embodiments of the disclosure, the melanoma has not earlier been treated with any MAPK pathway inhibitor. In some embodiments of the disclosure, the melanoma has not earlier been treated with any of the one or more MAPK pathway inhibitors according to any aspect or embodiment herein. For example, in particular embodiments of the disclosure, the melanoma has not earlier been treated with any one or more of vemurafenib, dabrafenib, encorafenib, sorafenib, trametinib, binimetinib, cobimetinib and selumetinib.

In some embodiments of the disclosure, the melanoma is resistant to at least one MAPK pathway inhibitor. In some embodiments of the disclosure, the melanoma is resistant to at least one of the one or more MAPK pathway inhibitors according to any aspect or embodiment herein. For example, in particular embodiments of the disclosure, the melanoma is resistant to any one or more of vemurafenib, dabrafenib, encorafenib, sorafenib, trametinib, binimetinib, cobimetinib and selumetinib.

In some embodiments of the disclosure, the melanoma has native (intrinsic) resistance to at least one MAPK pathway inhibitor. In some embodiments of the disclosure, the melanoma has native (intrinsic) resistance to at least one of the one or more MAPK pathway inhibitors according to any aspect or embodiment herein. For example, in particular embodiments of the disclosure, the melanoma has native (intrincic) resistance to any one or more of vemurafenib, dabrafenib, encorafenib, sorafenib, trametinib, binimetinib, cobimetinib and selumetinib.

In some embodiments of the disclosure, the melanoma has acquired resistance to at least one of the one or more inhibitors. The melanoma may, for example, be undergoing or has earlier undergone treatment with at least one of the one or more inhibitors according to any aspect or embodiment herein. Optionally, the melanoma may be a recurrent or relapsed melanoma.

In one embodiment of the disclosure, the melanoma is resistant or refractory to at least one of vemurafenib, dabrafenib, encorafenib and sorafenib. For example, the subject may have undergone treatment with vemurafenib, dabrafenib, encorafenib or sorafenib for a period of at least 2 months, such as at least 3 months, such as at least 7 months, such as at least 9 months, such as at least 12 months or more.

In one embodiment of the disclosure, the melanoma is resistant or refractory to at least one of trametinib, cobimetinib, binimetinib and selumetinib. For example, the subject may have undergone treatment with trametinib, cobimetinib, binimetinib or selumetinib for a period of at least 2 months, such as at least 3 months, such as at least 7 months, such as at least 9 months, such as at least 12 months or more.

In one embodiment of the disclosure, the melanoma is resistant or refractory to at least one of dabrafenib or trametinib, optionally both. For example, the subject may have undergone treatment with a combination of trametinib and dabrafenib for a period of at least 2 months, such as at least 3 months, such as at least 7 months, such as at least 9 months, such as at least 12 months or more.

In one embodiment of the disclosure, the melanoma is resistant or refractory to at least one of vemurafenib or trametinib, optionally both. For example, the subject may have undergone treatment with a combination of vemurafenib and dabrafenib for a period of at least 2 months, such as at least 3 months, such as at least 7 months, such as at least 9 months, such as at least 12 months or more.

In other embodiments of the disclosure, the melanoma to be treated according to any aspect or embodiment herein is not resistant to any of the one or more inhibitors. The melanoma may, for example, not have undergone any

treatment with any of the one or more inhibitors according to any aspect or embodiment herein. However, it is also possible that the melanoma is undergoing treatment with one or more such inhibitors, or has been treated with any of the one or more inhibitors, but resistance has not occurred. In such embodiments, the subject may, for example, have undergone treatment with vemurafenib, dabrafenib, encorafenib, sorafenib, trametinib, binimetinib, cobimetinib or selumetinib for a period of at least 2 months, such as at least 3 months, such as at least 7 months, such as at least 9 months, such as at least 12 months or more.

In one particular embodiment of the disclosure, the AXL-ADC provided by the present disclosure is for use in treating an AXL-expressing melanoma resistant to a therapeutic agent with which the melanoma is being or has been treated, wherein the therapeutic agent is vemurafenib, dabrafenib, encorafenib, sorafenib or a therapeutically effective analog or derivative of any thereof, and wherein the melanoma exhibits a mutation in BRAF. In particular, the melanoma exhibits a mutation in BRAF which renders the BRAF sensitive for inhibition by vemurafenib, dabrafenib, encorafenib, sorafenib or the therapeutically effective analog or derivative. Preferably, the mutation is an amino acid substitution. Specific residues for such mutations include, but are not limited to, V600 (*e.g.,* V600E, V600K and V600D), residue L597 (*e.g.,* L597R); and residue K601 (K601E). In one embodiment, the mutation is selected from V600E, V600D, V600K, L597R and K601E.

In one particular embodiment of the disclosure, the AXL-ADC provided by the present disclosure is for use in treating an AXL-expressing melanoma resistant to a therapeutic agent with which the melanoma is being or has been treated, wherein the therapeutic agent is trametinib, cobimetinib, binimetinib, selumetinib or a therapeutically effective analog or derivative of any thereof. The melanoma may harbour a mutation, such as an activating mutation, in NRAS. For example, the NRAS may have a mutation in, Q61 (*e.g.,* Q61R, Q61K and Q61L), G12 (*e.g.,* G12D, G12S, G12C and G12V), or G13 (G13D and G13R). In one embodiment, the melanoma harbours at least one mutation selected from Q61R, Q61K, Q61L G12D, G12S, G12C, G12V, G13D and G13R.

In one particular embodiment of the disclosure, the AXL-ADC provided by the present disclosure is for use in treating an AXL-expressing melanoma resistant to a therapeutic agent with which the melanoma is being or has been treated, wherein the therapeutic agent is LTT-462, ulixertinib, VTXKIIE, or a therapeutically effective analog or derivative of any thereof.

As for the AXL-ADC, a physician having ordinary skill in the art may readily determine and prescribe the effective amount of the pharmaceutical composition required. In relation hereto, when referring to a pharmaceutical composition it is to be understood also to comprise a composition as such, or vice versa. For example, the physician could start doses of the AXL-ADC employed in the pharmaceutical composition at levels lower than that required in order to achieve the desired therapeutic effect and gradually increase the dosage until the desired effect is achieved. In general, a suitable dose will be that amount of the compound which is the lowest dose effective to produce a therapeutic effect according to a particular dosage regimen. Such an effective dose will generally depend upon the factors described above.

For example, an "effective amount" for therapeutic use may be measured by its ability to stabilize the progression of disease. The ability of a compound to inhibit cancer may, for example, be evaluated in an animal model system predictive of efficacy in human tumors. Alternatively, this property of a composition may be evaluated by examining the ability of the compound to inhibit cell growth or to induce cytotoxicity by *in vitro* assays known to the skilled practitioner. A therapeutically effective amount of a therapeutic compound may decrease tumor size, or otherwise ameliorate symptoms in a subject. One of ordinary skill in the art would be able to determine such amounts based on such factors as the subject's size, the severity of the subject's symptoms, and the particular composition or route of administration selected. For example, as already indicated, the National Comprehensive Cancer Network (NCCN, www.nccn.org) and European Society for Medical Oncology (ESMO, www.esmo.org/Guidelines) guidelines for assessing cancer treatments can be used.

An exemplary, non-limiting range for a therapeutically effective amount of an AXL-ADC of the invention is 0.02-100 mg/kg, such as about 0.02-30 mg/kg, such as about 0.05-10 mg/kg, 0.1-5 mg/kg or 0.1-3 mg/kg, for example about 0.5-3 mg/kg or 0.5-2 mg/kg.

Administration may *e.g.* be intravenous, intramuscular, intraperitoneal, or subcutaneous, and for instance administered proximal to the site of the target.

Dosage regimens in the above methods of treatment and uses are adjusted to provide the optimum desired response (*e.g.*, a therapeutic response). For example, a single bolus may be administered, several divided doses may be administered over time or the dose may be proportionally reduced or increased as indicated by the exigencies of the therapeutic situation.

In one embodiment of the disclosure, the efficacy-safety window is optimized by lowering specific toxicity such as for example by lowering the drug-antibody ratio (DAR) and/or mixing of AXL-ADC with unlabeled anti-AXL antibody.

In one embodiment of the disclosure, the efficacy of the treatment is monitored during the therapy, *e.g.* at predefined points in time. Methods for measuring efficacy generally depend on the particular type of cancer and are well known to a person skilled in the art. In one embodiment, the efficacy may be monitored, by visualization of the disease area, or by other diagnostic methods described further herein, *e.g.* by performing one or more PET-CT scans, for example using a labeled anti-AXL antibody, fragment or mini-antibody derived from an AXL-specific antibody.

If desired, an effective daily dose of an AXL-ADC may be two, three, four, five, six or more sub-doses administered separately at appropriate intervals throughout the day, optionally, in unit dosage forms. In another embodiment, the AXL-ADCs are administered by slow continuous infusion over a long period, such as more than 24 hours, in order to minimize any unwanted side effects.

An effective dose of an AXL-ADC may also be administered using a weekly, biweekly or triweekly dosing period. The dosing period may be restricted to, *e.g.,* 8 weeks, 12 weeks or until clinical progression has been established. In one embodiment of the disclosure, an AXL-ADC is administered either once every 3 weeks (1Q3W) or three administrations over 4 weeks (3Q4W) so that the patient receives sixteen or twelve cycles of AXL-ADC at three week or four-week intervals for, *e.g.,* 48 weeks, extending or repeating the regimen as needed.

For example, in one embodiment of the disclosure, the AXL-ADC may be administered by infusion in a weekly dosage of between 10 and 500 mg/$m^2$, such as between 200 and 400 mg/$m^2$. Such administration may be repeated, *e.g.,* 1 to 8 times, such as 3 to 5 times. The administration may be performed by continuous infusion over a period of from 1 to 24 hours, such as from 1 to 12 hours.

In another embodiment of the disclosure, the AXL-ADC is administered by infusion every three weeks in a dosage of between 10 and 500 mg/$m^2$, such as between 50-200 mg/$m^2$. Such administration may be repeated, *e.g.,* 1 to 8 times, such as 3 to 5 times. The administration may be performed by continuous infusion over a period of from 1 to 24 hours, such as from 1 to 12 hours.

In one embodiment of the disclosure, an AXL-ADC is administered as a single dose of about 0.1-10 mg/kg, such as about 1-3 mg/kg, every week or every third week for up to twelve times, up to eight times, or until clinical progression. The administration may be performed by continuous infusion over a period of from 1 to 24 hours, such as from 1 to 12 hours.

In one embodiment of the disclosure, the AXL-ADC is administered in an amount of about 0.02-100 mg/kg, such as about 0.02-30 mg/kg, such as about 0.05-10 mg/kg either every 1 week (1Q1W), every 2 weeks (1Q2W) or every 3 weeks (1Q3W) or three administrations over 4 weeks (3Q4W). Typically, the patient may receives sixteen or twelve cycles of AXL-ADC at three week or four-week intervals for, *e.g.,* 48 weeks, extending, shortening or repeating the regimen as determined by the physician responsible. The administration may be performed by continuous infusion over a period of from 1 to 24 hours, such as from 1 to 12 hours.

Such regimens may be repeated one or more times as necessary, for example, after 6 months or 12 months. The dosage may be determined or adjusted by measuring the amount of compound of the present invention in the blood upon administration by for instance taking out a biological sample and using anti-idiotypic antibodies which target the antigen binding region of the anti-AXL antibodies.

In one embodiment of the disclosure, the AXL-ADCs are administered as maintenance therapy, such as, *e.g.,* once a week for a period of six months or more. As used herein, "maintenance therapy" means therapy for the purpose of avoiding or delaying the cancer's progression or return. Typically, if a cancer is in complete remission after the initial treatment, maintenance therapy can be used to avoid or delay a return of the cancer. If the cancer is advanced and complete remission has not been achieved after the initial treatment, maintenance therapy can be used to slow the growth of the cancer, *e.g.,* to lengthen the life of the patient.

In other non-limiting examples, treatment according to the present invention may be provided as a daily dosage of a compound of the present invention in an amount of about 0.1-100 mg/kg, such as about 0.1-50 mg/kg, such as about 0.2, 0.5, 0.9, 1.0, 1.1, 1.5, 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 45, 50, 60, 70, 80, 90 or 100 mg/kg, per day, on at least one of days 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40, or alternatively, at least one of weeks 1, 2, 3, 4, 5, 6, 7, 8, 9, 10, 11, 12, 13, 14, 15, 16, 17, 18, 19 or 20 after initiation of treatment, or any combination thereof, using single or divided doses every 24, 12, 8, 6, 4, or 2 hours, or any combination thereof.

Parenteral compositions may be formulated in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms of the present invention are dictated by and directly dependent on (a) the unique characteristics of the active compound and the particular therapeutic effect to be achieved, and (b) the limitations inherent in the art of compounding such an active compound for the treatment of sensitivity in individuals.

As described herein, an AXL-ADC is used in combination with one or more MAPK pathway inhibitors, e.g., one or more serine/threonine kinase inhibitors, optionally at least one serine/threonine kinase inhibitor to which the melanoma is resistant.

The AXL-ADC and the one or more MAPK pathway inhibitors such as serine/threonine kinase inhibitor(s) can be administered simultaneously, separately or sequentially. For example, in one embodiment, the combination is used for treating a melanoma patient which has not received prior treatment with the inhibitor, optionally not with any serine/-threonine kinase inhibitor. In another embodiment, the combination is used for treating a melanoma patient which has failed prior treatment with the inhibitor, *e.g.,* the serine/threonine kinase inhibitor. Efficient dosages and dosage regimens

for the AXL-ADC and inhibitor(s) depend on the melanoma and patient to be treated and may be determined by the persons skilled in the art.

In one embodiment of the disclosure, the dosages and dosage regimens for the one or more MAPK pathway inhibitors, *e.g.,* the one or more serine/threonine kinase inhibitors to be used in conjunction with the AXL-ADC are the same or essentially similar to those normally used in the treatment of a cancer, *e.g.,* melanoma with the one or more serine/threonine kinase inhibitors.

Vemurafenib may, for example, be administered orally at a dose of about 200-2000 mg, 500-1500 mg, such as about 1000 mg per day, *e.g.,* 960 mg, administered as 4 x 240 mg tablets q12hr (approximately 12 hr apart).

Dabrafenib may, for example, be administered orally to the subject at a dose of about 50-300 mg, such as about 100-200 mg, such as about 150 mg, once or twice daily or every 2 or 3 days. Preferably, the dabrafenib is administered as 150 mg orally twice daily, *e.g.,* at least 1hr before a meal or at least 2 hrs after a meal.

Encorafenib may, for example, be administered orally at a total dose of 600 mg, such as 400 mg, such as 300 mg, such as 200 mg, such as 100 mg once or twice daily or every 2, 3 or 4 days, such as 300 mg once daily (QD).

Sorafenib may, for example, be admistered orally at a total dose of 200-1600 mg, such as 1200 mg, such as 800 mg, such as 600 mg, such as 400 mg once or twice daily or every 2, 3 or 4 days, such as two tablets of 200 mg twice daily (equivalent to a total daily dose of 800 mg).

Trametinib may, for example, be administered to orally to the subject at a dose of about 0.1 to 10 mg, such as about 0.5 to 5 mg, such as about 2 mg, once or twice daily, or every 2 or 3 days. Preferaby, the trametinib is administered as 2 mg orally once a day, e.g., at a similar time every day without food, at least 1 hour before or 2 hours after a meal.

Cobimetinib may, for example, be administered orally to the subject at a dose of about 10 to 100 mg, such as about 30 to 80 mg, such as about 60 mg per day, optionally divided into 2, 3 or 4 separate doses. Preferably, the cobimetinib is administered at a dose of 60 mg a day (3 tablets of 20 mg) in 28 day cycles, wherein the drug is taken for 21 consecutive days, followed by a 7-day break.

Binimetinib may, for example, be administered orally to the subject at a dose of about 10-200 mg, such as 150 mg, such as 100 mg, such as 90 mg, such as 45 mg, such as 30 mg, such as 20 mg once or twice daily, or every 2, 3 or 4 days, such as 45 mg twice daily.

Selumetinib may, for example, be administered orally at a dose of about 50-225 mg, such as 75 mg, 100mg, 125mg, 150mg, 175mg, 200mg or 225mg once or twice a day, such as twice per day, optionally in a regimen where it is given for three days followed by four days off in four week cycles.

In one embodiment of the disclosure, the dosages of the MAPK pathway inhibitor(s), *e.g.,* the serine/threonine kinase inhibitor(s) are lower than those normally used, but the dosage regimen is otherwise similar. In one embodiment of the disclosure, the dosages of the MAPK pathway inhibitor(s), such as the serine/threonine kinase inhibitor(s), are similar to those normally used, but the dosage regimen is adjusted to fewer or less frequent administrations. In one embodiment of the disclosure, the dosages of the serine/threonine kinase inhibitors (s) are lower than those normally used and the dosage regimen is adjusted to fewer or less frequent administrations.

In one aspect, the disclosure relates to the use of an AXL-ADC in a method of treating a melanoma in a subject in need thereof, comprising administering to the subject (i) an ADC comprising an antibody binding to human AXL, optionally HuMax-AXL-ADC, and (ii) one or more MAPK pathway inhibitors, such as serine/threonine kinase inhibitor(s), wherein the ADC and the at least one inhibitor are administered simultaneously, separately or sequentially. In one embodiment of the disclosure, the one or more inhibitors of the MAPK pathway comprise or consist of a BRAF-inhibitor, a MEK-inhibitor, an ERK inhibitor or a combination of any two or more thereof, such as a BRAF inhibitor and a MEK inhibitor, a BRAF inhibitor and an ERK inhibitor, or a MEK inhibitor and an ERK inhibitor. In one embodiment of the disclosure, the melanoma is an AXL-expressing melanoma. In one embodiment of the disclosure, the melanoma is resistant to at least one of the one or more inhibitors. In one embodiment of the disclosure, the inhibitor is a BRAF inhibitor selected from vemurafenib, dabrafenib, encorafenib, sorafenib or a therapeutically effective analog or derivative thereof, and the melanoma exhibits a mutation in BRAF providing for inhibition of the kinase activity of the mutant BRAF by the BRAF inhibitor. In one embodiment of the disclosure, the inhibitor is a MEK inhibitor selected from trametinib, cobimetinib, binimetinib, selumetinib or a therapeutically effective analog or derivative thereof. The AXL-ADC may, *e.g.,* be administered in a therapeutically effective amount according to a dosage regimen described in more detail above. For example, as a non-limiting example, the AXL-ADC may be administered in an amount of about 0.02-100 mg/kg, such as about 0.02-30 mg/kg, such as about 0.05-10 mg/kg either every 1 week (1Q1W), every 2 weeks (1Q2W) or every 3 weeks (1Q3W) or three administrations over 4 weeks (3Q4W) so that the patient receives sixteen or twelve cycles of AXL-ADC at three week or four-week intervals for, *e.g.,* 48 weeks, extending, shortening or repeating the regimen as determined by the physician responsible.

In one aspect, the disclosure relates to the use of an AXL-ADC in a method of treating a melanoma in a subject, the method comprising administering to the subject (i) an ADC comprising an antibody binding to human AXL ,(ii) a BRAF inhibitor selected from vemurafenib, dabrafenib, encorafenib and sorafenib or a therapeutically effective analog or derivative of any thereof; and (iii) a MEK inhibitor selected from trametinib, cobimetinib, binimetinib and selumetinib,

or a therapeutically effective analog or derivative or any thereof; wherein the melanoma exhibits a mutation in BRAF providing for inhibition of the kinase activity of the mutant BRAF by the BRAF-inhibitor, and wherein the ADC, the BRAF-inhibitor and the MEK-inhibitor are administered simultaneously, separately or sequentially in therapeutically effective amounts. In one embodiment, the mutation is in a BRAF residue selected from V600, L597 and K601, such as a mutation selected from V600E, V600K, V600D, L597R and K601E, such as V600E. In a particular embodiment of the disclosure, the melanoma does not harbour an activating NRAS mutation.

In one embodiment of the disclosure, the invention relates to the use of an AXL-ADC in a method of treating a melanoma in a subject, wherein the melanoma exhibits a mutation in BRAF and the mutation providing for vemurafenib inhibition of BRAF kinase activity of the mutant BRAF, the method comprising administering to the subject (i) an ADC comprising an antibody binding to human AXL and (ii) vemurafenib, or a therapeutically effective analog or derivative thereof, wherein (i) and (ii) are administered simultaneously, separately or sequentially. In one embodiment of the disclosure, the melanoma is resistant to vemurafenib. For example, the melanoma may have been earlier treated with vemurafenib, or may be undergoing treatment with vemurafenib. Alternatively, the melanoma may have been earlier treated with another BRAF inhibitor, *e.g.,* dabrafenib, encorafenib or sorafenib; or may be undergoing treatment with another BRAF inhibitor, *e.g.,* dabrafenib, encorafenib or sorafenib. The melanoma may, for example, be a relapsed melanoma. In one embodiment, the melanoma is an AXL-expressing melanoma. In another embodiment of the disclosure, the melanoma is not resistant to vemurafenib and/or has not earlier been treated with vemurafenib. In one embodiment of the disclosure, the mutation is an amino acid substitution in residue V600, L597 and/or K601. In one embodiment of the disclosure, the mutation is selected from V600E, V600D, V600K, L597R and K601E. The vemurafenib is typically administered in a therapeutically effective amount according to a suitable dosage regimen. For example, vemurafenib may be administered orally at a dose of about 200-2000 mg, 500-1500 mg, such as about 1000 mg per day, *e.g.,* 960 mg, administered as 4 x 240 mg tablets q12hr (approximately 12 hr apart).

In one embodiment, the disclosure relates to the use of an AXL-ADC in a method of treating a melanoma in a subject, wherein the melanoma exhibits a mutation in BRAF and the mutation providing for dabrafenib inhibition of BRAF kinase activity of the mutant BRAF, the method comprising administering to the subject (i) an ADC comprising an antibody binding to human AXL and (ii) dabrafenib, or a therapeutically effective analog or derivative thereof, wherein (i) and (ii) are administered simultaneously, separately or sequentially. In one embodiment of the disclosure, the melanoma is resistant to dabrafenib. For example, the melanoma may have been earlier treated with dabrafenib, or may be undergoing treatment with dabrafenib. Alternatively, the melanoma may have been earlier treated with another BRAF inhibitor, *e.g.,* vemurafenib, encorafenib or sorafenib; or may be undergoing treatment with another BRAF inhibitor, *e.g.,* vemurafenib, encorafenib or sorafenib. The melanoma may, for example, be a relapsed melanoma. In one embodiment of the disclosure, the melanoma is an AXL-expressing melanoma. In another embodiment, the melanoma is not resistant to dabrafenib and/or has not earlier been treated with dabrafenib. In one embodiment of the disclosure, the mutation is an amino acid substitution in residue V600, L597 and/or K601. In one embodiment of the disclosure, the mutation is selected from V600E, V600D, V600K, L597R and K601E. The dabrafenib is typically administered in a therapeutically effective amount and according to a suitable dosage regimen. Dabrafenib may, for example, be administered orally to the subject at a dose of about 50-300 mg, such as about 100-200 mg, such as about 150 mg, once or twice daily or every 2 or 3 days. Preferably, the dabrafenib is administered as 150 mg orally twice daily, *e.g.,* at least 1hr before a meal or at least 2 hrs after a meal.

In one embodiment of the disclosure, the invention relates to the use of an AXL-ADC in a method of treating a melanoma in a subject, wherein the melanoma exhibits a mutation in BRAF and the mutation providing for encorafenib inhibition of BRAF kinase activity of the mutant BRAF, the method comprising administering to the subject (i) an ADC comprising an antibody binding to human AXL and (ii) encorafenib, or a therapeutically effective analog or derivative thereof, wherein (i) and (ii) are administered simultaneously, separately or sequentially. In one embodiment of the disclosure, the melanoma is resistant to encorafenib. For example, the melanoma may have been earlier treated with encorafenib, or may be undergoing treatment with encorafenib. Alternatively, the melanoma may have been earlier treated with another BRAF inhibitor, *e.g.,* vemurafenib, dabrafenib or sorafenib; or may be undergoing treatment with another BRAF inhibitor, *e.g.,* vemurafenib, dabrafenib or sorafenib. The melanoma may, for example, be a relapsed melanoma. In one embodiment, the melanoma is an AXL-expressing melanoma. In another embodiment of the disclosure, the melanoma is not resistant to encorafenib and/or has not earlier been treated with encorafenib. In one embodiment, the mutation is an amino acid substitution in residue V600, L597 and/or K601. In one embodiment of the disclosure, the mutation is selected from V600E, V600D, V600K, L597R and K601E. The encorafenib is typically administered in a therapeutically effective amount and according to a suitable dosage regimen. Encorafenib may, for example, be administered orally at a total dose of 600 mg, such as 400 mg, such as 300 mg, such as 200 mg, such as 100 mg once or twice daily or every 2, 3 or 4 days, such as 300 mg once daily.

In one embodiment, the disclosure relates to the use of an AXL-ADC in a method of treating a melanoma in a subject, optionally wherein the melanoma exhibits a mutation in BRAF, the method comprising administering to the subject (i) an ADC comprising an antibody binding to human AXL and (ii) sorafenib, or a therapeutically effective analog or

derivative thereof, wherein (i) and (ii) are administered simultaneously, separately or sequentially In one embodiment, the melanoma is resistant to sorafenib. For example, the melanoma may have been earlier treated with sorafenib, or may be undergoing treatment with sorafenib. Alternatively, the melanoma may have been earlier treated with another BRAF inhibitor, *e.g.,* vemurafenib, dabrafenib or encorafenib; or may be undergoing treatment with another BRAF inhibitor, *e.g.,* vemurafenib, dabrafenib or encorafenib. The melanoma may, for example, be a relapsed melanoma. In one embodiment of the disclosure, the melanoma is an AXL-expressing melanoma. In another embodiment of the disclosure, the melanoma is not resistant to sorafenib and/or has not earlier been treated with sorafenib. In one embodiment of the disclosure, the mutation is an amino acid substitution in residue V600, L597 and/or K601. In one embodiment of the disclosure, the mutation is selected from V600E, V600D, V600K, L597R and K601E. The sorafenib is typically administered in a therapeutically effective amount and according to a suitable dosage regimen. Sorafenib may, for example, be admistered orally at a total dose of 200-1600 mg, such as 1200 mg, such as 800 mg, such as 600 mg, such as 400 mg once or twice daily or every 2, 3 or 4 days, such as two tablets of 200 mg twice daily (equivalent to a total daily dose of 800 mg).

In one embodiment of the disclosure, the invention relates to the use of an AXL-ADC in a method of treating a melanoma in a subject, the method comprising administering to the subject (i) an ADC comprising an antibody binding to human AXL and (ii) trametinib, or a therapeutically effective analog or derivative thereof, wherein (i) and (ii) are administered simultaneously, separately or sequentially. In one embodiment, the melanoma is resistant to trametinib. For example, the melanoma may have been earlier treated with trametinib, or may be undergoing treatment with trametinib. Alternatively, the melanoma may have been earlier treated with another MEK inhibitor, *e.g.,* binimetinib, cobimetinib or selumetinib; or may be undergoing treatment with another MEK inhibitor, *e.g.,* binimetinib, cobimetinib or selumetinib. The melanoma may, for example, be a relapsed melanoma. In one embodiment of the disclosure, the melanoma is an AXL-expressing melanoma. In another embodiment of the disclosure, the melanoma is not resistant to trametinib and/or has not earlier been treated with trametinib. In one embodiment, the melanoma exhibits a mutation in NRAS, such as is in an NRAS residue selected from Q61, G12 and G13, such as a mutation in NRAS selected from Q61R, Q61K, Q61L, G12D, G12S, G12C, G12V, G13D and G13R. The trametinib is typically administered in a therapeutically effective amount and according to a suitable dosage regimen. The dabrafenib may, for example, be administered orally to the subject at a dose of about 50-300 mg, such as about 100-200 mg, such as about 150 mg, once or twice daily or every 2 or 3 days. Preferably, the dabrafenib is administered as 150 mg orally twice daily, *e.g.,* at least 1hr before a meal or at least 2 hrs after a meal.

In one embodiment, the disclosure relates to the use of an AXL-ADC in a method of treating a melanoma in a subject, the method comprising administering to the subject (i) an ADC comprising an antibody binding to human AXL and (ii) cobimetinib, or a therapeutically effective analog or derivative thereof, wherein (i) and (ii) are administered simultaneously, separately or sequentially. In one embodiment of the disclosure, the melanoma is resistant to cobimetinib. For example, the melanoma may have been earlier treated with cobimetinib, or may be undergoing treatment with cobimetinib. Alternatively, the melanoma may have been earlier treated with another MEK inhibitor, *e.g.,* trametinib, binimetinib or selumetinib; or may be undergoing treatment with another MEK inhibitor, *e.g.,* trametinib, binimetinib or selumetinib. The melanoma may, for example, be a relapsed melanoma. In one embodiment, the melanoma is an AXL-expressing melanoma. In another embodiment, the melanoma is not resistant to cobimetinib and/or has not earlier been treated with cobimetinib. In one embodiment, the melanoma exhibits a mutation in NRAS, such as is in an NRAS residue selected from Q61, G12 and G13, such as a mutation in NRAS selected from Q61R, Q61K, Q61L, G12D, G12S, G12C, G12V, G13D and G13R. The cobimetinib is typically administered in a therapeutically effective amount and according to a suitable dosage regimen. Cobimetinib may, for example, be administered orally to the subject at a dose of about 10 to 100 mg, such as about 30 to 80 mg, such as about 60 mg per day, optionally divided into 2, 3 or 4 separate doses. Preferably, the cobimetinib is administered at a dose of 60 mg a day (3 tablets of 20 mg) in 28 day cycles, wherein the drug is taken for 21 consecutive days, followed by a 7-day break.

In one embodiment of the disclosure, the invention relates to the use of an AXL-ADC in a method of treating a melanoma in a subject, the method comprising administering to the subject (i) an ADC comprising an antibody binding to human AXL and (ii) binimetinib, or a therapeutically effective analog or derivative thereof, wherein (i) and (ii) are administered simultaneously, separately or sequentially. In one embodiment, the melanoma is resistant to binimetinib. For example, the melanoma may have been earlier treated with binimetinib, or may be undergoing treatment with binimetinib. Alternatively, the melanoma may have been earlier treated with another MEK inhibitor, *e.g.,* trametinib, cobimetinib or selumetinib; or may be undergoing treatment with another MEK inhibitor, *e.g.,* trametinib, cobimetinib or selumetinib. The melanoma may, for example, be a relapsed melanoma. In one embodiment, the melanoma is an AXL-expressing melanoma. In another embodiment of the disclosure, the melanoma is not resistant to binimetinib and/or has not earlier been treated with binimetinib. In one embodiment of the disclosure, the melanoma exhibits a mutation in NRAS, such as is in an NRAS residue selected from Q61, G12 and G13, such as a mutation in NRAS selected from Q61R, Q61K, Q61L, G12D, G12S, G12C, G12V, G13D and G13R. The binimetinib is typically administered in a therapeutically effective amount and according to a suitable dosage regimen. Binimetinib may, for example, be administered orally to the subject at a dose of about 10-200 mg, such as 150 mg, such as 100 mg, such as 90 mg, such as 45 mg, such as 30 mg, such as 20 mg

once or twice daily, or every 2, 3 or 4 days, such as 45 mg twice daily.

In one embodiment of the disclosure, the invention relates to the use of an AXL-ADC in a method of treating a melanoma in a subject, the method comprising administering to the subject (i) an ADC comprising an antibody binding to human AXL and (ii) selumetinib, or a therapeutically effective analog or derivative thereof, wherein (i) and (ii) are administered simultaneously, separately or sequentially. In one embodiment of the disclosure, the melanoma is resistant to selumetinib. For example, the melanoma may have been earlier treated with selumetinib, or may be undergoing treatment with selumetinib. Alternatively, the melanoma may have been earlier treated with another MEK inhibitor, *e.g.,* trametinib, cobimetinib or binimetinib; or may be undergoing treatment with another MEK inhibitor, *e.g.,* trametinib, cobimetinib or binimetinib. The melanoma may, for example, be a relapsed melanoma. In one embodiment of the disclosure, the melanoma is an AXL-expressing melanoma. In another embodiment of the disclosure, the melanoma is not resistant to selumetinib and/or has not earlier been treated with selumetinib. In one embodiment of the disclosure, the melanoma exhibits a mutation in NRAS, such as is in an NRAS residue selected from Q61, G12 and G13, such as a mutation in NRAS selected from Q61R, Q61K, Q61L, G12D, G12S, G12C, G12V, G13D and G13R. The selumetinib is typically administered in a therapeutically effective amount and according to a suitable dosage regimen. Selumetinib may, for example, be administered orally at a dose of about 50-225 mg, such as 75 mg, 100mg, 125mg, 150mg, 175mg, 200mg or 225mg once or twice a day, such as twice per day, optionally in a regimen where it is given for three days followed by four days off in four week cycles.

In one embodiment of the AXL-ADC for use in a method according to any one of the preceding embodiments, the melanoma exhibits a mutation in NRAS, e.g., an activating NRAS mutation, such as is in an NRAS residue selected from Q61, G12 and G13, such as a mutation in NRAS selected from Q61R, Q61K, Q61L, G12D, G12S, G12C, G12V, G13D and G13R.

In one embodiment, the disclosure relates to the use of an AXL-ADC in a method of treating a melanoma in a subject, wherein the melanoma exhibits a mutation in BRAF and the mutation providing for dabrafenib inhibition of BRAF kinase activity of the mutant BRAF, the method comprising administering to the subject (i) an ADC comprising an antibody binding to human AXL, (ii) dabrafenib, or a therapeutically effective analog or derivative thereof and (iii) trametinib or a therapeutically effective analog or derivative thereof. In one embodiment of the disclosure, the melanoma is resistant to dabrafenib, trametinib or both. For example, the melanoma may have been earlier treated with dabrafenib, trametinib or both, or may be undergoing treatment with dabrafenib, trametinib or both. Alternatively, the melanoma may have been earlier treated with another BRAF inhibitor, MEK inhibitor, or both. The melanoma may, for example, be a relapsed melanoma. In one embodiment, the melanoma is an AXL-expressing melanoma. In another embodiment, the melanoma is not resistant to dabrafenib or trametinib and/or has not earlier been treated with dabrafenib or trametinib. In one embodiment of the disclosure, the mutation is an amino acid substitution in residue V600, L597 and/or K601. In one embodiment of the disclosure, the BRAF mutation is selected from V600E, V600D, V600K, L597R and K601E. The dabrafenib may, for example, be administered orally to the subject at a dose of about 50-300 mg, such as about 100-200 mg, such as about 150 mg, once or twice daily or every 2 or 3 days. Preferably, the dabrafenib is administered as 150 mg orally twice daily, e.g., at least 1hr before a meal or at least 2 hrs after a meal. The tramatenib may, for example, be administered orally at a dose of about 0.5 to 5 mg, such as about 1 to 4 mg, such as about 2-3 mg, such as about 2 mg, once or twice daily or every 2, 3 or 4 days, such as once daily.

In one embodiment, the disclosure relates to a method of treating a melanoma resistant to dabrafenib, trametinib or both in a subject, wherein the melanoma exhibits a mutation in BRAF and the mutation providing for dabrafenib inhibition of BRAF kinase activity of the mutant BRAF, the method comprising administering to the subject (i) an ADC comprising an antibody binding to human AXL, (ii) dabrafenib, or a therapeutically effective analog or derivative thereof and (iii) trametinib or a therapeutically effective analog or derivative thereof. In one embodiment of the disclosure, the cancer is an AXL-expressing cancer. In one embodiment, the mutation is an amino acid substitution in residue V600, L597 and/or K601. In one embodiment of the disclosure, the mutation is selected from V600E, V600D, V600K, L597R and K601E. The dabrafenib may, for example, be administered orally to the subject at a dose of about 50-300 mg, such as about 100-200 mg, such as about 150 mg, once or twice daily or every 2 or 3 days. Preferably, the dabrafenib is administered as 150 mg orally twice daily, *e.g.,* at least 1hr before a meal or at least 2 hrs after a meal. The trametenib may, for example, be administered orally at a dose of about 0.5 to 5 mg, such as about 1 to 4 mg, such as about 2-3 mg, such as about 2 mg, once or twice daily or every 2, 3 or 4 days, such as once daily.

In one embodiment, the disclosure relates to the use of an AXL-ADC in a method of treating a melanoma in a subject, wherein the melanoma exhibits a mutation in BRAF and the mutation providing for vemurafenib inhibition of BRAF kinase activity of the mutant BRAF, the method comprising administering to the subject (i) an ADC comprising an antibody binding to human AXL, (ii) vemurafenib, or a therapeutically effective analog or derivative thereof and (iii) trametinib or a therapeutically effective analog or derivative thereof. In one embodiment of the disclosure, the melanoma is resistant to vemurafenib, trametinib or both. For example, the melanoma may have been earlier treated with vemurafenib, trametinib or both, or may be undergoing treatment with vemurafenib, trametinib or both. Alternatively, the melanoma may have been earlier treated with another BRAF inhibitor, MEK inhibitor, or both. The melanoma may, for example, be a relapsed

melanoma. In one embodiment of the disclosure, the melanoma is an AXL-expressing melanoma. In another embodiment of the disclosure, the melanoma is not resistant to vemurafenib or trametinib and/or has not earlier been treated with vemurafenib or trametinib. In one embodiment, the BRAF mutation is an amino acid substitution in residue V600, L597 and/or K601. In one embodiment of the disclosure, the mutation is selected from V600E, V600D, V600K, L597R and K601E. The vemurafenib may, for example, be administered orally at a dose of about 200-2000 mg, 500-1500 mg, such as about 1000 mg per day, *e.g.,* 960 mg, administered as 4 x 240 mg tablets q12hr (approximately 12 hr apart). The tramatenib may, for example, be administered orally at a dose of about 0.5 to 5 mg, such as about 1 to 4 mg, such as about 2-3 mg, such as about 2 mg, once or twice daily or every 2, 3 or 4 days, such as once daily.

In one embodiment, the disclosure relates to the use of an AXL-ADC in a method of treating a melanoma in a subject, wherein the melanoma exhibits a mutation in BRAF and the mutation providing for encorafenib inhibition of BRAF kinase activity of the mutant BRAF, the method comprising administering to the subject (i) an ADC comprising an antibody binding to human AXL, (ii) encorafenib, or a therapeutically effective analog or derivative thereof and (iii) trametinib or a therapeutically effective analog or derivative thereof. In one embodiment of the disclosure, the melanoma is resistant to encorafenib, trametinib or both. For example, the melanoma may have been earlier treated with encorafenib, trametinib or both, or may be undergoing treatment with encorafenib, trametinib or both. Alternatively, the melanoma may have been earlier treated with another BRAF inhibitor, MEK inhibitor, or both. The melanoma may, for example, be a relapsed melanoma. In one embodiment of the disclosure, the melanoma is an AXL-expressing melanoma. In another embodiment of the disclosure, the melanoma is not resistant to encorafenib or trametinib and/or has not earlier been treated with encorafenib or trametinib. In one embodiment, the BRAF mutation is an amino acid substitution in residue V600, L597 and/or K601. In one embodiment, the mutation is selected from V600E, V600D, V600K, L597R and K601E. The encorafenib may, for example, be administered orally at a total dose of 600 mg, such as 400 mg, such as 300 mg, such as 200 mg, such as 100 mg once or twice daily or every 2, 3 or 4 days, such as once daily (QD). The tramatenib may, for example, be administered orally at a dose of about 0.5 to 5 mg, such as about 1 to 4 mg, such as about 2-3 mg, such as about 2 mg, once or twice daily or every 2, 3 or 4 days, such as once daily.

In one embodiment of the disclosure, the invention relates to the use of an AXL-ADC in a method of treating a melanoma in a subject, optionally wherein the melanoma exhibits a mutation in BRAF, the method comprising administering to the subject (i) an ADC comprising an antibody binding to human AXL, (ii) sorafenib, or a therapeutically effective analog or derivative thereof and (iii) trametinib or a therapeutically effective analog or derivative thereof. In one embodiment, the melanoma is resistant to sorafenib, trametinib or both. For example, the melanoma may have been earlier treated with sorafenib, trametinib or both, or may be undergoing treatment with sorafenib, trametinib or both. Alternatively, the melanoma may have been earlier treated with another BRAF inhibitor, MEK inhibitor, or both. The melanoma may, for example, be a relapsed melanoma. In one embodiment of the disclosure, the melanoma is an AXL-expressing melanoma. In another embodiment of the disclosure, the melanoma is not resistant to sorafenib or trametinib and/or has not earlier been treated with sorafenib or trametinib. In one embodiment of the disclosure, the BRAF mutation is an amino acid substitution in residue V600, L597 and/or K601. In one embodiment, the mutation is selected from V600E, V600D, V600K, L597R and K601E. The sorafenib may, for example, be admistered orally at a total dose of 200-1600 mg, such as 1200 mg, such as 800 mg, such as 600 mg, such as 400 mg once or twice daily or every 2, 3 or 4 days, such as two tablets of 200 mg twice daily (equivalent to a total daily dose of 800 mg). The tramatenib may, for example, be administered orally at a dose of about 0.5 to 5 mg, such as about 1 to 4 mg, such as about 2-3 mg, such as about 2 mg, once or twice daily or every 2, 3 or 4 days, such as once daily.

In one embodiment of the disclosure, the invention relates to the use of an AXL-ADC in a method of treating a melanoma in a subject, wherein the melanoma exhibits a mutation in BRAF and the mutation providing for dabrafenib inhibition of BRAF kinase activity of the mutant BRAF, the method comprising administering to the subject (i) an ADC comprising an antibody binding to human AXL, (ii) dabrafenib, or a therapeutically effective analog or derivative thereof and (iii) cobimetinib or a therapeutically effective analog or derivative thereof. In one embodiment of the disclosure, the melanoma is resistant to dabrafenib, cobimetinib or both. For example, the melanoma may have been earlier treated with dabrafenib, cobimetinib or both, or may be undergoing treatment with dabrafenib, cobimetinib or both. Alternatively, the melanoma may have been earlier treated, or may be undergoing treatment, with another BRAF inhibitor, MEK inhibitor or both. The melanoma may, for example, be a relapsed melanoma. In one embodiment of the disclosure, the melanoma is an AXL-expressing melanoma. In another embodiment, the melanoma is not resistant to dabrafenib or cobimetinib and/or has not earlier been treated with dabrafenib or cobimetinib. In one embodiment of the disclosure, the mutation is an amino acid substitution in residue V600, L597 and/or K601. In one embodiment of the disclosure, the BRAF mutation is selected from V600E, V600D, V600K, L597R and K601E. The dabrafenib may, for example, be administered orally to the subject at a dose of about 50-300 mg, such as about 100-200 mg, such as about 150 mg, once or twice daily or every 2 or 3 days. Preferably, the dabrafenib is administered as 150 mg orally twice daily, *e.g.,* at least 1hr before a meal or at least 2 hrs after a meal.

In one embodiment of the disclosure, the invention relates to the use of an AXL-ADC in a method of treating a melanoma in a subject, wherein the melanoma exhibits a mutation in BRAF and the mutation providing for vemurafenib

inhibition of BRAF kinase activity of the mutant BRAF, the method comprising administering to the subject (i) an ADC comprising an antibody binding to human AXL, (ii) vemurafenib, or a therapeutically effective analog or derivative thereof and (iii) cobimetinib or a therapeutically effective analog or derivative thereof. In one embodiment of the disclosure, the melanoma is resistant to vemurafenib, cobimetinib or both. For example, the melanoma may have been earlier treated with vemurafenib, cobimetinib or both, or may be undergoing treatment with vemurafenib, cobimetinib or both. Alternatively, the melanoma may have been earlier treated, or may be undergoing treatment, with another BRAF inhibitor, MEK inhibitor or both. The melanoma may, for example, be a relapsed melanoma. In one embodiment, the melanoma is an AXL-expressing melanoma. In another embodiment of the disclosure, the melanoma is not resistant to vemurafenib or cobimetinib and/or has not earlier been treated with vemurafenib or cobimetinib. In one embodiment of the disclosure, the mutation is an amino acid substitution in residue V600, L597 and/or K601. In one embodiment of the disclosure, the BRAF mutation is selected from V600E, V600D, V600K, L597R and K601E. The vemurafenib may, for example, be administered orally at a dose of about 200-2000 mg, 500-1500 mg, such as about 1000 mg per day, *e.g.,* 960 mg, administered as 4 x 240 mg tablets q12hr (approximately 12 hr apart).

In one embodiment of the disclosure, the invention relates to the use of an AXL-ADC in a method of treating a melanoma in a subject, wherein the melanoma exhibits a mutation in BRAF and the mutation providing for encorafenib inhibition of BRAF kinase activity of the mutant BRAF, the method comprising administering to the subject (i) an ADC comprising an antibody binding to human AXL, (ii) encorafenib, or a therapeutically effective analog or derivative thereof and (iii) cobimetinib or a therapeutically effective analog or derivative thereof. In one embodiment of the disclosure, the melanoma is resistant to encorafenib, cobimetinib or both. For example, the melanoma may have been earlier treated with encorafenib, cobimetinib or both, or may be undergoing treatment with encorafenib, cobimetinib or both. Alternatively, the melanoma may have been earlier treated, or may be undergoing treatment, with another BRAF inhibitor, MEK inhibitor or both. The melanoma may, for example, be a relapsed melanoma. In one embodiment, the melanoma is an AXL-expressing melanoma. In another embodiment of the disclosure, the melanoma is not resistant to encorafenib or cobimetinib and/or has not earlier been treated with encorafenib or cobimetinib. In one embodiment of the disclosure, the BRAF mutation is an amino acid substitution in residue V600, L597 and/or K601. In one embodiment of the disclosure, the mutation is selected from V600E, V600D, V600K, L597R and K601E. The encorafenib may, for example, be administered orally at a total dose of 600 mg, such as 400 mg, such as 300 mg, such as 200 mg, such as 100 mg once or twice daily or every 2, 3 or 4 days, such as once daily (QD).

In one embodiment of the disclosure, the invention relates to the use of an AXL-ADC in a method of treating a melanoma in a subject, optionally wherein the melanoma exhibits a mutation in BRAF, the method comprising administering to the subject (i) an ADC comprising an antibody binding to human AXL, (ii) sorafenib, or a therapeutically effective analog or derivative thereof and (iii) cobimetinib or a therapeutically effective analog or derivative thereof. In one embodiment of the disclosure, the melanoma is resistant to sorafenib, cobimetinib or both. For example, the melanoma may have been earlier treated with sorafenib, cobimetinib or both, or may be undergoing treatment with sorafenib, cobimetinib or both. Alternatively, the melanoma may have been earlier treated, or may be undergoing treatment, with another BRAF inhibitor, MEK inhibitor or both. The melanoma may, for example, be a relapsed melanoma. In one embodiment, the melanoma is an AXL-expressing melanoma. In another embodiment of the disclosure, the melanoma is not resistant to sorafenib or cobimetinib and/or has not earlier been treated with sorafenib or cobimetinib. In one embodiment of the disclosure, the BRAF mutation is an amino acid substitution in residue V600, L597 and/or K601. In one embodiment, the mutation is selected from V600E, V600D, V600K, L597R and K601E. The sorafenib may, for example, be admistered orally at a total dose of 200-1600 mg, such as 1200 mg, such as 800 mg, such as 600 mg, such as 400 mg once or twice daily or every 2, 3 or 4 days, such as two tablets of 200 mg twice daily (equivalent to a total daily dose of 800 mg).

The cobimetinib may, for example, be administered orally to the subject at a dose of about 10 to 100 mg, such as about 30 to 80 mg, such as about 60 mg per day, optionally divided into 2, 3 or 4 separate doses. Preferably, the cobimetinib is administered at a dose of 60 mg a day (3 tablets of 20 mg) in 28 day cycles, wherein the drug is taken for 21 consecutive days, followed by a 7-day break.

In one embodiment, the disclosure relates to the use of an AXL-ADC in a method of treating a melanoma in a subject, wherein the melanoma exhibits a mutation in BRAF and the mutation providing for dabrafenib inhibition of BRAF kinase activity of the mutant BRAF, the method comprising administering to the subject (i) an ADC comprising an antibody binding to human AXL, (ii) dabrafenib, or a therapeutically effective analog or derivative thereof and (iii) binimetinib or a therapeutically effective analog or derivative thereof. In one embodiment of the disclosure, the melanoma is resistant to dabrafenib, binimetinib or both. For example, the melanoma may have been earlier treated with dabrafenib, binimetinib or both, or may be undergoing treatment with dabrafenib, binimetinib or both. Alternatively, the melanoma may have been earlier treated, or may be undergoing treatment, with another BRAF inhibitor, MEK inhibitor or both. The melanoma may, for example, be a relapsed melanoma. In one embodiment of the disclosure, the melanoma is an AXL-expressing melanoma. In another embodiment of the disclosure, the melanoma is not resistant to dabrafenib or binimetinib and/or has not earlier been treated with dabrafenib or binimetinib. In one embodiment of the disclosure, the BRAF mutation is an

amino acid substitution in residue V600, L597 and/or K601. In one embodiment of the disclosure, the mutation is selected from V600E, V600D, V600K, L597R and K601E. The dabrafenib may, for example, be administered orally to the subject at a dose of about 50-300 mg, such as about 100-200 mg, such as about 150 mg, once or twice daily or every 2 or 3 days. Preferably, the dabrafenib is administered as 150 mg orally twice daily, *e.g.,* at least 1hr before a meal or at least 2 hrs after a meal.

In one embodiment, the disclosure relates to the use of an AXL-ADC in a method of treating a melanoma in a subject, wherein the melanoma exhibits a mutation in BRAF and the mutation providing for vemurafenib inhibition of BRAF kinase activity of the mutant BRAF, the method comprising administering to the subject (i) an ADC comprising an antibody binding to human AXL, (ii) vemurafenib, or a therapeutically effective analog or derivative thereof and (iii) binimetinib or a therapeutically effective analog or derivative thereof. In one embodiment of the disclosure, the melanoma is resistant to vemurafenib, binimetinib or both. For example, the melanoma may have been earlier treated with vemurafenib, binimetinib or both, or may be undergoing treatment with vemurafenib, binimetinib or both. Alternatively, the melanoma may have been earlier treated, or may be undergoing treatment, with another BRAF inhibitor, MEK inhibitor or both. The melanoma may, for example, be a relapsed melanoma. In one embodiment of the disclosure, the melanoma is an AXL-expressing melanoma. In another embodiment of the disclosure, the melanoma is not resistant to vemurafenib or binimetinib and/or has not earlier been treated with vemurafenib or binimetinib. In one embodiment of the disclosure, the BRAF mutation is an amino acid substitution in residue V600, L597 and/or K601. In one embodiment, the mutation is selected from V600E, V600D, V600K, L597R and K601E. The vemurafenib may, for example, be administered orally at a dose of about 200-2000 mg, 500-1500 mg, such as about 1000 mg per day, *e.g.,* 960 mg, administered as 4 x 240 mg tablets q12hr (approximately 12 hr apart).

In one embodiment, the disclosure relates to the use of an AXL-ADC in a method of treating a melanoma in a subject, wherein the melanoma exhibits a mutation in BRAF and the mutation providing for encorafenib inhibition of BRAF kinase activity of the mutant BRAF, the method comprising administering to the subject (i) an ADC comprising an antibody binding to human AXL, (ii) encorafenib, or a therapeutically effective analog or derivative thereof and (iii) binimetinib or a therapeutically effective analog or derivative thereof. In one embodiment of the disclosure, the melanoma is resistant to encorafenib, binimetinib or both. For example, the melanoma may have been earlier treated with encorafenib, binimetinib or both, or may be undergoing treatment with encorafenib, binimetinib or both. Alternatively, the melanoma may have been earlier treated, or may be undergoing treatment, with another BRAF inhibitor, MEK inhibitor or both. The melanoma may, for example, be a relapsed melanoma. In one embodiment of the disclosure, the melanoma is an AXL-expressing melanoma. In another embodiment of the disclosure, the melanoma is not resistant to encorafenib or binimetinib and/or has not earlier been treated with encorafenib or binimetinib. In one embodiment of the disclosure, the BRAF mutation is an amino acid substitution in residue V600, L597 and/or K601. In one embodiment of the disclosure, the mutation is selected from V600E, V600D, V600K, L597R and K601E. The encorafenib may, for example, be administered orally at a total dose of 600 mg, such as 400 mg, such as 300 mg, such as 200 mg, such as 100 mg once or twice daily or every 2, 3 or 4 days, such as once daily (QD).

In one embodiment, the disclosure relates to the use of an AXL-ADC in a method of treating a melanoma in a subject, optionally wherein the melanoma exhibits a mutation in BRAF, the method comprising administering to the subject (i) an ADC comprising an antibody binding to human AXL, (ii) sorafenib, or a therapeutically effective analog or derivative thereof and (iii) binimetinib or a therapeutically effective analog or derivative thereof. In one embodiment of the disclosure, the melanoma is resistant to sorafenib, binimetinib or both. For example, the melanoma may have been earlier treated with sorafenib, binimetinib or both, or may be undergoing treatment with sorafenib, binimetinib or both. Alternatively, the melanoma may have been earlier treated, or may be undergoing treatment, with another BRAF inhibitor, MEK inhibitor or both. The melanoma may, for example, be a relapsed melanoma. In one embodiment, the melanoma is an AXL-expressing melanoma. In another embodiment of the disclosure, the melanoma is not resistant to sorafenib or binimetinib and/or has not earlier been treated with sorafenib or binimetinib. In one embodiment of the disclosure, the mutation is an amino acid substitution in residue V600, L597 and/or K601. In one embodiment of the disclosure, the BRAF mutation is selected from V600E, V600D, V600K, L597R and K601E. The sorafenib may, for example, be admistered orally at a total dose of 200-1600 mg, such as 1200 mg, such as 800 mg, such as 600 mg, such as 400 mg once or twice daily or every 2, 3 or 4 days, such as two tablets of 200 mg twice daily (equivalent to a total daily dose of 800 mg).

The binimetinib may, for example, be administered orally to the subject at a dose of about 10-200 mg, such as 150 mg, such as 100 mg, such as 90 mg, such as 45 mg, such as 30 mg, such as 20 mg once or twice daily, or every 2, 3 or 4 days, such as 45 mg twice daily.

In one embodiment, the disclosure relates to the use of an AXL-ADC in a method of treating a melanoma in a subject, wherein the melanoma exhibits a mutation in BRAF and the mutation providing for dabrafenib inhibition of BRAF kinase activity of the mutant BRAF, the method comprising administering to the subject (i) an ADC comprising an antibody binding to human AXL, (ii) dabrafenib, or a therapeutically effective analog or derivative thereof and (iii) selumetinib or a therapeutically effective analog or derivative thereof. In one embodiment of the disclosure, the melanoma is resistant to dabrafenib, selumetinib or both. For example, the melanoma may have been earlier treated with dabrafenib, selumetinib or

both, or may be undergoing treatment with dabrafenib, selumetinib or both. Alternatively, the melanoma may have been earlier treated, or may be undergoing treatment, with another BRAF inhibitor, MEK inhibitor or both. The melanoma may, for example, be a relapsed melanoma. In one embodiment of the disclosure, the melanoma is an AXL-expressing melanoma. In another embodiment, the melanoma is not resistant to dabrafenib or selumetinib and/or has not earlier been treated with dabrafenib or selumetinib. In one embodiment of the disclosure, the mutation is an amino acid substitution in residue V600, L597 and/or K601. In one embodiment, the mutation is selected from V600E, V600D, V600K, L597R and K601E. The dabrafenib may, for example, be administered orally to the subject at a dose of about 50-300 mg, such as about 100-200 mg, such as about 150 mg, once or twice daily or every 2 or 3 days. Preferably, the dabrafenib is administered as 150 mg orally twice daily, *e.g.,* at least 1hr before a meal or at least 2 hrs after a meal.

In one embodiment, the disclosure relates to the use of an AXL-ADC in a method of treating a melanoma in a subject, wherein the melanoma exhibits a mutation in BRAF and the mutation providing for vemurafenib inhibition of BRAF kinase activity of the mutant BRAF, the method comprising administering to the subject (i) an ADC comprising an antibody binding to human AXL, (ii) vemurafenib, or a therapeutically effective analog or derivative thereof and (iii) selumetinib or a therapeutically effective analog or derivative thereof. In one embodiment of the disclosure, the melanoma is resistant to vemurafenib, selumetinib or both. For example, the melanoma may have been earlier treated with vemurafenib, selumetinib or both, or may be undergoing treatment with vemurafenib, selumetinib or both. Alternatively, the melanoma may have been earlier treated, or may be undergoing treatment, with another BRAF inhibitor, MEK inhibitor or both. The melanoma may, for example, be a relapsed melanoma. In one embodiment, the melanoma is an AXL-expressing melanoma. In another embodiment, the melanoma is not resistant to vemurafenib or selumetinib and/or has not earlier been treated with vemurafenib or selumetinib. In one embodiment of the disclosure, the BRAF mutation is an amino acid substitution in residue V600, L597 and/or K601. In one embodiment of the disclosure, the mutation is selected from V600E, V600D, V600K, L597R and K601E. The vemurafenib may, for example, be administered orally at a dose of about 200-2000 mg, 500-1500 mg, such as about 1000 mg per day, *e.g.,* 960 mg, administered as 4 x 240 mg tablets q12hr (approximately 12 hr apart).

In one embodiment, the disclosure relates to the use of an AXL-ADC in a method of treating a melanoma in a subject, wherein the melanoma exhibits a mutation in BRAF and the mutation providing for encorafenib inhibition of BRAF kinase activity of the mutant BRAF, the method comprising administering to the subject (i) an ADC comprising an antibody binding to human AXL, (ii) encorafenib, or a therapeutically effective analog or derivative thereof and (iii) selumetinib or a therapeutically effective analog or derivative thereof. In one embodiment of the disclosure, the melanoma is resistant to encorafenib, selumetinib or both. For example, the melanoma may have been earlier treated with encorafenib, selumetinib or both, or may be undergoing treatment with encorafenib, selumetinib or both. Alternatively, the melanoma may have been earlier treated, or may be undergoing treatment, with another BRAF inhibitor, MEK inhibitor or both. The melanoma may, for example, be a relapsed melanoma. In one embodiment, the melanoma is an AXL-expressing melanoma. In another embodiment of the disclosure, the melanoma is not resistant to encorafenib or selumetinib and/or has not earlier been treated with encorafenib or selumetinib. In one embodiment of the disclosure, the BRAF mutation is an amino acid substitution in residue V600, L597 and/or K601. In one embodiment of the disclosure, the mutation is selected from V600E, V600D, V600K, L597R and K601E. The encorafenib may, for example, be administered orally at a total dose of 600 mg, such as 400 mg, such as 300 mg, such as 200 mg, such as 100 mg once or twice daily or every 2, 3 or 4 days, such as once daily (QD).

In one embodiment, the disclosure relates to the use of an AXL-ADC in a method of treating a melanoma in a subject, optionally wherein the melanoma exhibits a mutation in BRAF, the method comprising administering to the subject (i) an ADC comprising an antibody binding to human AXL, (ii) sorafenib, or a therapeutically effective analog or derivative thereof and (iii) selumetinib or a therapeutically effective analog or derivative thereof. In one embodiment of the disclosure, the melanoma is resistant to sorafenib, selumetinib or both. For example, the melanoma may have been earlier treated with sorafenib, selumetinib or both, or may be undergoing treatment with sorafenib, selumetinib or both. Alternatively, the melanoma may have been earlier treated, or may be undergoing treatment, with another BRAF inhibitor, MEK inhibitor or both. The melanoma may, for example, be a relapsed melanoma. In one embodiment of the disclosure, the melanoma is an AXL-expressing melanoma. In another embodiment of the disclosure, the melanoma is not resistant to sorafenib or selumetinib and/or has not earlier been treated with sorafenib or selumetinib. In one embodiment of the disclosure, the mutation is an amino acid substitution in residue V600, L597 and/or K601. In one embodiment of the disclosure, the mutation is selected from V600E, V600D, V600K, L597R and K601E. The sorafenib may, for example, be admistered orally at a total dose of 200-1600 mg, such as 1200 mg, such as 800 mg, such as 600 mg, such as 400 mg once or twice daily or every 2, 3 or 4 days, such as two tablets of 200 mg twice daily (equivalent to a total daily dose of 800 mg).

Selumetinib may, for example, be administered orally at a dose of about 50-225 mg, such as 75 mg, 100mg, 125mg, 150mg, 175mg, 200mg or 225mg once or twice a day, such as twice per day, optionally in a regimen where it is given for three days followed by four days off in four week cycles.

In one embodiment of the disclosure of the AXL-ADC for use in a method according to any one of the preceding embodiments, the melanoma does not exhibit a mutation in NRAS, such as is in an NRAS residue selected from Q61, G12

and G13, such as a mutation in NRAS selected from Q61R, Q61K, Q61L, G12D, G12S, G12C, G12V, G13D and G13R. In such embodiments, one of the one or more inhibitors of the MAPK pathway, *e.g.,* the serine/threonine kinase inhibitor, may comprise or consist of a BRAFi, *e.g.,* vemurafenib, dabrafenib, encorafenib or sorafenib.

In a particular embodiment of the preceding aspects of the disclosure, the AXL-ADC is used in combination with one or more MAPK pathway inhibitors, such as at least one serine/threonine kinase inhibitor, to treat recurrent melanoma in a subject, where the melanoma recurred after an initial treatment with a serine/threonine kinase inhibitor. Should the cancer recur yet again after the initial treatment with AXL-ADC, the AXL-ADC can be used again, together with the at least one serine/threonine kinase inhibitor, to treat the recurrent cancer.

In one aspect of the disclosure, the invention relates to a method of selecting a subject suffering from a melanoma for treatment with a combination of an AXL-ADC and at least one S/Th KI, comprising determining

(a) whether the subject meets the criteria for treatment with a S/Th KI;
(b) whether AXL expression in the melanoma is associated with resistance to the S/Th KI; and
(c) selecting a subject meeting the criteria for treatment with the S/Th KI and suffering from a melanoma for which AXL expression is associated with resistance to the S/Th KI.

In one aspect, the disclosure relates to a method of treating a subject diagnosed with having a melanoma which is, or has a high tendency for becoming, resistant to vemurafenib or a therapeutically effective analog or derivative thereof, comprising administering a therapeutically effective amount of an ADC comprising an antibody binding to human AXL and at least one MAPK pathway inhibitor, such as, e.g., a serine/threonine kinase inhibitor.

In one aspect, the disclosure relates to a method of determining if a subject suffering from melanoma is suitable for treatment with a combination of (i) a BRAFi such as vemurafenib, dabrafenib, encorafenib, sorafenib or a therapeutically effective analog or derivative thereof, (ii) a MEKi such as dabrafenib, cobimetinib, binimetinib, selumetinib or a therapeutically effective analog or derivative thereof, and (iii) an ADC comprising an antibody which binds to human AXL, wherein the subject is undergoing or has undergone treatment with the BRAFi, the MEKI or both, and is determined or suspected to be resistant to the BRAFi, MEKi or both, thus determining that the subject is suitable for the treatment. In a further aspect of the disclosure it may be determined if the melanoma expresses AXL.

In one embodiment of the disclosure, the resistant melanoma to be treated with an anti-AXL-ADC has been determined to express AXL.

In one particular embodiment of the disclosure, this is achieved by detecting levels of AXL antigen or levels of cells which express AXL on their cell surface in a sample, e.g., a tumor sample such as a biopsy, taken from a patient. The patient may, for example, suffer from melanoma or or be at risk for developing a melanoma. The AXL antigen to be detected can be soluble AXL antigen, cell-associated AXL antigen, or both. The sample to be tested can, for example, be contacted with an anti-AXL antibody under conditions that allow for binding of the antibody to AXL, optionally along with a control sample and/or control antibody binding to an irrelevant antigen. Binding of the antibody to AXL can then be detected (*e.g.*, using an ELISA). When using a control sample along with the test sample, the level of anti-AXL antibody or anti-AXL antibody AXL complex is analyzed in both samples and a statistically significant higher level of anti-AXL antibody or anti-AXL antibody-AXL complex in the test sample shows a higher level of AXL in the test sample compared with the control sample, indicating a higher expression of AXL. Examples of conventional immunoassays useful for such purposes include, without limitation, ELISA, RIA, FACS assays, plasmon resonance assays, chromatographic assays, tissue immunohistochemistry, Western blot, and/or immunoprecipitation.

A tissue sample may be taken from a tissue known or suspected of containing AXL antigen and/or cells expressing AXL. For example, in situ detection of AXL expression may be accomplished by removing a histological specimen such as a tumor biopsy or blood sample from a patient, and providing the anti-AXL antibody to such a specimen after suitable preparation of the specimen. The antibody may be provided by applying or by overlaying the antibody to the specimen, which is then detected using suitable means.

In the above assays, the anti-AXL antibody can be labeled with a detectable substance to allow AXL-bound antibody to be detected.

The level of AXL expressed on cells in a sample can also be determined according to the method described in Example 17, where AXL expression on the plasma membrane of human tumor cell lines was quantified by indirect immunofluorescence using QIFIKIT analysis (DAKO, Cat nr K0078), using a monoclonal anti-AXL antibody (here: mouse monoclonal antibody ab89224; Abcam, Cambridge, UK). Briefly, a single-cell suspension is prepared, and optionally washed. The next steps are performed on ice. The cells are seeded, *e.g.,* at 100,000 cells per well or tube, and thereafter pelleted and resuspended in 50 μL antibody sample at a concentration of 10 μg/mL, optionally adding a control antibody to a parallel sample. After an incubation of 30 minutes at 4ºC, cells are pelleted and resuspended in 150 μL FACS buffer, and the amount of AXL determined by FACS analysis using, *e.g.,* a secondary, FITC-labelled antibody binding to the anti-AXL and control antibodies. For each cell line, the antibody binding capacity (ABC), an estimate for the number of AXL molecules expressed on the plasma membrane, was calculated using the mean fluorescence intensity of the AXL

antibody-stained cells, based on the equation of a calibration curve as described in Example 23 (interpolation of unknowns from the standard curve). In one embodiment, using the method of Example 23, the level of AXL on AXL-expressing cells is estimated to at least 5000, such as at least 8000, such as at least 13000.

In one particular embodiment of the disclosure, the presence or level of AXL-expressing cells in a melanoma is assessed by *in vivo* imaging of detectably labelled anti-AXL antibodies according to methods known in the art. A significantly higher signal from a site, such as the known or suspected site of a melanoma tumor, than background or other control indicates overexpression of AXL in the tumor.

*AXL-ADCs*

ADCs suitable for use in the context of the present invention can be prepared from any anti-AXL antibody, typically an antibody binding to an extracellular region of human AXL. In one embodiment of the disclosure, the AXL antibody also binds to an extracellular region of cynomolgus monkey AXL. Preferred anti-AXL antibodies are characterized by one or more of the AXL-binding properties, variable or hypervariable sequences, or a combination of binding and sequence properties, set out in the aspects and embodiments below. In a particular aspect of the disclosure, the antibody binds to AXL but does not compete for AXL binding with the ligand Growth Arrest-Specific 6 (Gas6). Most preferred are the specific anti-AXL antibodies whose sequences are described in Table 2, in particular the antibody designated 107 and antibodies sharing one or more AXL-binding properties or CDR, VH and/or VL sequences with antibody 107.

So, in one particular embodiment of any preceding aspect or embodiment, the anti-AXL antibody comprises at least one binding region comprising a VH region and a VL region, wherein the VH region comprises the CDR1, CDR2 and CDR3 sequences of SEQ ID Nos.: 36, 37 and 38, and the VL region comprises the CDR1, CDR2 and CDR3 sequences of SEQ ID Nos.: 39, GAS, and 40.

Preferably, the ADC comprises such an anti-AXL antibody linked to a cytotoxic agent which is an auristatin or a functional peptide analog or derivate thereof, such as, *e.g.,* monomethyl auristatin E, preferably via a maleimidocaproyl-valine-citrulline-*p*-aminobenzyloxy-carbonyl (mc-vc-PAB) linker.

Particularly preferred for the aspects and embodiments herein is an AXL-ADC wherein the anti-AXL antibody is a full-length IgG1 antibody comprising a VH region and a VL region, wherein the VH region comprises the CDR1, CDR2 and CDR3 sequences of SEQ ID NOs.: 36, 37 and 38, and the VL region comprises the CDR1, CDR2 and CDR3 sequences of SEQ ID NOs.: 39, GAS, and 40, optionally wherein the VH and VL region comprise SEQ ID NO:1 and SEQ ID NO:2, respectively, linked to monomethyl auristatin E via a maleimidocaproyl-valine-citrulline-*p*-aminobenzyloxy-carbonyl (mc-vc-PAB) linker. Such an AXL-ADC may also be referred to herein as "HuMax-AXL-ADC" or "IgG1-AXL-107-vcMMAE".

The term "antibody" as used herein is intended to refer to an immunoglobulin molecule, a fragment of an immunoglobulin molecule, or a derivative of either thereof, which has the ability to specifically bind to an antigen under typical physiological and/or tumor-specific conditions with a half-life of significant periods of time, such as at least about 30 minutes, at least about 45 minutes, at least about one hour, at least about two hours, at least about four hours, at least about 8 hours, at least about 12 hours, about 24 hours or more, about 48 hours or more, about 3, 4, 5, 6, 7 or more days, etc., or any other relevant functionally-defined period (such as a time sufficient to induce, promote, enhance, and/or modulate a physiological response associated with antibody binding to the antigen and/or time sufficient for the antibody to be internalized). The binding region (or binding domain which may be used herein, both having the same meaning) which interacts with an antigen, comprises variable regions of both the heavy and light chains of the immunoglobulin molecule. The constant regions of the antibodies (Abs) may mediate the binding of the immunoglobulin to host tissues or factors, including various cells of the immune system (such as effector cells) and components of the complement system such as C1q, the first component in the classical pathway of complement activation. As indicated above, the term antibody as used herein, unless otherwise stated or clearly contradicted by context, includes fragments of an antibody that retain the ability to specifically interact, such as bind, to the antigen. It has been shown that the antigen-binding function of an antibody may be performed by fragments of a full-length antibody. Examples of binding fragments encompassed within the term "antibody" include (i) a Fab' or Fab fragment, a monovalent fragment consisting of the VL, VH, CL and CH1 domains, or a monovalent antibody as described in WO 2007/059782; (ii) $F(ab')_2$ fragments, bivalent fragments comprising two Fab fragments linked by a disulfide bridge at the hinge region; (iii) an Fd fragment consisting essentially of the VH and CH1 domains; (iv) an Fv fragment consisting essentially of the VL and VH domains of a single arm of an antibody, (v) a dAb fragment (Ward *et al.,* 1989), which consists essentially of a VH domain and is also called domain antibody (Holt *et al.,* 2003); (vi) camelid or nanobodies (Revets *et al.,* 2005) and (vii) an isolated complementarity determining region (CDR). Furthermore, although the two domains of the Fv fragment, VL and VH, are coded for by separate genes, they may be joined, using recombinant methods, by a synthetic linker that enables them to be made as a single protein chain in which the VL and VH regions pair to form monovalent molecules (known as single chain antibodies or single chain Fv (scFv), see for instance Bird *et al.* (1988) and Huston *et al.* (1988). Such single chain antibodies are encompassed within the term antibody unless otherwise noted or clearly indicated by context. Although such fragments are generally included within the meaning of antibody, they collectively and each independently are unique features of the present invention, exhibiting

different biological properties and utility. These and other useful antibody fragments in the context of the present invention are discussed further herein. It also should be understood that the term antibody, unless specified otherwise, also includes polyclonal antibodies, monoclonal antibodies (mAbs), antibody-like polypeptides, such as chimeric antibodies and humanized antibodies, as well as 'antibody fragments' or 'fragments thereof' retaining the ability to specifically bind to the antigen (antigen-binding fragments) provided by any known technique, such as enzymatic cleavage, peptide synthesis, and recombinant techniques, and retaining the ability to be conjugated to a toxin. An antibody as generated can possess any isotype.

The term "immunoglobulin heavy chain" or "heavy chain of an immunoglobulin" as used herein is intended to refer to one of the heavy chains of an immunoglobulin. A heavy chain is typically comprised of a heavy chain variable region (abbreviated herein as VH) and a heavy chain constant region (abbreviated herein as CH) which defines the isotype of the immunoglobulin. The heavy chain constant region typically is comprised of three domains, CH1, CH2, and CH3. The term "immunoglobulin" as used herein is intended to refer to a class of structurally related glycoproteins consisting of two pairs of polypeptide chains, one pair of light (L) low molecular weight chains and one pair of heavy (H) chains, all four potentially inter-connected by disulfide bonds. The structure of immunoglobulins has been well characterized (see for instance Fundamental Immunology Ch. 7 (Paul, W., ed., 2nd ed. Raven Press, N.Y. (1989). Within the structure of the immunoglobulin, the two heavy chains are inter-connected via disulfide bonds in the so-called "hinge region". Equally to the heavy chains each light chain is typically comprised of several regions; a light chain variable region (abbreviated herein as VL) and a light chain constant region. The light chain constant region typically is comprised of one domain, CL. Furthermore, the VH and VL regions may be further subdivided into regions of hypervariability (or hypervariable regions which may be hypervariable in sequence and/or form of structurally defined loops), also termed complementarity determining regions (CDRs), interspersed with regions that are more conserved, termed framework regions (FRs). Each VH and VL is typically composed of three CDRs and four FRs, arranged from amino-terminus to carboxy-terminus in the following order: FR1, CDR1, FR2, CDR2, FR3, CDR3, FR4. CDR sequences are defined according to IMGT (see Lefranc *et al.* (1999) and Brochet *et al.* (2008)).

The term "antigen-binding region" or "binding region" as used herein, refers to a region of an antibody which is capable of binding to the antigen. The antigen can be any molecule, such as a polypeptide, *e.g.* present on a cell, bacterium, or virion. The terms "antigen" and "target" may, unless contradicted by the context, be used interchangeably in the context of the present invention.

The term "binding" as used herein refers to the binding of an antibody to a predetermined antigen or target, typically it is binding with an affinity corresponding to a $K_D$ of about $10^{-6}$ M or less, *e.g.* $10^{-7}$ M or less, such as about $10^{-8}$ M or less, such as about $10^{-9}$ M or less, about $10^{-10}$ M or less, or about $10^{-11}$ M or even less when determined by for instance surface plasmon resonance (SPR) technology in a BIAcore 3000 instrument using the antigen as the ligand and the antibody as the analyte, and binds to the predetermined antigen with an affinity corresponding to a $K_D$ that is at least ten-fold lower, such as at least 100 fold lower, for instance at least 1,000 fold lower, such as at least 10,000 fold lower, for instance at least 100,000 fold lower than its $K_D$ of binding to a non-specific antigen (*e.g.,* BSA, casein) other than the predetermined antigen or a closely-related antigen. The amount with which the $K_D$ is lower is dependent on the $K_D$ of the antibody, so that when the $K_D$ of the antibody is very low (that is, the antibody is highly specific), then the amount with which the affinity for the antigen is lower than the affinity for a non-specific antigen may be at least 10,000 fold. The term "$K_D$" (M), as used herein, refers to the dissociation equilibrium constant of a particular antibody-antigen interaction, and is obtained by dividing $k_d$ by $k_a$. Affinity, as used herein, and $K_D$ are inversely related, that is higher affinity is intended to refer to lower $K_D$, and lower affinity is intended to refer to higher $K_D$.

The term "$k_d$" ($sec^{-1}$), as used herein, refers to the dissociation rate constant of a particular antibody-antigen interaction. Said value is also referred to as the $k_{off}$ value or off-rate.

The term "$k_a$" ($M^{-1} \times sec^{-1}$), as used herein, refers to the association rate constant of a particular antibody-antigen interaction. Said value is also referred to as the $k_{on}$ value or on-rate.

The term "$K_A$" ($M^{-1}$), as used herein, refers to the association equilibrium constant of a particular antibody-antigen interaction and is obtained by dividing $k_a$ by $k_d$.

The term "AXL" as used herein, refers to the protein entitled AXL, which is also referred to as UFO or JTK11, a 894 amino acid protein with a molecular weight of 104-140 kDa that is part of the subfamily of mammalian TAM Receptor Tyrosine Kinases (RTKs). The molecular weight is variable due to potential differences in glycosylation of the protein. The AXL protein consists of two extracellular immunoglobulin-like (Ig-like) domains on the N-terminal end of the protein, two membrane-proximal extracellular fibronectin type III (FNIII) domains, a transmembrane domain and an intracellular kinase domain. AXL is activated upon binding of its ligand Gas6, by ligand-independent homophilic interactions between AXL extracellular domains, by autophosphorylation in presence of reactive oxygen species (Korshunov *et al.,* 2012) or by transactivation through EGFR (Meyer *et al.,* 2013), and is aberrantly expressed in several tumor types. In humans, the AXL protein is encoded by a nucleic acid sequence encoding the amino acid sequence shown in SEQ ID NO:130 (human AXL protein: Swissprot P30530; cynomolgus AXL protein: Genbank accession HB387229.1 (SEQ ID NO:147)).

The term "ligand-independent homophilic interactions" as used herein, refers to association between two AXL

molecules (expressed on neighboring cells) that occurs in absence of the ligand.

The term "antibody binding AXL" as used herein, refers to any antibody binding an epitope on the extracellular part of AXL.

The term "epitope" means a protein determinant capable of specific binding to an antibody. Epitopes usually consist of surface groupings of molecules such as amino acids, sugar side chains or a combination thereof and usually have specific three dimensional structural characteristics, as well as specific charge characteristics. Conformational and non-conformational epitopes are distinguished in that the binding to the former but not the latter is lost in the presence of denaturing solvents. The epitope may comprise amino acid residues which are directly involved in the binding, and other amino acid residues, which are not directly involved in the binding, such as amino acid residues which are effectively blocked or covered by the specific antigen binding peptide (in other words, the amino acid residue is within the footprint of the specific antigen binding peptide).

The term "ligand" as used herein, refers to a substance, such as a hormone, peptide, ion, drug or protein, that binds specifically and reversibly to another protein, such as a receptor, to form a larger complex. Ligand binding to a receptor may alter its chemical conformation, and determines its functional state. For instance, a ligand may function as agonist or antagonist.

The term "Growth Arrest-Specific 6" or "Gas6" as used herein, refers to a 721 amino acid protein, with a molecular weight of 75-80 kDa, that functions as a ligand for the TAM family of receptors, including AXL. Gas6 is composed of an N-terminal region containing multiple gamma-carboxyglutamic acid residues (Gla), which are responsible for the specific interaction with the negatively charged phospholipid membrane. Although the Gla domain is not necessary for binding of Gas6 to AXL, it is required for activation of AXL. Gas6 may also be termed as the "ligand to AXL".

The terms "monoclonal antibody", "monoclonal Ab", "monoclonal antibody composition", "mAb", or the like, as used herein refer to a preparation of antibody molecules of single molecular composition. A monoclonal antibody composition displays a single binding specificity and affinity for a particular epitope. Accordingly, the term "human monoclonal antibody" refers to antibodies displaying a single binding specificity which have variable and constant regions derived from human germline immunoglobulin sequences. The human monoclonal antibodies may be produced by a hybridoma which includes a B cell obtained from a transgenic or transchromosomal non-human animal, such as a transgenic mouse, having a genome comprising a human heavy chain transgene and a light chain transgene, fused to an immortalized cell.

In the context of the present invention the term "ADC" refers to an antibody drug conjugate, which in the context of the present invention refers to an anti-AXL antibody which is coupled to a therapeutic moiety, e.g., a cytotoxic moiety as described in the present application. It may *e.g.* be coupled with a linker to *e.g.* cysteine or with other conjugation methods to other amino acids. The moiety may *e.g.* be a drug or a toxin or the like.

As used herein, a "therapeutic moiety" means a compound which exerts a therapeutic or preventive effect when administered to a subject, particularly when delivered as an ADC as described herein. A "cytotoxic" or "cytostatic" moiety is a compound that is detrimental to (*e.g.,* kills) cells. Some cytotoxic or cytostatic moieties for use in ADCs are hydrophobic, meaning that they have no or only a limited solubility in water, *e.g.,* 1 g/L or less (very slightly soluble), such as 0.8 g/L or less, such as 0.6 g/L or less, such as 0.4 g/L or less, such as 0.3 g/L or less, such as 0.2 g/L or less, such as 0.1 g/L or less (practically insoluble). Exemplary hydrophobic cytotoxic or cytostatic moieties include, but are not limited to, certain microtubulin inhibitors such as auristatin and its derivatives, *e.g.,* MMAF and MMAE, as well as maytansine and its derivatives, *e.g.,* DM1.

In one embodiment of the disclosure, the antibody has a binding affinity ($K_D$) in the range of $0.3\times10^{-9}$ to $63\times10^{-9}$ M to AXL, and wherein said binding affinity is measured using a Bio-layer Interferometry using soluble AXL extracellular domain.

The binding affinity may be determined as described in Example 2. Thus, in one embodiment, the antibody has a binding affinity of $0.3\times10^{-9}$ to $63\times10^{-9}$ M to the antigen, wherein the binding affinity is determined by a method comprising the steps of;

i) loading anti-human Fc Capture biosensors with anti-AXL antibodies, and
ii) determining association and dissociation of soluble recombinant AXL extracellular domain by Bio-Layer Interferometry at different concentrations.

The term "soluble recombinant AXL extracellular domain" as used herein, refers to an AXL extracellular domain, corresponding to amino acids 1-447 of the full-length protein (SEQ ID NO:130; see Example 1) that has been expressed recombinantly. Due to absence of the transmembrane and intracellular domain, recombinant AXL extracellular domain is not attached to a, *e.g.* cell surface and stays in solution. It is well-known how to express a protein recombinantly, see *e.g.* Sambrook (1989), and thus, it is within the knowledge of the skilled person to provide such recombinant AXL extracellular domain.

In one embodiment of the disclosure, the antibody has a dissociation rate of $6.9\times10^{-5}$ $s^{-1}$ to $9.7\times10^{-3}$ $s^{-1}$ to AXL,

and wherein the dissociation rate is measured by Bio-layer Interferometry using soluble recombinant AXL extracellular domain.

Optionally, the antibody has a dissociation rate of 9.7×10-5 to 4.4×10-3 s-1 to AXL, and wherein the dissociation rate is measured by Bio-layer Interferometry using soluble recombinant AXL extracellular domain.

The binding affinity may be determined as described above (and in Example 2). Thus, in one embodiment, the antibody has a dissociation rate of $6.9\times10^{-5}$ $s^{-1}$ to $9.7\times10^{-3}$ $s^{-1}$ to AXL, and wherein the dissociation rate is measured by a method comprising the steps of

i) loading anti-human Fc Capture biosensors with anti-AXL antibodies, and
ii) determining association and dissociation of recombinant AXL extracellular domain by Bio-Layer Interferometry at different concentrations.

The term "dissociation rate" as used herein, refers to the rate at which an antigen-specific antibody bound to its antigen, dissociates from that antigen, and is expressed as $s^{-1}$. Thus, in the context of an antibody binding AXL, the term "dissociation rate", refers to the antibody binding AXL dissociates from the recombinant extracellular domain of AXL, and is expressed as $s^{-1}$.

In one aspect of the disclosure, the ADCs for the use of the present invention comprises an antibody-portion which binds to an extracellular domain of AXL without competing or interfering with Gas6 binding to AXL. In a particular embodiment of the disclosure, the antibody binds to the extracellular domain Ig1domain without competing or interfering with Gas6 binding to AXL. In one embodiment of the disclosure, the antibody binds to the extracellular domain Ig1 and show no more than a 20% reduction in maximal Gas6 binding to AXL. In one embodiment of the disclosure, the antibody show no more than a 15% reduction in maximal Gas6 binding to AXL. In one embodiment of the disclosure, the antibody show no more than a 10% reduction in maximal Gas6 binding to AXL. In one embodiment of the disclosure, the antibody show no more than a 5% reduction in maximal Gas6 binding to AXL. In one embodiment of the disclosure, the antibody show no more than a 4% reduction in maximal Gas6 binding to AXL In one embodiment of the disclosure, the antibody show no more than a 2% reduction in maximal Gas6 binding to AXL. In one embodiment of the disclosure, the antibody show no more than a 1% reduction in maximal Gas6 binding. In one embodiment of the disclosure the antibody binds to the Ig2 domain in the AXL extracellular domain without competing or interfering with Gas6 binding to AXL. In one embodiment of the disclosure, the antibody binds to the Ig2 domain in the AXL extracellular domain and show no more than a 20%, such as no more than 15%, such as no more than 10%, such as no more than 5%, such as no more than 4%, such as no more than 2%, such as no more than 1%, reduction in maximal Gas6 binding to AXL. The embodiment's ability to compete with or reduce Gas6 binding may be determined as disclosed in Example 2 or Example 12. In one embodiment the antibody binds to the Ig2 domain in the AXL extracellular domain without competing or interfering with maximal Gas6 binding to AXL.

In one embodiment, maximal antibody binding in the presence of Gas6 is at least 90%, such as at least 95%, such as at least 97%, such as at least 99%, such as 100%, of binding in absence of Gas6 as determined by a competition assay, wherein competition between said antibody binding to human AXL and said Gas6 is determined on A431 cells preincubated with Gas6 and without Gas6.

Competition between anti-AXL and the ligand Gas6 to AXL may be determined as described in Example 2 under the heading "Interference of anti-AXL binding with Gas6 binding". Thus, in one embodiment of the disclosure, the antibody does not compete for AXL binding with the ligand Gas6, wherein the competing for binding is determined in an assay comprising the steps of

i) incubating AXL-expressing cells with Gas6,
ii) adding anti-AXL antibodies to be tested,
iii) adding a fluorescently labelled secondary reagent detecting anti-AXL antibodies and
iv) analyzing the cells by FACS.

In another embodiment, the antibody does not compete for binding with the ligand Gas6, wherein the competing for binding is determined in an assay comprising the steps of

i) incubating AXL-expressing cells with anti-AXL antibodies,
ii) adding Gas6,
iii) adding a fluorescently labelled secondary reagent detecting Gas6, and
iv) analyzing the cells by FACS.

In one embodiment of the disclosure, the antibody modulates AXL-associated signaling in an AXL-expressing cell of the when the cell is contacted with the antibody.

In one embodiment of the disclosure, the antibody does not modulate AXL-associated signaling in an AXL-expressing cell of the when the cell is contacted with the antibody.

Non-limiting examples of modulation of AXL-associated signalling includes modulation of intracellular signaling pathways such as the PI3K/AKT, mitogen-activated protein kinase (MAPK), STAT or NF-κB cascades.

In one embodiment of the disclosure, the anti-AXL antibody or AXL-ADC competes for binding to AXL with an antibody comprising a variable heavy (VH) region and a variable light (VL) region selected from the group consisting of:

(a) a VH region comprising SEQ ID No:1 and a VL region comprising SEQ ID No:2 [**107**];
(b) a VH region comprising SEQ ID No:5 and a VL region comprising SEQ ID No:6 [**148**];
(c) a VH region comprising SEQ ID No:34 and a VL region comprising SEQ ID No:35 [**733**]
(d) a VH region comprising SEQ ID No:7 and a VL region comprising SEQ ID No:9 [**154**];
(e) a VH region comprising SEQ ID No:10 and a VL region comprising SEQ ID No:11 [**171**];
(f) a VH region comprising SEQ ID No:16 and a VL region comprising SEQ ID No:18 [**183**];
(g) a VH region comprising SEQ ID No:25 and a VL region comprising SEQ ID No:26 [**613**];
(h) a VH region comprising SEQ ID No:31 and a VL region comprising SEQ ID No:33 [**726**];
(i) a VH region comprising SEQ ID No:3 and a VL region comprising SEQ ID No:4 [**140**];
(j) a VH region comprising SEQ ID No:8 and a VL region comprising SEQ ID No:9 [**154-M103L**];
(k) a VH region comprising SEQ ID No:12 and a VL region comprising SEQ ID No:13 [**172**];
(l) a VH region comprising SEQ ID No:14 and a VL region comprising SEQ ID No:15 [**181**];
(m)a VH region comprising SEQ ID No:17 and a VL region comprising SEQ ID No:18 [**183-N52Q**];
(n) a VH region comprising SEQ ID No:19 and a VL region comprising SEQ ID No:20 [**187**];
(o) a VH region comprising SEQ ID No:21 and a VL region comprising SEQ ID No:22 [**608-01**];
(p) a VH region comprising SEQ ID No:23 and a VL region comprising SEQ ID No:24 [**610-01**];
(q) a VH region comprising SEQ ID No:27 and a VL region comprising SEQ ID No:28 [**613-08**];
(r) a VH region comprising SEQ ID No:29 and a VL region comprising SEQ ID No:30 [**620-06**]; and
(s) a VH region comprising SEQ ID No:32 and a VL region comprising SEQ ID No:33 [**726-M101L**].

When used herein in the context of an antibody and a Gas6 ligand or in the context of two or more antibodies, the term "competes with" or "cross-competes with" indicates that the antibody competes with the ligand or another antibody, *e.g.*, a "reference" antibody in binding to an antigen, respectively. Example 2 describes an example of how to test competition of an anti-AXL antibody with the AXL-ligand Gas6. Preferred reference antibodies for cross-competition between two antibodies are those comprising a binding region comprising the VH region and VL region of an antibody herein designated 107, 148, 733, 154, 171, 183, 613, 726, 140, 154-M103L, 172, 181, 183-N52Q, 187, 608-01, 610-01, 613-08, 620-06 or 726-M101L, as set forth in Table 4. A particularly preferred reference antibody is the antibody designated 107.

In one embodiment of the disclosure, the anti-AXL antibody binds to the same epitope on AXL as any one or more of the antibodies according to the aforementioned embodiment, as defined by their VH and VL sequences, *e.g.*, a VH region comprising SEQ ID No:1 and a VL region comprising SEQ ID No:2 [**107**].

Methods of determining an epitope to which an antibody binds are well-known in the art. Thus, the skilled person would know how to determine such an epitope. However, one example of determining whether an antibody binds within any epitope herein described may be by introducing point mutations into the extracellular domain of AXL extracellular domain, *e.g.,* for the purpose of identifying amino acids involved in the antibody-binding to the antigen. It is within the knowledge of the skilled person to introduce point mutation(s) in the AXL extracellular domain and test for antibody binding to point mutated AXL extracellular domains, since the effect of point mutations on the overall 3D structure is expected to be minimal.

An alternative method was used in Example 3, wherein the AXL domain specificity was mapped by preparing a panel of human-mouse chimeric AXL mutants where the human Ig1, Ig2, FN1 or FN2 domain had been replaced by its murine analog, and determining which mutant an anti-AXL antibody bound to. This method was based on the principle that these human AXL-specific antibodies recognized human but not mouse AXL. So, in separate and specific embodiments of the disclosure, the antibody binds to the Ig1 domain of AXL, the Ig2 domain of AXL, the FN1 domain of AXL, or the FN2 domain of AXL.

A more high-resolution epitope-mapping method, identifying AXL extracellular domain amino acids involved in antibody binding, was also used in this Example. Specifically, this method analyzed binding of the anti-AXL antibody to a library of AXL sequence variants generated by recombination of AXL sequences derived from species with variable levels of homology with the human AXL sequence (SEQ ID NO:130) in the extracellular domain. This method was based on the principle that these human AXL-specific antibodies recognize human AXL, but not the AXL from any of the other species used in the example.

So, in one embodiment, the antibody binds to an epitope within the Ig1 domain of AXL, and the antibody binding is dependent on one or more or all of the amino acids corresponding to positions L121 to Q129 or one or more or all of T112 to

Q124 of human AXL, wherein the numbering of amino acid residues refers to their respective positions in human AXL (SEQ ID NO:130). In one embodiment of the disclosure, the antibody binds to an epitope within the Ig1 domain of AXL, and antibody binding is dependent on the amino acids corresponding to positions L121 to Q129 or T112 to Q124 of human AXL. In a preferred embodiment of the disclosure antibody binding is dependent on one or more or all amino acids in position L121, G122, H123, Q124, T125, F126, V127, S128 and Q129, corresponding to the amino acids involved in the binding of the antibody herein designated 107. In one embodiment of the disclosure, antibody binding is dependent on one or more or all amino acid in position T112, G113, Q114, Y115, Q116, C117, L118,V119, F120, L121, G122, H123 and Q124.

In another embodiment, the antibody binds to an epitope within the Ig2 domain of AXL, and antibody binding is dependent on one or more or all of the amino acids corresponding to position D170 or the combination of D179 or one or more or all of the amino acids in positions T182 to R190 of human AXL. In one embodiment of the disclosure antibody binding is dependent on the amino acids in position T182, A183, P183, G184, H185, G186, P187, Q189 and R190.

In another embodiment, the antibody binds to an the FN1 domain of human AXL, and antibody binding is dependent on one or more or all of the amino acids corresponding to positions Q272 to A287 and G297 to P301 of human AXL. In one embodiment of the disclosure, antibody binding is dependent on the amino acids corresponding to positions Q272 to A287 and G297 to P301 of human AXL.

In another embodiment, the antibody binds to the FN2 domain of human AXL and antibody binding is dependent on one or more or all of the amino acids corresponding to positions A359, R386, and Q436 to K439 of human AXL.

In yet another embodiment, the antibody binds to an epitope within the Ig1 domain of AXL, and the epitope comprises or requires one or more or all of the amino acids corresponding to positions L121 to Q129 or one or more or all of T112 to Q124 of human AXL, wherein the numbering of amino acid residues refers to their respective positions in human AXL (SEQ ID NO:130). In one embodiment of the disclosure, the antibody binds to an epitope within the Ig1 domain of AXL, and the epitope comprises or requires the amino acids corresponding to positions L121 to Q129 or T112 to Q124 of human AXL. In a preferred embodiment of the disclosure the epitope comprises one or more or all amino acid in position L121, G122, H123, Q124, T125, F126, V127, S128 and Q129, corresponding to the amino acids involved in the binding of the antibody herein designated 107. In one embodiment of the disclosure, the epitope comprises one or more or all amino acid in position T112, G113, Q114, Y115, Q116, C117, L118,V119, F120, L121, G122, H123 and Q124.

In another embodiment, the antibody binds to an epitope within the Ig2 domain of AXL, and the epitope comprises or requires one or more or all of the amino acids corresponding to position D170 or the combination of D179 or one or more or all of the amino acids in positions T182 to R190 of human AXL. In one embodiment of the disclosure the epitope comprises or requires the amino acids in position T182, A183, P183, G184, H185, G186, P187, Q189 and R190.

In another embodiment, the antibody binds to an epitope within the FN1 domain of human AXL, which epitope comprises or requires one or more or all of the amino acids corresponding to positions Q272 to A287 and G297 to P301 of human AXL. In one embodiment of the disclosure, the epitope comprises or requires the amino acids corresponding to positions Q272 to A287 and G297 to P301 of human AXL.

In another embodiment, the antibody binds to an epitope within the FN2 domain of human AXL, which epitope comprises or requires one or more or all of the amino acids corresponding to positions A359, R386, and Q436 to K439 of human AXL.

In one embodiment of the disclosure, the antibody binds to an epitope within the FN1-like domain of human AXL.

In one embodiment of the disclosure, the antibody binds to an epitope on AXL which epitope is recognized by any one of the antibodies defined by

a)) a VH region comprising the CDR1, CDR2, and CDR3 sequences of SEQ ID Nos.: 36, 37, and 38, respectively; and a VL region comprising the CDR1, CDR2, and CDR3 sequences of SEQ ID Nos.: 39, GAS, and 40, respectively, [**107**];
b) a VH region comprising the CDR1, CDR2, and CDR3 sequences of SEQ ID Nos.: 46, 47, and 48, respectively; and a VL region comprising the CDR1, CDR2, and CDR3 sequences of SEQ ID Nos.: 49, AAS, and 50, respectively, [**148**];
c) a VH region comprising the CDR1, CDR2, and CDR3 sequences of SEQ ID Nos.: 114, 115, and 116, respectively, and a VL region comprising the CDR1, CDR2, and CDR3 sequences of SEQ ID Nos.: 117, DAS, and 118, respectively [**733**];
d) a VH region comprising the CDR1, CDR2, and CDR3 sequences of SEQ ID Nos.: 51, 52, and 53, respectively; and a VL region comprising the CDR1, CDR2, and CDR3 sequences of SEQ ID Nos.: 55, GAS, and 56, respectively [**154**];
e) a VH region comprising the CDR1, CDR2, and CDR3 sequences of SEQ ID Nos.: 51, 52, and 54, respectively; and a VL region comprising the CDR1, CDR2, and CDR3 sequences of SEQ ID Nos.: 55, GAS, and 56, respectively [**154-M103L**];
f) a VH region comprising the CDR1, CDR2, and CDR3 sequences of SEQ ID Nos.: 57, 58, and 59, respectively; and a VL region comprising the CDR1, CDR2, and CDR3 sequences of SEQ ID Nos.: 60, GAS, and 61, respectively, [**171**];
g) a VH region comprising the CDR1, CDR2, and CDR3 sequences of SEQ ID Nos.: 62, 63, and 64, respectively; and a VL region comprising the CDR1, CDR2, and CDR3 sequences of SEQ ID Nos.: 65, GAS, and 66, respectively, [**172**];
h) a VH region comprising the CDR1, CDR2, and CDR3 sequences of SEQ ID Nos.: 67, 68, and 69, respectively; and a

VL region comprising the CDR1, CDR2, and CDR3 sequences of SEQ ID Nos.: 70, GAS, and 71, respectively, [**181**];
i) a VH region comprising the CDR1, CDR2, and CDR3 sequences of SEQ ID Nos.: 72, 73, and 75, respectively; and a VL region comprising the CDR1, CDR2, and CDR3 sequences of SEQ ID Nos.: 76, ATS, and 77, respectively, [**183**];
j) a VH region comprising the CDR1, CDR2, and CDR3 sequences of SEQ ID Nos.: 72, 74, and 75, respectively; and a VL region comprising the CDR1, CDR2, and CDR3 sequences of SEQ ID Nos.: 76, ATS, and 77, respectively, [**183-N52Q**];
k) a VH region comprising the CDR1, CDR2, and CDR3 sequences of SEQ ID Nos.: 78, 79, and 80, respectively; and a VL region comprising the CDR1, CDR2, and CDR3 sequences of SEQ ID Nos.: 81, AAS, and 82, respectively, [**187**];
l) a VH region comprising the CDR1, CDR2, and CDR3 sequences of SEQ ID Nos.: 83, 84, and 85, respectively; and a VL region comprising the CDR1, CDR2, and CDR3 sequences of SEQ ID Nos.: 86, GAS, and 87, respectively, [**608-01**];
m) a VH region comprising the CDR1, CDR2, and CDR3 sequences of SEQ ID Nos.: 88, 89, and 90, respectively; and a VL region comprising the CDR1, CDR2, and CDR3 sequences of SEQ ID Nos.: 91, GAS, and 92, respectively, [**610-01**];
n) a VH region comprising the CDR1, CDR2, and CDR3 sequences of SEQ ID Nos.: 93, 94, and 95, respectively; and a VL region comprising the CDR1, CDR2, and CDR3 sequences of SEQ ID Nos.: 96, GAS, and 97, respectively, [**613**];
o) a VH region comprising the CDR1, CDR2, and CDR3 sequences of SEQ ID Nos.: 98, 99, and 100, respectively; and a VL region comprising the CDR1, CDR2, and CDR3 sequences of SEQ ID Nos.: 10, DAS, and 102, respectively, [**613-08**];
p) a VH region comprising the CDR1, CDR2, and CDR3 sequences of SEQ ID Nos.: 103, 104, and 105, respectively; and a VL region comprising the CDR1, CDR2, and CDR3 sequences of SEQ ID Nos.: 106, GAS, and 107, respectively, [**620-06**];
q) a VH region comprising the CDR1, CDR2, and CDR3 sequences of SEQ ID Nos.: 108, 109, and 110, respectively; and a VL region comprising the CDR1, CDR2, and CDR3 sequences of SEQ ID Nos.: 112, AAS, and 113, respectively, [**726**];
r) a VH region comprising the CDR1, CDR2, and CDR3 sequences of SEQ ID Nos.: 108, 109, and 111, respectively; and a VL region comprising the CDR1, CDR2, and CDR3 sequences of SEQ ID Nos.: 112, AAS, and 113, respectively, [**726-M101L**];
s) a VH region comprising the CDR1, CDR2, and CDR3 sequences of SEQ ID Nos.: 41, 42, and 43, respectively; and a VL region comprising the CDR1, CDR2, and CDR3 sequences of SEQ ID Nos.: 44, AAS, and 45, respectively, [**140**];
t) a VH region comprising the CDR1, CDR2, and CDR3 sequences of SEQ ID Nos.: 93, 94, and 95, respectively, and a VL region comprising the CDR1, CDR2, and CDR3 sequences of SEQ ID Nos.: 128, XAS, wherein X is D or G, and 129, respectively, [**613** / **613-08**];
u) a VH region comprising the CDR1, CDR2, and CDR3 sequences of SEQ ID Nos.: 46, 119, and 120, respectively; and a VL region comprising CDR1, CDR2, and CDR3 sequences of SEQ ID Nos.: 49, AAS, and 50, respectively, [**148 / 140**];
v) a VH region comprising the CDR1, CDR2, and CDR3 sequences of SEQ ID Nos.: 123, 124, and 125, respectively; and a VL region comprising CDR1, CDR2, and CDR3 sequences of SEQ ID Nos.: 60, GAS, and 61, respectively [**171** / **172** / **181**]; and
w) a VH region comprising the CDR1, CDR2, and CDR3 sequences of SEQ ID Nos.: 121, 109, and 122, respectively; and a VL region comprising the CDR1, CDR2, and CDR3 sequences of SEQ ID Nos.: 112, AAS, and 113, respectively [**726 / 187**]; and
x) a VH region comprising the CDR1, CDR2, and CDR3 sequences of SEQ ID Nos.:93, 126, and 127, respectively; and a VL region comprising the CDR1, CDR2, and CDR3 sequences of SEQ ID Nos.: 96, GAS, and 97, respectively [**613** / **608-01** / **610-01** / **620-06**].

In a particular embodiment of the disclosure, the antibody binds to an epitope on AXL which epitope is recognized by any one of the antibodies defined by comprising a binding regon comprising the VH and VL sequences of an antibody selected from those herein designated 107, 061, 137, 148, 154-M103L, 171, 183-N52Q, 511, 613, 726-M102L and 733. As shown in Example 16, these anti-AXL antibodies internalize, and are thus suitable for an ADC approach.
In one embodiment, the antibody comprises at least one binding region comprising a VH region and a VL region selected from the group consisting of:

(a) a VH region comprising the CDR1, CDR2, and CDR3 sequences of SEQ ID Nos.:36, 37, and 38, respectively; and a VL region comprising the CDR1, CDR2, and CDR3 sequences of SEQ ID Nos.:39, GAS, and 40, respectively, [**107**];
(b) a VH region comprising the CDR1, CDR2, and CDR3 sequences of SEQ ID Nos.:46, 47, and 48, respectively; and a VL region comprising the CDR1, CDR2, and CDR3 sequences of SEQ ID Nos.:49, AAS, and 50, respectively, [**148**];
(c) a VH region comprising the CDR1, CDR2, and CDR3 sequences of SEQ ID Nos.:114, 115, and 116, respectively,

and a VL region comprising the CDR1, CDR2, and CDR3 sequences of SEQ ID Nos.:117, DAS, and 118, respectively [**733**];

(d) a VH region comprising the CDR1, CDR2, and CDR3 sequences of SEQ ID Nos.:51, 52, and 53, respectively; and a VL region comprising the CDR1, CDR2, and CDR3 sequences of SEQ ID Nos.:55, GAS, and 56, respectively [**154**];

(e) a VH region comprising the CDR1, CDR2, and CDR3 sequences of SEQ ID Nos.:51, 52, and 54, respectively; and a VL region comprising the CDR1, CDR2, and CDR3 sequences of SEQ ID Nos.:55, GAS, and 56, respectively [**154-M103L**];

(f) a VH region comprising the CDR1, CDR2, and CDR3 sequences of SEQ ID Nos.:57, 58, and 59, respectively; and a VL region comprising the CDR1, CDR2, and CDR3 sequences of SEQ ID Nos.:60, GAS, and 61, respectively, [**171**];

(g) a VH region comprising the CDR1, CDR2, and CDR3 sequences of SEQ ID Nos.:62, 63, and 64, respectively; and a VL region comprising the CDR1, CDR2, and CDR3 sequences of SEQ ID Nos.:65, GAS, and 66, respectively, [**172**];

(h) a VH region comprising the CDR1, CDR2, and CDR3 sequences of SEQ ID Nos.:67, 68, and 69, respectively; and a VL region comprising the CDR1, CDR2, and CDR3 sequences of SEQ ID Nos.:70, GAS, and 71, respectively, [**181**];

(i) a VH region comprising the CDR1, CDR2, and CDR3 sequences of SEQ ID Nos.:72, 73, and 75, respectively; and a VL region comprising the CDR1, CDR2, and CDR3 sequences of SEQ ID Nos.:76, ATS, and 77, respectively, [**183**];

(j) a VH region comprising the CDR1, CDR2, and CDR3 sequences of SEQ ID Nos.:72, 74, and 75, respectively; and a VL region comprising the CDR1, CDR2, and CDR3 sequences of SEQ ID Nos.:76, ATS, and 77, respectively, [**183-N52Q**];

(k) a VH region comprising the CDR1, CDR2, and CDR3 sequences of SEQ ID Nos.:78, 79, and 80, respectively; and a VL region comprising the CDR1, CDR2, and CDR3 sequences of SEQ ID Nos.:81, AAS, and 82, respectively, [**187**];

(l) a VH region comprising the CDR1, CDR2, and CDR3 sequences of SEQ ID Nos.:83, 84, and 85, respectively; and a VL region comprising the CDR1, CDR2, and CDR3 sequences of SEQ ID Nos.:86, GAS, and 87, respectively, [**608-01**];

(m) a VH region comprising the CDR1, CDR2, and CDR3 sequences of SEQ ID Nos.:88, 89, and 90, respectively; and a VL region comprising the CDR1, CDR2, and CDR3 sequences of SEQ ID Nos.:91, GAS, and 92, respectively, [**610-01**];

(n) a VH region comprising the CDR1, CDR2, and CDR3 sequences of SEQ ID Nos.:93, 94, and 95, respectively; and a VL region comprising the CDR1, CDR2, and CDR3 sequences of SEQ ID Nos.:96, GAS, and 97, respectively, [**613**];

(o) a VH region comprising the CDR1, CDR2, and CDR3 sequences of SEQ ID Nos.:98, 99, and 100, respectively; and a VL region comprising the CDR1, CDR2, and CDR3 sequences of SEQ ID Nos.:101, DAS, and 102, respectively, [**613-08**];

(p) a VH region comprising the CDR1, CDR2, and CDR3 sequences of SEQ ID Nos.:103, 104, and 105, respectively; and a VL region comprising the CDR1, CDR2, and CDR3 sequences of SEQ ID Nos.:106, GAS, and 107, respectively, [**620-06**];

(q) a VH region comprising the CDR1, CDR2, and CDR3 sequences of SEQ ID Nos.:108, 109, and 110, respectively; and a VL region comprising the CDR1, CDR2, and CDR3 sequences of SEQ ID Nos.:112, AAS, and 113, respectively, [**726**];

(r) a VH region comprising the CDR1, CDR2, and CDR3 sequences of SEQ ID Nos.:108, 109, and 111, respectively; and a VL region comprising the CDR1, CDR2, and CDR3 sequences of SEQ ID Nos.:112, AAS, and 113, respectively, [**726-M101L**];

(s) a VH region comprising the CDR1, CDR2, and CDR3 sequences of SEQ ID Nos.:41, 42, and 43, respectively; and a VL region comprising the CDR1, CDR2, and CDR3 sequences of SEQ ID Nos.:44, AAS, and 45, respectively, [**140**];

(t) a VH region comprising the CDR1, CDR2, and CDR3 sequences of SEQ ID Nos.:93, 94, and 95, respectively, and a VL region comprising the CDR1, CDR2, and CDR3 sequences of SEQ ID Nos.:128, XAS, wherein X is D or G, and 129, respectively, [**613** / **613-08**];

(u) a VH region comprising the CDR1, CDR2, and CDR3 sequences of SEQ ID Nos.:46, 119, and 120, respectively; and a VL region comprising CDR1, CDR2, and CDR3 sequences of SEQ ID Nos.:49, AAS, and 50, respectively, [**148 / 140**];

(v) a VH region comprising the CDR1, CDR2, and CDR3 sequences of SEQ ID Nos.:123, 124, and 125, respectively; and a VL region comprising CDR1, CDR2, and CDR3 sequences of SEQ ID Nos.:60, GAS, and 61, respectively [**171**/ **172** / **181**]; and

(w) a VH region comprising the CDR1, CDR2, and CDR3 sequences of SEQ ID Nos.:121, 109, and 122, respectively; and a VL region comprising the CDR1, CDR2, and CDR3 sequences of SEQ ID Nos.:112, AAS, and 113, respectively [**726** / **187**]; and

(x) a VH region comprising the CDR1, CDR2, and CDR3 sequences of SEQ ID Nos.:93, 126, and 127, respectively; and a VL region comprising the CDR1, CDR2, and CDR3 sequences of SEQ ID Nos.:96, GAS, and 97, respectively [**613** / **608-01** / **610-01** / **620-06**].

In one embodiment, the antibody comprises at least one binding region comprising a VH region and a VL region selected from the group consisting of:

(a) a VH region comprising SEQ ID No:1 and a VL region comprising SEQ ID No:2 [**107**];
(b) a VH region comprising SEQ ID No:5 and a VL region comprising SEQ ID No:6 [**148**];
(c) a VH region comprising SEQ ID No:34 and a VL region comprising SEQ ID No:35 [**733**]
(d) a VH region comprising SEQ ID No:7 and a VL region comprising SEQ ID No:9 [**154**];
(e) a VH region comprising SEQ ID No:10 and a VL region comprising SEQ ID No:11 [**171**];
(f) a VH region comprising SEQ ID No:16 and a VL region comprising SEQ ID No:18 [**183**];
(g) a VH region comprising SEQ ID No:25 and a VL region comprising SEQ ID No:26 [**613**];
(h) a VH region comprising SEQ ID No:31 and a VL region comprising SEQ ID No:33 [**726**];
(i) a VH region comprising SEQ ID No:3 and a VL region comprising SEQ ID No:4 [**140**];
(j) a VH region comprising SEQ ID No:8 and a VL region comprising SEQ ID No:9 [**154-M103L**];
(k) a VH region comprising SEQ ID No:12 and a VL region comprising SEQ ID No:13 [**172**];
(l) a VH region comprising SEQ ID No:14 and a VL region comprising SEQ ID No:15 [**181**];
(m) a VH region comprising SEQ ID No:17 and a VL region comprising SEQ ID No:18 [**183-N52Q**];
(n) a VH region comprising SEQ ID No:19 and a VL region comprising SEQ ID No:20 [**187**];
(o) a VH region comprising SEQ ID No:21 and a VL region comprising SEQ ID No:22 [**608-01**];
(p) a VH region comprising SEQ ID No:23 and a VL region comprising SEQ ID No:24 [**610-01**];
(q) a VH region comprising SEQ ID No:27 and a VL region comprising SEQ ID No:28 [**613-08**];
(r) a VH region comprising SEQ ID No:29 and a VL region comprising SEQ ID No:30 [**620-06**]; and
(s) a VH region comprising SEQ ID No:32 and a VL region comprising SEQ ID No:33 [**726-M101L**].

The present invention also provides antibodies comprising functional variants of the VL region, VH region, or one or more CDRs of the antibodies mentioned above. A functional variant of a VL, VH, or CDR used in the context of an AXL antibody still allows the antibody to retain at least a substantial proportion (at least about 50%, 60%, 70%, 80%, 90%, 95%, 99% or more) of the affinity/avidity and/or the specificity/selectivity of the parent antibody and in some cases such an AXL antibody may be associated with greater affinity, selectivity and/or specificity than the parent antibody.

Such functional variants typically retain significant sequence identity to the parent antibody. The percent identity between two sequences is a function of the number of identical positions shared by the sequences (*i.e.,* % homology = # of identical positions/total # of positions x 100), taking into account the number of gaps, and the length of each gap, which need to be introduced for optimal alignment of the two sequences. The comparison of sequences and determination of percent identity between two sequences may be accomplished using a mathematical algorithm, which is well-known in the art.

The sequence identity between two amino acid sequences may, for example, be determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

(Identical Residues x 100)/(Length of Alignment - Total Number of Gaps in Alignment).

The VH, VL and/or CDR sequences of variants may differ from those of the parent antibody sequences through mostly conservative substitutions; for instance at least about 35%, about 50% or more, about 60% or more, about 70% or more, about 75% or more, about 80% or more, about 85% or more, about 90% or more, (*e.g.,* about 65-95%, such as about 92%, 93% or 94%) of the substitutions in the variant are conservative amino acid residue replacements.

The VH, VL and/or CDR sequences of variants may differ from those of the parent antibody sequences through mostly conservative substitutions; for instance 10 or less, such as 9 or less, 8 or less, 7 or less, 6 or less, 5 or less, 4 or less, 3 or less, 2 or less or 1 of the substitutions in the variant are conservative amino acid residue replacements.

Embodiments are also provided wherein mutations or substitutions of up to five mutations or substitutions are allowed across the three CDR sequences in the variable heavy chain and/or variable light chain of the preceding embodiment. The up to five mutations or substitutions may be distributed across the three CDR sequences of the variable heavy chain and the three CDR sequences of the variable light chain. The up to five mutations or substitutions may be distributed across the six CDR sequences of the binding region. The mutations or substitutions may be of conservative, physical or functional amino acids such that mutations or substitutions do not change the epitope or preferably do not modify binding affinity to the epitope more than 30 %, such as more than 20 % or such as more than 10%. The conservative,

physical or functional amino acids are selected from the 20 natural amino acids found i.e, Arg, His, Lys, Asp, Glu, Ser, Thr, Asn, Gln, Cys, Gly, Pro, Ala, Ile, Leu, Met, Phe, Trp, Tyr and Val.

So, in one embodiment, the antibody comprises at least one binding region comprising a VH region and a VL region selected from the group consisting of VH and VL sequences at least 90%, such as at least 95%, such as at least 97%, such as at least 99% identical to:

(a) a VH region comprising SEQ ID No:1 and a VL region comprising SEQ ID No:2 [**107**];
(b) a VH region comprising SEQ ID No:5 and a VL region comprising SEQ ID No:6 [**148**];
(c) a VH region comprising SEQ ID No:34 and a VL region comprising SEQ ID No:35 [**733**]
(d) a VH region comprising SEQ ID No:7 and a VL region comprising SEQ ID No:9 [**154**];
(e) a VH region comprising SEQ ID No:10 and a VL region comprising SEQ ID No: 11 [**171**];
(f) a VH region comprising SEQ ID No: 16 and a VL region comprising SEQ ID No:18 [**183**];
(g) a VH region comprising SEQ ID No:25 and a VL region comprising SEQ ID No:26 [**613**];
(h) a VH region comprising SEQ ID No:31 and a VL region comprising SEQ ID No:33 [**726**];
(i) a VH region comprising SEQ ID No:3 and a VL region comprising SEQ ID No:4 [**140**];
(j) a VH region comprising SEQ ID No:8 and a VL region comprising SEQ ID No:9 [**154-M103L**];
(k) a VH region comprising SEQ ID No:12 and a VL region comprising SEQ ID No: 13 [**172**];
(l) a VH region comprising SEQ ID No:14 and a VL region comprising SEQ ID No: 15 [**181**];
(m)a VH region comprising SEQ ID No:17 and a VL region comprising SEQ ID No:18 [**183-N52Q**];
(n) a VH region comprising SEQ ID No:19 and a VL region comprising SEQ ID No:20 [**187**];
(o) a VH region comprising SEQ ID No:21 and a VL region comprising SEQ ID No:22 [**608-01**];
(p) a VH region comprising SEQ ID No:23 and a VL region comprising SEQ ID No:24 [**610-01**];
(q) a VH region comprising SEQ ID No:27 and a VL region comprising SEQ ID No:28 [**613-08**];
(r) a VH region comprising SEQ ID No:29 and a VL region comprising SEQ ID No:30 [**620-06**]; and
(s) a VH region comprising SEQ ID No:32 and a VL region comprising SEQ ID No:33 [**726-M101L**].

The present disclosure also provides antibodies comprising functional variants of the VL region, VH region, or one or more CDRs of the antibodies of the examples. A functional variant of a VL, VH, or CDR used in the context of an AXL antibody still allows the antibody to retain at least a substantial proportion (at least about 50%, 60%, 70%, 80%, 90%, 95%, 99% or more) of the affinity/avidity and/or the specificity/selectivity of the parent antibody and in some cases such an AXL antibody may be associated with greater affinity, selectivity and/or specificity than the parent antibody.

Such functional variants typically retain significant sequence identity to the parent antibody. The percent identity between two sequences is a function of the number of identical positions shared by the sequences (*i.e.,* % homology = # of identical positions/total # of positions x 100), taking into account the number of gaps, and the length of each gap, which need to be introduced for optimal alignment of the two sequences. The comparison of sequences and determination of percent identity between two sequences may be accomplished using a mathematical algorithm, which is well-known in the art.

The VH, VL and/or CDR sequences of variants may differ from those of the parent antibody sequences through mostly conservative substitutions; for instance at least about 35%, about 50% or more, about 60% or more, about 70% or more, about 75% or more, about 80% or more, about 85% or more, about 90% or more, (*e.g.,* about 65-95%, such as about 92%, 93% or 94%) of the substitutions in the variant are conservative amino acid residue replacements.

The VH, VL and/or CDR sequences of variants may differ from those of the parent antibody sequences through mostly conservative substitutions; for instance 10 or less, such as 9 or less, 8 or less, 7 or less, 6 or less, 5 or less, 4 or less, 3 or less, 2 or less or 1 of the substitutions in the variant are conservative amino acid residue replacements.

Embodiments of the disclosure are also provided wherein mutations or substitutions of up to five mutations or substitutions are allowed across the three CDR sequences in the variable heavy chain and/or variable light chain of the preceding embodiment. The up to five mutations or substitutions may be distributed across the three CDR sequences of the variable heavy chain and the three CDR sequences of the variable light chain. The up to five mutations or substitutions may be distributed across the six CDR sequences of the binding region. The mutations or substitutions may be of conservative, physical or functional amino acids such that mutations or substitutions do not change the epitope or preferably do not modify binding affinity to the epitope more than 30 %, such as more than 20 % or such as more than 10%. The conservative, physical or functional amino acids are selected from the 20 natural amino acids found i.e, Arg, His, Lys, Asp, Glu, Ser, Thr, Asn, Gln, Cys, Gly, Pro, Ala, Ile, Leu, Met, Phe, Trp, Tyr and Val.

In one embodiment of the disclosure, the antibody comprises at least one binding region comprising a VH region and a VL region selected from the group consisting of VH and VL sequences at least 90%, such as at least 95%, such as at least 97%, such as at least 99% identical to:

(t) a VH region comprising SEQ ID No:1 and a VL region comprising SEQ ID No:2 [**107**];

(u) a VH region comprising SEQ ID No:5 and a VL region comprising SEQ ID No:6 [**148**];
(v) a VH region comprising SEQ ID No:34 and a VL region comprising SEQ ID No:35 [**733**]
(w) a VH region comprising SEQ ID No:7 and a VL region comprising SEQ ID No:9 [**154**];
(x) a VH region comprising SEQ ID No:10 and a VL region comprising SEQ ID No:11 [**171**];
(y) a VH region comprising SEQ ID No:16 and a VL region comprising SEQ ID No:18 [**183**];
(z) a VH region comprising SEQ ID No:25 and a VL region comprising SEQ ID No:26 [**613**];
(aa)a VH region comprising SEQ ID No:31 and a VL region comprising SEQ ID No:33 [**726**];
(bb) a VH region comprising SEQ ID No:3 and a VL region comprising SEQ ID No:4 [**140**];
(cc) a VH region comprising SEQ ID No:8 and a VL region comprising SEQ ID No:9 [**154-M103L**];
(dd) a VH region comprising SEQ ID No:12 and a VL region comprising SEQ ID No:13 [**172**];
(ee)a VH region comprising SEQ ID No:14 and a VL region comprising SEQ ID No:15 [**181**];
(ff) a VH region comprising SEQ ID No:17 and a VL region comprising SEQ ID No:18 [**183-N52Q**];
(gg)a VH region comprising SEQ ID No:19 and a VL region comprising SEQ ID No:20 [**187**];
(hh) a VH region comprising SEQ ID No:21 and a VL region comprising SEQ ID No:22 [**608-01**];
(ii) a VH region comprising SEQ ID No:23 and a VL region comprising SEQ ID No:24 [**610-01**];
(jj) a VH region comprising SEQ ID No:27 and a VL region comprising SEQ ID No:28 [**613-08**];
(kk) a VH region comprising SEQ ID No:29 and a VL region comprising SEQ ID No:30 [**620-06**]; and
(ll) a VH region comprising SEQ ID No:32 and a VL region comprising SEQ ID No:33 [**726-M101L**].

In one embodiment of the disclosure, the antibody comprises at least one binding region comprising the VH and VL CDR1, CDR2, and CDR3 sequences of an anti-AXL antibody known in the art, *e.g.,* an antibody described in any of Leconet *et al.* (2013), Li *et al.* (2009), Ye *et al.* (2010), Iida *et al.* (2014), WO 2012/175691 (INSERM), WO 2012/175692 (INSERM), WO 2013/064685 (Pierre Fabré Medicaments), WO 2013/090776 (INSERM), WO 2009/063965 (Chugai Pharmaceuticals), WO 2010/131733, WO 2011/159980 (Genentech), WO09062690 (U3 Pharma), WO2010130751 (U3 Pharma), WO2014093707 (Stanford University) and EP2228392A1 (Chugai). In one specific embodiment of the disclosure, the antibody is murine antibody 1613F12 or a chimeric or a humanized variant thereof as described in WO2014174111 (Pierre Fabré Medicament), wherein the VH and VL sequences of the mouse antibody 1613F12 are presented as SEQ ID:8 and SEQ ID:7, respectively. The VH sequence of the humanized antibody variant of 1613F12 is selected from the sequences disclosed therein as SEQ ID NO:29 to 49 and SEQ ID NO:82, and the VL sequence of the humanized antibody variant of 1613F12 is selected from the sequences disclosed therein as SEQ ID NO:17 to 28 and SEQ ID: 81. One specific antibody comprises the VH and VL sequences disclosed therein as SEQ ID NO:29 and 17, respectively. The VH CDR1, CDR2 and CDR3 sequences of mouse, chimeric and humanized 1613F12 are SEQ ID NO:4, 5 and 6, respectively and the VL CDR1, CDR2 and CDR3 sequences of mouse and humanized 1613F12 are disclosed therein as SEQ ID NO:1, 2, and 3, respectively. In one specific embodiment of the disclosure, the antibody is an antibody described in WO2011159980 (Hoffman-La Roche), particularly paragraphs [0127] through [0229] (pages 28-52). For example, the antibody may comprise the VH and VL hypervariable regions (HVR), or the VH and VL regions, of antibody YW327.6S2, which are disclosed therein as SEQ ID NOS:7, 8 and 9 (VH HVR1, 2 and 3, respectively), SEQ ID NOS:10, 11 and 12 (VL HVR1, 2 and 3, respectively) and SEQ ID NOS:103 and 104 (VH and VL sequences, respectively).

In one embodiment of the disclosure, the antibody mediates antibody-mediated crosslinking or clustering (*e.g.,* due to the Fc-region of AXL-bound antibodies binding to FcR-expressing cells) of AXL molecules on the surface of a cell, which can lead to apoptosis of the cell.

In one embodiment of the disclosure, the antibody induces an Fc-dependent cellular response such as ADCC or ADCP against an AXL-expressing cell after binding of the AXL-specific antibody to the plasma membrane of the AXL-expressing cell in the presence of effector cells. In this embodiment, the antibody-portion of the antibody is typically full-length and of an isotype leading to an ADCC or ADCP response, such as, *e.g.,* an IgG1,$\kappa$ isotype.

In one embodiment of the disclosure, the antibody induces a CDC response against an AXL-expressing cell after binding of the AXL-specific antibody to the plasma membrane of the AXL-expressing cell in the presence of complement proteins, such as complement proteins present in normal human serum, that may be activated. In this embodiment, the antibody is typically full-length and of an isotype capable of inducing activation of the complement system, such as, *e.g.,* an IgG1,$\kappa$ isotype.

The antibody and/or ADC may further be characterized by internalization upon binding to AXL. Accordingly, in one embodiment of the disclosure, the antibody and/or ADC is internalized and trafficked to lysosomes for specific (*i.e.* cleavable linker) or non-specific (non-cleavable linker) proteolytic cleavage of the anti-AXL antibody-linker-drug complex.

In one embodiment of the disclosure, the antibody interferes with AXL-mediated regulation of the innate or adaptive immune response, such as by binding of the antibody to AXL-expressing macrophages, dendritic cells or NK cells.

In one embodiment of the disclosure, the therapeutic moiety of the ADC is linked to the antibody moiety via a linker allowing for release of the drug once the ADC is internalized, *e.g.,* by a change in pH or reducing conditions. Suitable linker

technology is known in the art, as described herein.

In one embodiment, the antibody comprises a heavy chain of an isotype selected from the group consisting of IgG1, IgG2, IgG3, and IgG4.

The term "isotype" as used herein refers to the immunoglobulin class (for instance IgG1, IgG2, IgG3, IgG4, IgD, IgA, IgE, or IgM) or any allotype thereof, such as IgG1m(za) and IgG1m(f)) that is encoded by heavy chain constant region genes. Further, each heavy chain isotype can be combined with either a kappa (κ) or lambda (λ) light chain.

In one embodiment, the isotype is IgG1, such as human IgG1, optionally allotype IgG1m(f).

In one embodiment, the antibody is a full-length monoclonal antibody, optionally a full-length human monoclonal IgG1,κ antibody.

The term "full-length antibody" when used herein, refers to an antibody (*e.g.,* a parent or variant antibody) which contains all heavy and light chain constant and variable domains corresponding to those that are normally found in a wild-type antibody of that isotype. A full-length antibody according to the present invention may be produced by a method comprising the steps of (i) cloning the CDR sequences into a suitable vector comprising complete heavy chain sequences and complete light chain sequence, and (ii) expressing the complete heavy and light chain sequences in suitable expression systems. It is within the knowledge of the skilled person to produce a full-length antibody when starting out from either CDR sequences or full variable region sequences. Thus, the skilled person would know how to generate a full-length antibody according to the present invention.

In one embodiment of the disclosure, the antibody is a human antibody.

The term "human antibody", as used herein, is intended to include antibodies having variable and framework regions derived from human germline immunoglobulin sequences and a human immunoglobulin constant domain. The human antibodies of the invention may include amino acid residues not encoded by human germline immunoglobulin sequences (*e.g.,* mutations, insertions or deletions introduced by random or site-specific mutagenesis *in vitro* or by somatic mutation *in vivo*). However, the term "human antibody", as used herein, is not intended to include antibodies in which CDR sequences derived from the germline of another non-human species, such as a mouse, have been grafted onto human framework sequences.

As used herein, a human antibody is "derived from" a particular germline sequence if the antibody is obtained from a system using human immunoglobulin sequences, for instance by immunizing a transgenic mouse carrying human immunoglobulin genes or by screening a human immunoglobulin gene library, and wherein the selected human antibody is at least 90%, such as at least 95%, for instance at least 96%, such as at least 97%, for instance at least 98%, or such as at least 99% identical in amino acid sequence to the amino acid sequence encoded by the germline immunoglobulin gene. Typically, outside the heavy chain CDR3, a human antibody derived from a particular human germline sequence will display no more than 20 amino acid differences, *e.g.* no more than 10 amino acid differences, such as no more than 9, 8, 7, 6 or 5, for instance no more than 4, 3, 2, or 1 amino acid difference from the amino acid sequence encoded by the germline immunoglobulin gene.

The antibody according to the present invention may comprise amino acid modifications in the immunoglobulin heavy and/or light chains. In a particular embodiment, amino acids in the Fc region of the antibody may be modified.

The term "Fc region" as used herein, refers to a region comprising, in the direction from the N- to C-terminal end of the antibody, at least a hinge region, a CH2 region and a CH3 region. An Fc region of the antibody may mediate the binding of the immunoglobulin to host tissues or factors, including various cells of the immune system (such as effector cells) and components of the complement system.

The term "hinge region" as used herein refers to the hinge region of an immunoglobulin heavy chain. Thus, for example the hinge region of a human IgG1 antibody corresponds to amino acids 216-230 according to the Eu numbering as set forth in Kabat *et al.* (1991). However, the hinge region may also be any of the other subtypes as described herein.

The term "CH1 region" or "CH1 domain" as used herein refers to the CH1 region of an immunoglobulin heavy chain. Thus, for example the CH1 region of a human IgG1 antibody corresponds to amino acids 118-215 according to the Eu numbering as set forth in Kabat *et al.* (1991) . However, the CH1 region may also be any of the other subtypes as described herein.

The term "CH2 region" or "CH2 domain" as used herein refers to the CH2 region of an immunoglobulin heavy chain. Thus, for example the CH2 region of a human IgG1 antibody corresponds to amino acids 231-340 according to the Eu numbering as set forth in Kabat *et al.* (1991) . However, the CH2 region may also be any of the other subtypes as described herein.

The term "CH3 region" or "CH3 domain" as used herein refers to the CH3 region of an immunoglobulin heavy chain. Thus for example the CH3 region of a human IgG1 antibody corresponds to amino acids 341-447 according to the Eu numbering as set forth in Kabat *et al.* (1991). However, the CH3 region may also be any of the other subtypes as described herein.

In another embodiment of the disclosure, the antibody is an effector-function-deficient antibody, a stabilized IgG4 antibody or a monovalent antibody.

In one particular embodiment of the disclosure, the heavy chain has been modified such that the entire hinge

region has been deleted.

In one embodiment of the disclosure, the sequence of the antibody has been modified so that it does not comprise any acceptor sites for N-linked glycosylation.

In one embodiment of the disclosure, the antibody is a single-chain antibody.

In further aspect, the present disclosure invention relates to a multispecific antibody comprising at least a first binding region of an antibody according to any aspect or embodiment herein described, and a second binding region which binds a different target or epitope than the first binding region. The term "multispecific antibody" as used herein, refers to antibodies wherein the binding regions bind to at least two, such as at least three, different antigens or at least two, such as at least three, different epitopes on the same antigen.

In one embodiment, the present disclosure relates to the use of an ADC comprising a bispecific antibody comprising a first binding region of an antibody according to any aspect or embodiments herein described, and a second binding region which binds a different target or epitope than the first binding region.

The term "bispecific" as used herein, refers to binding molecules, such as antibodies wherein the binding regions of the binding molecule bind to two different antigens or two different epitopes on the same antigen.

The term "bispecific antibody" refers to an antibody having specificities for at least two different, typically non-overlapping, epitopes. Such epitopes may be on the same or different targets. If the epitopes are on different targets, such targets may be on the same cell or different cells, cell types or structures, such as extracellular tissue.

The term "different target" as used herein, refers to another protein, molecule or the like than AXL or an AXL fragment.

Examples of bispecific antibody molecules which may be used in the present invention comprise (i) a single antibody that has two arms comprising different antigen-binding regions, (ii) a single chain antibody that has specificity to two different epitopes, *e.g.,* via two scFvs linked in tandem by an extra peptide linker; (iii) a dual-variable-domain antibody (DVD-Ig™), where each light chain and heavy chain contains two variable domains in tandem through a short peptide linkage (Wu *et al.,* 2010); (iv) a chemically-linked bispecific (Fab')2 fragment; (v) a Tandab®, which is a fusion of two single chain diabodies resulting in a tetravalent bispecific antibody that has two binding sites for each of the target antigens; (vi) a flexibody, which is a combination of scFvs with a diabody resulting in a multivalent molecule; (vii) a so called "dock and lock" molecule (Dock-and-Lock®), based on the "dimerization and docking domain" in Protein Kinase A, which, when applied to Fabs, can yield a trivalent bispecific binding protein consisting of two identical Fab fragments linked to a different Fab fragment; (viii) a so-called Scorpion molecule, comprising, *e.g.,* two scFvs fused to both termini of a human Fab-arm; and (ix) a diabody.

In one embodiment, the bispecific antibody of the present disclosure is a diabody, a cross-body, such as CrossMabs, or a bispecific antibody obtained via a controlled Fab arm exchange (such as described in WO 2011/131746, Genmab A/S).

Examples of different classes of bispecific antibodies include but are not limited to (i) IgG-like molecules with complementary CH3 domains to force heterodimerization; (ii) recombinant IgG-like dual targeting molecules, wherein the two sides of the molecule each contain the Fab fragment or part of the Fab fragment of at least two different antibodies; (iii) IgG fusion molecules, wherein full length IgG antibodies are fused to extra Fab fragment or parts of Fab fragment; (iv) Fc fusion molecules, wherein single chain Fv molecules or stabilized diabodies are fused to heavy-chain constant-domains, Fc-regions or parts thereof; (v) Fab fusion molecules, wherein different Fab-fragments are fused together, fused to heavy-chain constant-domains, Fc-regions or parts thereof; and (vi) ScFv-and diabody-based and heavy chain antibodies (*e.g.,* domain antibodies, Nanobodies®) wherein different single chain Fv molecules or different diabodies or different heavy-chain antibodies (*e.g.* domain antibodies, Nanobodies®) are fused to each other or to another protein or carrier molecule fused to heavy-chain constant-domains, Fc-regions or parts thereof.

Examples of IgG-like molecules with complementary CH3 domains molecules include but are not limited to the Triomab® (Trion Pharma/Fresenius Biotech, WO/2002/020039), Knobs-into-Holes (Genentech, WO9850431), CrossMAbs (Roche, WO 2009/080251, WO 2009/080252, WO 2009/080253), electrostatically-matched Fc-heterodimeric molecules (Amgen, EP1870459 and WO2009089004; Chugai, US201000155133; Oncomed, WO2010129304), LUZ-Y (Genentech), DIG-body, PIG-body and TIG-body (Pharmabcine), Strand Exchange Engineered Domain body (SEEDbody) (EMD Serono, WO2007110205), Bispecific IgG1 and IgG2 (Pfizer/Rinat, WO11143545), Azymetric scaffold (Zymeworks/Merck, WO2012058768), mAb-Fv (Xencor, WO2011028952), XmAb (Xencor), Bivalent bispecific antibodies (Roche, WO2009/080254), Bispecific IgG (Eli Lilly), DuoBody® molecules (Genmab A/S, WO 2011/131746), DuetMab (Medimmune, US2014/0348839), Biclonics (Merus, WO 2013/157953), NovImmune (κλBodies, WO 2012/023053), FcΔAdp (Regeneron, WO 2010/151792), (DT)-Ig (GSK/Domantis), Two-in-one Antibody or Dual Action Fabs (Genentech, Adimab), mAb2 (F-Star, WO2008003116), Zybodies™ (Zyngenia), CovX-body (CovX/Pfizer), FynomAbs (Covagen/Janssen Cilag), DutaMab (Dutalys/Roche), iMab (MedImmune), Dual Variable Domain (DVD)-IgTM (Abbott, US 7,612,18), dual domain double head antibodies (Unilever; Sanofi Aventis, WO20100226923), Ts2Ab (MedImmune/AZ), BsAb (Zymogenetics), HERCULES (Biogen Idec, US007951918), scFv-fusions (Genentech/Roche, Novartis, Immunomedics, Changzhou Adam Biotech Inc, CN 102250246), TvAb (Roche, WO2012025525,

WO2012025530), ScFv/Fc Fusions, SCORPION (Emergent BioSolutions/Trubion, Zymogenetics/BMS), Interceptor (Emergent), Dual Affinity Retargeting Technology (Fc-DARTTM) (MacroGenics, WO2008/157379, WO2010/080538), BEAT (Glenmark), Di-Diabody (Imclone/Eli Lilly) and chemically crosslinked mAbs (Karmanos Cancer Center), and covalently fused mAbs (AIMM therapeutics).

Examples of recombinant IgG-like dual targeting molecules include but are not limited to Dual Targeting (DT)-Ig (GSK/Domantis), Two-in-one Antibody (Genentech), Cross-linked Mabs (Karmanos Cancer Center), mAb2 (F-Star, WO2008003116), Zybodies™ (Zyngenia), approaches with common light chain (Crucell/Merus, US 7,262,028), κλBodies (NovImmune) and CovX-body (CovX/Pfizer).

Examples of IgG fusion molecules include but are not limited to Dual Variable Domain (DVD)-Ig™ (Abbott, US 7,612,181), Dual domain double head antibodies (Unilever; Sanofi Aventis, WO20100226923), IgG-like Bispecific (ImClone/Eli Lilly), Ts2Ab (MedImmune/AZ) and BsAb (Zymogenetics), HERCULES (Biogen Idec, US 7,951,918), scFv fusion (Novartis), scFv fusion (Changzhou Adam Biotech Inc, CN 102250246) and TvAb (Roche, WO2012025525, WO2012025530).

Examples of Fc fusion molecules include but are not limited to ScFv/Fc Fusions (Academic Institution), SCORPION (Emergent BioSolutions/Trubion, Zymogenetics/BMS), Dual Affinity Retargeting Technology (Fc-DART™) (MacroGenics, WO2008157379 and WO2010080538) and Dual(ScFv)2-Fab (National Research Center for Antibody Medicine - China).

Examples of Fab fusion bispecific antibodies include but are not limited to F(ab)2 (Medarex/AMGEN), Dual-Action or Bis-Fab (Genentech), Dock-and-Lock® (DNL) (ImmunoMedics), Bivalent Bispecific (Biotecnol) and Fab-Fv (UCB-Celltech).

Examples of ScFv-, diabody-based and domain antibodies include but are not limited to Bispecific T Cell Engager (BiTE®) (Micromet, Tandem Diabody (Tandab™) (Affimed), Dual Affinity Retargeting Technology (DART) (MacroGenics), Single-chain Diabody (Academic), TCR-like Antibodies (AIT, ReceptorLogics), Human Serum Albumin ScFv Fusion (Merrimack) and COMBODY (Epigen Biotech), dual targeting nanobodies® (Ablynx), dual targeting heavy chain only domain antibodies.

A bispecific antibody for use as an ADC according the present invention may be generated by introducing modifications in the constant region of the antibody.

In one particular embodiment of the disclosure, the bispecific antibody comprises a first and a second heavy chain, each of the first and second heavy chain comprises at least a hinge region, a CH2 and CH3 region, wherein in the first heavy chain at least one of the amino acids in the positions corresponding to positions selected from the group consisting of K409, T366, L368, K370, D399, F405, and Y407 in a human IgG1 heavy chain has been substituted, and in the second heavy chain at least one of the amino acids in the positions corresponding to a position selected from the group consisting of F405, T366, L368, K370, D399, Y407, and K409 in a human IgG1 heavy chain has been substituted, and wherein the first and the second heavy chains are not substituted in the same positions.

In one embodiment of the disclosure, in the first heavy chain the amino acid in the position corresponding to K409 in a human IgG1 heavy chain is not K, L or M and optionally the amino acid in the position corresponding to F405 in a human IgG1 heavy chain is F, and in the second heavy chain the amino acid in the position corresponding to F405 in a human IgG1 heavy chain is not F and the amino acid in the position corresponding to K409 in a human IgG1 heavy chain is K.

In one embodiment of the disclosure, in the first heavy chain, the amino acid in the position corresponding to F405 in a human IgG1 heavy chain is not F, R, and G, and in the second heavy chain the amino acids in the positions corresponding to a position selected from the group consisting of; T366, L368, K370, D399, Y407, and K409 in a human IgG1 heavy chain has been substituted.

In one embodiment of the disclosure, the amino acid in position corresponding to K409 in a human IgG1 heavy chain is another than K, L or M in the first heavy chain, and in the second heavy chain the amino acid in position corresponding to F405 in a human IgG1 heavy chain is not F and optionally the amino acid in the position corresponding to K409 in a human IgG1 heavy chain is K.

In one embodiment of the disclosure, the amino acid in the position corresponding to F405 in a human IgG1 heavy chain is L in said first heavy chain, and the amino acid in the position corresponding to K409 in a human IgG1 heavy chain is R in said second heavy chain, or vice versa.

Thus, in one embodiment of the disclosure, the amino acid in the position corresponding to K409 in a human IgG1 heavy chain is R in the first heavy chain, and the amino acid in the position corresponding to F405 in a human IgG1 heavy chain is L in the second heavy chain.

Unless otherwise stated or contradicted by context, the amino acids of the constant region sequences are herein numbered according to the Eu-index of numbering (described in Kabat, 1991). The terms "Eu-index of numbering" and "Eu numbering as set forth in Kabat" may be used interchangeably and have the same meaning and purpose. Thus, an amino acid or segment in one sequence that "corresponds to" an amino acid or segment in another sequence is one that aligns with the other amino acid or segment using a standard sequence alignment program such as ALIGN, ClustalW or similar, typically at default settings and has at least 50%, at least 80%, at least 90%, or at least 95% identity to a human IgG1 heavy

chain. It is well-known in the art how to align a sequence or segment in a sequence and thereby determine the corresponding position in a sequence to an amino acid position according to the present invention.

The term "amino acid corresponding to position" as used herein refers to an amino acid position number in a human IgG1 heavy chain.

The term "amino acid" and "amino acid residue" may herein be used interchangeably, and are not to be understood limiting.

In the context of the present invention, the amino acid may be defined by conservative or non-conservative amino acids, and may therefore be classified accordingly. Amino acid residues may also be divided into classes defined by alternative physical and functional properties. Thus, classes of amino acids may be reflected in one or both of the following lists:

Amino acid residue of conservative class:

| | |
|---|---|
| Acidic Residues: | D and E |
| Basic Residues: | K, R, and H |
| Hydrophilic Uncharged Residues: | S, T, N, and Q |
| Aliphatic Uncharged Residues: | G, A, V, L, and I |
| Non-polar Uncharged Residues: | C, M, and P |
| Aromatic Residues: | F, Y, and W |

Alternative Physical and Functional Classifications of Amino Acid Residues:

| | |
|---|---|
| Alcohol group-containing residues: | S and T |
| Aliphatic residues: | I, L, V, and M |
| Cycloalkenyl-associated residues: | F, H, W, and Y |
| Hydrophobic residues: | A, C, F, G, H, I, L, M, R, T, V, W, and Y |
| Negatively charged residues: | D and E |
| Polar residues: | C, D, E, H, K, N, Q, R, S, and T |
| Positively charged residues: | H, K, and R |
| Small residues: | A, C, D, G, N, P, S, T, and V |
| Very small residues: | A, G, and S |
| Residues involved in turn formation: | A, C, D, E, G, H, K, N, Q, R, S, P, and T |
| Flexible residues: | Q, T, K, S, G, P, D, E, and R |

In the context of the present invention, a substitution in an antibody is indicated as:
Original amino acid - position - substituted amino acid;
Referring to the well-recognized nomenclature for amino acids, the three letter code, or one letter code, is used, including the codes "Xaa" or "X" to indicate any amino acid residue. Thus, Xaa or X may typically represent any of the 20 naturally occurring amino acids. The term "naturally occurring" as used herein refers to any one of the following amino acid residues; glycine, alanine, valine, leucine, isoleucine, serine, threonine, lysine, arginine, histidine, aspartic acid, asparagine, glutamic acid, glutamine, proline, tryptophan, phenylalanine, tyrosine, methionine, and cysteine. Accordingly, the notation "K409R" or "Lys409Arg" means, that the antibody comprises a substitution of Lysine with Arginine in amino acid position 409.

Substitution of an amino acid at a given position to any other amino acid is referred to as: Original amino acid - position; or *e.g.* "K409"

For a modification where the original amino acid(s) and/or substituted amino acid(s) may comprise more than one, but not all amino acid(s), the more than one amino acid may be separated by "," or "/". For example, the substitution of Lysine with Arginine, Alanine, or Phenylalanine in position 409 is:
"Lys409Arg,Ala,Phe" or "Lys409Arg/Ala/Phe" or "K409R,A,F" or "K409R/A/F" or "K409 to R, A, or F".

Such designation may be used interchangeably in the context of the invention but have the same meaning and

purpose.

Furthermore, the term "a substitution" embraces a substitution into any one or the other nineteen natural amino acids, or into other amino acids, such as non-natural amino acids. For example, a substitution of amino acid K in position 409 includes each of the following substitutions: 409A, 409C, 409D, 409E, 409F, 409G, 409H, 409I, 409L, 409M, 409N, 409Q, 409R, 409S, 409T, 409V, 409W, 409P, and 409Y. This is, by the way, equivalent to the designation 409X, wherein the X designates any amino acid other than the original amino acid. These substitutions may also be designated K409A, K409C, etc. or K409A,C, etc. or K409A/C/etc. The same applies by analogy to each and every position mentioned herein, to specifically include herein any one of such substitutions.

The antibody according to the invention may also comprise a deletion of an amino acid residue. Such deletion may be denoted "del", and includes, *e.g.,* writing as K409del. Thus, in such embodiments, the Lysine in position 409 has been deleted from the amino acid sequence.

In one embodiment of the disclosure, both the first and the second binding region of the bispecific antibody bind AXL. However, the first binding region comprises a different set of CDR sequences than the second binding region. Thus, in a particular embodiment of the disclosure, the bispecific antibody comprising a first and a second binding region, and a first and a second heavy chain, wherein the first and the second binding regions each comprise a VH and VL region selected from the group consisting of;

a) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 46, 47, and 48, respectively; and a second VL region comprising the CDR1, CDR2, and CDR3 sequences of SEQ ID Nos.: 49, AAS, and 50, respectively, [**148**];

b) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 114, 115, and 116, respectively, and a second VL region comprising the CDR1, CDR2, and CDR3 sequences of SEQ ID Nos.: 117, DAS, and 118, respectively [**733**];

c) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 41, 42, and 43, respectively; and a second VL region comprising the CDR1, CDR2, and CDR3 sequences of SEQ ID Nos.: 44, AAS, and 45, respectively, [**140**];

d) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 51, 52, and 55, respectively; and a second VL region comprising the CDR1, CDR2, and CDR3 sequences of SEQ ID Nos.: 55, GAS, and 56, respectively. [**154**];

e) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 51, 52, and 54, respectively; and a second VL region comprising the CDR1, CDR2, and CDR3 sequences of SEQ ID Nos.: 55, GAS, and 56, respectively. [**154-M103L**];

f) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 57, 58, and 59, respectively; and a second VL region comprising the CDR1, CDR2, and CDR3 sequences of SEQ ID Nos.: 60, GAS, and 61, respectively, [**171**];

g) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 62, 63, and 64, respectively; and a second VL region comprising the CDR1, CDR2, and CDR3 sequences of SEQ ID Nos.: 65, GAS, and 66, respectively, [**172**];

h) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 67, 68, and 69, respectively; and a second VL region comprising the CDR1, CDR2, and CDR3 sequences of SEQ ID Nos.: 70, GAS, and 71, respectively, [**181**];

i) a first VH region comprising the CDR1, CDR2, and CDR3 sequences of SEQ ID Nos.: 36, 37, and 38, respectively;

and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 72, 73, and 75, respectively; and a second VL region comprising the CDR1, CDR2, and CDR3 sequences of SEQ ID Nos.: 76, ATS, and 77, respectively, [**183**];

j) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 72, 74, and 75, respectively; and a second VL region comprising the CDR1, CDR2, and CDR3 sequences of SEQ ID Nos.: 76, ATS, and 77, respectively, [**183-N52Q**];

k) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 78, 79, and 80, respectively; and a second VL region comprising the CDR1, CDR2, and CDR3 sequences of SEQ ID Nos.: 81, AAS, and 82, respectively, [**187**];

l) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 83, 84, and 85, respectively; and a second VL region comprising the CDR1, CDR2, and CDR3 sequences of SEQ ID Nos.: 86, GAS, and 87, respectively, [**608-01**];

m) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 88, 89, and 90, respectively; and a second VL region comprising the CDR1, CDR2, and CDR3 sequences of SEQ ID Nos.: 91, GAS, and 92, respectively, [**610-01**];

n) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 94, 95, and 95, respectively; and a second VL region comprising the CDR1, CDR2, and CDR3 sequences of SEQ ID Nos.: 96, GAS, and 97, respectively, [**613**];

o) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 98, 99, and 100, respectively; and a second VL region comprising the CDR1, CDR2, and CDR3 sequences of SEQ ID Nos.: 101, DAS, and 102, respectively, [**613-08**];

p) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 103, 104, and 105, respectively; and a second VL region comprising the CDR1, CDR2, and CDR3 sequences of SEQ ID Nos.: 106, GAS, and 107, respectively, [**620-06**];

q) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 108, 109, and 110, respectively; and a second VL region comprising the CDR1, CDR2, and CDR3 sequences of SEQ ID Nos.: 112, AAS, and 113, respectively, [**726**];

r) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 108, 109, and 111, respectively; and a second VL region comprising the CDR1, CDR2, and CDR3 sequences of SEQ ID Nos.: 112, AAS, and 113, respectively, [**726-M101L**];

s) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 114, 115, and 116, respectively, and a second VL region comprising the CDR1, CDR2, and CDR3 sequences of SEQ ID Nos.: 117, DAS, and 118, respectively [**733**];

t) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 41, 42, and 43, respectively; and a second VL region comprising the CDR1, CDR2, and CDR3 sequences of SEQ ID Nos.:

44, AAS, and 45, respectively, [**107**];

u) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 51, 52, and 55, respectively; and a second VL region comprising the CDR1, CDR2, and CDR3 sequences of SEQ ID Nos.: 55, GAS, and 56, respectively. [**154**];

v) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 51, 52, and 54, respectively; and a second VL region comprising the CDR1, CDR2, and CDR3 sequences of SEQ ID Nos.: 55, GAS, and 56, respectively. [**154-M103L**];

w) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 57, 58, and 59, respectively; and a second VL region comprising the CDR1, CDR2, and CDR3 sequences of SEQ ID Nos.: 60, GAS, and 61, respectively, [**171**];

x) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 62, 63, and 64, respectively; and a second VL region comprising the CDR1, CDR2, and CDR3 sequences of SEQ ID Nos.: 65, GAS, and 66, respectively, [**172**];

y) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 67, 68, and 69, respectively; and a second VL region comprising the CDR1, CDR2, and CDR3 sequences of SEQ ID Nos.: 70, GAS, and 71, respectively, [**181**];

z) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 72, 73, and 75, respectively; and a second VL region comprising the CDR1, CDR2, and CDR3 sequences of SEQ ID Nos.: 76, ATS, and 77, respectively, [**183**];

aa) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 72, 74, and 75, respectively; and a second VL region comprising the CDR1, CDR2, and CDR3 sequences of SEQ ID Nos.: 76, ATS, and 77, respectively, [**183-N52Q**];

bb) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 78, 79, and 80, respectively; and a second VL region comprising the CDR1, CDR2, and CDR3 sequences of SEQ ID Nos.: 81, AAS, and 82, respectively, [**187**];

cc) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 83, 84, and 85, respectively; and a second VL region comprising the CDR1, CDR2, and CDR3 sequences of SEQ ID Nos.: 86, GAS, and 87, respectively, [**608-01**];

dd) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 88, 89, and 90, respectively; and a second VL region comprising the CDR1, CDR2, and CDR3 sequences of SEQ ID Nos.: 91, GAS, and 92, respectively, [**610-01**];

ee) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 94, 95, and 95, respectively; and a second VL region comprising the CDR1, CDR2, and CDR3 sequences of SEQ ID Nos.: 96, GAS, and 97, respectively, [**613**];

ff) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50,

respectively, [**148**]; and a second VH region comprising the CDR1, CDR2, and CDR3 sequences of SEQ ID Nos.: 98, 99, and 100, respectively; and a second VL region comprising the CDR1, CDR2, and CDR3 sequences of SEQ ID Nos.: 101, DAS, and 102, respectively, [**613-08**];

gg) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 103, 104, and 105, respectively; and a second VL region comprising the CDR1, CDR2, and CDR3 sequences of SEQ ID Nos.: 106, GAS, and 107, respectively, [**620-06**];

hh) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 108, 109, and 110, respectively; and a second VL region comprising the CDR1, CDR2, and CDR3 sequences of SEQ ID Nos.: 112, AAS, and 113, respectively, [**726**];

ii) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 108, 109, and 111, respectively; and a second VL region comprising the CDR1, CDR2, and CDR3 sequences of SEQ ID Nos.: 112, AAS, and 113, respectively, [**726-M101L**];

jj) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 41, 42, and 43, respectively; and a second VL region comprising the CDR1, CDR2, and CDR3 sequences of SEQ ID Nos.: 44, AAS, and 45, respectively, [**140**];

kk) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 51, 52, and 55, respectively; and a second VL region comprising the CDR1, CDR2, and CDR3 sequences of SEQ ID Nos.: 55, GAS, and 56, respectively. [**154**];

ll) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 51, 52, and 54, respectively; and a second VL region comprising the CDR1, CDR2, and CDR3 sequences of SEQ ID Nos.: 55, GAS, and 56, respectively. [**154-M103L**];

mm) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 57, 58, and 59, respectively; and a second VL region comprising the CDR1, CDR2, and CDR3 sequences of SEQ ID Nos.: 60, GAS, and 61, respectively, [**171**];

nn) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 62, 63, and 64, respectively; and a second VL region comprising the CDR1, CDR2, and CDR3 sequences of SEQ ID Nos.: 65, GAS, and 66, respectively, [**172**];

oo) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 67, 68, and 69, respectively; and a second VL region comprising the CDR1, CDR2, and CDR3 sequences of SEQ ID Nos.: 70, GAS, and 71, respectively, [**181**];

pp) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 72, 73, and 75, respectively; and a second VL region comprising the CDR1, CDR2, and CDR3 sequences of SEQ ID Nos.: 76, ATS, and 77, respectively, [**183**];

qq) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 72, 74, and 75, respectively; and a second VL region comprising the CDR1, CDR2, and CDR3 sequences of SEQ ID Nos.: 76, ATS, and 77, respectively, [**183-N52Q**];

rr) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 78, 79, and 80, respectively; and a second VL region comprising the CDR1, CDR2, and CDR3 sequences of SEQ ID Nos.: 81, AAS, and 82, respectively, [**187**];

ss) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 83, 84, and 85, respectively; and a second VL region comprising the CDR1, CDR2, and CDR3 sequences of SEQ ID Nos.: 86, GAS, and 87, respectively, [**608-01**];

tt) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 88, 89, and 90, respectively; and a second VL region comprising the CDR1, CDR2, and CDR3 sequences of SEQ ID Nos.: 91, GAS, and 92, respectively, [**610-01**];

uu) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 94, 95, and 95, respectively; and a second VL region comprising the CDR1, CDR2, and CDR3 sequences of SEQ ID Nos.: 96, GAS, and 97, respectively, [**613**];

vv) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**];and a second VH region comprising the CDR1, CDR2, and CDR3 sequences of SEQ ID Nos.: 98, 99, and 100, respectively; and a second VL region comprising the CDR1, CDR2, and CDR3 sequences of SEQ ID Nos.: 101, DAS, and 102, respectively, [**613-08**];

ww) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 103, 104, and 105, respectively; and a second VL region comprising the CDR1, CDR2, and CDR3 sequences of SEQ ID Nos.: 106, GAS, and 107, respectively, [**620-06**];

xx) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 108, 109, and 110, respectively; and a second VL region comprising the CDR1, CDR2, and CDR3 sequences of SEQ ID Nos.: 112, AAS, and 113, respectively, [**726**]; and

yy) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 108, 109, and 111, respectively; and a second VL region comprising the CDR1, CDR2, and CDR3 sequences of SEQ ID Nos.: 112, AAS, and 113, respectively, [**726-M101L**];

Antibodies conjugated to a cytotoxic agent, drug or the like are also known as antibody-drug conjugates (ADC). An ADC may have a half-life of sufficient periods of time for the antibody-drug conjugate to be internalized, degraded and induce cell killing by the released toxin.

Thus, an ADC can comprise an anti-AXL antibody or bispecific antibody and a therapeutic moiety, such as a cytotoxic agent, a chemotherapeutic drug, or the like. The cytotoxic agent, chemotherapeutic drug or the like may be conjugated to the antibody or the bispecific antibody via a linker.

ADCs are often designed such that the cytotoxic payload is inactive when conjugated to the antibody. The cytotoxic payload may be released intracellularly upon internalization of the ADC after binding to the plasma-membrane of cells, or alternatively in response to proteolytic activity in the tumor microenvironment. The term "internalized" or "internalization" as used herein, refers to a biological process in which molecules such as the AXL-ADC are engulfed by the cell membrane and drawn into the interior of the cell. It may also be referred to as "endocytosis".

Accordingly, in some instances it may be desired to use antibodies which undergo internalization. Such antibodies that have good internalization properties may be suited for conjugation to a cytotoxic agent, drug, or the like, optionally via a linker, which is designed to be cleaved intracellularly.

Once internalized, the ADC may be delivered to lysosomes in most cases, where effective drug release takes advantage of the catabolic environment found with these organelles. It is typically a linker that connects the antibody with a cytotoxic agent. Thus, specialized linkers have been designed to be cleaved only in a specific microenvironment found in

or on the target tumor cell or in the tumor microenvironment. Examples include linkers that are cleaved by acidic conditions, reducing conditions, or specific proteases.

Stability of the antibody-linker-drug in circulation is important because this allows antibody-mediated delivery of the drug to specific target cells. In addition, the long circulating half-life of the ADC provides exposure for several days to weeks post injection. Drugs that are conjugated through non-cleavable linkers and protease-cleavable linkers are generally more stable in circulation than disulfide and hydrazone linkers, although the stability of the latter two linkers can be tuned by altering the neighboring chemical structure (Alley *et al.,* 2010).

In one embodiment, the therapeutic moiety is a cytotoxic agent. A cytotoxin or cytotoxic agent includes any agent that is detrimental to (*e.g.,* kills) cells. Suitable cytotoxic agents for forming ADCs for use in the present invention include taxol, tubulysins, duostatins, cytochalasin B, gramicidin D, ethidium bromide, emetine, mitomycin, etoposide, tenoposide, vincristine, vinblastine, colchicin, doxorubicin, daunorubicin, dihydroxy anthracin dione, maytansine or an analog or derivative thereof, mitoxantrone, mithramycin, actinomycin D, 1-dehydrotestosterone, glucocorticoids, procaine, tetracaine, lidocaine, propranolol, and puromycin; calicheamicin or analogs or derivatives thereof; antimetabolites (such as methotrexate, 6-mercaptopurine, 6-thioguanine, cytarabine, fludarabin, 5-fluorouracil, decarbazine, hydroxyurea, asparaginase, gemcitabine, cladribine), alkylating agents (such as mechlorethamine, thioepa, chlorambucil, melphalan, carmustine (BSNU), lomustine (CCNU), cyclophosphamide, busulfan, dibromomannitol, streptozotocin, dacarbazine (DTIC), procarbazine, mitomycin C, cisplatin and other platinum derivatives, such as carboplatin; as well as duocarmycin A, duocarmycin SA, CC-1065 (a.k.a. rachelmycin), or analogs or derivatives of CC-1065), dolastatin, auristatin, pyrrolo [2,1-c][1,4] benzodiazepins (PDBs), indolinobenzodiazepine (IGNs) or analogues thereof, antibiotics (such as dactinomycin (formerly actinomycin), bleomycin, daunorubicin (formerly daunomycin), doxorubicin, idarubicin, mithramycin, mitomycin, mitoxantrone, plicamycin, anthramycin (AMC)), anti-mitotic agents (*e.g.,* tubulin-targeting agents), such as diphtheria toxin and related molecules (such as diphtheria A chain and active fragments thereof and hybrid molecules); ricin toxin (such as ricin A or a deglycosylated ricin A chain toxin), cholera toxin, a Shiga-like toxin (SLT-I, SLT-II, SLT-IIV), LT toxin, C3 toxin, Shiga toxin, pertussis toxin, tetanus toxin, soybean Bowman-Birk protease inhibitor, Pseudomonas exotoxin, alorin, saporin, modeccin, gelanin, abrin A chain, modeccin A chain, alpha-sarcin, Aleurites fordii proteins, dianthin proteins, Phytolacca americana proteins (PAPI, PAPII, and PAP-S), momordica charantia inhibitor, curcin, crotin, sapaonaria officinalis inhibitor, gelonin, mitogellin, restrictocin, phenomycin, and enomycin toxins. Other suitable conjugated molecules include antimicrobial/lytic peptides such as CLIP, Magainin 2, mellitin, Cecropin, and P18; ribonuclease (RNase), DNase I, Staphylococcal enterotoxin-A, pokeweed antiviral protein, diphtherin toxin, and Pseudomonas endotoxin. See, for example, Pastan et al., Cell 47, 641 (1986) and Goldenberg, Calif. A Cancer Journal for Clinicians 44, 43 (1994). Therapeutic agents that may be administered in combination with anti-AXL antibodies or antibody-drug conjugates for use according to the present invention as described elsewhere herein, such as, *e.g.,* anti-cancer cytokines or chemokines, are also candidates for therapeutic moieties useful for conjugation to an antibody for use according to the present invention.

The term "cytotoxic agent" as used herein, refers to any agent that is detrimental to (*e.g.,* kills) cells. For a description of these classes of drugs which are well known in the art, and their mechanisms of action, see Goodman et al. (1990). Additional techniques relevant to the preparation of antibody immunotoxins are provided in for instance Vitetta *et al.* (1993) and US 5,194,594.

In one embodiment of the disclosure, the cytotoxic agent is linked to said antibody, or fragment thereof, with a cleavable linker, such as *N*-succinimydyl 4-(2-pyridyldithio)-pentanoate (SSP), maleimidocaproyl-valine-citrulline-*p*-aminobenzyloxycarbonyl (mc-vc-PAB) or AV-1 K-lock valine-citrulline.

The term "cleavable linker" as used herein, refers to a subset of linkers that are catalyzed by specific proteases in the targeted cell or in the tumor microenvironment, resulting in release of the cytotoxic agent. Examples of cleavable linkers are linkers based on chemical motifs including disulfides, hydrazones or peptides. Another subset of cleavable linker, adds an extra linker motif between the cytotoxic agent and the primary linker, *i.e.* the site that attaches the linker-drug combination to the antibody. In some embodiments of the disclosure, the extra linker motif is cleavable by a cleavable agent that is present in the intracellular environment (e. g. within a lysosome or endosome or caveola). The linker can be, e. g. a peptidyl linker that is cleaved by an intracellular peptidase or protease enzyme, including but not limited to, a lysosomal or endosomal protease. In some embodiments of the disclosure, the peptidyl linker is at least two amino acids long or at least three amino acids long. Cleaving agents can include cathepsins B and D and plasmin, all of which are known to hydrolyze dipeptide drug derivatives resulting in the release of active drug inside the target cells (see e. g. Dubowchik and Walker, 1999, Pharm. Therapeutics 83:67-123). In a specific embodiment, the peptidyl linker cleavable by an intracellular protease is a Val-Cit (valine-citrulline) linker or a Phe-Lys (phenylalanine-lysine) linker (see *e.g.* US6214345, which describes the synthesis of doxorubicin with the Val-Cit linker). An advantage of using intracellular proteolytic release of the therapeutic agent is that the agent is typically attenuated when conjugated and the serum stabilities of the conjugates are typically high.

In another embodiment of the disclosure, the cytotoxic agent is linked to said antibody, or fragment thereof, with a non-cleavable linker, such as succinimidyl-4(*N*-maleimidomethyl)cyclohexane-1-carboxylate (MCC) or maleimidocaproyl (MC).

The term "noncleavable linker" as used herein, refers to a subset of linkers which, in contrast to cleavable linkers, do not comprise motifs that are specifically and predictably recognized by intracellular or extracellular proteases. Thus, ADCs based on non-cleavable linkers are not released or cleaved form the antibody until the complete antibody-linker-drug complex is degraded in the lysosomal compartment. Examples of a non-cleavable linker are thioethers. In yet another embodiment, the linker unit is not cleavable and the drug is released by antibody degradation (see US 2005/0238649). Typically, such a linker is not substantially sensitive to the extracellular environment. As used herein, "not substantially sensitive to the extracellular environment" in the context of a linker means that no more than 20%, typically no more than about 15%, more typically no more than about 10%, and even more typically no more than about 5%, no more than about 3%, or no more than about 1% of the linkers, in a sample of antibody drug conjugate compound, are cleaved when the antibody drug conjugate compound is present in an extracellular environment (*e.g.* plasma). Whether a linker is not substantially sensitive to the extracellular environment can be determined for example by incubating with plasma the antibody drug conjugate compound for a predetermined time period (*e.g.* 2, 4, 8, 16 or 24 hours) and then quantitating the amount of free drug present in the plasma.

In one embodiment of the disclosure, cytotoxic agent is selected from the group: DNA-targeting agents, *e.g.* DNA alkylators and cross-linkers, such as calicheamicin, duocarmycin, rachelmycin (CC-1065), pyrrolo[2,1-c][1,4] benzodiazepines (PBDs), and indolinobenzodiazepine (IGN); microtubule-targeting agents, such as duostatin, such as duostatin-3, auristatin, such as monomethylauristatin E (MMAE) and monomethylauristatin F (MMAF), dolastatin, maytansine, *N*(2')-deacetyl-*N*(2')-(3-marcapto-1-oxopropyl)-maytansine (DM1), and tubulysin; and nucleoside analogs; or an analogs, derivatives, or prodrugs thereof.

In one embodiment, the AXL-ADC comprises a combination of;

i) a cleavable linker and a cytotoxic agent having bystander kill capacity;
ii) a cleavable linker and a cytotoxic agent not having bystander kill capacity;
iii) a non-cleavable linker and a cytotoxic agent having bystander kill capacity; or
iv) a non-cleavable linker and a cytotoxic agent not having bystander kill capacity.

The term "bystander killing effect", "bystander kill", "bystander kill capacity" or "bystander cytotoxicity" as used herein, refers to the effect where the cytotoxic agent that is conjugated to the antibody by either a cleavable or non-cleavable linker has the capacity to diffuse across cell membranes after the release from the antibody and thereby cause killing of neighboring cells. When the cytotoxic agent is conjugated by a cleavable or non-cleavable linker, it may be either the cytotoxic agent only or the cytotoxic agent with a part of the linker that has the bystander kill capacity. The capacity to diffuse across cell membranes is related to the hydrophobicity of the the cytotoxic agent or the combination of the cytotoxic agent and the linker. Such cytotoxic agents may advantageously be membrane-permeable toxins, such as MMAE that has been released from the antibody by proteases. Especially in tumors with heterogeneous target expression and in solid tumors where antibody penetration may be limited, a bystander killing effect may be desirable.

The term "no bystander kill capacity", "no bystander killing effect", "no-bystander kill" or "no bystander cytotoxicity" as used herein, refers to the effect where the cytotoxic agent that is conjugated to the antibody by either a cleavable or non-cleavable linker does not have the capacity to diffuse across cell membranes after release from the antibody. Thus, such cytotoxic agents or combinations of the cytotoxic agent with the linker, will not be able to kill neighboring cells upon release from the antibody. It is believed without being bound by theory, that such combinations of a cytotoxic agent and either a cleavable or non-cleavable linker will only kill cells expressing the target that the antibody binds.

A stable link between the antibody and cytotoxic agent is an important factor of an ADC. Both cleavable and non-cleavable types of linkers have been proven to be safe in preclinical and clinical trials.

In one embodiment of the disclosure, the cytotoxic agent is chosen from the group of microtubule targeting agents, such as auristatins and maytansinoids.

The term "microtubule-targeting agent" as used herein, refers to an agent or drug which inhibits mitosis (cell division). Microtubules are structures that are essential for proper separation of DNA during cell division, and microtubule function critically depends on 'dynamic instability', *i.e.* the process in which microtubule structures are continuously elongated and shortened. Microtubule-targeting agents disrupt or stabilize microtubules, which prevents formation of the mitotic spindle, resulting in mitotic arrest and apoptosis. The microtubule-targeting agents can be derived from *e.g.* natural substances such as plant alkaloids, and prevent cells from undergoing mitosis by disrupting or stabilizing microtubule polymerization, thus preventing formation of the mitotic spindle and subsequent cell division, resulting in inhibition of cancerous growth. Examples of microtubule-targeting agents are paclitaxel, docetaxel, vinblastine, vincristine, vinorelbine, duostatins, auristatins, maytansanoids, tubulysins, and dolastatin.

In one embodiment of the disclosure, the cytotoxic agent is auristatins or auristatin peptide analogs and derivates (US 5,635,483;US 5,780,588). Auristatins have been shown to interfere with microtubule dynamics, GTP hydrolysis and

nuclear and cellular division (Woyke *et al.,* 2001) and have anti-cancer (US 5,663,149) and anti-fungal activity (Pettit, 1998). The auristatin drug moiety may be attached to the antibody via a linker, through the N (amino) terminus or the C (terminus) of the peptidic drug moiety.

Exemplary auristatin embodiments include the N-terminus-linked monomethyl auristatin drug moieties $D_E$ and $D_F$, disclosed in Senter *et al.* (2004) and described in US 2005/0238649.

In a particular embodiment of the disclosure, the cytotoxic agent is monomethyl auristatin E (MMAE);

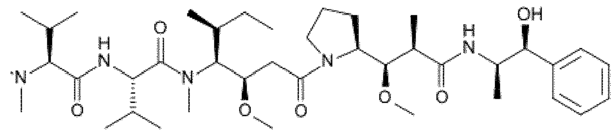

wherein the antibody is linked to MMAE at the nitrogen (N) on the left-hand side of the chemical structure above by the appropriate linker.

In one embodiment of the disclosure, the cytotoxic agent monomethyl auristatin E (MMAE) is linked to the antibody via a valine-citrulline (VC) linker.

In another embodiment of the disclosure, the cytotoxic agent monomethyl auristatin E (MMAE) is linked to the antibody via a valine-citrulline (VC) linker and the maleimidocaproyl (MC)linker, wherein the combination of the cytotoxic agent and the linkers has the chemical structure;

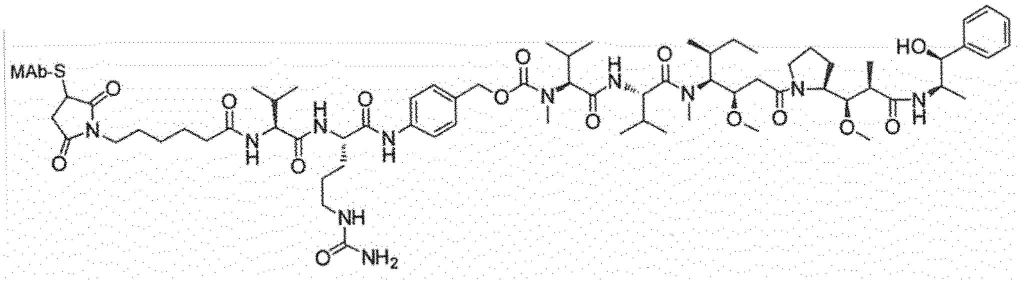

wherein MAb is the antibody.

In one embodiment of the disclosure, the cytotoxic agent is monomethyl auristatin F (MMAF);

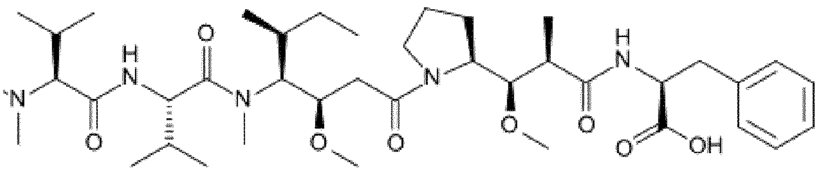

wherein the antibody is linked to MMAF at the nitrogen (N) on the left-hand side of the chemical structure above by the appropriate linker.

In one embodiment of the disclosure, the cytotoxic agent monomethyl auristatin F (MMAF) is linked to the antibody via a maleimidocaproyl (mc)-linker, wherein the combination of the cytotoxic agent and linker has the chemical structure;

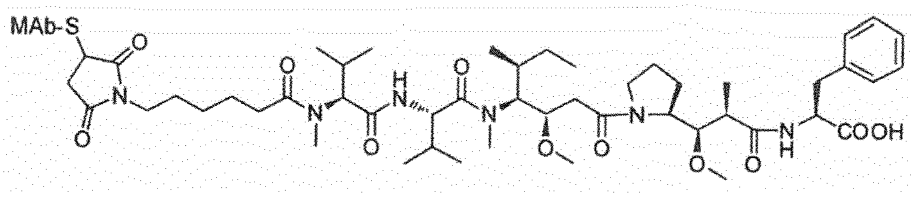

wherein MAb is the antibody.

In one embodiment of the disclosure, the cytotoxic agent is duostatin3.

In another particular embodiment of the disclosure, the cytotoxic agent is a DNA-targeting agent.

The term "DNA-targeting agent" as used herein, refers to a specific class of cytotoxic agents which are able to alkylate and/or cross-link DNA. An example of such a DNA-acting agent is IGN agents comprising indolino-benzodiazepinedimers and pyrrolo[2,1-c][1,4]benzodiazepines (PBDs) which are highly potent by virtue of their ability to alkylate and cross-link DNA. Another example is IGN agents comprising indolino-benzodiazepinemonomers which are highly potent by virtue of the ability to alkylate only DNA. Duocarmycins are another class of DNA-acting agents. Duocarmycins are small-molecule, synthetic DNA minor groove binding alkylating agents. These compounds are suitable to target solid tumors as well as hematological tumors.

In one embodiment of the disclosure, the AXL-ADC comprises two to four cytotoxic molecules per antibody. Depending on the chemical properties of the toxin and the linker-toxin combination, two to four cytotoxic molecules per antibody may be superior to more heavily loaded conjugates that are cleared more rapidly from the circulation than less loaded conjugates. The cytotoxic agent loading is represented by p and is the average number of cytotoxic agent moieties per antibody in a molecule (also designated as the drug to antibody ratio, DAR). The cytotoxic agent loading may range from 1 to 20 drug moieties per antibody and may occur on amino acids with useful functional groups such as, but not limited to, amino or sulfhydryl groups, as in lysine or cysteine.

In one embodiment of the disclosure, the number of cytotoxic agents per antibody is from 1 to 8, such as 2 to 7, such as 2 to 6, such as 2 to 5, such as 2 to 4, and such as 2 to 3.

In another embodiment of the disclosure, the AXL-ADC comprises four to eight cytotoxic molecules per antibody. In another embodiment of the disclosure, the AXL-ADC comprises six to ten cytotoxic molecules per antibody. In yet another embodiment of the disclosure, the AXL-ADC comprises 10 to 30, such as 15 to 25, such as 20, cytotoxic molecules per antibody.

Depending on the way of conjugation, p may be limited by the number of attachment sites on the antibody, for example where the attachment is a cysteine thiol or a lysine. Generally, antibodies do not contain many free and reactive cysteine thiol groups which may be linked to a drug moiety as most cysteine thiol residues in antibodies exist as disulfide bridges. Therefore, in those embodiments of the disclosure, where the cytotoxic agent is conjugated via a cysteine thiol, the antibody may be reduced with reducing agent such as dithiothreitol (DTT) or tricarbonylethylphosphine (TCEP), under partial or fully reducing conditions, to generate reactive cysteine thiol groups. In certain embodiments of the disclosure, the drug loading for an ADC of the invention ranges from 1 to about 8, as a maximum of 8 free cysteine thiol groups becomes available after (partial) reduction of the antibody (there are 8 cysteines involved in inter-chain disulfide bonding).

In one embodiment of the disclosure, the drug linker moiety is vcMMAE. The vcMMAE drug linker moiety and conjugation methods are disclosed in WO 2004/010957; US 7,659,241; US 7,829,531; and US 7,851,437 (Seattle Genetics). vcMMAE is formed by conjugation of the linker mc-vc-PAB and the cytotoxic moiety MMAE, and the vcMMAE drug linker moiety is bound to the anti-AXL antibodies at the cysteine residues using a method similar to those disclosed therein, *e.g.,* as described in Example 8.

In one embodiment of the disclosure, the drug linker moiety is mcMMAF. The mcMMAF drug linker moiety and conjugation methods are disclosed in US 7,498,298; US 7,994,135 and WO 2005/081711 (Seattle Genetics), and the mcMMAF drug linker moiety is bound to the anti-AXL antibodies at the cysteine residues using a method similar to those disclosed therein.

In one embodiment of the disclosure, the cytotoxic agent is linked to 1 or 2 lysines within the antibody amino acid sequence by K-Lock™ conjugation as described in WO 2013/173391, WO 2013/173392 and WO 2013/173393 (Concortis Biosystems). Duostatin3 (also known as Duo3) may also be bound to the anti-AXL antibodies at the lysine residues using a method similar to those described therein.

Other linker technologies may be used in the anti-AXL antibody drug conjugates for the use of the invention, such as linkers comprising a hydroxyl group.

In one embodiment of the disclosure, the linker is attached to free cysteine residues of the anti-AXL antibody obtained by (partial) reduction of the anti-AXL antibody.

In a particular embodiment, the linker is mc-vc-PAB and the cytotoxic agent is MMAE; In one embodiment of the disclosure the linker SSP and the cytotoxic agent is DM1.

In a particular embodiment of the disclosure, the linker is MMC and the cytotoxic agent is DM1; or the linker is MC and the cytotoxic agent is MMAF.

In a particular embodiment of the disclosure, the linker is the cleavable linker AV1-K lock and the cytotoxic agent is duostatin3.

In one embodiment of the disclosure the AXL-ADC comprises the linker mc-vc-PAB, the cytotoxic agent MMAE and an antibody wherein the at least one binding region comprises a VH region and a VL region selected from the group consisting of;

In one embodiment, the antibody comprises at least one binding region comprising a VH region and a VL region selected from the group consisting of:

(a) a VH region comprising the CDR1, CDR2, and CDR3 sequences of SEQ ID Nos.:36, 37, and 38, respectively; and a VL region comprising the CDR1, CDR2, and CDR3 sequences of SEQ ID Nos.:39, GAS, and 40, respectively, [**107**];
(b) a VH region comprising the CDR1, CDR2, and CDR3 sequences of SEQ ID Nos.:46, 47, and 48, respectively; and

a VL region comprising the CDR1, CDR2, and CDR3 sequences of SEQ ID Nos.:49, AAS, and 50, respectively, [**148**];
(c) a VH region comprising the CDR1, CDR2, and CDR3 sequences of SEQ ID Nos.:114, 115, and 116, respectively, and a VL region comprising the CDR1, CDR2, and CDR3 sequences of SEQ ID Nos.:117, DAS, and 118, respectively [**733**];
(d) a VH region comprising the CDR1, CDR2, and CDR3 sequences of SEQ ID Nos.:51, 52, and 53, respectively; and a VL region comprising the CDR1, CDR2, and CDR3 sequences of SEQ ID Nos.:55, GAS, and 56, respectively [**154**];
(e) a VH region comprising the CDR1, CDR2, and CDR3 sequences of SEQ ID Nos.:51, 52, and 54, respectively; and a VL region comprising the CDR1, CDR2, and CDR3 sequences of SEQ ID Nos.:55, GAS, and 56, respectively [**154-M103L**];
(f) a VH region comprising the CDR1, CDR2, and CDR3 sequences of SEQ ID Nos.:57, 58, and 59, respectively; and a VL region comprising the CDR1, CDR2, and CDR3 sequences of SEQ ID Nos.:60, GAS, and 61, respectively, [**171**];
(g) a VH region comprising the CDR1, CDR2, and CDR3 sequences of SEQ ID Nos.:62, 63, and 64, respectively; and a VL region comprising the CDR1, CDR2, and CDR3 sequences of SEQ ID Nos.:65, GAS, and 66, respectively, [**172**];
(h) a VH region comprising the CDR1, CDR2, and CDR3 sequences of SEQ ID Nos.:67, 68, and 69, respectively; and a VL region comprising the CDR1, CDR2, and CDR3 sequences of SEQ ID Nos.:70, GAS, and 71, respectively, [**181**];
(i) a VH region comprising the CDR1, CDR2, and CDR3 sequences of SEQ ID Nos.:72, 73, and 75, respectively; and a VL region comprising the CDR1, CDR2, and CDR3 sequences of SEQ ID Nos.:76, ATS, and 77, respectively, [**183**];
(j) a VH region comprising the CDR1, CDR2, and CDR3 sequences of SEQ ID Nos.:72, 74, and 75, respectively; and a VL region comprising the CDR1, CDR2, and CDR3 sequences of SEQ ID Nos.:76, ATS, and 77, respectively, [**183-N52Q**];
(k) a VH region comprising the CDR1, CDR2, and CDR3 sequences of SEQ ID Nos.:78, 79, and 80, respectively; and a VL region comprising the CDR1, CDR2, and CDR3 sequences of SEQ ID Nos.:81, AAS, and 82, respectively, [**187**];
(l) a VH region comprising the CDR1, CDR2, and CDR3 sequences of SEQ ID Nos.:83, 84, and 85, respectively; and a VL region comprising the CDR1, CDR2, and CDR3 sequences of SEQ ID Nos.:86, GAS, and 87, respectively, [**608-01**];
(m)a VH region comprising the CDR1, CDR2, and CDR3 sequences of SEQ ID Nos.:88, 89, and 90, respectively; and a VL region comprising the CDR1, CDR2, and CDR3 sequences of SEQ ID Nos.:91, GAS, and 92, respectively, [**610-01**];
(n) a VH region comprising the CDR1, CDR2, and CDR3 sequences of SEQ ID Nos.:93, 94, and 95, respectively; and a VL region comprising the CDR1, CDR2, and CDR3 sequences of SEQ ID Nos.:96, GAS, and 97, respectively, [**613**];
(o) a VH region comprising the CDR1, CDR2, and CDR3 sequences of SEQ ID Nos.:98, 99, and 100, respectively; and a VL region comprising the CDR1, CDR2, and CDR3 sequences of SEQ ID Nos.:101, DAS, and 102, respectively, [**613-08**];
(p) a VH region comprising the CDR1, CDR2, and CDR3 sequences of SEQ ID Nos.:103, 104, and 105, respectively; and a VL region comprising the CDR1, CDR2, and CDR3 sequences of SEQ ID Nos.:106, GAS, and 107, respectively, [**620-06**];
(q) a VH region comprising the CDR1, CDR2, and CDR3 sequences of SEQ ID Nos.:108, 109, and 110, respectively; and a VL region comprising the CDR1, CDR2, and CDR3 sequences of SEQ ID Nos.:112, AAS, and 113, respectively, [**726**];
(r) a VH region comprising the CDR1, CDR2, and CDR3 sequences of SEQ ID Nos.:108, 109, and 111, respectively; and a VL region comprising the CDR1, CDR2, and CDR3 sequences of SEQ ID Nos.:112, AAS, and 113, respectively, [**726-M101L**];
(s) a VH region comprising the CDR1, CDR2, and CDR3 sequences of SEQ ID Nos.:41, 42, and 43, respectively; and a VL region comprising the CDR1, CDR2, and CDR3 sequences of SEQ ID Nos.:44, AAS, and 45, respectively, [**140**];
(t) a VH region comprising the CDR1, CDR2, and CDR3 sequences of SEQ ID Nos.:93, 94, and 95, respectively, and a VL region comprising the CDR1, CDR2, and CDR3 sequences of SEQ ID Nos.:128, XAS, wherein X is D or G, and 129, respectively, [**613** / **613-08**];
(u) a VH region comprising the CDR1, CDR2, and CDR3 sequences of SEQ ID Nos.:46, 119, and 120, respectively; and a VL region comprising CDR1, CDR2, and CDR3 sequences of SEQ ID Nos.:49, AAS, and 50, respectively, [**148/140**];
(v) a VH region comprising the CDR1, CDR2, and CDR3 sequences of SEQ ID Nos.:123, 124, and 125, respectively; and a VL region comprising CDR1, CDR2, and CDR3 sequences of SEQ ID Nos.:60, GAS, and 61, respectively [**171** / **172** / **181**]; and
(w) a VH region comprising the CDR1, CDR2, and CDR3 sequences of SEQ ID Nos.:121, 109, and 122, respectively; and a VL region comprising the CDR1, CDR2, and CDR3 sequences of SEQ ID Nos.:112, AAS, and 113, respectively [**726** / **187**]; and
(x) a VH region comprising the CDR1, CDR2, and CDR3 sequences of SEQ ID Nos.:93, 126, and 127, respectively; and a VL region comprising the CDR1, CDR2, and CDR3 sequences of SEQ ID Nos.:96, GAS, and 97, respectively

[**613 / 608-01 / 610-01 / 620-06**].

In another alternative embodiment of the disclosure, an anti-AXL antibody drug conjugate comprises a conjugated nucleic acid or nucleic acid-associated molecule. In one such embodiment, the conjugated nucleic acid is a cytotoxic ribonuclease, an antisense nucleic acid, an inhibitory RNA molecule (*e.g.,* a siRNA molecule) or an immunostimulatory nucleic acid (*e.g.,* an immunostimulatory CpG motif-containing DNA molecule).

In another alternative embodiment of the disclosure, an anti-AXL antibody is conjugated to an aptamer or a ribozyme or a functional peptide analog or derivate thereof.

In another alternative embodiment of the disclosure, anti-AXL antibody drug conjugates comprising one or more radiolabeled amino acids are provided. A radiolabeled anti-AXL antibody may be used for both diagnostic and therapeutic purposes (conjugation to radiolabeled molecules is another possible feature). Non-limiting examples of labels for polypeptides include $^{3}H$, $^{14}C$, $^{15}N$, $^{35}S$, $^{90}Y$, $^{99}Tc$, and $^{125}I$, $^{131}I$, and $^{186}Re$. Methods for preparing radiolabeled amino acids and related peptide derivatives are known in the art (see for instance Junghans *et al.* (1996); US 4,681,581; US 4,735,210; US 5,101,827; US 5,102,990; US 5,648,471; and US 5,697,902. For example, a halogen radioisotope may be conjugated by a chloramine T method.

In one embodiment of the disclosure, the antibody is conjugated to a radioisotope or to a radioisotope-containing chelate. For example, the antibody can be conjugated to a chelator linker, *e.g.* DOTA, DTPA or tiuxetan, which allows for the antibody to be complexed with a radioisotope. The antibody may also or alternatively comprise or be conjugated to one or more radiolabeled amino acids or other radiolabeled molecules. A radiolabeled anti-AXL antibody may be used for both diagnostic and therapeutic purposes. Non-limiting examples of radioisotopes include $^{3}H$, $^{14}C$, $^{15}N$, $^{35}S$, $^{90}Y$, $^{99}Tc$, $^{125}I$, $^{111}In$, $^{131}I$, $^{186}Re$, $^{213}Bs$, $^{225}Ac$ and $^{227}Th$.

Anti-AXL antibodies may also be chemically modified by covalent conjugation to a polymer to for instance increase their circulating half-life. Exemplary polymers, and methods to attach them to peptides, are illustrated in for instance US 4,766,106; US 4,179,337; US 4,495,285 and US 4,609,546. Additional polymers include polyoxyethylated polyols and polyethylene glycol (PEG) (*e.g.,* a PEG with a molecular weight of between about 1,000 and about 40,000, such as between about 2,000 and about 20,000). This may for example be used if the anti-AXL antibody is a fragment.

Any method known in the art for conjugating the anti-AXL antibody to the conjugated molecule(s), such as those described above, may be employed, including the methods described by Hunter *et al.* (1974), Pain *et al.* (1981) and Nygren (1982). Such antibodies may be produced by chemically conjugating the other moiety to the N-terminal side or C-terminal side of the anti-AXL antibody (*e.g.,* an anti-AXL antibody H or L chain) (see, *e.g.,* Kanemitsu, 1994). Such conjugated antibody derivatives may also be generated by conjugation at internal residues or sugars, or non-naturally occurring amino acids or additional amino acids that have been introduced into the antibody constant domain, where appropriate.

The agents may be coupled either directly or indirectly to an anti-AXL antibody. One example of indirect coupling of a second agent is coupling via a spacer moiety to cysteine or lysine residues in the antibody. In one embodiment of the disclosure, an anti-AXL antibody is conjugated, via a spacer or linker, to a prodrug molecule that can be activated *in vivo* to a therapeutic drug. After administration, the spacers or linkers are cleaved by tumor cell-associated enzymes or other tumor-specific conditions, by which the active drug is formed. Examples of such pro-drug technologies and linkers are described in WO 2002/083180, WO 2004/043493, WO 2007/018431, WO 2007/089149, WO 2009/017394 and WO 2010/62171 (Syngenta BV). Suitable antibody-pro-drug technology and duocarmycin analogs can also be found in US 6,989,452 (Medarex).

In one embodiment of the disclosure, the anti-AXL antibody is attached to a chelator linker, *e.g.* tiuxetan, which allows for the antibody to be conjugated to a radioisotope.

In one aspect, the present disclosure relates to an ADC comprising an antibody binding to human AXL, for use in treating melanoma in a subject in combination with a BRAF inhibitor, a MEK-inhibitor or a combination of a BRAF inhibitor and a MEK inhibitor, wherein the ADC comprises an antibody comprising at least one binding region comprising a VH region comprising the CDR1, CDR2, and CDR3 sequences of SEQ ID Nos.: 36, 37, and 38, respectively; and a VL region comprising the CDR1, CDR2, and CDR3 sequences of SEQ ID Nos.: 39, GAS, and 40, respectively, [**107**], linked to MMAE via an mc-vc-PAB linker, and the AXL-ADC and the at least one inhibitor are administered simultaneously, separately or sequentially.

In one embodiment of the disclosure, the at least one binding region comprises a VH region comprising SEQ ID NO:1 and a VL region comprising SEQ ID NO:2. Optionally, the isotype of the antibody is IgG1, *e.g.,* allotype IgG1m(f). The anitbody may be a full-length monoclonal antibody, such as a a full-length monoclonal IgG1,$\kappa$ antibody.

In one embodiment of the disclosure, the BRAF inhibitor is selected from the group consisting of vemurafenib, dabrafenib, encorafenib and sorafenib, and the melanoma exhibits a mutation in a BRAF residue selected from V600, L597 and K601, such as a mutation in BRAF selected from V600E, V600K, V600D, L597R and K601E. In one embodiment of the disclosure, the BRAF inhibitor is vemurafenib. In one embodiment of the disclosure, the BRAF inhibitor is dabrafenib. In one embodiment, the BRAF inhibitor is encorafenib. In one embodiment of the disclosure, the BRAF inhibitor is sorafenib.

In one embodiment of the disclosure, the melanoma exhibits a mutation in NRAS, such as in an NRAS residue selected from Q61, G12 and G13, such as a mutation in NRAS selected from Q61R, Q61K, Q61L, G12D, G12S, G12C, G12V, G13D and G13R.

In one embodiment of the disclosure, the MEK inhibitor is selected from the group consisting of trametinib, cobimetinib, binimetinib and selumetinib. In one embodiment, the MEK inhibitor is trametinib. In one embodiment of the disclosure, the MEK inhibitor is cobimetinib. In one embodiment of the disclosure, the MEK inhibitor is binimetinib. In one embodiment of the disclosure, the MEK inhibitor is selumetinib.

In one embodiment, AXL-ADC is used in combination with a BRAF inhibitor and a MEK inhibitor selected from (a) to (p):

(a) vemurafenib and trametinib;
(b) vemurafenib and cobimetinib;
(c) vemurafenib and binimetinib;
(d) vemurafenib and selumetinib;
(e) dabrafenib and trametinib;
(f) dabrafenib and cobimetinib;
(g) dabrafenib and binimetinib;
(h) dabrafenib and selumetinib;
(i) encorafenib and trametinib;
(j) encorafenib and cobimetinib;
(k) encorafenib and binimetinib;
(l) encorafenib and selumetinib;
(m) sorafenib and trametinib
(n) sorafenib and cobimetinib;
(o) sorafenib and binimetinib; and
(p) sorafenib and selumetinib.

*Compositions and kits*

The AXL-ADC for use according to the present invention can be administered in the form of a composition. In one aspect, the composition is a pharmaceutical composition comprising the AXL-ADC and a pharmaceutical carrier.

In one embodiment of the disclosure, the AXL-ADC or pharmaceutical composition comprising the AXL-ADC is for use in treating a melanoma in combination with the at least one MAPK pathway inhibitor, *e.g.,* at least one serine/threonine kinase inhibitor, according to any preceding aspect or embodiment. Typically, the AXL-ADC and the inhibitor of the combination are separately administered and formulated as separate pharmaceutical compositions.

In one embodiment of the disclosure, however, the pharmaceutical composition comprising the AXL-ADC further comprises the at least one serine/threonine kinase inhibitor with which the neoplasm is being or has been treated, *e.g.,* a BRAF inhibitor, MEK inhibitor or combination thereof. The AXL-ADCs for use according to the present invention in combination with the at least one serine/threonine kinase inhibitor can be also be provided in the form of a kit, for simultaneous, separate or sequential administration, wherein the kit may further comprise instructions for use.

In one embodiment of the disclosure, the serine/threonine kinase inhibitor in the combination, composition or kit is selected from vemurafenib, dabrafenib, encorafenib, sorafenib, PLX4720, trametinib, cobimetinib, binimetinib, selumetinib, VTX11E and LTT-4620.

In one embodiment of the disclosure, the BRAF inhibitor in the combination, composition or kit is vemurafenib or a therapeutically effective analog or derivative thereof, such as dabrafenib, encorafenib, sorafenib or PLX4720. In one embodiment of the disclosure, the BRAF inhibitor is vemurafenib. In one embodiment of the disclosure, the BRAF-inhibitor is dabrafenib. In one embodiment, the BRAF inhibitor is encorafenib. In one embodiment of the disclosure, the BRAF-inhibitor is sorafenib.

In one embodiment of the disclosure, the serine/threonine kinase inhibitor in the combination, composition or kit comprises at least one BRAF-inhibitor and at least one MEK-inhibitor, wherein the at least one BRAF-inhibitor is selected from vemurafenib, dabrafenib and a combination thereof, and wherein the MEK-inhibitor is selected from selumetinib (AZD6244) and trametinib, and a combination thereof. For example, the combination, composition or kit may comprise dabrafenib and trametinib; vemurafenib and trametinib; dabrafenib, vemurafenib and trametinib; dabrafenib and selumetinib; or vemurafenib and selumetinib. Alternatively, the combination, composition or kit may comprise

(a) vemurafenib and trametinib;
(b) vemurafenib and cobimetinib;
(c) vemurafenib and binimetinib;

(d) vemurafenib and selumetinib;
(e) dabrafenib and trametinib;
(f) dabrafenib and cobimetinib;
(g) dabrafenib and binimetinib;
(h) dabrafenib and selumetinib;
(i) encorafenib and trametinib;
(j) encorafenib and cobimetinib;
(k) encorafenib and binimetinib;
(l) encorafenib and selumetinib;
(m) sorafenib and trametinib
(n) sorafenib and cobimetinib;
(o) sorafenib and binimetinib; or
(p) sorafenib and selumetinib.

The kits can further include, if desired, one or more of various conventional pharmaceutical kit components, such as, for example, containers with one or more pharmaceutically acceptable carriers, additional containers, etc., as will be readily apparent to those skilled in the art. Printed instructions, either as inserts or as labels, indicating quantities of the components to be administered, guidelines for administration, and/or guidelines for mixing the components, can also be included in the kit.
The pharmaceutical compositions may be formulated with pharmaceutically acceptable carriers or diluents as well as any other known adjuvants and excipients in accordance with conventional techniques such as those disclosed in Remington: The Science and Practice of Pharmacy (1995).
The pharmaceutically acceptable carriers or diluents as well as any other known adjuvants and excipients should be suitable for the AXL-ADC and the chosen mode of administration. Suitability for carriers and other components of pharmaceutical compositions is determined based on the lack of significant negative impact on the desired biological properties of the chosen compound or pharmaceutical composition (*e.g.*, less than a substantial impact (10% or less relative inhibition, 5% or less relative inhibition, etc.) upon antigen binding).
A pharmaceutical composition may also include diluents, fillers, salts, buffers, detergents (e. g., a nonionic detergent, such as Tween-20 or Tween-80), stabilizers (*e.g.*, sugars or protein-free amino acids), preservatives, tissue fixatives, solubilizers, and/or other materials suitable for inclusion in a pharmaceutical composition.
The actual dosage levels of the active ingredients in the pharmaceutical compositions may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.
The pharmaceutical composition may be administered by any suitable route and mode. Suitable routes of administering a compound of the present invention *in vivo* and *in vitro* are well known in the art and may be selected by those of ordinary skill in the art.
In one embodiment, the pharmaceutical composition is administered parenterally.
The terms "parenteral administration" and "administered parenterally" as used herein refers to modes of administration other than enteral and topical administration, usually by injection, and include epidermal, intravenous, intramuscular, intra-arterial, intrathecal, intracapsular, intra-orbital, intracardiac, intradermal, intraperitoneal, intratendinous, transtracheal, subcutaneous, subcuticular, intra-articular, subcapsular, subarachnoid, intraspinal, intracranial, intrathoracic, epidural and intrasternal injection and infusion.
In one embodiment of the disclosure, the pharmaceutical composition is administered by intravenous or subcutaneous injection or infusion.
Pharmaceutically acceptable carriers include any and all suitable solvents, dispersion media, coatings, antibacterial and antifungal agents, isotonicity agents, antioxidants and absorption-delaying agents, and the like that are physiologically compatible with an AXL-ADC or therapeutic agent for the use according to the present invention.
Examples of suitable aqueous and non-aqueous carriers which may be employed in the pharmaceutical compositions include water, saline, phosphate-buffered saline, ethanol, dextrose, polyols (such as glycerol, propylene glycol, polyethylene glycol, and the like), and suitable mixtures thereof, vegetable oils, such as olive oil, corn oil, peanut oil, cottonseed oil, and sesame oil, carboxymethyl cellulose colloidal solutions, tragacanth gum and injectable organic esters, such as ethyl oleate, and/or various buffers. Other carriers are well known in the pharmaceutical arts.
Pharmaceutically acceptable carriers include sterile aqueous solutions or dispersions and sterile powders for the

extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions is contemplated.

Proper fluidity may be maintained, for example, by the use of coating materials, such as lecithin, by the maintenance of the required particle size in the case of dispersions, and by the use of surfactants.

Pharmaceutical compositions may also comprise pharmaceutically acceptable antioxidants for instance (1) water-soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal-chelating agents, such as citric acid, ethylenediamine tetraacetic acid (EDTA), sorbitol, tartaric acid, phosphoric acid, and the like.

Pharmaceutical compositions may also comprise isotonicity agents, such as sugars, polyalcohols, such as mannitol, sorbitol, glycerol or sodium chloride in the compositions.

The pharmaceutical compositions may also contain one or more adjuvants appropriate for the chosen route of administration such as preservatives, wetting agents, emulsifying agents, dispersing agents, preservatives or buffers, which may enhance the shelf life or effectiveness of the pharmaceutical composition. The AXL-ADCs or therapeutic agents for the uses of the present invention may be prepared with carriers that will protect the compound against rapid release, such as a controlled release formulation, including implants, transdermal patches, and micro-encapsulated delivery systems. Such carriers may include gelatin, glyceryl monostearate, glyceryl distearate, biodegradable, biocompatible polymers such as ethylene vinyl acetate, polyanhydrides, polyglycolic acid, collagen, poly-ortho-esters, and polylactic acid alone or with a wax, or other materials well known in the art. Methods for the preparation of such formulations are generally known to those skilled in the art. See *e.g.,* Robinbson: Sustained and Controlled Release Drug Delivery Systems (1978).

In one embodiment of the disclosure, the compounds may be formulated to ensure proper distribution *in vivo.* Pharmaceutically acceptable carriers for parenteral administration include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions is contemplated. Other active or therapeutic compounds may also be incorporated into the compositions.

Pharmaceutical compositions for injection must typically be sterile and stable under the conditions of manufacture and storage. The composition may be formulated as a solution, microemulsion, liposome, or other ordered structure suitable to high drug concentration. The carrier may be an aqueous or a non-aqueous solvent or dispersion medium containing for instance water, ethanol, polyols (such as glycerol, propylene glycol, polyethylene glycol, and the like), and suitable mixtures thereof, vegetable oils, such as olive oil, and injectable organic esters, such as ethyl oleate. The proper fluidity may be maintained, for example, by the use of a coating such as lecithin, by the maintenance of the required particle size in the case of dispersion and by the use of surfactants. In many cases, it will be preferable to include isotonic agents, for example, sugars, polyalcohols such as glycerol, mannitol, sorbitol, or sodium chloride in the composition. Prolonged absorption of the injectable compositions may be brought about by including in the composition an agent that delays absorption, for example, monostearate salts and gelatin. Sterile injectable solutions may be prepared by incorporating the active compound in the required amount in an appropriate solvent with one or a combination of ingredients *e.g.* as enumerated above, as required, followed by sterilization microfiltration. Generally, dispersions are prepared by incorporating the active compound into a sterile vehicle that contains a basic dispersion medium and the required other ingredients *e.g.* from those enumerated above. In the case of sterile powders for the preparation of sterile injectable solutions, examples of methods of preparation are vacuum-drying and freeze-drying (lyophilization) that yield a powder of the active ingredient plus any additional desired ingredient from a previously sterile-filtered solution thereof.

Sterile injectable solutions may be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, examples of methods of preparation are vacuum-drying and freeze-drying (lyophilization) that yield a powder of the active ingredient plus any additional desired ingredient from a previously sterile-filtered solution thereof.

*Production of anti-AXL antibodies*

The antibodies for use as ADCs according to the invention can be prepared recombinantly in a host cell, using nucleic acid constructs, typically in the form of one or more expression vectors. In one embodiment, the nucleic acid construct encodes one or more sequences set out in Table 1. In a further embodiment of the disclosure, the expression vector further comprises a nucleic acid sequence encoding the constant region of a light chain, a heavy chain or both light

and heavy chains of an antibody, *e.g.* a human IgG1, κ monoclonal antibody.

The expressed anti-AXL antibody may subsequently be conjugated to a moiety as described herein. In another embodiment the anti-AXL antibody may subsequently be used to generate a bispecific antibody as described herein, before conjugation.

The expression vector may be any suitable vector, including chromosomal, non-chromosomal, and synthetic nucleic acid vectors (a nucleic acid sequence comprising a suitable set of expression control elements). Examples of such vectors include derivatives of SV40, bacterial plasmids, phage DNA, baculovirus, yeast plasmids, vectors derived from combinations of plasmids and phage DNA, and viral nucleic acid (RNA or DNA) vectors. In one embodiment of the disclosure, an anti-AXL antibody-encoding nucleic acid is comprised in a naked DNA or RNA vector, including, for example, a linear expression element (as described in for instance Sykes and Johnson (1997), a compacted nucleic acid vector (as described in for instance US 6,077,835 and/or WO 00/70087), a plasmid vector such as pBR322, pUC 19/18, or pUC 118/119, a "midge" minimally-sized nucleic acid vector (as described in for instance Schakowski *et al.* (2001)), or as a precipitated nucleic acid vector construct, such as a calcium phosphate-precipitated construct (as described in for instance WO 00/46147; Benvenisty and Reshef, 1986; Wigler *et al.,* 1978; and Coraro and Pearson, 1981). Such nucleic acid vectors and the usage thereof are well known in the art (see for instance US 5,589,466 and US 5,973,972).

In one embodiment of the disclosure, the vector is suitable for expression of the anti-AXL antibody in a bacterial cell. Examples of such vectors include expression vectors such as BlueScript (Stratagene), pIN vectors (Van Heeke and Schuster, 1989), pET vectors (Novagen, Madison WI) and the like).

An expression vector may also or alternatively be a vector suitable for expression in a yeast system. Any vector suitable for expression in a yeast system may be employed. Suitable vectors include, for example, vectors comprising constitutive or inducible promoters such as alpha factor, alcohol oxidase and PGH (reviewed in Ausubel *et al.,* 1987, and Grant *et al.,* 1987).

A nucleic acid construct and/or vector may also comprise a nucleic acid sequence encoding a secretion/localization sequence, which can target a polypeptide, such as a nascent polypeptide chain, to the periplasmic space or into cell culture media. Such sequences are known in the art, and include secretion leader or signal peptides, organelle targeting sequences (e. g., nuclear localization sequences, ER retention signals, mitochondrial transit sequences, chloroplast transit sequences), membrane localization/anchor sequences (e. g., stop transfer sequences, GPI anchor sequences), and the like.

In an expression vector, the anti-AXL antibody-encoding nucleic acids may comprise or be associated with any suitable promoter, enhancer, and other expression-facilitating elements. Examples of such elements include strong expression promoters (*e.g.*, human CMV IE promoter/enhancer as well as RSV, SV40, SL3-3, MMTV, and HIV LTR promoters), effective poly (A) termination sequences, an origin of replication for plasmid product in E. coli, an antibiotic resistance gene as selectable marker, and/or a convenient cloning site (*e.g.*, a polylinker). Nucleic acids may also comprise an inducible promoter as opposed to a constitutive promoter such as CMV IE (the skilled artisan will recognize that such terms are actually descriptors of a degree of gene expression under certain conditions).

In one embodiment, the anti-AXL-antibody-encoding expression vector may be positioned in and/or delivered to the host cell or host animal via a viral vector.

The host cell can be a recombinant eukaryotic or prokaryotic host cell, such as a transfectoma, which produces an anti-AXL antibody as defined herein or a bispecific molecule of the invention as defined herein. Examples of host cells include yeast, bacterial and mammalian cells, such as CHO or HEK cells or derivatives thereof. For example, in one embodiment of the disclosure, the cell comprises a nucleic acid stably integrated into the cellular genome that comprises a sequence coding for expression of the anti-AXL antibody. In another embodiment of the disclosure, the cell comprises a non-integrated nucleic acid, such as a plasmid, cosmid, phagemid, or linear expression element, which comprises a sequence coding for expression of the anti-AXL antibody.

The term "recombinant host cell" (or simply "host cell"), as used herein, is intended to refer to a cell into which an expression vector has been introduced. It should be understood that such terms are intended to refer not only to the particular subject cell, but also to the progeny of such a cell. Because certain modifications may occur in succeeding generations due to either mutation or environmental influences, such progeny may not, in fact, be identical to the parent cell, but are still included within the scope of the term "host cell" as used herein. Recombinant host cells include, for example, transfectomas, such as CHO cells, HEK-293 cells, PER.C6, NS0 cells, and lymphocytic cells, and prokaryotic cells such as *E. coli* and other eukaryotic hosts such as plant cells and fungi.

The term "transfectoma", as used herein, includes recombinant eukaryotic host cells expressing the antibody or a target antigen, such as CHO cells, PER.C6, NS0 cells, HEK-293 cells, plant cells, or fungi, including yeast cells.

The antibody may alternatively be produced from a hybridoma prepared from murine splenic B cells obtained from mice immunized with an antigen of interest, for instance in form of cells expressing the antigen on the surface, or a nucleic acid encoding an extracellular region of AXL. Monoclonal antibodies may also be obtained from hybridomas derived from antibody-expressing cells of immunized humans or non-human mammals such as rabbits, rats, dogs, primates, etc.

Human antibodies may be generated using transgenic or transchromosomal mice, *e.g.* HuMAb mice, carrying parts of the human immune system rather than the mouse system. The HuMAb mouse contains a human immunoglobulin gene minilocus that encodes unrearranged human heavy (μ and y) and κ light chain immunoglobulin sequences, together with targeted mutations that inactivate the endogenous μ and κ chain loci (Lonberg *et al.,* 1994a). Accordingly, the mice mount a human antibody response upon immunization, the introduced human heavy and light chain transgenes, undergo class switching and somatic mutation to generate high affinity human IgG,κ monoclonal antibodies (Lonberg *et al.*, 1994b; Lonberg and Huszar, 1995; Harding and Lonberg, 1995). The preparation of HuMAb mice is described in detail in Taylor *et al.,* 1992; Chen *et al.,* 1993; Tuaillon *et al.,* 1994; and Fishwild *et al.,* 1996. See also US 5,545,806; US 5,569,825; US 5,625,126; US 5,633,425; US 5,789,650; US 5,877,397; US 5,661,016; US 5,814,318; US 5,874,299; US 5,770,429; US 5,545,807; WO 98/024884; WO 94/025585; WO 93/001227; WO 92/022645; WO 92/003918; and WO 01/009187. Splenocytes from these transgenic mice may be used to generate hybridomas that secrete human monoclonal antibodies according to well-known techniques. In addition human antibodies may be generated from transgenic mice or rats to produce human-rat chimeric antibodies that can be used as a source for the recombinant production of fully human monoclonal antibodies.

Further, human antibodies may be identified through display-type technologies, including, without limitation, phage display, retroviral display, ribosomal display, mammalian display, yeast display and other techniques known in the art, and the resulting molecules may be subjected to additional maturation, such as affinity maturation, as such techniques are well known in the art.

*Table 2 - Sequences*

| SEQ ID NO: | Name | Amino acid sequence | Comment |
|---|---|---|---|
| 1 | 107 VH | EVQLLESGGGLVQPGGSLRLSCAAS**GFTFSSYA**MNWVRQAPGKGLEWVST**TSGSGAST**YYADSVKGRFTISRDNSKNTLYLQMNSLRAEDTAVYYC**AKIWIAFDI**WGQGTMVTVSS | HCo12-BalbC Ig1 domain binding Ab |
| 2 | 107 VL | EIVLTQSPGTLSLSPGERATLSCRAS**QSVSSSY**LAWYQQKPGQAPRLLIY**GAS**SRATGIPDRFSGSGSGTDFTLTISRLEPEDFAVYYC**QQYGSSPYT**FGQGTKLEIK | |
| 3 | 140 VH | EVQLLESGGGLVQPGGSLRLSCAAS**GFTFSSYA**MTWVRQAPGKGLEWVSA**ISISGAST**FYADSVKGRFTISRDNSKNTLSLQMNSLRAEDTAVYFC**RGYSGYVYDAFDI**WGQGTMVTVSS | |
| 4 | 140 VL | DIQMTQSPSSLSASVGDRVTITCRAS**QGISNW**LAWYQQKPEKAPKSLIY**AAS**SLQSGVPSRFSGSGSGTDFTLTISSLQPEDFATYYC**QQYNSYPLT**FGGGTKVEIK | |
| 5 | 148 VH | EVQLLESGGGLVQPGGSLRLSCAAS**GFTFSSYA**MTWVRQAPGKGLEWVSA**ISISGGST**FYADSVKGRFTISRDNSKNTLYLQMNSLRAEDTAVYYC**RGYSGYVYDAFDF**WGQGTMVTVSS | HCo12-BalbC Ig2 domain binding Ab |
| 6 | 148 VL | DIQMTQSPSSLSASVGDRVTITCRAS**QGISNW**LAWYQQKPEKAPKSLIY**AAS**SLQSGVPSRFSGSGSGTDFTLTISSLQPEDFATYYC**QQYNSYPLT**FGGGTKVEIK | |
| 7 | 154 VH | EVQLLDSGGGLVQPGGSLRLSCAAS**GFTFSSYA**MSWVRQAPGKGLEWVSA**ISIGGGNA**YYADSVKGRFTISRDNSKNTLYLQMNSLRAADTAVYYC**AKPGFIMVRGPLDY**WGQGALVTVSS | HCo12-BalbC FN1 domain binding Ab |
| 8 | 154-M103L VH | EVQLLDSGGGLVQPGGSLRLSCAAS**GFTFSSYA**MSWVRQAPGKGLEWVSA**ISIGGGNA**YYADSVKGRFTISRDNSKNTLYLQMNSLRAADTAVYYC**AKPGFILVRGPLDY**WGQGALVTVSS | |

(continued)

| SEQ ID NO: | Name | Amino acid sequence | Comment |
|---|---|---|---|
| 9 | 154 VL | EIVLTQSPGTLSLSPGERATLSCRAS**QSVSNSY**LAWYQQKPGQAPRLLIY**GAS**SRATGIPDRFSGSGSGTDFTLTISRLEPEDFAVYYC**QQYGSSPYT**FGQGTKLEIK | |
| 10 | 171 VH | EVQLLESGGGLVQPGGSLRLSCAAS**GFTFSSYA**MSWVRQAPGKGLEWVSD**ISVSGGST**YYADSVKGRFTISRDNSKNTLYLQMNSLRAEDTAVYYC**AKEGYIWFGESLSYAFDI**WGQGTMVTVSS | HCo17-BalbC Ig2 domain binding Ab |
| 11 | 171 VL | EIVLTQSPGTLSLSPGERATLSCRAS**QSVSSSY**LAWYQQKPGQAPRLLIY**GAS**SRATGIPDRFSGSGSGTDFTLTISRLEPEDFAVYYC**QQYGRSFT**FGPGTKVDIK | |
| 12 | 172 VH | EVQLLESGGGLVQPGGSLRLSCAAS**GFTFSNYA**MSWVRQAPGKGLEWVSD**ISVSGGST**YYADSVKGRFTISRDNSKNTLYLQMNSLRAEDTAVYYC**AKEGYIWFGESLSYAFDI**WGQGTMVTVSS | |
| 13 | 172 VL | EIVLTQSPGTLSLSPGERATLSCRAS**QSVSSSY**LAWYQQKPGQAPRLLIY**GAS**SRATGIPDRFSGSGSGTDFTLTISRLEPEDFAVYYC**QQYGRSFT**FGPGTKVDIK | |
| 14 | 181 VH | EVQLLESGGGLVQPGGSLRLSCAAS**GFTFSSYA**MSWVRQAPGKGLEWVSD**ISVSGGST**YYADSVKGRFTISRDNSKNTLYLHMNSLRAEDTAVYYC**AKEGYIWFGESLSYAFDI**WGQGTMVTVSS | |
| 15 | 181 VH | EIVLTQSPGTLSLSPGERATLSCRAS**QSVSSSY**LAWYQQKPGQAPRLLIY**GAS**SRATGIPDRFSGSGSGTDFTLTISRLEPEDFAVYYC**QQYGRSFT**FGPGTKVDIK | |
| 16 | 183 VH | QVQLQQWGAGLLKPSETLSLTCAVY**GGSFSGYY**WSWIRQPPGKGLEWIGE**INQSGST**NYNPSLKSRVTISVDTSKNQFSLKLSSVTAADTSVYYC**ASGNWDHFFDY**WGQGTLVTVSS | HCo17-BalbC FN1 domain binding Ab |
| 17 | 183-N52Q VH | QVQLQQWGAGLLKPSETLSLTCAVY**GGSFSGYY**WSWIRQPPGKGLEWIGE**IQQSGST**NYNPSLKSRVTISVDTSKNQFSLKLSSVTAADTSVYYC**ASGNWDHFFDY**WGQGTLVTVSS | |
| 18 | 183 VL | DIQMTQSPSSVSASVGDRVTITCRAS**QGISSW**LAWYQHKPGKAPKLLIY**ATS**SLQSGVTSRFSGSGSGTDFTLTISSLQPEDFATYYC**QQAKSFPWT**FGQGTKVEIK | |
| 19 | 187 VH | QVPLQQWGAGLLKPSETLSLTCAVY**GGSFSGYH**WSWIRQPPGKGLEWIGE**ISHSGRT**NYNPSLKSRVTISIDTSKNQFSLKLSSVTAADTAVYYC**ASFITMIRGTIITHFDY**WGQGTLVTVSS | |
| 20 | 187 VL | DIQMTQSPSSLSASVGDRVTITCRAS**QGISSW**LAWYQQKPEKAPKSLIY**AAS**SLQSGVPSRFSGSGSGTDFTLTISSLQPEDFATYYC**QQYHSYPYT**FGQGTKLEIK | |

(continued)

| SEQ ID NO: | Name | Amino acid sequence | Comment |
|---|---|---|---|
| 21 | 608-01 VH | QVQLVQSGAEVKKPGSSVKVSCKAS**GGTFSSYA**ISWVRQAPGQGLEWMGR**IIPIFGIA**NYVQKFQGRVTITADKSTSTAYMELSSLRAEDTAVYYC**ARRGDYYGSGSPDVFDI**WGQGTMVTVSS | |
| 22 | 608-01 VL | EIVLTQSPGTLSLSPGERATLSCRAS**QSVSSSY**LAWYQQKPGQAPRLLIY**GAS**SRATGIPDRFSGSGSGTDFTLTISRLEPEDFAVYYC**QQYGSSYT**FGQGTKLEIK | |
| 23 | 610-01 VH | QVQLVQSGAEVKKPGSSVKVSCKAS**GGTFSSYA**ISWVRQAPGQGLEWMGR**IIPIFGIA**NYVQKFQGRVTITADKSTSTAYMELSSLRAEDTAVYYC**ARRGNYYGSGSPDVFDI**WGQGTMVTVSS | |
| 24 | 610-01 VL | EIVLTQSPGTLSLSPGERATLSCRAS**QSVSSSY**LAWYQQKPGQAPRLLIY**GAS**SRATGIPDRFSGSGSGTDFTLTISRLEPEDFAVYYC**QQYGSSYT**FGQGTKLEIK | |
| 25 | 613 VH | QVQLVQSGAEVKKPGSSVKVSCKAS**GGTFSSYA**INWMRQAPGQGLEWMGR**IIPIFGIV**NYAQKFQGRVTLTADKSTSTAYMELSSLRSEDTAVYYC**ARRGNYYGSGSPDVFDI**WGQGTMVTVSS | HCo20 Ig1 domain binding Ab |
| 26 | 613 VL | EIVLTQSPGTLSLSPGERATLSCRAS**QSVSSSY**LAWYQQKPGQAPRLLIY**GAS**SRATGIPDRFSGSGSGTDFTLTISRLEPEDFAVYYC**QQYGSSYT**FGQGTKLEIK | |
| 27 | 613-08 VH | QVQLVQSGAEVKKPGSSVKVSCKAS**GGTFSSYA**INWMRQAPGQGLEWMGR**IIPIFGIV**NYAQKFQGRVTLTADKSTSTAYMELSSLRSEDTAVYYC**ARRGNYYGSGSPDVFDI**WGQGTMVTVSS | |
| 28 | 613-08 VL | EIVLTQSPATLSLSPGERATLSCRAS**QSVSSY**LAWYQQKPGQAPRLLIY**DAS**NRATGIPARFSGSGSGTDFTLTISSLEPEDFAVYYC**QQRSNWLT**FGGGTKVEIK | |
| 29 | 620-06 VH | QVQLVQSGAEVKKPGSSVKVSCKAS**GGTFSSYA**ISWVRQAPGQGLEWMGR**IIPIFGIA**NYAQKFQGRVTITADKSTSTAYMELSSLRSEDTAVYYC**ARRGNYYGSGSPDVFDI**WGQGTMVTVSS | |
| 30 | 620-06 VL | EIVLTQSPGTLSLSPGERATLSCRAS**QSVSSSY**LAWYQQKPGQAPRLLIY**GAS**SRATGIPDRFSGSGSGTDFTLTISRLEPEDFAVYYC**QQYGSSYT**FGQGTKLEIK | |
| 31 | 726 VH | QVQLQQWGAGLLKPSETLSLTCAID**GGSFSGYY**WSWIRQPPGKGLEWIGE**ISHSGRT**NYNPSLKSRVTISIDTSKNQFSLKLSSVAAADTAVYYC**ARFITMIRGAIITHFDY**WGQGALVTVSS | HCo17-BalbC FN2 domain binding Ab |
| 32 | 726-M101L VH | QVQLQQWGAGLLKPSETLSLTCAID**GGSFSGYY**WSWIRQPPGKGLEWIGE**ISHSGRT**NYNPSLKSRVTISIDTSKNQFSLKLSSVAAADTAVYYC**ARFITLIRGAIITHFDY**WGQGALVTVSS | |

(continued)

| SEQ ID NO: | Name | Amino acid sequence | Comment |
|---|---|---|---|
| 33 | 726 VL | DIQMTQSPSSLSASVGDRVTITCRAS**QGISSW**LAWYQQKPEKAPKSLIY**AAS**SLQSGVPSRFSGSGSGTDFTLTISSLQPEDFATYYC**QQYHSYPYT**FGQGTKLEIK | |
| 34 | 733 VH | QVQLVESGGGVVQPGRSLRLSCAAS**GFSFSTYA**MHWVRQAPGKGLEWVAV**ISYDGDNK**YSADSVKGRFTISRDNSKNTLYLQMNSLRAEDTAVYYC**ARGRKLGIDAFDI**WGQGTMVTVSS | HCo17-BalbC FN1 domain binding Ab |
| 35 | 733 VL | AIQLTQSPSSLSASVGDRVTITCRAS**QGISSA**LAWYQQKPGKAPKLLIY**DAS**SLESGVPSRFSGSGSGTDFTLTISGLQPEDFATYYC**QQFNSYPFT**FGPGTKVDIK | |
| 36 | 107 VH CDR1 | GFTFSSYA | |
| 37 | 107 VH CDR2 | TSGSGAST | |
| 38 | 107 VH CDR3 | AKIWIAFDI | |
| 39 | 107 VL CDR1 | QSVSSSY | |
| | 107 VL CDR2 | GAS | |
| 40 | 107 VL CDR3 | QQYGSSPYT | |
| 41 | 140 VH CDR1 | GFTFSSYA | |
| 42 | 140 VH CDR2 | ISISGAST | |
| 43 | 140 VH CDR3 | RGYSGYVYDAFDI | |
| 44 | 140 VL CDR1 | QGISNW | |
| | 140 VL CDR2 | AAS | |
| 45 | 140 VL CDR3 | QQYNSYPLT | |
| 46 | 148 VH CDR1 | GFTFSSYA | |
| 47 | 148 VH CDR2 | ISISGGST | |
| 48 | 148 VH CDR3 | RGYSGYVYDAFDF | |
| 49 | 148 VL CDR1 | QGISNW | |
| | 148 VL CDR2 | AAS | |
| 50 | 148 VL CDR3 | QQYNSYPLT | |
| 51 | 154 VH CDR1 | GFTFSSYA | |
| 52 | 154 VH CDR2 | ISIGGGNA | |
| 53 | 154 VH CDR3 | AKPGFIMVRGPLDY | |
| 54 | 154-M103L VH CDR3 | AKPGFILVRGPLDY | |
| 55 | 154 VL CDR1 | QSVSNSY | |
| | 154 VL CDR2 | GAS | |
| 56 | 154 VL CDR3 | QQYGSSPYT | |
| 57 | 171 VH CDR1 | GFTFSSYA | |
| 58 | 171 VH CDR2 | ISVSGGST | |
| 59 | 171 VH CDR3 | AKEGYIWFGESLSYAFDI | |

(continued)

| SEQ ID NO: | Name | Amino acid sequence | Comment |
|---|---|---|---|
| 60 | 171 VL CDR1 | QSVSSSY | |
| | 171 VL CDR2 | GAS | |
| 61 | 171 VL CDR3 | QQYGRSFT | |
| 62 | 172 VH CDR1 | GFTFSNYA | |
| 63 | 172 VH CDR2 | ISVSGGST | |
| 64 | 172 VH CDR3 | AKEGYIWFGESLSYAFDI | |
| 65 | 172 VL CDR1 | QSVSSSY | |
| | 172 VL CDR2 | GAS | |
| 66 | 172 VL CDR3 | QQYGRSFT | |
| 67 | 181 VH CDR1 | GFTFSSYA | |
| 68 | 181 VH CDR2 | ISVSGGST | |
| 69 | 181 VH CDR3 | AKEGYIWFGESLSYAFDI | |
| 70 | 181 VL CDR1 | QSVSSSY | |
| | 181 VL CDR2 | GAS | |
| 71 | 181 VL CDR3 | QQYGRSFT | |
| 72 | 183 VH CDR1 | GGSFSGYY | |
| 73 | 183 VH CDR2 | INQSGST | |
| 74 | 183-N52Q VH CDR2 | IQQSGST | |
| 75 | 183 VH CDR3 | ASGNWDHFFDY | |
| 76 | 183 VL CDR1 | QGISSW | |
| | 183 VL CDR2 | ATS | |
| 77 | 183 VL CDR3 | QQAKSFPWT | |
| 78 | 187 VH CDR1 | GGSFSGYH | |
| 79 | 187 VH CDR2 | ISHSGRT | |
| 80 | 187 VH CDR3 | ASFITMIRGTIITHFDY | |
| 81 | 187 VL CDR1 | QGISSW | |
| | 187 VL CDR2 | AAS | |
| 82 | 187 VL CDR3 | QQYHSYPYT | |
| 83 | 608-01 VH CDR1 | GGTFSSYA | |
| 84 | 608-01 VH CDR2 | IIPIFGIA | |
| 85 | 608-01 VH CDR3 | ARRGDYYGSGSPDVFDI | |
| 86 | 608-01 VL CDR1 | QSVSSSY | |
| | 608-01 VL CDR2 | GAS | |
| 87 | 608-01 VL CDR3 | QQYGSSYT | |

(continued)

| SEQ ID NO: | Name | Amino acid sequence | Comment |
|---|---|---|---|
| 88 | 610-01 VH CDR1 | GGTFSSYA | |
| 89 | 610-01 VH CDR2 | IIPIFGIA | |
| 90 | 610-01 VH CDR3 | ARRGNYYGSGSPDVFDI | |
| 91 | 610-01 VL CDR1 | QSVSSSY | |
| | 610-01 VL CDR2 | GAS | |
| 92 | 610-01 VL CDR3 | QQYGSSYT | |
| 93 | 613 VH CDR1 | GGTFSSYA | |
| 94 | 613 VH CDR2 | IIPIFGIV | |
| 95 | 613 VH CDR3 | ARRGNYYGSGSPDVFDI | |
| 96 | 613 VL CDR1 | QSVSSSY | |
| | 613 VL CDR2 | GAS | |
| 97 | 613 VL CDR3 | QQYGSSYT | |
| 98 | 613-08 VH CDR1 | GGTFSSYA | |
| 99 | 613-08 VH CDR2 | IIPIFGIV | |
| 100 | 613-08 VH CDR3 | ARRGNYYGSGSPDVFDI | |
| 101 | 613-08 VL CDR1 | QSVSSY | |
| | 613-08 VL CDR2 | DAS | |
| 102 | 613-08 VL CDR3 | QQRSNWLT | |
| 103 | 620-06 VH CDR1 | GGTFSSYA | |
| 104 | 620-06 VH CDR2 | IIPIFGIA | |
| 105 | 620-06 VH CDR3 | ARRGNYYGSGSPDVFDI | |
| 106 | 620-06 VL CDR1 | QSVSSSY | |
| | 620-06 VL CDR2 | GAS | |
| 107 | 620-06 VL CDR3 | QQYGSSYT | |
| 108 | 726 VH CDR1 | GGSFSGYY | |
| 109 | 726 VH CDR2 | ISHSGRT | |

(continued)

| SEQ ID NO: | Name | Amino acid sequence | Comment |
|---|---|---|---|
| 110 | 726 VH CDR3 | ARFITMIRGAIITHFDY | |
| 111 | 726-M101L VH CDR3 | ARFITLIRGAIITHFDY | |
| 112 | 726 VL CDR1 | QGISSW | |
| | 726 VL CDR2 | AAS | |
| 113 | 726 VL CDR3 | QQYHSYPYT | |
| 114 | 733 VH CDR1 | GFSFSTYA | |
| 115 | 733 VH CDR2 | ISYDGDNK | |
| 116 | 733 VH CDR3 | ARGRKLGIDAFDI | |
| 117 | 733 VL CDR1 | QGISSA | |
| | 733 VL CDR2 | DAS | |
| 118 | 733 VL CDR3 | QQFNSYPFT | |
| 119 | Ig2 domain VH CDR2 | ISISGXST - wherein X is A or G | |
| 120 | Ig2 domain VH CDR3 | RGYSGYVYDAFDX - wherein X is I or F | |
| 121 | FN2 domain VH CDR1 | GGSFSGYX - wherein X is H or Y | |
| 122 | FN2 domain VH CDR3 | AX1FITMIRGX2IITHFDY - wherein X1 is S or R; and X2 is T or A | |
| 123 | FN1 domain VH CDR1 | GFTFSXYA - wherein X is S or N | |
| 124 | FN1 domain VH CDR2 | ISVSGGST | |
| 125 | FN1 domain VH CDR3 | AKEGYIWFGESLSYAFDI | |
| 126 | Ig1 domain VH CDR2 | IIPIFGIX - wherein X is A or V | |
| 127 | Ig1 domain VH CDR3 | ARRGXYYGSGSPDVFDI - wherein X is D or N | |
| 128 | Ig1 domain VL CDR1 | QSVXSSY - wherein X is S or del | |
| | Ig1 domain VL CDR2 | XAS - wherein X is D or G | |
| 129 | Ig1 domain VL CDR3 | QQX1X2X3X4X5T - wherein X1 is R or Y; X2 is S or G; X3 is N or S; X4 is W or S; and X5 is L or Y | |

(continued)

| SEQ ID NO: | Name | Amino acid sequence | Comment |
|---|---|---|---|
| 130 | Human AXL protein (Swis-sprot P30530) | MAWRCPRMGRVPLAWCLALCGWACMAPRGTQAEESPFVGN<br>PGNITGARGLTGTLRCQLQVQGEPPEVHWLRDGQILELADSTQT<br>QVPLGEDEQDDWIVVSQLRITSLQLSDTGQYQCLVFLGHQTFVS<br>QPGYVGLEGLPYFLEEPEDRTVAANTPFNLSCQAQGPPEPVDLL<br>WLQDAVPLATAPGHGPQRSLHVPGLNKTSSFSCEAHNAKGVTT<br>SRTATITVLPQQPRNLHLVSRQPTELEVAWTPGLSGIYPLTHCTL<br>QAVLSDDGMGIQAGEPDPPEEPLTSQASVPPHQLRLGSLHPHT<br>PYHIRVACTSSQGPSSWTHWLPVETPEGVPLGPPENISATRNGS<br>QAFVHWQEPRAPLQGTLLGYRLAYQGQDTPEVLMDIGLRQEVT<br>LELQGDGSVSNLTVCVAAYTAAGDGPWSLPVPLEAWRPGQAQ<br>PVHQLVKEPSTPAFSWPWWYVLLGAVVAAACVLILALFLVHRRK<br>KETRYGEVFEPTVERGELVVRYRVRKSYSRRTTEATLNSLGISEELK<br>EKLRDVMVDRHKVALGKTLGEGEFGAVMEGQLNQDDSILKVA<br>VKTMKIAICTRSELEDFLSEAVCMKEFDHPNVMRLIGVCFQGSER<br>ESFPAPVVILPFMKHGDLHSFLLYSRLGDQPVYLPTQMLVKFMA<br>DIASGMEYLSTKRFIHRDLAARNCMLNENMSVCVADFGLSKKIY<br>NGDYYRQGRIAKMPVKWIAIESLADRVYTSKSDVWSFGVTMW<br>EIATRGQTPYPGVENSEIYDYLRQGNRLKQPADCLDGLYALMSR<br>CWELNPQDRPSFTELREDLENTLKALPPAQEPDEILYVNMDEGG<br>GYPEPPGAAGGADPPTQPDPKDSCSCLTAAEVHPAGRYVLCPST<br>TPSPAQPADRGSPAAPGQEDGA | |
| 131 | Mus musculus AXL | MAWRCPRMGRVPLAWCLALCGWACMYPYDVPDYAAHKDTQ<br>TEAGSPFVGNPGNITGARGLTGTLRCELQVQGEPPEVVWLRDG<br>QILELADNTQTQVPLGEDWQDEWKVVSQLRISALQLSDAGEYQ<br>CMVHLEGRTFVSQPGFVGLEGLPYFLEEPEDKAVPANTPFNLSC<br>QAQGPPEPVTLLWLQDAVPLAPVTGHSSQHSLQTPGLNKTSSFS<br>CEAHNAKGVTTSRTATITVLPQRPHHLHVVSRQPTELEVAWTPG<br>LSGIYPLTHCNLQAVLSDDGVGIWLGKSDPPEDPLTLQVSVPPH<br>QLRLEKLLPHTPYHIRISCSSSQGPSPWTHWLPVETTEGVPLGPP<br>ENVSAMRNGSQVLVRWQEPRVPLQGTLLGYRLAYRGQDTPEV<br>LMDIGLTREVTLELRGDRPVANLTVSVTAYTSAGDGPWSLPVPL<br>EPWRPGQGQPLHHLVSEPPPRAFSWPWWYVLLGAVVAAACV<br>LILALFLVHRRKKETRYGEVFEPTVERGELVVRYRVRKSYSRRTTE<br>ATLNSLGISEELKEKLRDVMVDRHKVALGKTLGEGEFGAVMEGQ<br>LNQDDSILKVAVKTMKIAICTRSELEDFLSEAVCMKEFDHPNVM<br>RLIGVCFQGSERESFPAPVVILPFMKHGDLHSFLLYSRLGDQPVYL<br>PTQMLVKFMADIASGMEYLSTKRFIHRDLAARNCMLNENMSV<br>CVADFGLSKKIYNGDYYRQGRIAKMPVKWIAIESLADRVYTSKSD<br>VWSFGVTMWEIATRGQTPYPGVENSEIYDYLRQGNRLKQPADC<br>LDGLYALMSRCWELNPQDRPSFTELREDLENTLKALPPAQEPDEI<br>LYVNMDEGGGYPEPPGAAGGADPPTQPDPKDSCSCLTAAEVH<br>PAGRYVLCPSTTPSPAQPADRGSPAAPGQEDGA | |

(continued)

| SEQ ID NO: | Name | Amino acid sequence | Comment |
|---|---|---|---|
| 132 | Homo sapiens AXL - Mus musculus Ig1 domain | MAWRCPRMGRVPLAWCLALCGWACMAPRGTQAEESPFVGN PGNITGARGLTGTLRCQLQVQGEPPEVHWLRDGQILELADSTQT QVPLGEDEQDDWIVVSQLRITSLQLSDTGQYQCLVFLGHQTFVS QPGYVGLEGLPYFLEEPEDKAVPANTPFNLSCQAQGPPEPVTLL WLQDAVPLAPVTGHSSQHSLQTPGLNKTSSFSCEAHNAKGVTT SRTATITVLPQQPRNLHLVSRQPTELEVAWTPGLSGIYPLTHCTL QAVLSDDGMGIQAGEPDPPEEPLTSQASVPPHQLRLGSLHPHT PYHIRVACTSSQGPSSWTHWLPVETPEGVPLGPPENISATRNGS QAFVHWQEPRAPLQGTLLGYRLAYQGQDTPEVLMDIGLRQEVT LELQGDGSVSNLTVCVAAYTAAGDGPWSLPVPLEAWRPGQAQ PVHQLVKEPSTPAFSWPWWYVLLGAVVAAACVLILALFLVHRRK KETRYGEVFEPTVERGELVVRYRVRKSYSRRTTEATLNSLGISEELK EKLRDVMVDRHKVALGKTLGEGEFGAVMEGQLNQDDS ILKVAVKTMKIAICTRSELEDFLSEAVCMKEFDHPNVMRLIGVCF QGSERESFPAPVVILPFMKHGDLHSFLLYSRLGDQPVYLPTQML VKFMADIASGMEYLSTKRFIHRDLAARNCMLNENMSVCVADFG LSKKIYNGDYYRQGRIAKMPVKWIAIESLADRVYTSKSDVWSFG VTMWEIATRGQTPYPGVENSEIYDYLRQGNRLKQPADCLDGLY ALMSRCWELNPQDRPSFTELREDLENTLKALPPAQEPDEILYVN MDEGGGYPEPPGAAGGADPPTQPDPKDSCSCLTAAEVHPAGR YVLCPSTTPSPAQPADRGSPAAPGQEDGA | |
| 133 | Homo sapiens AXL - Mus musculus Ig2 domain | MAWRCPRMGRVPLAWCLALCGWACMAPRGTQAEESPFVGN PGNITGARGLTGTLRCQLQVQGEPPEVHWLRDGQILELADSTQT QVPLGEDEQDDWIVVSQLRITSLQLSDTGQYQCLVFLGHQTFVS QPGYVGLEGLPYFLEEPEDKAVPANTPFNLSCQAQGPPEPVTLL WLQDAVPLAPVTGHSSQHSLQTPGLNKTSSFSCEAHNAKGVTT SRTATITVLPQQPRNLHLVSRQPTELEVAWTPGLSGIYPLTHCTL QAVLSDDGMGIQAGEPDPPEEPLTSQASVPPHQLRLGSLHPHT PYHIRVACTSSQGPSSWTHWLPVETPEGVPLGPPENISATRNGS QAFVHWQEPRAPLQGTLLGYRLAYQGQDTPEVLMDIGLRQEVT LELQGDGSVSNLTVCVAAYTAAGDGPWSLPVPLEAWRPGQAQ PVHQLVKEPSTPAFSWPWWYVLLGAVVAAACVLILALFLVHRRK KETRYGEVFEPTVERGELVVRYRVRKSYSRRTTEATLNSLGISEELK EKLRDVMVDRHKVALGKTLGEGEFGAVMEGQLNQDDS ILKVAVKTMKIAICTRSELEDFLSEAVCMKEFDHPNVMRLIGVCF QGSERESFPAPVVILPFMKHGDLHSFLLYSRLGDQPVYLPTQML VKFMADIASGMEYLSTKRFIHRDLAARNCMLNENMSVCVADFG LSKKIYNGDYYRQGRIAKMPVKWIAIESLADRVYTSKSDVWSFG VTMWEIATRGQTPYPGVENSEIYDYLRQGNRLKQPADCLDGLY ALMSRCWELNPQDRPSFTELREDLENTLKALPPAQEPDEILYVN MDEGGGYPEPPGAAGGADPPTQPDPKDSCSCLTAAEVHPAGR YVLCPSTTPSPAQPADRGSPAAPGQEDGA | |

(continued)

| SEQ ID NO: | Name | Amino acid sequence | Comment |
|---|---|---|---|
| 134 | Homo sapiens AXL - Mus musculus FN1 domain | MAWRCPRMGRVPLAWCLALCGWACMAPRGTQAEESPFVGN PGNITGARGLTGTLRCQLQVQGEPPEVHWLRDGQILELADSTQT QVPLGEDEQDDWIVVSQLRITSLQLSDTGQYQCLVFLGHQTFVS QPGYVGLEGLPYFLEEPEDRTVAANTPFNLSCQAQGPPEPVDLL WLQDAVPLATAPGHGPQRSLHVPGLNKTSSFSCEAHNAKGVTT SRTATITVLPQRPHHLHVVSRQPTELEVAWTPGLSGIYPLTHCNL QAVLSDDGVGIWLGKSDPPEDPLTLQVSVPPHQLRLEKLLPHTP YHIRISCSSSQGPSPWTHWLPVETTEGVPLGPPENISATRNGSQA FVHWQEPRAPLQGTLLGYRLAYQGQDTPEVLMDIGLRQEVTLE LQGDGSVSNLTVCVAAYTAAGDGPWSLPVPLEAWRPGQAQPV HQLVKEPSTPAFSWPWWYVLLGAVVAAACVLILALFLVHRRKKE TRYGEVFEPTVERGELVVRYRVRKSYSRRTTEATLNSLGISEELKEK LRDVMVDRHKVALGKTLGEGEFGAVMEGQLNQDDS ILKVAVKTMKIAICTRSELEDFLSEAVCMKEFDHPNVMRLIGVCF QGSERESFPAPVVILPFMKHGDLHSFLLYSRLGDQPVYLPTQML VKFMADIASGMEYLSTKRFIHRDLAARNCMLNENMSVCVADFG LSKKIYNGDYYRQGRIAKMPVKWIAIESLADRVYTSKSDVWSFG VTMWEIATRGQTPYPGVENSEIYDYLRQGNRLKQPADCLDGLY ALMSRCWELNPQDRPSFTELREDLENTLKALPPAQEPDEILYVN MDEGGGYPEPPGAAGGADPPTQPDPKDSCSCLTAAEVHPAGR YVLCPSTTPSPAQPADRGSPAAPGQEDGA | |
| 135 | Homo sapiens AXL - Mus musculus FN2 domain | MAWRCPRMGRVPLAWCLALCGWACMAPRGTQAEESPFVGN PGNITGARGLTGTLRCQLQVQGEPPEVHWLRDGQILELADSTQT QVPLGEDEQDDWIVVSQLRITSLQLSDTGQYQCLVFLGHQTFVS QPGYVGLEGLPYFLEEPEDRTVAANTPFNLSCQAQGPPEPVDLL WLQDAVPLATAPGHGPQRSLHVPGLNKTSSFSCEAHNAKGVTT SRTATITVLPQQPRNLHLVSRQPTELEVAWTPGLSGIYPLTHCTL QAVLSDDGMGIQAGEPDPPEEPLTSQASVPPHQLRLGSLHPHT PYHIRVACTSSQGPSSWTHWLPVETPEGVPLGPPENVSAMRNG SQVLVRWQEPRVPLQGTLLGYRLAYRGQDTPEVLMDIGLTREVT LELRGDRPVANLTVSVTAYTSAGDGPWSLPVPLEPWRPGQGQP LHHLVSEPPPRAFSWPWWYVLLGAVVAAACVLILALFLVHRRKK ETRYGEVFEPTVERGELVVRYRVRKSYSRRTTEATLNSLGISEELKE KLRDVMVDRHKVALGKTLGEGEFGAVMEGQLNQDDSILKVAV KTMKIAICTRSELEDFLSEAVCMKEFDHPNVMRLIGVCFQGSERE SFPAPVVILPFMKHGDLHSFLLYSRLGDQPVYLPTQMLVKFMAD IASGMEYLSTKRFIHRDLAARNCMLNENMSVCVADFGLSKKIYN GDYYRQGRIAKMPVKWIAIESLADRVYTSKSDVWSFGVTMWEI ATRGQTPYPGVENSEIYDYLRQGNRLKQPADCLDGLYALMSRC WELNPQDRPSFTELREDLENTLKALPPAQEPDEILYVNMDEGGG YPEPPGAAGGADPPTQPDPKDSCSCLTAAEVHPAGRYVLCPSTT PSPAQPADRGSPAAPGQEDGA | |
| 136 | 511 VH | EVQLLESGGGLVQPGGSLRLSCAAS**GFTFSSYA**MNWVRQAPGK GLEWVSG**ISGSGGHT**YHADSVKGRFTISRDNSKNTLYLQMNSLR AEDTAVYYC**AKDRYDILTGYYNLLDY**WGQGTLVTVSS | Ig2 domain binding Ab |

(continued)

| SEQ ID NO: | Name | Amino acid sequence | Comment |
|---|---|---|---|
| 137 | 511 VH CDR1 | GFTFSSYA | |
| 138 | 511 VH CDR2 | ISGSGGHT | |
| 139 | 511 VH CDR3 | AKDRYDILTGYYNLLDY | |
| 140 | 511 VL | DIQMTQSPSSLSASVGDRVTITCRAS**QGISSW**LAWYQQKPEEAPKSLIY**AAS**SLQSGVPSRFSGSGSGTDFTLTISSLQPEDFATYYC**QQYNSYPLT**FGGGAKVEIK | |
| 141 | 511 VL CDR1 | QGISSW | |
| | 511 VL CDR2 | AAS | |
| 142 | 511 VL CDR3 | QQYNSYPLT | |
| 143 | 061 VH | QVQLVQSGAEVKKPGASVKVSCKASGYAFTGYGISWVRQAPGQGLEWIGWISAYNGNTNYVQNLQDRVTMTTDTSTSTAYMELRSLRSDDTAVYYCARDHISMLRGIIIRNYWGQGTLVTVSS | Ig1 domain binding Ab |
| 144 | 061 VL | EIVLTQSPATLSLSPGERATLSCRASQSVSSYLAWYQQKPGQAPRLLIYDASNRATGIPARFSGSGSGTDFTLTISSLEPEDFAVYYCQQRSSWPRLTFGGGTKVEIK | |
| 145 | 137 VH | QVQLVQSGAEVKKPGSSVKVSCKASGGTFSRYAISWVRQAPGQGLEWMGRIIPIVGIANYAQKFQGRVTLTADKSTSTAYMELSSLRSEDTAVYYCAREAGYSSSWYAEYFQHWGQGTLVTVSS | |
| 146 | 137 VL | EIVLTQSPGTLSLSPGERATLSCRASQSVSSNYLAWYQQKPGQAPRLLIYGASSRATGFPDRFSGSGSGTDFTLTISRLEPEDFAVYYCQQYGSSPYTFGQGTKLEIK | |
| 147 | Cynomolgus monkey AXL (GenBank number HB387229.1) | AWRCPRMGRVPLAWCLALCGWVCMAPRGTQAEESPFVGNPGNITGARGLTGTLRCQLQVQGEPPEVHWLRDGQILELADSTQTQVPLGEDEQDDWIVVSQLRIASLQLSDAGQYQCLVFLGHQNFVSQPGYVGLEGLPYFLEEPEDRTVAANTPFNLSCQAQGPPEPVDLLWLQDAVPLATAPGHGPQRNLHVPGLNKTSSFSCEAHNAKGVTTSRTATITVLPQQPRNLHLVSRQPTELEVAWTPGLSGIYPLTHCTLQAVLSDDGMGIQAGEPDPPEEPLTLQASVPPHQLRLGSLHPHTPYHIRVACTSSQGPSSWTHWLPVETPEGVPLGPPENISATRNGSQAFVHWQEPRAPLQGTLLGYRLAYQGQDTPEVLMDIGLRQEVTLELQGDGSVSNLTVCVAAYTAAGDGPWSLPVPLEAWRPGQAQPVHQLVKETSAPAFSWPWWYILLGAVVAAACVLILALFLVHRRKKETRYGEVFEPTVERGELVVRYRVRKSYSRRTTEATLNSLGISEELKEKLRDVMVDRHKVALGKTLGEGEFGAVMEGQLNQDDSILKVAVKTMKIAICTRSELEDFLSEAVCMKEFDHPNVMRLIGVCFQGSERESFPAPVVILPFMKHGDLHSFLLYSRLGDQPVYLPTQMLVKFMADIASGMEYLSTKRFIHRDLAARNCMLNENMSVCVADFGLSKKIYNGDYYRQGRIAKMPVKWIAIESLADRVYTSKSDVWSFGVTMWEIATRGQTPYPGVENSEIYDYLRQGNRLKQPADCLDGLYALMSRCWELNPQDRPSFTELREDLENTLKALPPAQEPDEILYVNMDEGGGYPEPPGAAGGADPPTQLDPKDSCSCLTSAEVHPAGRYVLCPSTAPSPAQPADRGSPAAPGQEDGA | |
| 148-153 | | See Example 3 | |

The present invention is further illustrated by the following examples which should not be construed as further limiting.

EXAMPLES

*Example 1 - Generation of AXL antibodies and AXL ADCs*

First set of AXL-specific antibodies

A first set of AXL-specific monoclonal antibodies (antibodies IgG1-AXL-061, IgG1-AXL-107, IgG1-AXL-183, IgG1-AXL-613, IgG1-AXL-726, IgG1-AXL-511, IgG1-AXL-137, IgG1-AXL-148, IgG1-AXL-154, IgG1-AXL-171, IgG1-AXL-733) were produced by immunizing transgenic mice with AXL protein constructs or cells as described below. For details on immunization procedures, hybridoma generation and mass spectrometry of purified antibodies, see Example 1 of WO 2016/005593 A1.

Expression constructs for AXL

The following codon-optimized constructs for expression of various full-length AXL variants were generated: human (*Homo sapiens*) AXL (Genbank accession no. NP_068713.2), human-cynomolgus monkey chimeric AXL in which the human extracellular domain (ECD) was replaced with the ECD of cynomolgus monkey (*Macaca fascicularis*) AXL (translation of Genbank accession HB387229.1; aa 1-447), human-mouse chimeric AXL in which the human ECD was replaced with the ECD of mouse (*Mus musculus*) AXL (Genbank accession NP_033491.2; aa 1-441), human-mouse chimeric AXL in which the human Ig-like domain I (aa 1-134, also termed "Ig1 domain" herein) was replaced with the Ig-like domain I of mouse AXL, human-mouse chimeric AXL in which the human Ig-like domain II (aa 148-194, also termed "Ig2 domain" herein) was replaced by the Ig-like domain II of mouse AXL, human-mouse chimeric ALX in which the human FNIII-like domain I (aa 227-329, also termed "FN1 domain" herein) was replaced with the FNIII-like domain I of mouse AXL, human-mouse chimeric AXL in which the human FNIII-like domain II (aa 340-444, also termed "FN2 domain" herein) was replaced by the FNIII-like domain II of mouse AXL. In addition, the following codon-optimized constructs for various AXL ECD variants were generated: the extracellular domain (ECD) of human AXL (aa 1-447) with a C-terminal His tag (AXLECDHis), the FNIII-like domain II of human AXL (aa 327-447) with a N-terminal signal peptide and a C-terminal His tag (AXL-FN2ECDHis), and the Ig1 and Ig2 domains of human AXL (aa 1-227) with a C-terminal His tag (AXL-Ig12ECDHis).

The constructs contained suitable restriction sites for cloning and an optimal Kozak (GCCGCCACC) sequence (Kozak *et al.,* 1999). The constructs were cloned in the mammalian expression vector pcDNA3.3 (Invitrogen).

AXL expression in EL4 cells

EL4 cells were stable transfected with the pcDNA3.3 vector containing the full human AXL coding sequence and stable clones were selected after selection with the antibiotic agent, G418, (Geneticin).

Purification of His-tagged AXL

AXLECDHis, AXL-FN2ECDHis, and AXL-Ig12ECDHis were expressed in HEK-293F cells. The His-tag enables purification with immobilized metal affinity chromatography. In this process, a chelator fixed onto the chromatographic resin is charged with $Co^{2+}$ cations. His-tagged protein containing supernatants were incubated with the resin in batch mode (*i.e.* solution). The His-tagged protein binds strongly to the resin beads, while other proteins present in the culture supernatant do not bind or bind weakly compared to the His-tagged proteins. After incubation the beads are retrieved from the supernatant and packed into a column. The column is washed in order to remove weakly bound proteins. The strongly bound His-tagged proteins are then eluted with a buffer containing imidazole, which competes with the binding of His to $Co^{2+}$. The eluent is removed from the protein by buffer exchange on a desalting column.

Homogeneous antigen specific screening assay

The presence of anti-AXL antibodies in sera of immunized mice or HuMab (human monoclonal antibody) hybridoma or transfectoma culture supernatant was determined by homogeneous antigen specific screening assays using Fluorometric Micro volume Assay Technology (FMAT; Applied Biosystems, Foster City, CA, USA). For this, two different test designs with combinations of either 4 or 8 cell based assays were used.

The 4 cell based assay test design was used for the testing of sera from immunized mice and as primary screening test for hybridoma or transfectoma culture supernatant. In the 4 assay test design samples were analyzed for

binding of human antibodies to A431 (DSMZ) and MDA-MB-231 cells (both expressing AXL at the cell surface) as well as binding to TH1021-AXL (HEK-293F cells transiently expressing full length human AXL; produced as described above) and HEK293 wild-type cells (negative control which does not express AXL), respectively.

Hybridoma or transfectoma culture supernatant samples were additionally subjected to an 8 cell based assay test design. In the 8 assay test design samples were analyzed for binding of human antibodies to TH1021-hAXL (HEK-293F cells transiently expressing the human AXL), TH1021-cAXL (HEK-293F cells transiently expressing human-cynomolgus AXL chimeras in which the human ECD had been replaced with the ECD of cynomolgus monkey AXL), TH1021-mAXL (HEK-293F cells transiently expressing human-mouse AXL chimeras in which the human ECD had been replaced with the ECD of mouse AXL), TH1021-mIg1 (HEK-293F cells transiently expressing the human AXL with the Ig-like domain I being replaced by the Ig-like domain I of mouse AXL), TH1021-mIg2 (HEK-293F cells transiently expressing human AXL with the Ig-like domain II being replaced by the Ig-like domain II of mouse AXL), TH1021-mFN1 (HEK-293F cells transiently expressing human AXL with the FNIII-like domain I being replaced by the FNIII-like domain I of mouse AXL), TH1021-mFN2 (HEK-293F cells transiently expressing human AXL with the FNIII-like domain II being replaced by the FNIII-like domain II of mouse AXL), and HEK293 wild-type cells (negative control which does not express AXL), respectively.

Samples were added to the cells to allow binding to AXL. Subsequently, binding of HuMab was detected using a fluorescent conjugate (Goat anti-Human IgG Fc gamma-DyLight649; Jackson ImmunoResearch). The AXL specific humanized mouse antibody A0704P (produced in HEK-293F cells) was used as a positive control and HuMab-mouse pooled serum and ChromPure Human IgG, whole molecule (Jackson ImmunoResearch), respectively, were used as negative controls. The samples were scanned using an Applied Biosystems 8200 Cellular Detection System (8200 CDS) and mean fluorescence was used as read-out. Samples were stated positive when counts were higher than 50 and counts $\times$ fluorescence was at least three times higher than the negative control.

Sequence analysis of the AXL antibody variable domains and cloning in expression vectors

Total RNA was prepared from 0.2 to $5\times10^6$ hybridoma cells and 5'-RACE-Complementary DNA (cDNA) was prepared from 100 ng total RNA, using the SMART RACE cDNA Amplification kit (Clontech), according to the manufacturer's instructions. VH and VL coding regions were amplified by PCR and cloned directly, in frame, in the pG1f and pKappa expression vectors, by ligation independent cloning (Aslanidis, C. and P.J. de Jong, Nucleic Acids Res 1990;18(20): 6069-74). For each antibody, 12 VL clones and 12 VH clones were sequenced. The resulting sequences are shown in Table 2. CDR sequences were defined according to IMGT (Lefranc *et al.,* 1999 and Brochet, 2008). Clones with a correct Open Reading Frame (ORF) were selected for further study and expression. Vectors of all combinations of heavy chains and light chains that were found were transiently co-expressed in FreestyleTM 293-F cells using 293fectin.

For antibodies IgG1-AXL-154, IgG1-AXL-183 and IgG1-AXL-726, the following variants with point mutations in the variable domains were generated: IgG1-AXL-154-M103L, IgG1-AXL-183-N52Q and IgG1-AXL-726-M101L. Mutants were generated by site-directed mutagenesis using the Quickchange II mutagenesis kit (Stratagene).

AXL control antibodies

In some of the Examples a comparison antibody against AXL was used (IgG1-YW327.6S2) that has been previously described (EP 2 220 131, U3 Pharma; WO 2011/159980, Genentech). The VH and VL sequences for these AXL-specific antibodies were cloned into the pG1f and pKappa expression vectors.

b12 antibody

In some of the examples the antibody b12, a gp120 specific antibody (Barbas, 1993) was used as a negative control.

Expression

Antibodies were expressed as IgG1,$\kappa$. Plasmid DNA mixtures encoding both heavy and light chains of antibodies were transiently transfected to Freestyle HEK293F cells (Invitrogen, US) using 293fectin (Invitrogen, US) essentially as described by the manufacturer.

Purification of antibodies

Culture supernatant was filtered over 0.2 $\mu$m dead-end filters, loaded on 5 mL MabSelect SuRe columns (GE Health Care) and eluted with 0.1 M sodium citrate-NaOH, pH 3. The eluate was immediately neutralized with 2M Tris-HCl,

pH 9 and dialyzed overnight to 12.6 mM NaH2PO4, 140 mM NaCl, pH 7.4 (B.Braun). Alternatively, subsequent to purification, the eluate was loaded on a HiPrep Desalting column and the antibody was exchanged into 12.6 mM NaH2PO4, 140 mM NaCl, pH 7.4 (B.Braun) buffer. After dialysis or exchange of buffer, samples were sterile filtered over 0.2 μm dead-end filters. Purity was determined by SDS-PAGE and IgG concentration was measured using an Octet (Fortebio, Menlo Park, USA). Purified antibodies were stored at 4°C.

AXL-specific antibody 511

The antibody IgG1-AXL-511 was produced by immunizing transgenic mice with AXL protein constructs or cells as described below, using the below-described selection procedure. For details on immunization procedure, hybridoma generation, isolation of RNA from spleen cells, primer sequences, LEE PCR, and determination and selection of HC and LC sequences, see WO 2016/005593 A1.

Expression constructs for AXL

The following codon-optimized constructs for expression of various full-length AXL variants were generated: human (*Homo sapiens*) AXL (Genbank accession no. NP_068713.2), human-cynomolgus monkey chimeric AXL in which the human extracellular domain (ECD) was replaced with the ECD of cynomolgus monkey (*Macaca fascicularis*) AXL (translation of Genbank accession HB387229.1; aa 1-447), human-mouse chimeric AXL in which the human ECD was replaced with the ECD of mouse (*Mus musculus*) AXL (Genbank accession NP_033491.2; aa 1-441), human-mouse chimeric AXL in which the human Ig-like domain I (aa 1-147, also termed "Ig1 domain" herein) was replaced with the Ig-like domain I of mouse AXL, human-mouse chimeric AXL in which the human Ig-like domain II (aa 148-227, also termed "Ig2 domain" herein) was replaced by the Ig-like domain II of mouse AXL, human-mouse chimeric ALX in which the human FNIII-like domain I (aa 228-326, also termed "FN1 domain" herein) was replaced with the FNIII-like domain I of mouse AXL, human-mouse chimeric AXL in which the human FNIII-like domain II (aa 327-447, also termed "FN2 domain" herein) was replaced by the FNIII-like domain II of mouse AXL. In addition, the following codon-optimized constructs for various AXL ECD variants were generated: the extracellular domain (ECD) of human AXL (aa 1-447) with a C-terminal His tag (AXLECDHis), the FNIII-like domain II of human AXL (aa 327-447) with a N-terminal signal peptide and a C-terminal His tag (AXL-FN2ECDHis), and the Ig1 and Ig2 domains of human AXL (aa 1-227) with a C-terminal His tag (AXL-Ig12ECDHis).

The constructs contained suitable restriction sites for cloning and an optimal Kozak (GCCGCCACC) sequence (Kozak et al. (1999) Gene 234: 187-208). The constructs were cloned in the mammalian expression vector pcDNA3.3 (Invitrogen).

AXL expression in EL4 cells

EL4 cells were stable transfected with the pcDNA3.3 vector containing the full length human AXL coding sequence and stable clones were selected after selection with the antibiotic agent, G418, (Geneticin).

Purification of His-tagged AXL

AXLECDHis, AXL-FN2ECDHis, and AXL-Ig12ECDHis were expressed in HEK293F cells and purified with immobilized metal affinity chromatography.

Transient expression in HEK-293 cells

Antibodies were expressed as IgG1,κ. Plasmid DNA mixtures encoding both heavy and light chains of antibodies were transiently transfected in Freestyle 293-F (HEK293F) cells (Life technologies, USA) using 293fectin (Life technologies) essentially as described by Vink, T., et al. (2014) ('A simple, robust and highly efficient transient expression system for producing antibodies', Methods, 65 (1), 5-10).

For LEE expression of Abs 1 μl of the HC LEE PCR reaction mixture, 1 μl of the LC PCR reaction mixture and 1 μl of a 30 ng/ μl enhancing mix containing a mix of 3 expression enhancing plasmids as described in Vink, T., *et al.* (2014), were mixed and transfected in HEK293F cells in a total volume of 100 μl using 293 fectin as transfection reagent, according to the instructions of the manufacturer (Life technologies), using 96 well plates as vessel, essentially as described supra.

AXLECDHis ELISA

ELISA plates (Greiner, Netherlands) were coated with 100 μl / well of 0.5 μg/ ml AXLECDHis in Phosphate

buffered saline (PBS) and incubated for 16 hours at room temperature (RT). The coating solution was removed and the wells were blocked by adding 150 µl PBSTC (PBS containing 0.1 % tween-20 and 2% chicken serum) well and incubating for 1 hour at RT. The plates were washed three times with 300 µl PBST (PBS containing 0.1 % tween-20)/well and 100 µl of test solution was added, followed by an incubation of 1 hour at RT. After washing three times with 300 µl of PBST/well, 100 µl antibody goat anti human IgG coupled with horse radish peroxidase (diluted 1/3000) was added and incubated for 1 hour at RT. After washing three times with 300 µl of PBST/well, 100 µl of ABTS (1mg/ml) solution was added and incubated at RT until sufficient signal was observed and the reaction was stopped by adding 100 µl of 2 % oxalic acid solution. 96 well plates were measured on an ELISA reader at 405 nm.

Diversity screen

Samples were analyzed for binding of antibodies to TH1021-hAXL (HEK293F cells transiently expressing the human AXL), TH1021-cAXL (HEK293F cells transiently expressing human-cynomolgus AXL chimeras in which the human ECD had been replaced with the ECD of cynomolgus monkey AXL), TH1021-mAXL (HEK293F cells transiently expressing human-mouse AXL chimeras in which the human ECD had been replaced with the ECD of mouse AXL), TH1021-mIg1 (HEK293F cells transiently expressing the human AXL with the Ig-like domain I being replaced by the Ig-like domain I of mouse AXL), TH1021-mIg2 (HEK293F cells transiently expressing human AXL with the Ig-like domain II being replaced by the Ig-like domain II of mouse AXL), TH1021-mFN1 (HEK293F cells transiently expressing human AXL with the FNIII-like domain I being replaced by the FNIII-like domain I of mouse AXL), TH1021-mFN2 (HEK293F cells transiently expressing human AXL with the FNIII-like domain II being replaced by the FNIII-like domain II of mouse AXL), and HEK293F cells (negative control which does not express AXL), respectively.

Samples from the LEE expression were added to the cells to allow binding to the various AXL constructs. Subsequently, binding of antibodies was detected using a fluorescent conjugate (Goat anti-Human IgG Fc gamma-DyLight649; Jackson ImmunoResearch). The samples were scanned using an Applied Biosystems 8200 Cellular Detection System (8200 CDS) and mean fluorescence was used as read-out. Samples were stated positive when counts were higher than 50 and counts × fluorescence was at least three times higher than the negative control.

Binding affinity of antibody 511

The affinity of one anti-AXL antibody (clone 511) was determined.

Affinity was determined using Bio-Layer Interferometry on a ForteBio OctetRED384. Anti-human Fc Capture (AHC) biosensors (ForteBio, Portsmouth, UK; cat no. 18-5064) were loaded for 150 s with hIgG (1 µg/mL) aiming at a loading response of 1 nm. After a baseline (150 s) the association (1000 s) and dissociation (2000 s) of AXLECDHis (as described in Example 1) was determined, using a concentration range of 10 µg/mL - 0.16 µg/mL (218 nM - 3 nM) with 2-fold dilution steps. For calculations, the theoretical molecular mass of AXLECDHis based on the amino acid sequence was used, i.e. 46 kDa. Experiments were carried out on an OctetRED384, while shaking at 1000 rpm and at 30°C. Each antibody was tested in three independent experiments.

Data was analyzed with ForteBio Data Analysis Software v7.0.3.1, using the 1:1 model and a global full fit with 1000 s association time and 1000 s dissociation time unless stated otherwise. A dissociation time of 1000 s (instead of the 2000 s dissociation time that was acquired) was used since this resulted in better fits. Data traces were corrected by subtraction of a reference curve (antibody without AXLECDHis), the Y-axis was aligned to the last 5 s of the baseline, and interstep correction as well as Savitzky-Golay filtering was applied.

The affinity ($K_D$) of clone 511 for AXL was $23*10^{-9}$M ($k_{on}$ $1.7*10^5$ 1/Ms and a $k_{dis}$ of $3.9*10^{-3}$1/s).

Duostatin-3 synthesis.

Preparation of compound 3:

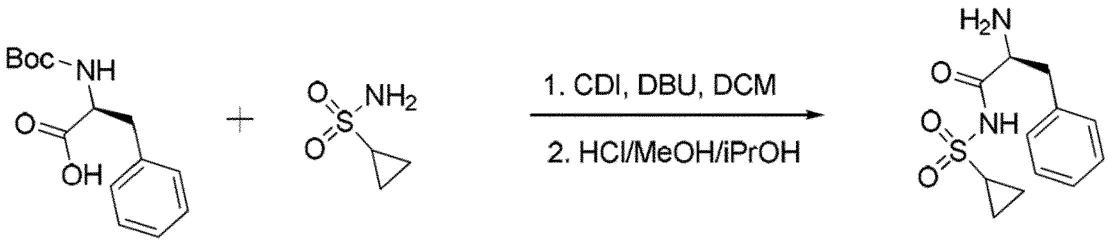


1 2 3

To a solution of Boc-L-phenylalanine 1 (5.36 g *et al.,* 20.2 mmol) in 30 mL of methylene chloride (DCM), carbonyldiimidazole (CDI, 4.26 g, 26.3 mmol) was added and stirred for 1 hour. Then added a solution of 2 (3.67 g, 30.3 mmol) and 2,4-diaminobutyric acid (DBU, 4.5 mL, 30 mmol) in 15 mL of DCM. The mixture was heated at 40°C for 16 hours. The mixture was diluted with 60 mL of DCM and 40 mL of water, then neutralized to pH 7 with conc. HCl. The DCM extract was collected, washed with 0.2M HCl (60 mL), then with brine (60 mL), dried over Na2SO4, and evaporated to give 7.47 g of Boc protected sulfonamide. This material was suspended in 40 mL of methanol, then 200 mL of 6N HCl/isopropanol was added and the mixture was stirred for 2 hours. The solvent was evaporated under vacuum, 100 mL of ether was then added. The precipitate was collected by filtration and dried to give compound 3 as HCl salt (5.93 g, 96%); MS m/z 269.1 (M+H).

Preparation of compound 5:

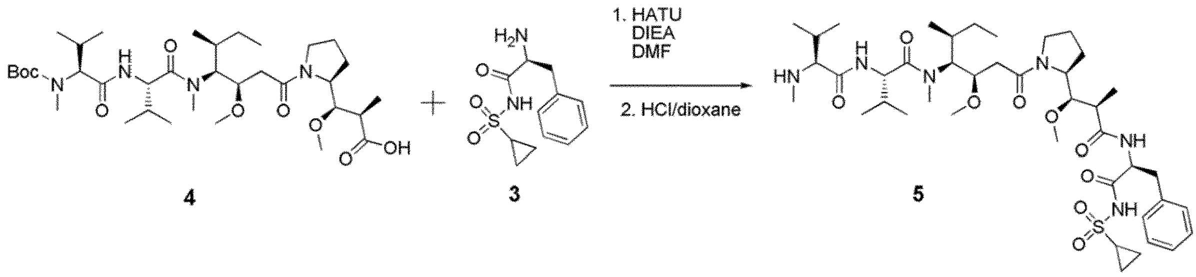


4 3 5

To a solution of compound 4 (1.09 g, 1.6 mmol) in 10 mL of N,N-Dimethylformamide (DMF) was added 2-(IH-7-azabenzotriazol-I-yl)-I,I ,3,3-tetramethyl uranium hexafluorophosphate (HATU, 0.61 g, 1.6 mmol), diisopropylethylamine (DIEA, 0.56 mL), and compound 3 (0.49 g, 1.6 mmol) in that order. The mixture was stirred for 1 hour and diluted with 100 mL of water and 4 mL of acetic acid. The precipitate was collected by filtration, dried under vacuum and added to 10 mL of 4M HCl/dioxane. After 30 min *et al.,* 200 mL of ether was added and insoluble precipitate was collected and purified by HPLC to give compound 5 as tetrahydrofuran salt (TFA, 1.3 g, 88%); MS m/z 835.5 (M+H). Compound 5 is referred to as duostatin-3 throughout the manuscript.

Preparation of compound 7:

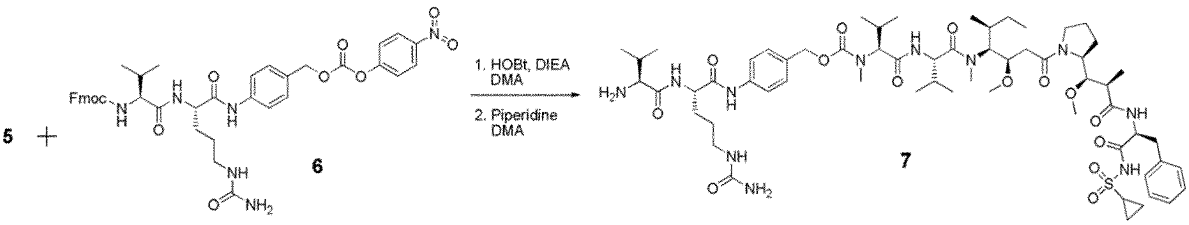


5 + 6 7

To a solution of compound 5 (500 mg, 0.527 mmol) in 5 mL of DMF was added compound 6 (483 mg, 0.631 mmol), N-Hydroxybenzotriazole (HOBt, 40 mg, 0.296 mmol), and DIEA (0.27 mL). The mixture was stirred for 16 hours after which 0.4 mL of piperidine was added. After 1 hour, the mixture was diluted with 100 mL of ether and the precipitate was collected and dried to give compound 7 as HCl salt (640 mg, 95 %); MS m/z 1240.7 (M+H).

Preparation of compound 9:

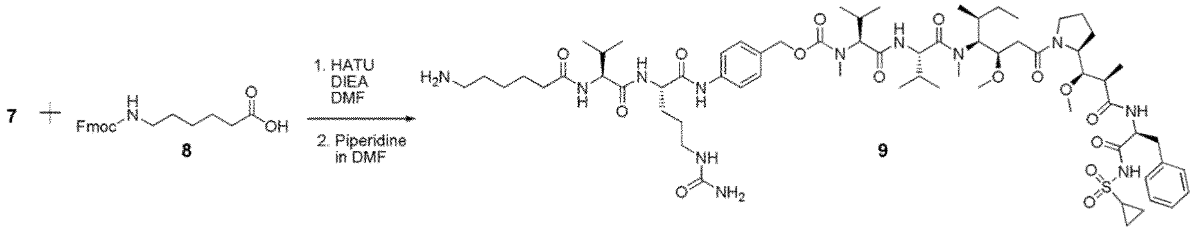


To a solution of compound 8 (219 mg, 0.62 mmol) in 5 mL of DMF was added HATU (236 mg, 0.62 mmol), DIEA (0.15 mL), and compound 7 (316 mg, 1.6 mmol), respectively. After 1 hour, 0.2 mL of piperidine was added and the mixture was stirred for 30 min, then purified by HPLC to give compound 9 as TFA salt (235 mg, 64 %); MS m/z 1353.8 (M+H).

Preparation of compound 11:

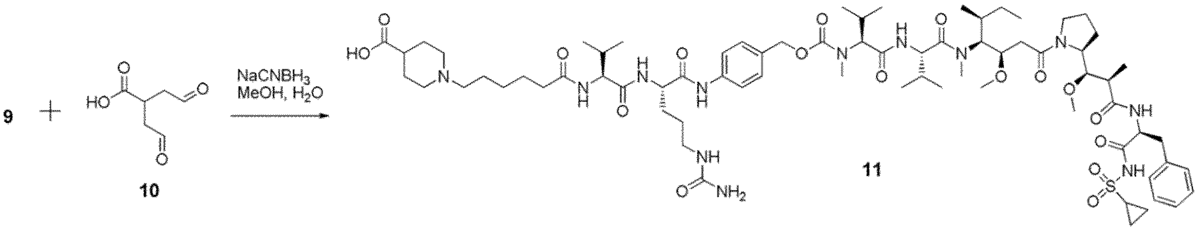


To a solution of compound 9 (235 mg, 0.16 mmol) in 2 mL of methanol and 1 mL of water was added a solution of dialdehyde 10 (1.6 mL of 0.3M in iPrOH) and NaCNBH3 (180 mg, 2.85 mmol). The mixture was stirred for 2 hours at RT, and then purified by HPLC giving rise to compound 11 as TFA salt (126 mg, 50 %); MS m/z 1465.8 (M+H)

Generation of AXL-specific antibody-drug conjugates (ADC)

Purified AXL antibodies IgG1-AXL-148, IgG1-AXL-183 and IgG1-AXL-726 as well as the negative control antibody IgG1-b12 were conjugated with Duostatin-3 by Concortis Biosystems, Inc. (San Diego, CA) through covalent conjugation using the K-lock AV1-valine-citruline (vc) linker (WO 2013/173391, WO 2013/173392 and WO 2013/173393 by Concortis Biosystems).
The anti-AXL antibody drug conjugates were subsequently analyzed for concentration (by absorbance at 280 nm), the drug to antibody ratio (the 'DAR') by reverse phase chromatography (RP-HPLC) and hydrophobic interaction chromatography (HIC), the amount of unconjugated drug (by reverse phase chromatography), the percentage aggregation (by size-exclusion chromatography, SEC-HPLC) and the endotoxin levels (by LAL). The results were as follows (Table 3):

*Table 3*

| | IgG1-AXL-148-vcDuostatin3 | IgG1-AXL-183-vcDuostatin3 | IgG1-AXL-726-vcDuostatin3 | IgG1-b12-vcDuostatin3 |
|---|---|---|---|---|
| Concentration (mg/mL) | 6.57 | 3.40 | 5.93 | 3.36 |
| DAR by HIC-HPLC | 1.71 | 1.79 | 1.77 | 2.05 |
| % unconjugated drug | 6.67 | 4.16 | 5.38 | 4.19 |
| % aggregate by SEC-HPLC | 3.71% | 3.35 | 3.42 | 1.75 |

*Example 2 - Binding characteristics of AXL antibodies*

Binding affinity of AXL antibodies

The affinities of the panel of 9 anti-AXL antibodies as well as 3 variants of these antibodies with single amino acid mutations in the variable domains (IgG1-AXL-154-M103L, IgG1-AXL-183-N52Q, IgG1-AXL-726-M101L), were determined.

Affinities were determined using Bio-Layer Interferometry on a ForteBio OctetRED384. Anti-human Fc Capture (AHC) biosensors (ForteBio, Portsmouth, UK; cat no. 18-5064) were loaded for 150 s with hIgG (1 μg/mL) aiming at a loading response of 1 nm. After a baseline (150 s) the association (1000 s) and dissociation (2000 s) of AXLECDHis (as described in Example 1) was determined, using a concentration range of 10 μg/mL - 0.16 μg/mL (218 nM - 3 nM) with 2-fold dilution steps. For calculations, the theoretical molecular mass of AXLECDHis based on the amino acid sequence was used, i.e. 46 kDa. Experiments were carried out on an OctetRED384, while shaking at 1000 rpm and at 30°C. Each antibody was tested in three independent experiments.

Data was analyzed with ForteBio Data Analysis Software v7.0.3.1, using the 1:1 model and a global full fit with 1000 s association time and 1000 s dissociation time unless stated otherwise. A dissociation time of 1000 s (instead of the 2000 s dissociation time that was acquired) was used since this resulted in better fits. For antibody IgG1-AXL-154 and IgG1-AXL-154-M103L a dissociation time of 500 s was used. For IgG1-AXL-012 and IgG1-AXL-094 dissociation times of 200 s were used. Data traces were corrected by subtraction of a reference curve (antibody without AXLECDHis), the Y-axis was aligned to the last 5 s of the baseline, and interstep correction as well as Savitzky-Golay filtering was applied.

The affinities ($K_D$) of the anti-AXL antibodies ranged from $0.3*10^{-9}$M to $63*10^{-9}$M (Table 4). For mutant IgG1-AXL-183-N52Q the $K_D$ was lower than for wild-type IgG1-AXL-183, due to an approximately 2.5-fold higher dissociation rate. The observed kinetics of the other two mutants were similar to the kinetics of the wild-type IgGs.

*Table 4*

| Antibody | Binding affinity (OCTET) | | |
|---|---|---|---|
| | KD (M) | Kon (1/Ms) | Kd is (1/s) |
| IgG1-AXL-107 | 16*10-9 | $2.8*10^5$ | $4.1*10^{-3}$ |
| IgG1-AXL-148 | $20*10^{-9}$ | $2.3*10^5$ | $4.4*10^{-3}$ |
| IgG1-AXL-154 | $7.2*10^{-9}$ | $2.6*10^5$ | 1.9*10-3 |
| IgG1-AXL-154-M103L | 7.8*10-9 | $2.7*10^5$ | 2.0*10-3 |
| IgG1-AXL-171 | 17*10-9 | $1.1*10^5$ | 1.8*10-3 |
| IgG1-AXL-183 | $10.2*10^{-9}$ | $4.1*10^4$ | $4.2*10^{-4}$ |
| IgG1-AXL-183-N52Q | $24*10^{-9}$ | $4.2*10^4$ | 1.0*10-3 |
| IgG1-AXL-613 | 1.5*10-9 | $5.4*10^5$ | $8.0*10^{-4}$ |
| IgG1-AXL-726 | 0.6*10-9 | $2.4*10^5$ | $1.3*10^{-4}$ |
| IgG1-AXL-726-M101L | 0.3*10-9 | $2.1*10^5$ | $6.9*10^{-5}$ |
| IgG1-AXL-733 | $63*10^{-9}$ | $1.6*10^5$ | $9.7*10^{-3}$ |

Binding of AXL antibodies to human, mouse and cynomolgus AXL

HEK293T cells were transiently transfected with expression constructs for full length human AXL, human AXL with a cynomolgus monkey extracellular domain (ECD) or human AXL with a mouse ECD (see Example 1). Binding of HuMab-AXL antibodies to these cells was evaluated by flow cytometry. Transfected HEK293 cells were incubated with serial dilutions of AXL-antibodies (final concentration range 0.0024-10 μg/mL) for 30 minutes at 4ºC. After washing three times in PBS/0.1% BSA/0.02% azide, cells were incubated with R-Phycoerythrin (PE)-conjugated goat-anti-human IgG F(ab')2 (Jackson ImmunoResearch Laboratories, Inc., West Grove, PA; cat. No. 109-116-098) diluted 1/100 in PBS/0.1% BSA/0.02% azide (final volume 100 μL). Next, cells were washed twice in PBS/0.1% BSA/0.02% azide, resuspended in 120 μL PBS/0.1% BSA/0.02% azide and analyzed on a FACS CantoII (BD Biosciences).

Binding curves were analyzed using non-linear regression (sigmoidal dose-response with variable slope) using GraphPad Prism V5.04 software (GraphPad Software, San Diego, CA, USA).

**Figure 1A** shows that the HuMab-AXL antibodies showed dose-dependent binding to the HEK293 cells

expressing human AXL-ECD. Furthermore, HuMab-AXL antibodies recognized AXL with a cynomolgus monkey ECD, with $EC_{50}$ values in the same range as for fully human AXL (**Figure 1B).** In contrast, binding of HuMabs to AXL with a mouse ECD was low (IgG1-AXL-107, IgG1-AXL-154, IgG1-AXL-154-M103L, IgG1-AXL-733, IgG1-AXL-183, IgG1-AXL-183-N52Q) or not detectable (IgG1-AXL-171, IgG1-AXL-613, IgG1-AXL-726, IgG1-AXL-726-M101L, IgG1-AXL-148; **Figure 1C**). As expected, the negative control antibody IgG1-b12 showed (**Figure 1**) no binding to cells expressing any of the AXL variants. Table 5 shows the EC50 values and standard deviations for binding of the anti-AXL antibodies to human AXL or human AXL with a cynomolgus AXL ECD (determined in at least 3 experiments). EC50 values for binding to human AXL with a mouse AXL ECD could not be determined to very low or absent binding.

*Table 5*

| Antibody | Binding EC50 (μg/mL) | |
|---|---|---|
| | human AXL Average (s.d.) | cynomolgus AXL Average (s.d.) |
| IgG1-AXL-107 | 0.050 (0.004) | 0.149 (0.021) |
| IgG1-AXL-154 | 0.105 (0.003) | 0.160 (0.027) |
| IgG1-AXL-154-M103L | 0.110 (0.038) | 0.161 (0.042) |
| IgG1-AXL-171 | 0.073 (0.023) | 0.157 (0.057) |
| IgG1-AXL-613 | 0.040 (0.023) | 0.146 (0.023) |
| IgG 1-AXL-726 | 0.288 (0.206) | 0.349 (0.160) |
| IgG1-AXL-726-M101L | 0.184 (0.117) | 0.250 (0.066) |
| IgG1-AXL-733 | 0.176 (0.094) | 0.254 (0.114) |
| IgG1-AXL-148 | 0.094 (0.059) | 0.152 (0.080) |
| IgG1-AXL-183 | 0.526 (0.177) | 0.309 (0.086) |
| IgG1-AXL-183-N52Q | 0.350 (0.206) | 0.324 (0.121) |

Competition between AXL antibodies and Gas6 for AXL binding

It was tested whether the AXL ligand Gas6 interfered with binding of the AXL antibodies to AXL. Therefore, AXL-positive A431 cells were incubated for 15 minutes at 4°C with 10 μg/mL recombinant human Gas6 (R&D Systems, Abingdon, UK; cat. No. 885-GS). Subsequently, serial dilutions of AXL antibodies were prepared (final concentration range 0.014-10 μg/mL), added to the cells and incubated for 30 minutes at 4ºC. After washing three times in PBS/0.1% BSA/0.02% azide, cells were incubated in 100 μL with secondary antibody at 4°C for 30 min in the dark. As a secondary antibody binding the Fc region, R-Phycoerythrin (PE)-conjugated goat-anti-human IgG F(ab')2 (Jackson ImmunoResearch Laboratories, Inc., West Grove, PA; cat. No. 109-116-098) diluted 1/100 in PBS/0.1% BSA/0.02% azide, was used. Next, cells were washed twice in PBS/0.1% BSA/0.02% azide, resuspended in 120 μL PBS/0.1% BSA/0.02% azide and analyzed on a FACS Cantoll (BD Biosciences).

Alternatively, A431 cells were pre-incubated with 10 μg/mL AXL antibodies (15 minutes, 4°C) to assess if the AXL ligand Gas6 could still bind in presence of AXL antibodies. After antibody pre-incubation, serial dilutions of recombinant human Gas6 (R&D Systems, Abingdon, UK; cat. No. 885-GS) were added to the cells at final concentrations of 0.001-20 μg/mL and incubated for 30 minutes at 4ºC. After washing three times in PBS/0.1% BSA/0.02% azide, cells were incubated with mouse anti-Gas6 IgG2a (R&D Systems; cat no. MAB885) at 4°C for 30 min. After washing three times in PBS/0.1% BSA/0.02% azide, cells were incubated with FITC-labelled goat anti-mouse IgG (Dako, Heverlee, Belgium; cat no. F049702) at 4°C for 30 min in the dark. Next, cells were washed twice in PBS/0.1% BSA/0.02% azide, resuspended in 120 μL PBS/0.1% BSA/0.02% azide and analyzed on a FACS Cantoll (BD Biosciences).

Binding curves were analyzed using non-linear regression (sigmoidal dose-response with variable slope) using GraphPad Prism V5.04 software (GraphPad Software, San Diego, CA, USA).

In experiments (n=3) in which A431 cells were pre-incubated with Gas6, the maximal binding values of anti-AXL antibodies was comparable to antibody binding in absence of Gas6 (maximal binding after Gas6 pre-incubation was 90-108% of binding without Gas6 pre-incubation) (Table 6). The $EC_{50}$ values for AXL antibody binding with or without Gas6 pre-incubation were in the same range, or somewhat enhanced after Gas6 pre-incubation (Table 6).

The binding of control AXL antibody YW327.6S2 to A431 cells was greatly reduced in the presence of Gas6 compared to binding without Gas. Maximal binding of YW327.6S2 in the presence of Gas6 was 19% of binding without Gas6, and the EC50 value for binding to A431 cells was 21-fold higher when cells had been pre-incubated with Gas6.

In experiments in which A431 cells were pre-incubated with anti-AXL antibodies, Gas6 binding was evaluated (n=3). Binding of Gas6 to A431 cells was similar with or without pre-incubation with HuMab-AXL antibodies. Average EC50 concentrations of Gas6 binding when cells were pre-incubated with HuMabs (0.34-0.83 μg/mL) and maximal Gas6 binding were similar to Gas6 binding in the presence of negative control antibody b12 (EC50 concentration: 0.40 μg/mL; 95-115% of Gas6 binding in the presence of the b12 control antibody). The binding of Gas6 to A431 cells was greatly reduced in the presence of control AXL antibody YW327.6S2 compared to pre-incubation with b12 (the EC50 concentration was 14-fold higher). Maximal binding of Gas6 in the presence of control antibody YW327.6S2 was 17% of binding in the presence of negative control antibody b12.

*Table 6*

| Antibody | Antibody binding to A431 cells | | | Gas6 binding to A431 cells | |
|---|---|---|---|---|---|
| | EC50 w/o Gas6 EC50 (μg/mL) mean (s.d.) | EC50 in presence of Gas6 (μg/mL) mean (s.d.) | Maximal binding in presence of Gas6 (% of binding in absence of Gas6) mean (s.d.) | EC50 in presence of AXL antibodies (μg/mL) mean (s.d.) | Maximal binding in presence of AXL antibodies (% of binding in prescence of control antibody) mean (s.d.) |
| IgG1-AXL-107 | 0.16 (0.17) | 0.94 (1.18) | 91 (5) | 0.78 (0.54) | 96 (8) |
| IgG1-AXL-148 | 0.11 (0.13) | 0.20 (0.30) | 93 (5) | 0.73 (0.52) | 106 (7) |
| IgG1-AXL-154 | 0.42 (0.55) | 0.76 (0.78) | 99 (13) | 0.44 (0.28) | 95 (10) |
| IgG1-AXL-171 | 0.18 (0.21) | 0.32 (0.40) | 95 (5) | 0.69 (0.42) | 108 (5) |
| IgG1-AXL-183 | 0.69 (0.72) | 1.19 (1.11) | 90 (19) | 0.34 (0.13) | 115 (8) |
| IgG1-AXL-511 | 0.12 (0.11) | 0.30 (0.31) | 93 (15) | 0.74 (0.44) | 113 (6) |
| IgG1-AXL-613 | 0.09 (0.09) | 0.10 (0.10) | 108 (22) | 0.57 (0.36) | 100 (11) |
| IgG1-AXL-726 | 0.32 (0.35) | 0.55 (0.69) | 97 (10) | 0.77 (0.58) | 98 (10) |
| IgG1-AXL-733 | 0.49 (0.51) | 0.62 (0.23) | 93 (5) | 0.83 (0.54) | 96 (5) |
| YW327.6S2 | 0.09 (0.09) | 1.90 (1.04) * | 41 (24) | 5.53 (7.09)* | 17 (10) |
| b12 | n.a.[a] | n.a. | n.a. | 0.40 (0.11) | 100 |

[a] n.a., not applicable
* EC50 values less accurate due to low binding.

*Example 3 - Epitope mapping studies anti-AXL antibody panel*

Determining the AXL domain specificity using human-mouse AXL chimeric molecules

The AXL domain specificity of the AXL antibodies was determined using a panel of human-mouse chimeric AXL mutants. Five different chimeric AXL molecules were generated, in which either the human Ig-like domain I (Ig1), the Ig-like domain II (Ig2), the human FNIII-like domain I (FN1) or the human FNIII-like domain II domain (FN2) were replaced with their murine homologs.

The following codon-optimized constructs for expression of the AXL human-mouse chimeras were generated and expressed in HEK293F cells as described in Example 1:

***Homo sapiens* AXL (p33-HAHs-AXL):** (SEQ ID NO:148)

MAWRCPRMGRVPLAWCLALCGWACMYPYDVPDYAAPRGTQAEESPFVGNPGNITGARGLTG
TLRCQLQVQGEPPEVHWLRDGQILELADSTQTQVPLGEDEQDDWIVVSQLRITSLQLSDTGQYQCLVFLGHQTFV
SQPGYVGLEGLPYFLEEPEDRTVAANTPFNLSCQAQGPPEPVDLLWLQDAVPLATAPGHGPQRSLHVPGLNKTSS
FSCEAHNAKGVTTSRTATITVLPQQPRNLHLVSRQPTELEVAWTPGLSGIYPLTHCTLQAVLSNDGMGIQAGEPDP
PEEPLTSQASVPPHQLRLGSLHPHTPYHIRVACTSSQGPSSWTHWLPVETPEGVPLGPPENISATRNGSQAFVHW
QEPRAPLQGTLLGYRLAYQGQDTPEVLMDIGLRQEVTLELQGDGSVSNLTVCVAAYTAAGDGPWSLPVPLEAWR
PGQAQPVHQLVKEPSTPAFSWPWWYVLLGAVVAAACVLILALFLVHRRKKETRYGEVFEPTVERGELVVRYRVRK
SYSRRTTEATLNSLGISEELKEKLRDVMVDRHKVALGKTLGEGEFGAVMEGQLNQDDSILKVAVKTMKIAICTRSEL
EDFLSEAVCMKEFDHPNVMRLIGVCFQGSERESFPAPVVILPFMKHGDLHSFLLYSRLGDQPVYLPTQMLVKFMA
DIASGMEYLSTKRFIHRDLAARNCMLNENMSVCVADFGLSKKIYNGDYYRQGRIAKMPVKWIAIESLADRVYTSKS
DVWSFGVTMWEIATRGQTPYPGVENSEIYDYLRQGNRLKQPADCLDGLYALMSRCWELNPQDRPSFTELREDLE
NTLKALPPAQEPDEILYVNMDEGGGYPEPPGAAGGADPPTQPDPKDSCSCLTAAEVHPAGRYVLCPSTTPSPAQP
ADRGSPAAPGQEDGA

***Mus musculus* AXL (p33-HAMm-AXL):** (SEQ ID NO:149)

MAWRCPRMGRVPLAWCLALCGWACMYPYDVPDYAAHKDTQTEAGSPFVGNPGNITGARGL
TGTLRCELQVQGEPPEVVWLRDGQILELADNTQTQVPLGEDWQDEWKVVSQLRISALQLSDAGEYQCMVHLEG
RTFVSQPGFVGLEGLPYFLEEPEDKAVPANTPFNLSCQAQGPPEPVTLLWLQDAVPLAPVTGHSSQHSLQTPGLNK
TSSFSCEAHNAKGVTTSRTATITVLPQRPHHLHVVSRQPTELEVAWTPGLSGIYPLTHCNLQAVLSDDGVGIWLGK
SDPPEDPLTLQVSVPPHQLRLEKLLPHTPYHIRISCSSSQGPSPWTHWLPVETTEGVPLGPPENVSAMRNGSQVLV
RWQEPRVPLQGTLLGYRLAYRGQDTPEVLMDIGLTREVTLELRGDRPVANLTVSVTAYTSAGDGPWSLPVPLEPW
RPGQGQPLHHLVSEPPPRAFSWPWWYVLLGAVVAAACVLILALFLVHRRKKETRYGEVFEPTVERGELVVRYRVR
KSYSRRTTEATLNSLGISEELKEKLRDVMVDRHKVALGKTLGEGEFGAVMEGQLNQDDSILKVAVKTMKIAICTRSE
LEDFLSEAVCMKEFDHPNVMRLIGVCFQGSERESFPAPVVILPFMKHGDLHSFLLYSRLGDQPVYLPTQMLVKFM
ADIASGMEYLSTKRFIHRDLAARNCMLNENMSVCVADFGLSKKIYNGDYYRQGRIAKMPVKWIAIESLADRVYTSK
SDVWSFGVTMWEIATRGQTPYPGVENSEIYDYLRQGNRLKQPADCLDGLYALMSRCWELNPQDRPSFTELREDL
ENTLKALPPAQEPDEILYVNMDEGGGYPEPPGAAGGADPPTQPDPKDSCSCLTAAEVHPAGRYVLCPSTTPSPAQ
PADRGSPAAPGQEDGA

***Homo sapiens* AXL - *Mus musculus* Ig1 domain (p33-AXL-mIgI):** (SEQ ID NO:150)

MGRVPLAWWLALCCWGCAAHKDTQTEAGSPFVGNPGNITGARGLTGTLRCELQVQGEPPEVV
WLRDGQILELADNTQTQVPLGEDWQDEWKVVSQLRISALQLSDAGEYQCMVHLEGRTFVSQPGFVGLEGLPYFL
EEPEDRTVAANTPFNLSCQAQGPPEPVDLLWLQDAVPLATAPGHGPQRSLHVPGLNKTSSFSCEAHNAKGVTTSR
TATITVLPQQPRNLHLVSRQPTELEVAWTPGLSGIYPLTHCTLQAVLSDDGMGIQAGEPDPPEEPLTSQASVPPHQ
LRLGSLHPHTPYHIRVACTSSQGPSSWTHWLPVETPEGVPLGPPENISATRNGSQAFVHWQEPRAPLQGTLLGYR
LAYQGQDTPEVLMDIGLRQEVTLELQGDGSVSNLTVCVAAYTAAGDGPWSLPVPLEAWRPGQAQPVHQLVKEP
STPAFSWPWWYVLLGAVVAAACVLILALFLVHRRKKETRYGEVFEPTVERGELVVRYRVRKSYSRRTTEATLNSLGIS
EELKEKLRDVMVDRHKVALGKTLGEGEFGAVMEGQLNQDDSILKVAVKTMKIAICTRSELEDFLSEAVCMKEFDHP
NVMRLIGVCFQGSERESFPAPVVILPFMKHGDLHSFLLYSRLGDQPVYLPTQMLVKFMADIASGMEYLSTKRFIHR
DLAARNCMLNENMSVCVADFGLSKKIYNGDYYRQGRIAKMPVKWIAIESLADRVYTSKSDVWSFGVTMWEIATR
GQTPYPGVENSEIYDYLRQGNRLKQPADCLDGLYALMSRCWELNPQDRPSFTELREDLENTLKALPPAQEPDEILY
VNMDEGGGYPEPPGAAGGADPPTQPDPKDSCSCLTAAEVHPAGRYVLCPSTTPSPAQPADRGSPAAPGQEDGA

***Homo sapiens* AXL - *Mus musculus* Ig2 domain (p33-AXL-mig2):** (SEQ ID NO:151)

MAWRCPRMGRVPLAWCLALCGWACMAPRGTQAEESPFVGNPGNITGARGLTGTLRCQLQV
QGEPPEVHWLRDGQILELADSTQTQVPLGEDEQDDWIVVSQLRITSLQLSDTGQYQCLVFLGHQTFVSQPGYVGL
EGLPYFLEEPEDKAVPANTPFNLSCQAQGPPEPVTLLWLQDAVPLAPVTGHSSQHSLQTPGLNKTSSFSCEAHNAK
GVTTSRTATITVLPQQPRNLHLVSRQPTELEVAWTPGLSGIYPLTHCTLQAVLSDDGMGIQAGEPDPPEEPLTSQAS
VPPHQLRLGSLHPHTPYHIRVACTSSQGPSSWTHWLPVETPEGVPLGPPENISATRNGSQAFVHWQEPRAPLQGT
LLGYRLAYQGQDTPEVLMDIGLRQEVTLELQGDGSVSNLTVCVAAYTAAGDGPWSLPVPLEAWRPGQAQPVHQ
LVKEPSTPAFSWPWWYVLLGAVVAAACVLILALFLVHRRKKETRYGEVFEPTVERGELVVRYRVRKSYSRRTTEATL
NSLGISEELKEKLRDVMVDRHKVALGKTLGEGEFGAVMEGQLNQDDS

ILKVAVKTMKIAICTRSELEDFLSEAVCMKEFDHPNVMRLIGVCFQGSERESFPAPVVILPFMKHG
DLHSFLLYSRLGDQPVYLPTQMLVKFMADIASGMEYLSTKRFIHRDLAARNCMLNENMSVCVADFGLSKKIYNGD
YYRQGRIAKMPVKWIAIESLADRVYTSKSDVWSFGVTMWEIATRGQTPYPGVENSEIYDYLRQGNRLKQPADCLD
GLYALMSRCWELNPQDRPSFTELREDLENTLKALPPAQEPDEILYVNMDEGGGYPEPPGAAGGADPPTQPDPKD
SCSCLTAAEVHPAGRYVLCPSTTPSPAQPADRGSPAAPGQEDGA

***Homo sapiens* AXL - *Mus musculus* FN1 domain (p33-AXL-mFN1):** (SEQ ID NO:152)

MAWRCPRMGRVPLAWCLALCGWACMAPRGTQAEESPFVGNPGNITGARGLTGTLRCQLQVQGEPPEVHWLR
DGQILELADSTQTQVPLGEDEQDDWIVVSQLRITSLQLSDTGQYQCLVFLGHQTFVSQPGYVGLEGLPYFLEEPED
RTVAANTPFNLSCQAQGPPEPVDLLWLQDAVPLATAPGHGPQRSLHVPGLNKTSSFSCEAHNAKGVTTSRTATIT
VLPQRPHHLHVVSRQPTELEVAWTPGLSGIYPLTHCNLQAVLSDDGVGIWLGKSDPPEDPLTLQVSVPPHQLRLEK
LLPHTPYHIRISCSSSQGPSPWTHWLPVETTEGVPLGPPENISATRNGSQAFVHWQEPRAPLQGTLLGYRLAYQGQ
DTPEVLMDIGLRQEVTLELQGDGSVSNLTVCVAAYTAAGDGPWSLPVPLEAWRPGQAQPVHQLVKEPSTPAFS
WPWWYVLLGAVVAAACVLILALFLVHRRKKETRYGEVFEPTVERGELVVRYRVRKSYSRRTTEATLNSLGISEELKEK
LRDVMVDRHKVALGKTLGEGEFGAVMEGQLNQDDSILKVAVKTMKIAICTRSELEDFLSEAVCMKEFDHPNVMR
LIGVCFQGSERESFPAPVVILPFMKHGDLHSFLLYSRLGDQPVYLPTQMLVKFMADIASGMEYLSTKRFIHRDLAAR
NCMLNENMSVCVADFGLSKKIYNGDYYRQGRIAKMPVKWIAIESLADRVYTSKSDVWSFGVTMWEIATRGQTPY
PGVENSEIYDYLRQGNRLKQPADCLDGLYALMSRCWELNPQDRPSFTELREDLENTLKALPPAQEPDEILYVNMDE
GGGYPEPPGAAGGADPPTQPDPKDSCSCLTAAEVHPAGRYVLCPSTTPSPAQPADRGSPAAPGQEDGA

***Homo sapiens* AXL - *Mus musculus* FN2 domain (p33-AXL-mFN2):** (SEQ ID NO:153)

MAWRCPRMGRVPLAWCLALCGWACMAPRGTQAEESPFVGNPGNITGARGLTGTLRCQLQV
QGEPPEVHWLRDGQILELADSTQTQVPLGEDEQDDWIVVSQLRITSLQLSDTGQYQCLVFLGHQTFVSQPGYVGL
EGLPYFLEEPEDRTVAANTPFNLSCQAQGPPEPVDLLWLQDAVPLATAPGHGPQRSLHVPGLNKTSSFSCEAHNA
KGVTTSRTATITVLPQQPRNLHLVSRQPTELEVAWTPGLSGIYPLTHCTLQAVLSDDGMGIQAGEPDPPEEPLTSQ
ASVPPHQLRLGSLHPHTPYHIRVACTSSQGPSSWTHWLPVETPEGVPLGPPENVSAMRNGSQVLVRWQEPRVPL
QGTLLGYRLAYRGQDTPEVLMDIGLTREVTLELRGDRPVANLTVSVTAYTSAGDGPWSLPVPLEPWRPGQGQPLH
HLVSEPPPRAFSWPWWYVLLGAVVAAACVLILALFLVHRRKKETRYGEVFEPTVERGELVVRYRVRKSYSRRTTEAT
LNSLGISEELKEKLRDVMVDRHKVALGKTLGEGEFGAVMEGQLNQDDSILKVAVKTMKIAICTRSELEDFLSEAVC
MKEFDHPNVMRLIGVCFQGSERESFPAPVVILPFMKHGDLHSFLLYSRLGDQPVYLPTQMLVKFMADIASGMEYL
STKRFIHRDLAARNCMLNENMSVCVADFGLSKKIYNGDYYRQGRIAKMPVKWIAIESLADRVYTSKSDVWSFGVT
MWEIATRGQTPYPGVENSEIYDYLRQGNRLKQPADCLDGLYALMSRCWELNPQDRPSFTELREDLENTLKALPPA
QEPDEILYVNMDEGGGYPEPPGAAGGADPPTQPDPKDSCSCLTAAEVHPAGRYVLCPSTTPSPAQPADRGSPAA
PGQEDGA

Binding of 1 μg/mL anti-AXL antibody to the human-mouse AXL chimeras was determined by flow cytometry, as described in Example 2. IgG1-b12 was included as an isotype control IgG1.

All anti-AXL antibodies showed binding to human AXL (**Figure 2A**), whereas binding was abrogated or strongly reduced when the human AXL ECD was replaced with its murine homolog (**Figure 2B**). The human-mouse cross-reactive monoclonal AXL antibody YW327.6S2 was included to confirm expression of hsAXL-mmECD.

Anti-AXL antibody 107 and 613 showed strongly reduced binding to hsAXL-mmIg1 (**Figure 2C**), indicating recognition of an epitope in the AXL Ig1 domain. IgG1-AXL-148 and IgG1-AXL-171 showed strongly reduced binding to hsAXL-mmlg2 (**Figure 2D**), indicating recognition of an epitope in the AXL Ig2 domain. IgG1-AXL-154, IgG1-AXL-183 and IgG1-AXL-733 showed reduced binding to hsAXL-mmFN1 (**Figure 2E**), indicative of a binding epitope in the AXL FN1 domain. Finally, binding of IgG1-AXL-726 was lost in hsAXL-mmFN2 (**Figure 2F**), indicating recognition of an epitope within the FN2 domain.

AXL domain specificity for all anti-AXL antibodies is summarized in Table 7.

*Table 7*

| Antibody | AXL domain specificity | AXL aa's involved in binding |
|---|---|---|
| IgG1-AXL-107 | Ig1 | L121-Q129 |
| IgG1-AXL-148 | Ig2 | D170-R190 |
| IgG1-AXL-154 | Fn1 | Q272-A287, G297-P301 |
| IgG1-AXL-154-M103L | n.d.[a] | n.d. |
| IgG1-AXL-171 | Ig2 | P170, T182-R190 |
| IgG1-AXL-183 | Fn1 | Not resolved |
| IgG1-AXL-183-N52Q | n.d. | n.d. |
| IgG1-AXL-613 | Ig1 | T112-Q124 |
| IgG1-AXL-726 | Fn2 | A359, R386, Q436-K439 |
| IgG1-AXL-726-M101L | n.d. | n.d. |
| IgG1-AXL-733 | Fn1 | Not resolved |
| IgG1-AXL-061 | Ig1 | I97-Q124 |
| IgG1-AXL-137 | Ig1 | Q57, E92-T105 |
| YW327.6S2 | Ig1 | G39-D59 |
| [a]n.d., not determined | | |

<u>High resolution epitope mapping to identify amino acids in the AXL extracellular domain involved in binding of AXL antibodies</u>

To identify amino acids in the AXL extracellular domain involved in binding of anti-AXL antibodies, a library of AXL sequence variants was generated by recombination of AXL sequences derived from species with variable levels of homology with the human AXL sequence in the extracellular domain. Briefly, an expression plasmid encoding human AXL (Hs) was mixed with cloning plasmids encoding *Mus musculus* (Mm), *Monodelphis domestica* (Md; opossum) *Anolis corolinensis* (Ac; lizard) and *Tetraodon nigroviridis* (Tn; pufferfish) AXL homologs or vice versa. A combination of two primers specific to either the cloning or the expression vector was used to perform a PCR amplifying the AXL extracellular domain (ECD) with abbreviated elongation time, forcing melting and reannealing of nascent DNA replication strands during PCR cycling. Full length ECD was amplified using a nested PCR, again specific to recombination products containing termini originating from both vectors.

Resulting AXL ECD PCR products were cloned into an expression vector creating full length AXL, and resulting plasmids were sequenced, ranked by maximal difference to the template vectors and selected to create a minimal ensemble with maximal differentiation power. Plasmids encoding AXL homologs from Hs, Mm, Md, Ac and Tn, four human/mouse chimeric plasmids encoding Hs AXL with murine Ig1, Ig2, Fn1 or Fn2 domains, and the sixteen most differentiating plasmids from the recombination library were transfected into HEK293-F cells according to the specifications supplied by the manufacturer (Life technologies). FACS binding data using 1 $\mu$g/mL anti-AXL antibodies were deconvoluted by scoring per amino acid if mutation did (+1) or did not (-1) correlate with loss of binding, after which a baseline correction and normalization to a scale of -5 to +5 was applied, resulting in an impact score per amino acid over the full ECD.

The deconvoluted binding data is summarized in Table 7 as the amino acids involved in binding. Antibodies whose binding sites could not be mapped to high resolution due to a lack of recombination events in the proximity of the binding site, are indicated as not resolved.

*Example 4 - Fc-mediated effector functions*

<u>Antibody-dependent cell-mediated cytotoxicity (ADCC)</u>

The ability of anti-AXL antibodies to induce ADCC of A431 epidermoid carcinoma cells was determined as explained below. As effector cells, peripheral blood mononuclear cells from healthy volunteers (UMC Utrecht, The Netherlands) were used.

*Labeling of target cells*

A431 cells were collected ($5\times10^6$ cells) in culture medium (RPMI 1640 culture medium supplemented with 10% fetal calf serum (FSC)), to which 100 μCi $^{51}$Cr (Chromium-51; Amersham Biosciences Europe GmbH, Roosendaal, The Netherlands) had been added, and the mixture was incubated in a 37°C water bath for 1 hour (hr) while shaking. After washing of the cells (twice in PBS, 1200 rpm, 5 min), the cells were resuspended in RPMI1640/10% FSC and counted by trypan blue exclusion. Cells were diluted to a density of $1\times10^5$ cells/mL.

*Preparation of effector cells*

Peripheral blood mononuclear cells (healthy volunteers, UMC Utrecht, Utrecht, The Netherlands) were isolated from 45 mL of freshly drawn heparin blood by Ficoll (Bio Whittaker; lymphocyte separation medium, cat 17-829E) according to the manufacturer's instructions. After resuspension of cells in RPMI1640/10% FSC, cells were counted by trypan blue exclusion and diluted to a density of $1\times10^7$ cells/mL.

*ADCC set up*

50 μl of $^{51}$Cr-labeled targets cells were pipetted into 96-well plates, and 50 μl of antibody were added, diluted in RPMI1640/10% FSC (3-fold dilutions at final concentrations range 0.01-10 μg/mL). Cells were incubated (room temperature (RT), 15 min), and 50 μl effector cells were added, resulting in an effector to target ratio of 100:1 (for determination of maximal lysis, 100 μl 5% Triton-X100 was added instead of effector cells; for determination of spontaneous lysis, 50 μL target cells and 100 μL RPMI1640/10% FSC were used). Cells were incubated overnight at 37°C and 5% $CO_2$. After spinning down cells (1200 rpm, 10 min), 70 μL of supernatant was harvested into micronic tubes, and counted in a gamma counter. The percentage specific lysis was calculated as follows:

% specific lysis = (cpm sample- cpm target cells only)/(cpm maximal lysis - cpm target cells only), wherein cpm is counts per minute.

IgG1-AXL-183-N52Q, and IgG1-AXL-733 induced 15 to 21% ADCC in A431 cells at a concentration of 10 μg/mL (**Figure 3**). IgG1-AXL-148, IgG1-AXL-726-M101L, IgG1-AXL-171, IgG1-AXL-613, IgG1-AXL-107, and IgG1-AXL-154-M103L did not induce significant ADCC in A431 cell at concentrations up to 10 μg/mL (**Figure 3**).

*Example 5 - Binding characteristics of AXL antibody-drug conjugates (AXL-ADCs)*

HEK293T cells were transiently transfected with expression constructs for full-length human AXL (see Example 1). Binding of anti-AXL antibodies and AXL-ADCs to these cells was evaluated by flow cytometry. Transiently transfected HEK293 cells were incubated with serial dilutions of anti-AXL antibodies or AXL-ADCs (4-fold dilutions; final concentration range 0.003-10 μg/mL) for 30 minutes at 4ºC. After washing three times in PBS/0.1% BSA/0.02% azide, cells were incubated in 100 μL with secondary antibody at 4°C for 30 min in the dark. As a secondary antibody, R-Phycoerythrin (PE)-conjugated goat-anti-human IgG F(ab')2 (Jackson ImmunoResearch Laboratories, Inc., West Grove, PA; cat. No. 109-116-098) diluted 1/100 in PBS/0.1% BSA/0.02% azide, was used. Next, cells were washed twice in PBS/0.1% BSA/0.02% azide, resuspended in 120 μL PBS/0.1% BSA/0.02% azide and analyzed on a FACS Cantoll (BD Biosciences).

Binding curves were analyzed using non-linear regression (sigmoidal dose-response with variable slope) using GraphPad Prism V5.04 software (GraphPad Software, San Diego, CA, USA).

**Figure 4** shows that binding of the anti-AXL antibodies to the HEK293 cells expressing human AXL-ECD was similar to the binding of the AXL-ADCs.

*Example 6 - In vitro cytotoxicity induced by AXL-specific antibody drug conjugates*

LCLC-103H cells (human large cell lung cancer) cells were cultured in RPMI 1640 with L-Glutamine (Cambrex; cat.no. BE12-115F) supplemented with 10% (vol/vol) heat inactivated Cosmic Calf Serum (Perbio; cat.no. SH30087.03), 2 mM L-glutamine (Cambrex; cat.no. US17-905C), 50 IU/mL penicillin, and 50 μg/mL streptomycin (Cambrex; cat.no. DE17-603E). MDA-MB-231 cells (human breast cancer) were cultured in DMEM (Cambrex; cat.no. BE12-709F) supplemented with 10% (vol/vol) heat inactivated Cosmic Calf Serum (Perbio; cat.no. SH30087.03), 1 mM Sodium Pyruvate (Cambrex; cat.no. BE13-115E), 2 mM L-glutamine (Cambrex; cat.no. US17-905C), 100 μM MEM NEAA (Invitrogen; cat.no. 11140), 50 IU/mL penicillin, and 50 μg/mL streptomycin (Cambrex; cat.no. DE17-603E). The cell

lines were maintained at 37°C in a 5% (vol/vol) CO2 humidified incubator. LCLC-103H and MDA-MB-231 cells were cultured to near confluency, after which cells were trypsinized, resuspended in culture medium and passed through a cell strainer (BD Falcon, cat.no. 352340) to obtain a single cell suspension. $1\times10^3$ cells were seeded in each well of a 96-well culture plate, and cells were incubated for 30 min at room temperature and subsequently for 5 hrs at 37ºC, 5% CO2 to allow adherence to the plate.

Serial dilutions (4-fold; final concentrations ranging from 0.00015 to 10 μg/mL) of AXL antibody drug conjugates (AXL-ADCs; see Example 1) were prepared in culture medium and added to the plates. Incubation of cells with 1 μM staurosporin (#S6942-200, Sigma) was used as reference for 100% tumor cell kill. Untreated cells were used as reference for 0% tumor cell kill. Plates were incubated for 5 days at 37ºC, 5% CO2. Next, CellTiter-Glo Reagent (Promega; cat.no. G7571) was added to the wells (20 μL per well) and plates were incubated for 1.5 hours at 37ºC, 5% CO2. Subsequently, 180 μL per well was transferred to white 96-well Optiplate™ plates (PerkinElmer, Waltham, MA; cat.no. 6005299), which were incubated for 30 min at room temperature. Finally, luminescence was measured on an EnVision multiplate reader (Envision, Perkin Elmer).

AXL-ADCs IgG1-AXL-148-vcDuo3, IgG1-AXL-183-vcDuo3, and IgG1-AXL-726-vcDuo3 induced cytotoxicity in LCLC-103H cells, with IC50 values between 0.01 and 0.06 μg/mL, as shown in **Figure 5A.** Similarly, **Figure 5B** shows that these AXL-ADCs induced cytoxicity of MDA-MB-231 cells with IC50 values between 0.005 and 0.015 μg/mL.

*Example 7 - Antibody VH and VL variants that allow binding to AXL*

Protein sequences of the VH and VL regions of the anti-AXL antibody panel (described in Example 1) were aligned and compared for AXL binding to identify critical or permissive changes of amino acid residues in the VH or VL regions. Therefore, antibodies with identical VH or VL regions were grouped and compared for binding to human AXL and differences in VL or VH sequences, respectively. Binding to human AXL transiently expressed by HEK-293F cells was assessed in the homogeneous antigen specific screening assay as described in Example 1. Numbering of amino acid positions for the alignments done in the present example was done based on the sequences put forth in **Figure 6,** *i.e.* the first amino acid in the shown sequence was numbered as position '1', the second as position '2', etc.

First, antibodies with identical VL sequences were grouped.

IgG1-AXL-148 and IgG1-AXL-140 were found to have an identical VL sequence, and showed 1 amino acid difference in the HC CDR3 region (F for I at amino acid position 109; **Figure 6A**). Both antibodies bound to human AXL (Table 8), indicating that the amino acid at position 109 is not essential for antibody binding, assuming that a mutation identified in the CDR2 region (G for A at the amino acid position 56) does not compensate for loss of binding (**Figure 6A**).

IgG1-AXL-726 and IgG1-AXL-187 were found to have an identical VL sequence and both antibodies bound to human AXL (Table 8). Two amino acid residue changes in the HC CDR3 region (R for S at position 97 and A for T at position 105; **Figure 6B**) were allowed without losing binding, assuming that mutations identified in the CDR1 (Y for H at position 32) and/or in the framework regions (P3Q, V24I, Y25D, T86A and T117A) do not compensate for loss of binding (**figure 6B**).

IgG1-AXL-171, IgG1-AXL-172 and IgG1-AXL-181 were found to have an identical VL sequence and all antibodies bound to human AXL (Table 8). The CDR3 regions of these three antibodies were identical, but an amino acid residue change in the HC CDR1 (S for N at position 31) or the framework region (H for Q at position 82) was allowed without losing binding (**Figure 6C**).

IgG1-AXL-613, IgG1-AXL-608-01, IgG1-AXL-610-01 and IgG1-AXL-620-06 were found to have an identical VL sequence, and showed one amino acid difference in the HC CDR3 region (N for D at amino acid position 101; **Figure 6D**). All antibodies bound to human AXL (Table 8), indicating that the amino acid at position 101 is not essential, assuming that mutations identified in the HC CDR2 (V for A at position 58) and/or in the framework regions (N35S, M37V, A61V, L70I, S88A) do not compensate for loss of binding (**Figure 6D**).

Next, antibodies with identical VH sequences were grouped.

IgG1-AXL-613 and IgG1-AXL-613-08 were found to have an identical VH sequence, and showed five amino acid differences in the CDR3 region of the LC (RSNWL for YGSSY at positions 92 to 96; **Figure 6E**). Both antibodies bound to human AXL (Table 8), indicating that the variation of amino acid at positions 92 to 96 are allowed and do not affect antibody binding, assuming that mutations identified in the CDR1 (deletion of the S at position 30), CDR2 (G51D), and/or in the framework regions (G9A, S54N, R78S, Q100G, L104V) do not compensate for loss of binding (**Figure 6E**).

*Table 8*

| Antibody | EC50 (μg/mL) | Maximal binding (Arbitrary units) |
|---|---|---|
| IgG1-AXL-140 | 0.0026 | 2889 |
| IgG1-AXL-148 | 0.0036 | 3499 |

(continued)

| Antibody | EC50 (μg/mL) | Maximal binding (Arbitrary units) |
|---|---|---|
| IgG1-AXL-171 | 0.003 | 2575 |
| IgG1-AXL-172 | 0.0055 | 5378 |
| IgG1-AXL-181 | 0.008 | 3598 |
| IgG1-AXL-187 | 0.0065 | 2563 |
| IgG1-AXL-608-01 | 0.0035 | 3318 |
| IgG1-AXL-610-01 | 0.0023 | 2947 |
| IgG1-AXL-613 | 0.0072 | 5211 |
| IgG1-AXL-613-08 | 0.0242 | 2209 |
| IgG1-AXL-620-06 | 0.0034 | 4352 |
| IgG1-AXL-726 | 0.0471 | 3154 |

*Example 8 - Conjugation of MMAE to anti-AXL antibodies and In vitro cytotoxicity induced by MMAE-conjugated AXL antibodies*

Conjugation of MMAE to anti-AXL antibodies

Anti-AXL antibodies were purified by Protein A chromatography according to standard procedures and conjugated to vcMMAE. The drug-linker vcMMAE was alkylated to the cysteines of the reduced antibodies according to procedures described in the literature (see Sun *et al.,* 2005; McDonagh *et al.,* 2006; and Alley *et al.,* 2008). The reaction was quenched by the addition of an excess of N-acetylcysteine. Any residual unconjugated drug was removed by purification and the final anti-AXL antibody drug conjugates were formulated in PBS. The anti-AXL antibody drug conjugates were subsequently analyzed for concentration (by absorbance at 280 nm), the drug to antibody ratio (DAR) by reverse phase chromatography (RP-HPLC) and hydrophobic interaction chromatography (HIC), the amount of unconjugated drug (by reverse phase chromatography), the percentage aggregation (by size-exclusion chromatography, SEC-HPLC) and the endotoxin levels (by LAL). The results are shown below in **Table 9.**

*Table 9 - Overview of different characteristics of the antibody-drug conjugates.*

| | ADC, IgG1-AXL- | | | | | | | | | |
|---|---|---|---|---|---|---|---|---|---|---|
| Assay | 107 | 148 | 154-M103L | 171 | 183-N52Q | 511 | 613 | 726-M101L | 733 | IgG1-b12 |
| Concentration (mg/mL) | 7.18 | 9.63 | 6.57 | 3.69 | 6.71 | 5.77 | 6.17 | 7.37 | 7.71 | 1.58 |
| DAR by HIC | 3.97 | 3.96 | 3.71 | 3.65 | 3.92 | 3.87 | 4.23 | 4.12 | 4.08 | 4.00 |
| % unconjugated antibody | 4.68 | 5.58 | 6.13 | 7.11 | 8.68 | 8.35 | 5.13 | 4.99 | 3.74 | 1.89 |
| % aggregate by SEC-HPLC | 6.3 | 2.28 | 2.9 | 3.3 | 5.2 | 5.1 | 6.4 | 4.0 | 3.5 | 2.5 |
| Endotoxin (EU/mg) | 2.3 | 1.2 | 2.6 | 3.1 | 5.9 | 4.5 | 2.0 | 3.6 | 7.6 | 11.5 |

Cell culture

LCLC-103H cells (human large cell lung cancer) and A431 cells (DMSZ, Braunschweig, Germany) were cultured in RPMI 1640 with L-Glutamine (Cambrex; cat.no. BE12-115F) supplemented with 10% (vol/vol) heat inactivated Cosmic Calf Serum (Perbio; cat.no. SH30087.03), 2 mM L-glutamine (Cambrex; cat.no. US17-905C), 50 IU/mL penicillin, and 50 μg/mL streptomycin (Cambrex; cat.no. DE17-603E). MDA-MB231 cells were cultured in DMEM with high glucose and HEPES (Lonza #BE12-709F), Donor Bovine Serum with Iron (Life Technologies #10371-029), 2 mM L-glutamine (Lonza

#BE17 -605E), 1 mM Sodium Pyruvate (Lonza #BE13-115E), and MEM Non-Essential Amino Acids Solution (Life Technologies #11140). The cell lines were maintained at 37°C in a 5% (vol/vol) $CO_2$ humidified incubator. LCLC-103H, A431 and MDA-MB231 cells were cultured to near confluency, after which cells were trypsinized, resuspended in culture medium and passed through a cell strainer (BD Falcon, cat.no. 352340) to obtain a single cell suspension. $1\times10^3$ cells were seeded in each well of a 96-well culture plate, and cells were incubated for 30 min at room temperature and subsequently for 5 hrs at 37ºC, 5% $CO_2$ to allow adherence to the plate.

Cytotoxicity assay

Serial dilutions (final concentrations ranging from 0.00015 to 10 μg/mL) of MMAE-conjugated AXL-antibodies were prepared in culture medium and added to the plates. Incubation of cells with 1 μM staurosporin (#56942-200, Sigma) was used as reference for 100% tumor cell kill. Untreated cells were used as reference for 100% cell growth. Plates were incubated for 5 days at 37ºC, 5% $CO_2$. Next, CellTiter-Glo Reagent (Promega; cat.no. G7571) was added to the wells (20 μL per well) and plates were incubated for 1.5 hours at 37ºC, 5% $CO_2$. Subsequently, 180 μL per well was transferred to white 96-well Optiplate™ plates (PerkinElmer, Waltham, MA; cat.no. 6005299), which were incubated for 30 min at room temperature. Finally, luminescence was measured on an EnVision multiplate reader (Envision, Perkin Elmer).

MMAE-conjugated AXL-antibodies induced 50% cell kill in LCLC-103H cells at concentrations between 0.004 and 0.219 μg/mL as shown in **Table 10** and **Figure 7.**

Similarly, AXL-ADCs efficiently induced cytotoxicity in A431 cells (**Table 11**) and **Figure 15A**) and MDA-MB231 cells (**Table 11** and **Figure 15B**).

*Table 10 - Cytotoxicity of MMAE-conjugated -AXL-antibodies in LCLC-103H cells (EC50 values)*

| **ADC** | **EC50 (μg/mL)** |
|---|---|
| IgG1-AXL- 613-vcMMAE | 0.004 |
| IgG1-AXL- 148-vcMMAE | 0.012 |
| IgG1-AXL- 171-vcMMAE | 0.018 |
| IgG1-AXL- 726-M101L-vcMMAE | 0.018 |
| IgG1-AXL- 107-vcMMAE | 0.022 |
| IgG1-AXL- 511-vcMMAE | 0.032 |
| IgG1-AXL- 154-M103L-vcMMAE | 0.044 |
| IgG1-AXL- 183-N52Q-vcMMAE | 0.113 |
| IgG1-AXL- 733-vcMMAE | 0.219 |

*Table 11. Cytotoxicity of MMAE-conjugated AXL antibodies in A431 and MDA-MB-231 cells (EC50 values).*

| **ADC** | **EC50 (μg/mL)** | | | |
|---|---|---|---|---|
| | **A431 (n=3)** | | **MDA-MB231 (n=2)** | |
| | **Mean** | **s.d.** | **Mean** | **s.d.** |
| IgG1-AXL-107-vcMMAE* | 0.154 | 0.066 | 0.037 | 0.005 |
| IgG1-AXL-148-vcMMAE | 0.070 | 0.013 | 0.012 | 0.004 |
| IgG1-AXL-154-M103L-vcMMAE | 0.719 | 0.091 | 0.396 | 0.195 |
| IgG1-AXL-171-vcMMAE | 0.206 | 0.074 | 0.035 | 0.006 |
| IgG1-AXL-183-N52Q-vcMMAE | 1.157 | 0.160 | 0.139 | 0.028 |
| IgG1-AXL-511-vcMMAE | 0.093 | 0.020 | 0.052 | 0.003 |
| IgG1-AXL-613-vcMMAE | 0.109 | 0.078 | 0.005 | 0.001 |
| IgG1-AXL-726-M101L-vcMMAE | 0.270 | 0.157 | 0.022 | 0.002 |
| IgG1-AXL-733-vcMMAE | 1.253 | 0.228 | 0.881 | 0.182 |
| *IgG1-AXL-107-vcMMAE is also referred to as "HuMax-AXL-ADC" herein | | | | |

*Example 9 - Therapeutic treatment of LCLC-103H tumor xenografts in SCID mice with MMAE-conjugated anti-AXL antibodies*

The *in vivo* efficacy of MMAE-conjugated anti-AXL antibodies was determined in established subcutaneous (SC) LCLC-103H xenograft tumors in SCID mice. $5 \times 10^6$ LCLC-103H (large cell lung carcinoma) tumor cells (obtained from Leibniz-Institut DSMZ-Deutsche Sammlung von Mikroorganismen und Zellkulturen GmbH (DSMZ)) in 200 $\mu$L PBS were injected subcutaneously in the right flank of female SCID mice. Starting 14-21 days after tumor cell inoculation, when the average tumor size was >100-200 $mm^3$ and distinct tumor growth was observed, a single injection with 1 mg/kg (20 $\mu$g/mouse) IgG1-AXL-vcMMAE antibodies (as described in Supplementary Example 1) or control (unconjugated IgG1-b12) was given intraperitoneally (100 $\mu$L/mouse). Tumor volume was determined at least two times per week. Tumor volumes ($mm^3$) were calculated from caliper (PLEXX) measurements as: $0.52 \times (\text{length}) \times (\text{width})^2$.

The panel of anti-AXL-vcMMAE antibodies showed a broad range of anti-tumor activity in established SC LCLC-103H tumors (**Figure 8**). Clones IgG1-AXL-733-vcMMAE, IgG1-AXL-107-vcMMAE and IgG1-AXL-148-vcMMAE induced tumor regression, clones AXL-171-vcMMAE, IgG1-AXL-511-vcMMAE and IgG1-AXL-613-vcMMAE induced tumor growth inhibition, and clones IgG1-AXL-154-M103L-vcMMAE, IgG1-AXL-183-N52Q-vcMMAE, and IgG1-AXL-726-M101L-vcMMAE showed no or only minor tumor growth inhibition.

Statistical analysis on the last day that all groups were intact (day 30) using One Way ANOVA (Dunnett's multiple comparisons test versus control IgG1- b12) indicated a highly significant difference ($p<0.0001$) in tumor volume between IgG1-b12 versus IgG1-AXL-733-vcMMAE, IgG1-AXL-107-vcMMAE and IgG1-AXL-148-vcMMAE. Treatment with these clones led in some mice within these groups to complete tumor reduction. Treatment with clones IgG1-AXL-171-vcMMAE, IgG1-AXL-511-vcMMAE and IgG1-AXL-613-vcMMAE also showed significant tumor growth inhibition compared to IgG1-b12, but the differences were less pronounced ($p<0.05$ to $p<0.001$). The tumor growth of mice treated with clones IgG1-AXL-154-M103L-vcMMAE, IgG1-AXL-183-N52Q-vcMMAE, and IgG1-AXL-726-M101L-vcMMAE was not significant affected compared to the IgG1-b12 control.

Anti-tumor activity of anti-AXL-vcMMAE antibodies was observed in various other *in vivo* tumor models. In two cell line-derived xenograft models (A431; epidermoid adenocarcinoma, and MDA-MB-231; breast cancer) anti-AXL-vcMMAE antibodies induced tumor growth inhibition, and tumor regression was induced by anti-AXL-vcMMAE antibodies in two patient-derived xenograft models from patients with pancreas cancer and cervical cancer.

*Example 10 - Anti-tumor efficacy of AXL-ADCs in a pancreas cancer patient-derived xenograft (PDX) model with heterogeneous target expression*

The anti-tumor activity of IgG1-AXL-107-vcMMAE, IgG1-AXL-148-vcMMAE, and IgG1-AXL-733-vcMMAE was determined in the PAXF1657 pancreas cancer PDX model (experiments performed by Oncotest, Freiburg, Germany). Human pancreas tumor tissue was subcutaneously implanted in the left flank of 5-7 weeks old female NMRI nu/nu mice. Randomization of animals was performed as follows: animals bearing a tumor with a volume between 50 - 250 $mm^3$, preferably 80 - 200 $mm^3$, were distributed in 7 experimental groups (8 animals per group), considering a comparable median and mean of group tumor volume. At day of randomization (day 0), the 3 ADCs were dosed intravenously (i.v.) at either 4 mg/kg or 2 mg/kg, and the control group received a single dose of IgG1-b12 (4 mg/kg). Tumor volumes ($mm^3$) were monitored twice weekly and were calculated from caliper (PLEXX) measurements as: $0.52 \times (\text{length}) \times (\text{width})^2$.

Staining of PAXF1657 tumors was performed with standard immunohistochemistry techniques. Briefly, frozen tissues were fixated with acetone for 10 minutes and endogenous peroxidase was exhausted using hydrogen peroxidase. Subsequently, tissue sections were blocked with normal mouse serum and staining was performed by incubation with 5 $\mu$g/mL of a pool of 5 IgG1-AXL antibodies (IgG1-AXL-061, IgG1-AXL-137, IgG1-AXL-148, IgG1-AXL-183, IgG1-AXL-726). After incubation with the secondary, horseradish peroxidase (HRP) conjugated antibody, HRP was visualized with amino-ethyl carbazole (AEC; resulting in a red color). Each slide was counterstained with hematoxylin (blue) to identify nuclei and coverslipped in glycergel. Immunostained tissue slices were digitized on manual Zeiss microscope (AxioSkop) at 10x and 40x magnifications.

**Figure 9** shows heterogeneous AXL expression in PAXF1657 tumors. Whereas strong AXL staining is observed in some tumor cells, other cells do not show AXL staining. In black and white photo the AXL staining appears as dark grey. Hematoxylin staining (nuclei) appears as light grey.

**Figure 10A** shows that treatment of mice with 2 mg/kg IgG1-AXL-107-vcMMAE, IgG1-AXL-148-vcMMAE and IgG1-AXL-733-vcMMAE significantly reduced the growth of PAXF1657 tumors compared to the control group. At a dose of 4 mg/kg IgG1-AXL-107-vcMMAE, IgG1-AXL-148-vcMMAE and IgG1-AXL-733-vcMMAE induced tumor regression of PAXF1657 tumors. On day 14 after treatment, the average tumor size in mice that had been treated with 2mg/kg or 4 mg/kg IgG1-AXL-107-MMAE, IgG1-AXL-148-MMAE or IgG1-AXL-733-MMAE was significantly smaller than in mice that had been treated with an isotype control IgG (IgG1-b12) ($p<0.001$; Tukey's multiple comparison test).

Treatment of mice with unconjugated IgG1-AXL-148 did not result in anti-tumor activity in the PAXF1657 model

(**Figure 10B**). Conjugated IgG1-AXL-148-vcMMAE, however, induced dose-dependent antitumor activity in this model (**Figure 10B**), illustrating that the therapeutic capacity of AXL-ADCs is dependent on the cytotoxic activity of MMAE.

Moreover, treatment of mice with the untargeted ADC IgG1-b12-vcMMAE did not show anti-tumor activity in the PAXF1657 model (**Figure 10C**), illustrating that the therapeutic capacity of AXL-ADCs also depends on specific target binding.

*Example 11 - AXL antibodies binding to the Ig1 domain*

The AXL domain specificity of AXL antibodies IgG1-AXL-061, IgG1-AXL-107, IgG1-AXL-137, and IgG1-AXL-613 was determined using a panel of human-mouse chimeric AXL mutants. The human-mouse cross-reactive monoclonal AXL antibody YW327.6S2 was included to confirm expression of hsAXL-mmECD. IgG1-b12 was included as isotype control antibody. Five different chimeric AXL molecules were generated and expressed in HEK293F as described in Example 3. In brief, the human Ig-like domain I (Ig1), the Ig-like domain II (Ig2), the human FNIII-like domain I (FN1) or the human FNIII-like domain II domain (FN2) were replaced with their murine homologs. Binding of 1 μg/mL anti-AXL antibody to the human-mouse AXL chimeras was determined by flow cytometry, as described in Example 2.

All anti-AXL antibodies showed binding to human AXL (**Figure 11A**), whereas binding was abrogated when the human AXL ECD was replaced with its murine homolog (**Figure 11B**). As expected, the human-mouse cross-reactive monoclonal AXL antibody YW327.6S2 showed binding to hsAXL-mmECD, confirming proper expression of hsAXL-mmECD.

AXL antibodies IgG1-AXL-061, IgG1-AXL-107, IgG1-AXL-137, and IgG1-AXL-613 showed strongly reduced binding to hsAXL-mmlg1 (**Figure 11C**), illustrating recognition of an epitope in the AXL Ig1 domain. In line with this, binding of IgG1-AXL-061, IgG1-AXL-107, IgG1-AXL-137, and IgG1-AXL-613 to hsAXL-mmlg2 (**Figure 11D**), hsAXL-mmFN1 (**Figure 11E**) or hsAXL-mmFN2 (**Figure 11F**) was not affected. The human-mouse cross-reactive monoclonal AXL antibody YW327.6S2 showed binding to all chimeric AXL variants, confirming proper expression of these proteins.

*Example 12 - AXL antibodies IgG1-AXL-107 and IgG1-AXL-613 bind to the Ig1 domain but do not compete with Gas6 binding*

It was tested whether the binding of the AXL antibodies IgG1-AXL-061, IgG1-AXL-107, IgG1-AXL-137, or IgG1-AXL-613 interfered with binding of AXL ligand Gas6 to AXL. Therefore, binding of Gas6 to A431 cells that had been pre-incubated with 10 μg/mL AXL antibodies was tested as described in Example 2. Pre-incubation with AXL antibody YW327.6S2, that was described to compete with Gas6 for AXL binding, IgG1-b12 (isotype control) or medium (negative control) were included as controls.

Binding curves were analyzed using non-linear regression (sigmoidal dose-response with variable slope) using GraphPad Prism V5.04 software (GraphPad Software, San Diego, CA, USA).

**Figure 12** and **Table 12** shows that binding of Gas6 to A431 cells that had been pre-incubated with IgG1-AXL-107 and IgG1-AXL-613 antibodies was similar to the IgG1-b12 and medium controls. This illustrates that binding of IgG1-AXL-107 and IgG1-AXL-613 to AXL does not interfere with Gas6 binding, as shown in Example 2. The binding of Gas6 to A431 cells was largely reduced in the presence of IgG1-AXL-061, IgG1-AXL-137 and control AXL antibody YW327.6S2 compared to the IgG1-b12 and medium controls.

In experiments in which A431 cells were pre-incubated with Gas6, the maximal binding values of IgG1-AXL-107 and IgG1-AXL-613 were comparable to antibody binding in absence of Gas6 (maximal binding after Gas6 pre-incubation was 91-108% of binding without Gas6 pre-incubation) (**Table 12**). The $EC_{50}$ values for IgG1-AXL-107 and IgG1-AXL-613 binding with or without Gas6 pre-incubation were in the same range, or somewhat higher after Gas6 pre-incubation (**Table 12**), illustrating that IgG1-AXL-107 and IgG1-AXL-613 do not compete with Gas6 binding.

Similar to control antibody YW327.6S2, the binding of IgG1-AXL-061 and IgG1-AXL-137 to A431 cells was greatly reduced in the presence of Gas6 compared to binding without Gas6 (maximal binding after Gas6 pre-incubation was 40-43% of binding without Gas6 pre-incubation; **Table 12**). The $EC_{50}$ values for IgG1-AXL-061 and IgG1-AXL-137 could not properly be determined after Gas6 pre-incubation (**Table 12**). This shows that IgG1-AXL-061 and IgG1-AXL-137 compete with Gas6 for binding to AXL.

These data demonstrate that antibodies binding to the AXL Ig1 domain have differential effect on Gas6 binding.

*Table 12*

| Antibody | Antibody binding to A431 cells | | | Gas6 binding to A431 cells | |
|---|---|---|---|---|---|
| | EC50 w/o Gas6 EC50 (μg/mL) mean (s.d.) | EC50 in presence of Gas6 (μg/mL) mean (s.d.) | Maximal binding in presence of Gas6 (% of binding in absence of Gas6) mean (s.d.) | EC50 in presence of AXL antibodies (μg/mL) mean (s.d.) | Maximal binding in presence of AXL antibodies (% of binding in presence of control antibody) mean (s.d.) |
| IgG1-AXL-061 | 0.15 (n.a.) | *n.a.* | 43 (28) | *n.a.* | 22 (8) |
| IgG1-AXL-107 | 0.16 (0.17) | 0.94 (1.18) | 91 (5) | 0.78 (0.54) | 96 (8) |
| IgG1-AXL-137 | 0.11 (0.10) | *n.a.* | 40 (18) | *n.a* | 36 (4) |
| IgG1-AXL-613 | 0.09 (0.09) | 0.10 (0.10) | 108 (22) | 0.57 (0.36) | 100 (11) |
| YW327.6S2 | 0.09 (0.09) | 1.90 (1.04)* | 41 (24) | 5.53 (7.09)* | 17 (10) |
| b12 | n.a.[a] | n.a. | n.a. | 0.40 (0.11) | 100 |

[a] n.a., not applicable
* EC50 values less accurate due to low binding.

*Example 13 - In vivo anti-tumor efficacy of AXL-ADCs in xenograft models with and without autocrine (endogenous) Gas6 production*

Gas6 production of A431 and LCLC-103H tumor cells

It was tested whether A431 cells and LCLC-103H cells produce Gas6. Therefore, cells were grown in complete culture medium for 3 days. Gas6 levels in supernatant were determined using the Quantikine Human Gas6 ELISA (R&D Systems, Minneapolis, MN) according to manufacturer's instructions. This assay uses the quantitative sandwich ELISA technique. A monoclonal Ab specific for human Gas6 has been pre-coated onto a microplate. Standards and samples are pipetted into the wells and any human Gas6 present is bound by the immobilized Ab. After washing away any unbound substances, an enzyme-linked polyclonal Ab specific for human Gas6 is added to the wells. Following a wash to remove any unbound Ab-enzyme reagent, a substrate is added to the wells and color develops in proportion to the amount of human Gas6 bound in the initial step. The color development is stopped and the intensity of the color is measured.

Cell culture medium conditioned by A431 cells was found to contain 2576 ng/mL Gas6, while the concentration of Gas6 in medium conditioned by LCLC-103H cells was more than 20-fold less (**Table 13**).

*Table 13 - Gas6 production in tumor cell conditioned medium.*

| Cell line | Gas6 in supernatant (ng/mL) |
|---|---|
| LCLC-103H | 126 |
| A431 | 2576 |

Anti-tumor activity of AXL-ADCs *in vivo*

The *in vivo* anti-tumor activity of IgG1-AXL-061-vcMMAE (Ig1 binder), IgG1-AXL-107-vcMMAE (Ig1-binder), IgG1-AXL-137-vcMMAE (Ig1-binder), IgG1-AXL-148-vcMMAE (Ig2-binder), IgG1-AXL-183-vcMMAE (FN1-binder), and IgG1-AXL-726-vcMMAE (FN2-binder) was determined in the A431 (epidermoid carcinoma) tumor model, that produces high levels of Gas6, and the LCLC-103H (large cell lung carcinoma) tumor model, that produces low levels of Gas6.

Tumor induction was performed by subcutaneous injection of 5 × $10^6$ A431 or LCLC-103H tumor cells (both obtained from Leibniz-Institut - Deutsche Sammlung von Mikroorganismen und Zellkulturen GmbH (DSMZ)) in 200 μL PBS in the right flank of female SCID mice. Treatment was started 14-21 days after tumor cell inoculation, when the

average tumor size was >100-200 $mm^3$ and distinct tumor growth was observed. Mice received a single injection or a total of 4 biweekly intraperitoneal injections with IgG1-AXL-vcMMAE ADCs or control antibody (unconjugated IgG1-b12), as indicated. Tumor volume was determined at least two times per week. Tumor volumes ($mm^3$) were calculated from caliper (PLEXX) measurements as: 0.52 × (length) × (width)$^2$.

**Figure 13A** shows that treatment of mice with 3 mg/kg IgG1-AXL-107-vcMMAE, IgG1-AXL-148-vcMMAE and IgG1-AXL-733-vcMMAE induced growth inhibition of A431 tumors.

**Figure 13B** shows that treatment of mice with 3 mg/kg IgG1-AXL-148-vcMMAE, IgG1-AXL-183-vcMMAE (FN1 binder) and IgG1-AXL-726-vcMMAE (FN2 binder) induced growth inhibition of A431 tumors. In contrast, clones IgG1-AXL-061-vcMMAE and IgG1-AXL-137-vcMMAE did not show anti-tumor activity in the A431 xenograft model.

**Figure 14A** shows that treatment of mice with 3 mg/kg IgG1-AXL-061-vcMMAE, IgG1-AXL-137-vcMMAE, IgG1-AXL-148-vcMMAE, IgG1-AXL-183-vcMMAE and IgG1-AXL-726-vcMMAE induced tumor regression in the LCLC-103H xenograft model. Similarly, treatment of mice with 1 mg/kg IgG1-AXL-107-vcMMAE or 1 mg/kg IgG1-AXL-613-vcMMAE induced regression of LCLC-103H tumors (**Figure 14B**).

In summary, all AXL-ADCs showed anti-tumor activity in the LCLC-103H xenograft model that produces low levels of Gas6. In the A431 xenograft model, that produces high levels of Gas6, anti-tumor activity was only observed for those AXL-ADCs that did not compete with the AXL ligand Gas6.

*Example 14 - AXL expression in different tumor indications*

Expression of AXL was evaluated in freshly cut paraffin embedded and formalin fixated (FFPE) tumor tissue micro arrays (TMA) comprising tissue cores from patients with thyroid, esophageal, ovarian, pancreatic, lung, breast, cervical or endometrial cancer, or malignant melanoma. TMAs were obtained from US BioMax.

FFPE tumor array slides were deparaffinized and subjected to antigen retrieval (pH 6) and endogenous peroxidase was exhausted by incubation with 0.1% H2O2 in citrate/phosphate buffer. To detect AXL expression, the TMAs were incubated with rabbit-anti-AXL ( Santa Cruz, cat nr: sc-20741) at a concentration of 1 μg/mL for 60 min (room temperature (RT)). To identify (tumor) cells of epithelial origin, TMAs were incubated with rabbit-anti-cytokeratin (Abcam, cat. Nr. ab9377) at a dilution of 1:50 for 60 min (RT). After a washing step, the TMAs were incubated with peroxidase conjugated, anti-rabbit IgG dextran polymer (ImmunoLogic, cat no: DPVR55HRP) to detect binding of rabbit Anti-AXL and rabbit anti-cytokeratin antibodies. Finally, binding of anti-rabbit IgG dextran polymer was visualized with di-amino-benzadine (DAB; brown color; DAKO, cat no: K346811). In the TMA with malignant melanoma tissue cores, binding of anti-rabbit IgG dextran polymer was visualized with amino-ethyl carbazole (AEC; red color; Vector, SK4200). Nuclei in TMAs were visualized with hematoxylin (blue color).

AXL and cytokeratin immunostained TMAs were digitized with an Aperio slide scanner at 20× magnification and immunostaining was quantified with tissue image analysis software (Definiens Tissue Studio software, version 3.6.1), using a cell-based algorithm. The algorithm was designed to identify and quantify the percentage of AXL- or cytokeratin-positive cells in the biopsies (range 0 - 100%) and to quantify AXL staining intensity in AXL-positive tumor cells (optical density (OD); range 0 - 3) in each tumor core. Tumor cells were scored AXL positive, when AXL OD was at least 0.1. The percentage of AXL positive tumor cells per tumor core (range 0 - 100%) was calculated by dividing the total number of AXL positive cells by the total number of cytokeratin-positive cells in sequential tumor cores. The average AXL staining intensity (OD) in each tumor core was calculated by dividing the sum of AXL OD of all AXL positive tumor cells by the number of AXL positive tumor cells.

Tumor array from patients with malignant melanoma were scored manually. AXL staining intensity was scored as either weak (1+), moderate (2+) or strong (3+) and the percentage AXL positive melanoma cells was scored in 10% intervals (range 0 - 100%).

**Figure 16** provides a graphical representation of AXL expression in tumor cores of thyroid, esophageal, ovarian, breast, lung, pancreatic, cervical and endometrial cancer. **Table 14** shows the percentage of tumor cores that showed AXL expression in more than 10% of tumor cells, for each indication. **Figure 17** shows a representative example of a tissue core immunostained for AXL, for each indication. The figures illustrate heterogeneous expression of AXL in the tumor issue.

*Table 14*

| Tumor indication | Subtype | % tumor cores (patients) with >10% AXL-positive tumor cells |
|---|---|---|
| Esophageal cancer | Adenocarcinoma (n=19) | 73 |
| | Squamous cell carcinoma (n=60) | 55 |
| Ovarian cancer | All subtypes (n=52) | 90 |
| Pancreatic cancer | All subtypes (n=58) | 60 |

(continued)

| Tumor indication | Subtype | % tumor cores (patients) with >10% AXL-positive tumor cells |
|---|---|---|
| Lung cancer (NSCLC) | Squamous cell carcinoma SSC (n=52) | 63 |
| | Adenocarcinoma (n=48) | 67 |
| Lung cancer (SCLC) | SCLC (n=5) | 60 |
| Thyroid cancer | All subtypes (n=48) | 92 |
| Uterine cancer | All subtypes (n=60) | 88 |
| Breast cancer | TNBC (n=54) | 24 |
| Cervical cancer | All subtypes (n=54) | 93 |
| Melanoma | Malignant melanoma (n=67) | 6 |
| Abbreviations used: NSCLC, non small cell lung cancer; SLCL, small cell lung cancer;TNBC, triple negative breast cancer | | |

*Example 15 - AXL antibodies specifically bind AXL but not other TAM receptor family members.*

Expression of human AXL, MER, and TYRO3 in HEK-293F cells

The following codon-optimized constructs for expression of various full-length proteins were generated: human (Homo sapiens) AXL (Genbank accession no. NP_068713.2), human MER (Genbank accession no. EAW52096.1, and human TYRO3 (Genbank accession no. Q06418.1). The constructs contained suitable restriction sites for cloning and an optimal Kozak (GCCGCCACC) sequence (Kozak *et al.,* 1999). The constructs were cloned in the mammalian expression vector pcDNA3.3 (Invitrogen)

FreestyleTM 293-F (a HEK-293 subclone adapted to suspension growth and chemically defined Freestyle medium, (HEK-293F)) cells were obtained from Invitrogen and transfected with the expression plasmids using 293fectin (Invitrogen), according to the manufacturer's instructions and grown for 24-48 hours.

Binding study of AXL antibodies to human AXL, human MER, or human TYRO3

HEK-293F cells transiently transfected with expression constructs for full length human AXL, MER, or TYRO3 were evaluated for binding of HuMab-AXL antibodies by flow cytometry. Transfected HEK-293F cells were incubated with serial dilutions of AXL-antibodies (4-fold dilutions; final concentration range 0.002-10 μg/mL) for 30 minutes at 4ºC. After washing three times in PBS/0.1% BSA/0.02% azide, cells were incubated with R-Phycoerythrin (PE)-conjugated goat-anti-human IgG F(ab')2 (Jackson ImmunoResearch Laboratories, Inc., West Grove, PA; cat. No. 109-116-098) diluted 1/100 in PBS/0.1% BSA/0.02% azide (final volume 100 μL). Next, cells were washed twice in PBS/0.1% BSA/0.02% azide, resuspended in 120 μL PBS/0.1% BSA/0.02% azide and analyzed on a FACS Cantoll (BD Biosciences). Staining with mouse anti-human Mer (R&D Systems, cat. Mab8912) and mouse anti-human Tyro3 (Dtk) (R&D Systems, cat. MAB859) were included as controls for expression, IgG1-b12 was included as a non-binding isotype control antibody. Binding curves were analyzed using non-linear regression (sigmoidal dose-response with variable slope) using GraphPad Prism V5.04 software (GraphPad Software, San Diego, CA, USA).

**Figure 18A** shows that Humab-AXL antibodies showed dose-dependent binding to the HEK293 cells expressing human AXL. In contrast, no binding of HuMab-AXL antibodies to cells expressing MER **(Figure 18B)** or TYRO3 **(Figure 18C)** or to untransfected HEK293 cells **(Figure 18D)** was observed. Staining with MER- and Tyro3-specific antibodies confirmed that transfected cells showed proper expression of MER **(Figure 18F)** or TYRO3 **(Figure 18G),** respectively.

*Example 16 - Internalization of cell surface bound AXL antibodies*

Internalization of cell surface bound HuMab-AXL evaluated by flow cytometry

Internalization of cell surface bound HuMab-AXL antibodies to MDA-MB-231 and Calu-1 cells (human lung carcinoma cell line; ATCC, catalognumber HTB-54) was evaluated by flow cytometry. 50,000 cells were seeded in 96-well tissue culture plates and allowed to attach for 6 hrs at 37°C. Plates were incubated at 4°C for 30 minutes before incubation with serial dilutions of AXL-antibodies (final concentration range 0.0032-10 μg/mL) at 4°C for 1 hour. Subsequently, the

medium was replaced by tissue culture medium without antibody and cells were incubated overnight (16-18 hours) at 37°C or 4°C. Subsequently, the cells were detached with 40 μL warm trypsin solution, washed with ice-cold PBS/0.1% BSA/0.02% azide, and incubated for 30 minutes at 4°C with R-Phycoerythrin (PE)-conjugated goat-anti-human IgG F(ab')2 (Jackson ImmunoResearch Laboratories, Inc., West Grove, PA; cat. No. 109-116-098) diluted 1/100 in PBS/0.1% BSA/0.02% azide (final volume 100 μL), to detect AXL-antibodies on the cell surface. Finally, cells were washed twice in PBS/0.1% BSA/0.02% azide, resuspended in 120 μL PBS/0.1% BSA/0.02% azide and analyzed on a FACS Cantoll (BD Biosciences).

Binding curves were analyzed using non-linear regression (sigmoidal dose-response with variable slope) using GraphPad Prism V5.04 software (GraphPad Software, San Diego, CA, USA).

**Figure 19** shows that, for all AXL HuMab antibodies and at all concentrations tested, more antibody was detected on the plasma membrane of cells that had been incubated at 4°C after antibody binding, compared to cells that had been incubated at 37°C. This illustrates that, at 37°C, AXL antibodies are internalized upon binding to the plasma membrane.

Fab-TAMRA/QSY7 internalization and intracellular degradation assay

Internalization of AXL antibodies was assessed in the Fab-TAMRA/QSY7 internalization assay. This assay uses a fluorophore (TAMRA) and quencher (QSY7) pair. In close proximity, for example, upon conjugation to the same protein, TAMRA fluorescence is quenched by QSY7. In this example, goat-anti-human IgG Fab-fragments (Jackson Immunoresearch) were conjugated with TAMRA/QSY7 (Fab-TAMRA/QSY7) as described (Ogawa et al., Mol Pharm 2009;6(2):386-395), and AXL HuMab (1.5 μg/mL) were preincubated with Fab-TAMRA/QSY7 (12 μg/mL; 30 min, 4ºC). The complex was subsequently added to LCLC-103H cells and incubated for 24 h incubation in the dark, under shaking conditions (200 rpm, 37ºC). Internalization of the HuMab-Fab-TAMRA/QSY7 complex and intracellular degradation in the endosomes and lysosomes causes dissociation of TAMRA/QSY7, resulting in dequenching of TAMRA. TAMRA fluorescence of LCLC-103H cells that had been incubated with AXL antibodies complexed with Fab-TAMRA/QSY7 was measured on a FACS Canto-II (BD Biosciences).

As shown in **Figure 20,** the fluorescence intensity of LCLC-103H cells was enhanced upon incubation with AXL-antibody-Fab-TAMRA/QSY7 complex, compared to IgG1-b12-Fab-TAMRA/QSY7 or Fab-TAMRA/QSY7 alone. This illustrates that AXL antibodies are internalized upon binding to LCLC-103H cells.

*Example 17 - Resistance to the BRAF inhibitor PLX4720 is associated with upregulated Axl protein expression and enhanced sensitivity to IgG1-AXL-107-vcMMAE in vitro and in vivo*

In a panel of established human melanoma cell lines (CDX) and patient derived low passage melanoma cell lines (PDX), Axl protein expression levels were evaluated in relation to their intrinsic or acquired resistance to growth inhibition by treatment with the BRAF inhibitor PLX4720, an analogue to the clinically approved BRAF inhibitor vemurafenib. In addition, the sensitivity of melanoma cells to treatment with IgG1-AXL-107-vcMMAE was evaluated *in vitro* and *in vivo.*

Cell culture

SKMEL147 was obtained from the Laboratory of Reuven Agami at the Netherlands Cancer Institute. A875 was obtained from Thermo Fischer, COLO679 from Sigma, SKMEL28 and A375 cells from ATCC. Melanoma cell lines were cultured in DMEM supplemented with 10% fetal bovine serum (Sigma), 100 U/ml penicillin and 0.1 mg/ml streptomycin (all Gibco). The cell lines were maintained at 37°C in a 5% (vol/vol) $CO_2$ humidified incubator.

Generation of PLX4720 resistant cell lines

BRAF inhibitor sensitive cell lines (SKMEL28, and A375) were cultured in the presence of increasing concentrations of the BRAF inhibitor PLX4720 (Selleck Chemicals, Houston, TX, USA, Company: Selleck Chemicals, Houston, TX, USA, Catalog number: S1152,) up to 3 μM to establish the corresponding PLX4720 resistant SKMEL28R, and A375R. All drug-resistant cell lines were permanently cultured in the presence of 3 μM of PLX4720.

Generation of patient derived low passage (PDX) melanoma cell lines

The Medical Ethical Board of the Antoni van Leeuwenhoek hospital, Netherlands Cancer Institute has approved the collection and use of human tissue. Animal experiments were approved by the animal experimental committee of the institute and performed according to applicable rules and regulations. Human tumor material was obtained during surgery, or by taking tumor biopsies from malignant melanoma patients using a 14-gauge needle. Tumor fragments of ~5mm$^3$ were used for subcutaneous implantation in NOD.Cg-*Prkdc$^{scid}$ Il2rg$^{tm1Wjl}$*/SzJ mice, which was performed under anesthesia.

Tumor outgrowth was measured twice per week with a caliper. Before reaching the a tumor size of 1000 $mm^3$, mice were sacrificed, tumors were removed and tumor pieces were dissociated into single cells suspensions, plated on 10-cm dishes and grown as primary cell cultures in DMEM + 10% FBS (Sigma) + 100 U/ml penicillin and 0.1 mg/ml streptomycin (all Gibco).

Western blot analysis

Expression of Axl and MITF was determined using Western blot analysis. The proteins in the cell lysate were separated on a 4-12% SDS-PAGE gel and transferred to PVDF membrane that was subsequently stained with antibody specific for Axl (sc-1096 Santa Cruz) in 5% BSA in PBS-Tween, or to a nitrocellulose membrane stained with MITF (ab12039 Abcam) in 5% non-fat dry milk in PBS-Tween. To control for gel loading, antibodies against vinculin or beta-actin were used.

Quantification of AXL expression on the plasma membrane of melanoma cell lines

AXL expression on the plasma membrane of human tumor cell lines was quantified by indirect immunofluorescence using QIFIKIT analysis (DAKO, Cat nr K0078). Axl was detected using the mouse monoclonal antibody ab89224 (Abcam, Cambridge, UK). Adherent cells were trypsinized and passed through a cell strainer to obtain single cell suspensions. Cells were pelleted by centrifugation for 5 minutes at 1,200 rpm, washed with PBS and resuspended at a concentration of $1\times10^6$ cells/mL. The next steps were performed on ice. 100 μL of the single cell suspensions (100,000 cells per well) were seeded in polystyrene 96-well round-bottom plates (Greiner Bio-One, Cat nr 650101). Cells were pelleted by centrifugation for 3 minutes at 300xg and resuspended in 50 μL antibody sample or mouse IgG1 isotype control sample (cat number QF2040741, lot number MA1-10406, Pierce) at a concentration of 10 μg/mL. After an incubation of 30 minutes at 4ºC, cells were pelleted and resuspended in 150 μL FACS buffer (PBS containing 0.1 % BSA). Set-up and calibration beads were added to the plate according to the manufacturer's instructions. Cells and beads in parallel were washed two more times with 150 μL FACS buffer and resuspended in 50 μL FITC-conjugated goat-antimouse IgG (1/50; DAKO, cat.no. K0078). Secondary antibody was incubated for 30 minutes at 4ºC in the dark. Cells and beads were washed twice with 150 μL FACS buffer and resuspended in 100 μL FACS buffer. Immunofluorescence was measured on a FACS Calibur (BD Biosciences) by recording 10,000 events within the gate of viable cells. The mean fluorescence intensity of the calibration beads was used to calculate the calibration curve using GraphPad Prism software (GraphPad Software, San Diego, CA, USA). For each cell line, the antibody binding capacity (ABC), an estimate for the number of AXL molecules expressed on the plasma membrane, was calculated using the mean fluorescence intensity of the AXL antibody-stained cells, based on the equation of the calibration curve (interpolation of unknowns from the standard curve, using GraphPad Software).

*In vitro* cytotoxicity

Cells were cultured to near confluency, after which cells were trypsinized, resuspended in culture medium and passed through a cell strainer (BD Falcon, cat.no. 352340) to obtain single cell suspensions. Cells were plated in a 96-well format using the following seeding densities: 2000 cells/well for established cell lines, 4000 cells/well for PDX-derived cell lines. IgG1-AXL-107-vcMMAE was added 4 hours after seeding. Serial dilutions (10-fold; final concentrations ranging from 0.0001 to 10 μg/mL) of IgG1-AXL-107-vcMMAE were prepared in culture medium and added to the plates. After 5 days (for CD samples) or 8 (PDX samples) days of incubation at 37ºC, 5% $CO_2$, CellTiter-Glo Reagent (Promega; cat.no. G7571) was added to the wells and the Luminescent Cell Viability Assay (Promega, Madison, WI) was performed according to the manufacturer's protocol. Luminescence was measured by the Infinite M200 microplate reader (Tecan) and viability was calculated as follows: % viability = (luminescence sample of interest - luminescence PAO)/(average luminescence of control vehicle treated - luminescence PAO), with PAO representing 5 μM phenyl arsine oxide for 100% cell killing.

SKMEL147 melanoma xenograft model

The anti-tumor activity of IgG1-AXL-107-vcMMAE was evaluated in the subcutaneous melanoma model SKMEL147 in NMRI nude mice. Mice were subcutaneously injected in the left flank with $2.5\times10^5$ SKMEL147 melanoma cells, which express high levels of Axl (see Figure 21 and Table 15), that were resuspended 1:1 in matrigel in a total volume of 100 μL. Tumors were measured three times weekly with a caliper, and when tumors were 100 mm3 the animals were randomized over the following treatment groups: IgG1-b12 (4 mg/kg), IgG1-b12-vcMMAE (4 mg/kg), IgG1-107 (4 mg/kg), IgG1-107-vcMMAE (2 mg/kg), and IgG1-107-vcMMAE (4 mg/kg).

On day 12 and day 19 after tumor cell injection (day 1 and day 8 of randomization) the test compounds were

injected into the tail vein of the animals in a total volume of 100 μL. Animals were sacrificed when the size of the tumor exceeded 1000 mm3.

Treatment of a mixed population of SKMEL28 wild type cells and SKMEL28 cells resistant to PLX4720 with AXL-ADC, BRAF inhibitor and/or MEK inhibitor.

SKMEL28 wild-type cells and SKMEL28 cells resistant to PLX4720 (SKMEL28-R) were transfected with expression vectors of the fluorophores mCherry (red) or GFP (green), respectively. Subsequently, cells were seeded in a 1:1 ratio, with 50.000 cells of each cell line in a 6-well plate (in total 100.000 cells/well). After 3 hours, the following compounds were added to the wells: IgG1-AXL-107-vcMMAE (1 μg/mL), IgG1-b12-MMAE (1 μg/mL; isotype control ADC), PLX4720 (10 μM; BRAF inhibitor), dabrafenib (1 μM; BRAF inhibitor), and/or trametinib (0.1 μM; MEK inhibitor). After 4 days, cells were trypsinized, washed once in PBS + 1% BSA and analyzed by flow cytometry.

Immunohistochemistry

Expression of AXL was evaluated in freshly cut paraffin embedded and formalin fixated (FFPE) whole tissues (WT) with malignant melanoma. Staining was performed manually in Sequenza Slide Racks (Ted Pella Inc., Redding, CA, USA; cat. no. 36105).

Prior to staining, FFPE tissue slides were deparaffinized in 100% xylene (Sigma-Aldrich, cat. no. 16446; three times, 5 min.) and dehydrated in 96% ethanol (Sigma Aldrich, cat. no. 32294; two times, 5 min.) at RT. Thereafter, antigen retrieval was performed. IHC slides were incubated in citrate buffer (pH6; DAKO; cat. no. S2369) for 5 min. and blocked for endogenous peroxidase in citrate/phosphate buffer (0.43 M citric acid, 0.35 M $Na_2HPO_4.2H_2O$; pH5.8) at RT for 15 min. Slides were incubated in 10% normal human serum (CLB/Sanquin, cat. no. K1146) in PBS, prior to incubation with primary antibodies. Axl expression was determined by incubation with 3 μg/mL rabbit polyclonal anti-human Axl antibody H-124 in PBS supplemented with 2% normal human serum at RT for 60 min. Slides were washed in PBS supplemented with 0.1% Tween-20 (twice, 3 min.) and binding of rabbit antibodies specific for Axl were detected with undiluted Bright Vision poly-HRP-anti-rabbit IgG. HRP was visualized with 3-amino-9-ethylcarbazole (AEC) chromophore (red color; Sigma, cat. no. A6926-100TAB); nuclei were counterstained with hematoxylin (DAKO, cat. no. S3309). Slides were analyzed by a certified pathologist at the Netherlands Cancer Institute (NKI, Amsterdam, The Netherlands), who scored the intensity and localization of Axl staining in each sample. Examples are shown in **Figure 27.**

Results

AXL expression:

AXL expression was evaluated in a panel of established melanoma cell lines **(Table 15)** and low passage primary melanoma lines (PDX, **Table 16).** AXL expression, as determined by western blot **(Figure 21),** was inversely correlated with MITF expression in established cell lines **(Figure 21A)** as well as clinical patient-derived samples **(Figure 21B).** In the established cell line panel, Axl expression was also determined by quantitative flow cytometry. An example of an AXL negative and positive cell line is shown in **Figure 22.** Axl expression levels (expressed as ABC) for all cell lines are listed in **Table 15,** along with the BRAF mutation status of the cell lines.

AXL-ADC Cytotoxicity *in vitro:*

Next, sensitivity of the established melanoma cell lines and PDX panel to IgG1-AXL-107-vcMMAE was evaluated in viability assays. Cells were exposed to increasing concentrations of IgG1-AXL-107-vcMMAE (range $1 \times 10^{-4}$ to 10 μg/mL) for 5 days after which the cell viability was determined. Results are summarized in **Table 15 and 16,** dose-response curves are shown in **Figure 23 and 24. Figure 23** shows that all 4 AXL expressing cell lines (SKMEL147, A875, A375R, SKMEL28R), three of which were resistant to PLX4720, are sensitive to treatment with IgG1-AXL-107-vcMMAE. The two AXL negative cell lines COLO679 and SKMEL28 did not show changes in viability upon treatment with IgG1-AXL-107-vcMMAE. Three PLX4720-resistant PDX samples were tested in viability assays with IgG1-AXL-107-vcMMAE. **Figure 24** shows that the two AXL high expressing PDX cultures, MO16 and MO19R, were sensitive to treatment with IgG1-AXL-107-vcMMAE, whereas the AXL low expressing PDX culture M082 did not show a different response from that seen with the IgG1-b12-vcMMAE control treatment.

*Table 15. Characteristics of the melanoma cell line panel.*

| Cell line | AXL expression (western blot) | AXL expression (FACS) Receptor number (ABC) | BRAF | NRAS | PLX4720 sensitivity | HuMax-AXL-ADC sensitivity |
|---|---|---|---|---|---|---|
| SKMEL147 | + | 34981 | Wt | Q61R | resistant | Sensitive |
| A875 | + | 37079 | V600E | wildtype | sensitive | Sensitive |
| COLO679 | - | BLQ | V600E | Wt | untested | Resistant |
| A375R | + | 14228 | V600E | Wt | resistant | Sensitive |
| SKMEL28 | - | BLQ | V600E | Wt | sensitive | Resistant |
| SKMEL28R | + | 63809 | V600E | Wt | resistant | Sensitive |
| *BLQ = Below Limit of Quantitation (<3300, lowest ABC value of calibration beads) | | | | | | |

*Table 16. Characteristics of the patient-derived melanoma cultures*

| Name | BRAF/NRAS status | AXL expression (western Blot) | AXL expression Receptor number (ABC, FACS) | PLX4720 sensitivity | HuMax-AXL-ADC sensitivity |
|---|---|---|---|---|---|
| M016 | $NRAS^{Q61R}$ | + | 13688 | resistant | Sensitive |
| M019R | $BRAF^{V600E}$ | ++ | 25988 | resistant | Sensitive |
| M082 | $BRAF^{V600E}$ | (low) | 3376 | resistant | Insensitive |

Treatment with AXL-ADC *in vivo:*

In the SKMEL147 melanoma xenograft model, mice treated with IgG1-b12, IgG1-b12-vcMMAE, or IgG1-AXL-107 did not show tumor growth inhibition. *IgG1-AXL-107-vcMMAE* induced tumor growth inhibition at 2 mg/kg, and at a dose of 4 mg/kg IgG1-AXL-107-vcMMAE induced strong tumor regression, which lasted until around day 50 **(Figure 25A).**

HuMax-AXL-ADC at a dose of 4 mg/kg thus showed a profound anti-tumor effect, but tumors started to grow out again after day 50. Four mice that showed tumor regrowth upon initial tumor regression with 4 mg/kg IgG1-AXL-107-vcMMAE were retreated with a single dose of 4 mg/kg IgG1-AXL-107-vcMMAE on days 55, while for comparison two other mice were observed.

Retreatment with 4 mg/kg IgG1-AXL-107-vcMMAE resulted in tumor regression in all four mice, whereas the 2 mice that were observed, showed tumor growth **(Figure 25B).** Two of the four retreated mice showed tumor regression that remained at least until day 80, while tumor regrowth was observed around day 70 in the two other retreated mice **(Figure 25B).**

Combination treatments:

In the mixed population of SKMEL28 wt cells and SKMEL28 PLX4720-resistant cells, compared to the untreated control, total cell numbers were reduced with 74-62 % when cell mixtures were treated with IgG1-AXL-107-vcMMAE, PLX4720, or dabrafenib **(Figure 26A).** Treatment of cell mixtures with the combinations of IgG1-AXL-107-vcMMAE and PLX4720, IgG1-AXL-107-vcMMAE and dabrafenib, dabrafenib and trametinib, or dabrafenib, trametinib and IgG1-AXL-107-vcMMAE induced 81-92 % reduction of total cell numbers compared to untreated cells **(Figure 26A).**

To evaluate if specific cell populations were eradicated, the ratio of green (GFP-positive SKMEL28-R cells) and red (mCherry-positive SKMEL28 cells) was determined. As expected, untreated and IgG1-b12-vcMMAE treatment did not affect the GFP/mCherry ratio, as total cell numbers were also unaffected **(Figure 26B).** Treatment with IgG1-AXL-107-vcMMAE resulted in a strongly reduced GFP/mCherry ratio **(Figure 26B),** indicating specific killing of SKMEL28-R cells. Conversely, treatment with BRAF inhibitors PLX4720 or dabrafenib increased the GFP/mCherry ratio **(Figure 26B),** indicating specific killing of SKMEL28 cells. Combinations of IgG1-AXL-107-vcMMAE and PLX4720, dabrafenib and trametinib, or dabrafenib, trametinib and IgG1-AXL-107-vcMMAE showed ratios closer to 1 **(Figure 26B),** indicating that both cell types were killed with similar efficacy. Treatment with the combination of IgG1-AXL-107-vcMMAE and dabrafenib resulted in a strongly reduced GFP/mCherry ratio **(Figure 26B),** indicating more efficient killing of SKMEL28-R cells at the concentrations used.

Immunohistochemistry:

In total 45 samples were analyzed, of which 3 did not contain any tumor material and were thus excluded from analysis. In addition, 7 matched pre - and post vemurafenib samples from the same patients were included, and 1 matched pre - and post dabrafenib/trametinib sample. In 41/42 samples Axl expression was detected in subsets of the melanoma region. Staining intensity differed per patient tumor **(Table 17).**

Furthermore, upregulation of Axl expression (as measured by increase of staining intensity by pathologist) was observed in 4/7 matched pre- and post vemurafenib samples **(Table 17).**

*Table 17. Axl staining in tumor tissue from melanoma patients.*

| Case nr. | Treatment | Pre-/posttreatment | Matched sample | Axl staining tumor cells[a] | Comments |
|---|---|---|---|---|---|
| 1 | vemurafenib | post | NA | Partially + | |
| 2 | vemurafenib | post | 17 | Weakly + to + | |
| 3 | dabr/tram | post | NA | ++ to +++ | |
| 4 | vemurafenib | post | NA | Focally + | |
| 5 | vemurafenib | post | NA | Partially weakly + | |
| 6 | dabr/tram | post | 40 | NA | very necrotic |
| 7 | dabr/tram | pre | 16 | Sporadic + | |
| 8 | vemurafenib | post | 38 | Sporadic + | the weakly positive cells at the edge of the tumor could be the result of staining artefact |
| 9 | vemurafenib | post | NA | - | |
| 10 | vemurafenib | post | NA | Partially weakly + | |
| 11 | vemurafenib | post | NA | Weakly + | many melanophages + |
| 12 | vemurafenib | post | NA | Locally weakly + | some melanophages + |
| 13 | vemurafenib | post | NA | ++ to +++ | |
| 14 | vemurafenib | post | 39 | Weakly + | many melanophages + |
| 15 | vemurafenib | post | 24 | Weakly + | |
| 16 | dabr/tram | post | 7 | Weakly + | |
| 17 | vemurafenib | pre | 2 | Partially + | |
| 18 | vemurafenib | 18 stable disease post | NA | Weakly + | |
| 19 | vemurafenib | post | NA | Locally + to ++ | |
| 20 | vemurafenib | 20 stable disease post | NA | Weakly + | |
| 21 | vemurafenib | post | NA | Weakly + | |
| 22 | vemurafenib | post | NA | Partially + | many melanophages + |

(continued)

| Case nr. | Treatment | Pre-/posttreatment | Matched sample | Axl staining tumor cells[a] | Comments |
|---|---|---|---|---|---|
| 23 | vemurafenib | post | NA | + to ++ | |
| 24 | vemurafenib | pre | 15 | Sporadic + | |
| 25 | vemurafenib | post | NA | Sporadic + | |
| 26 | vemurafenib | pre | 44 | Weakly + | many melanophages + |
| 27 | vemurafenib | post | NA | Partially and weakly + | |
| 28 | vemurafenib | 28 stable disease post | NA | Weakly + | limited amount of tumor cells are present |
| 29 | vemurafenib | post | NA | Partially and weakly + | |
| 30 | vemurafenib | post | NA | Partially + | |
| 31 | vemurafenib | post | NA | Partially + | |
| 32 | vemurafenib | post | NA | + | small amount of tumor cells/melanophages with melanin |
| 33 | vemurafenib | post | NA | Locally weakly + | |
| 34 | vemurafenib | post | NA | Weakly + to + | |
| 35 | vemurafenib | post | NA | Weakly + | |
| 36 | vemurafenib | post | NA | weakly + | many melanophages + |
| 37 | vemurafenib | post | NA | Partially weakly + | |
| 38 | vemurafenib | pre | 8 | Weakly + to + | |
| 39 | vemurafenib | pre | 14 | + | the positive cells are present in the sinuses of the lymph nodes. It is not certain whether they are tumor cells or macrophage since these cells contain rather rich cytoplasm |
| 40 | dabr/tram | pre | 6 | NA | no neoplastic lesions are encountered |
| 41 | vemurafenib | post | NA | NA | no neoplastic lesions are encountered |
| 42 | vemurafenib | post | NA | Partially + | partial negative areas could be due to staining artefact |
| 43 | vemurafenib | post | NA | Weakly + to + | |
| 44 | vemurafenib | post | 26 | + to ++ | |

(continued)

| Case nr. | Treatment | Pre-/posttreatment | Matched sample | Axl staining tumor cells[a] | Comments |
|---|---|---|---|---|---|
| 45 | vemurafenib | post | NA | Partially weakly + | |
| [a]-: negative; positive staining intensity: weakly +< + < ++ < +++; positive staining area: sporadic < focal < local < partial; NA: not available | | | | | |

*Example 18 - Generation and characterization of PDX-derived, BRAF mutant melanoma models*

Generation of patient-derived low passage (PDX) melanoma cell cultures

The Medical Ethical Board of the Antoni van Leeuwenhoek hospital, Netherlands Cancer Institute has approved the collection and use of human tissue. Animal experiments were approved by the animal experimental committee of the institute and performed according to applicable rules and regulations. Human tumor material was obtained during surgery, or by taking tumor biopsies from malignant melanoma patients using a 14-gauge needle. Tumor fragments of ~5mm$^3$were used for subcutaneous implantation in NOD.Cg-*Prkdc*$^{scid}$ *Il2rg*$^{tm1Wjl}$/SzJ mice, which was performed under anesthesia. Tumor outgrowth was measured twice per week with a caliper. Before reaching a tumor size of 1000 mm$^3$, mice were sacrificed, tumors were removed and tumor pieces were dissociated into single cells suspensions, plated on 10-cm dishes and grown as primary cell cultures in DMEM + 10% FBS (Sigma) + 100 U/ml penicillin and 0.1 mg/ml streptomycin (all Gibco). The M019R cell culture was derived from tumor material of a melanoma patient containing a *BRAF* V600E-mutation who was intrinsically resistant to vemurafenib.

Quantification of AXL expression on the plasma membrane of melanoma cell lines

AXL expression on the plasma membrane of human tumor cell lines was quantified by indirect immunofluorescence using QIFIKIT analysis (DAKO, Cat nr K0078). Axl was detected using the mouse monoclonal antibody ab89224 (Abcam, Cambridge, UK). Adherent cells were trypsinized and passed through a cell strainer to obtain single cell suspensions. Cells were pelleted by centrifugation for 5 minutes at 1,200 rpm, washed with PBS and resuspended at a concentration of $1\times10^6$ cells/mL. The next steps were performed on ice. 100 μL of the single cell suspensions (100,000 cells per well) were seeded in polystyrene 96-well round-bottom plates (Greiner Bio-One, Cat nr 650101). Cells were pelleted by centrifugation for 3 minutes at 300xg and resuspended in 50 μL antibody sample or mouse IgG1 isotype control sample (cat number QF2040741, lot number MA1-10406, Pierce) at a concentration of 10 μg/mL. After an incubation of 30 minutes at 4ºC, cells were pelleted and resuspended in 150 μL FACS buffer (PBS containing 0.1 % BSA). Set-up and calibration beads were added to the plate according to the manufacturer's instructions. Cells and beads in parallel were washed two more times with 150 μL FACS buffer and resuspended in 50 μL FITC-conjugated goat-antimouse IgG (1/50; DAKO, cat.no. K0078). Secondary antibody was incubated for 30 minutes at 4ºC in the dark. Cells and beads were washed twice with 150 μL FACS buffer and resuspended in 100 μL FACS buffer. Immunofluorescence was measured on a FACS Calibur (BD Biosciences) by recording 10,000 events within the gate of viable cells. The mean fluorescence intensity of the calibration beads was used to calculate the calibration curve using GraphPad Prism software (GraphPad Software, San Diego, CA, USA). For each cell line, the antibody binding capacity (ABC), an estimate for the number of AXL molecules expressed on the plasma membrane, was calculated using the mean fluorescence intensity of the AXL antibody-stained cells, based on the equation of the calibration curve (interpolation of unknowns from the standard curve, using GraphPad Software).

*In vitro* cytotoxicity

Cells were cultured to near confluency, after which cells were trypsinized, resuspended in culture medium and passed through a cell strainer (BD Falcon, cat.no. 352340) to obtain single cell suspensions. PDX-derived cell cultures were plated in a 96-well format at a density of 4000 cells/well. IgG1-AXL-107-vcMMAE, PLX4720 (Selleck Chemicals, Houston, TX, USA; Cat no: S1152), or trametinib (Selleck Chemicals; Cat no S2673) was added 4 hours after seeding. Serial dilutions of test reagents were prepared in culture medium and added to the plates. After 8 days of incubation at 37ºC, 5% $CO_2$, CellTiter-Glo Reagent (Promega; cat.no. G7571) was added to the wells and the Luminescent Cell Viability Assay (Promega, Madison, WI) was performed according to the manufacturer's protocol. Luminescence was

measured by the Infinite M200 microplate reader (Tecan) and viability was calculated as follows: % viability = (luminescence sample of interest - luminescence PAO)/(average luminescence of control vehicle treated - luminescence PAO), with PAO representing 5 μM phenyl arsine oxide for 100% cell killing.

Results

The PDX-derived melanoma cell cultures M019R and M009R were obtained as described above and characterized for Axl expression levels, *BRAF* and *NRAS* mutational status, and *in vitro* sensitivity to PLX4720, trametinib, or IgG1-AXL-107-vcMMAE. The results are summarized in Table 18. Axl expression levels in M009R were heterogeneous, meaning that only a subpopulation of cells had detectable levels of Axl as evaluated by flow cytometry.

*Table 18. Characteristics of the BRAF mutant melanoma cell cultures*

| | | | | **drug sensitivity *in vitro*** | | |
|---|---|---|---|---|---|---|
| **Cell line** | **Axl expression (FACS) Receptor number (ABC)** | **BRAF** | **NRAS** | **PLX4720 sensitivity[a]** | **trametinib sensitivity[a]** | **IgG1-AXL-107-vcMMAE Sensitivity[b]** |
| M019R | 25988 | V600E | wt | insensitive | insensitive | sensitive |
| M009R | Heterogeneous (~3% AXL positive cells) | V600E | wt | n.a. | n.a. | insensitive |

Abbreviations used: FACS, fluorescence-activated cell sorting; ABC, antibody binding capacity; wt, wild-type; n.a.
[a]Insensitive cell lines show no significant cell death at a concentration of 3 μM PLX4720 or 0.1 μM trametinib
[b]Insensitive cell lines show no significant cell death or cell death comparable to IgG1-b12-vcMMAE at a concentration of 1 μg/mL IgG1-AXL-107-vcMMAE.

*Example 19 - Anti-tumor activity of IgG1-AXL-107-vcMMAE alone and in combination with BRAFi/MEKi in a resistant BRAF mutant melanoma model* (*M019R*) *in vivo*

BRAF-mutant M019R xenograft model derived from a malignant melanoma patient

The anti-tumor activity of *IgG1-AXL-107-vcMMAE* was evaluated in the subcutaneous melanoma model M019R in NMRI nude mice. Mice were subcutaneously injected in the left flank with $2.5 \times 10^5$ M019R melanoma cells, that had been resuspended 1:1 in matrigel in a total volume of 100 μL. Tumors were measured two times weekly with a caliper, and when tumors were 100 mm3 on day 62 after tumor cell inoculation, the animals were randomized over the following 3 treatment groups (7 or 8 mice per group): IgG1-b12-vcMMAE ('control ADC'; 4 mg/kg, i.v.), IgG1-AXL-107-vcMMAE (4 mg/kg, i.v.), and the BRAF-inhibitor dabrafenib (30 mg/kg, oral gavage) plus the MEK-inhibitor trametinib (0.1 mg/kg, oral gavage).

On day 0 and day 7 after randomization the ADCs were injected into the tail vein of the animals in a total volume of 100 μL. Starting on the day of randomization, dabrafenib and trametinib were given orally on a daily basis until the second randomization. Animals were sacrificed when the size of the tumor exceeded 1000 $mm^3$.

On day 30 after the first randomization, mice that were treated with the combination of dabrafenib plus trametinib were divided in three treatment groups: dabrafenib plus trametinib (n=3), IgG1-AXL-107-vcMMAE (n=5), or the triple combination of dabrafenib, trametinib, and IgG1-AXL-107-vcMMAE (n=5). On day 0 and day 7 after randomization the ADCs were injected into the tail vein of the animals in a total volume of 100 μL. Groups receiving dabrafenib and trametinib were treated on a daily basis starting on the day of randomization until the end of the study. Animals were sacrificed when the size of the tumor exceeded 1000 $mm^3$. Survival was analyzed with Graphpad Prism software using a tumor size cutoff of 900 $mm^3$. Differences in survival between groups were analyzed using the Mantel-Cox test.

Results

*In vitro* data showed that the BRAF mutant PDX-derived cell culture M019R expresses Axl on the cell surface (Example 17) and is resistant to the BRAF inhibitor PLX4720, which is consistent with clinical resistance to vemurafinib of the patient from which this model was derived. Furthermore, IgG1-AXL-107-vcMMAE efficiently induced killing of M019R cells *in vitro* (Example 17).

M019R cells were transplanted in nude mice and the antitumor efficacy of IgG1-AXL-107-vcMMAE was evaluated. Control treatments with IgG1-b12-vcMMAE or dabrafenib plus trametinib (in combination) did not result in

significant tumor growth inhibition in this model (Figure 28). However, IgG1-AXL-107-vcMMAE (4 mg/kg) induced tumor regression, and tumor outgrowth was not observed until 25 days after discontinuation of treatment (day 39 after randomization) (Figure 28). Using a Mann-Whitney test, performed on day 33, it was shown that the differences in tumor size between IgG1-AXL-107-vcMMAE and IgG1-b12-vcMMAE treatment (p=0.0005), and between IgG1-AXL-107-vcMMAE and dabrafenib/trametinib treatment (p<0.0001) were highly significant.

Mice that received initiation treatment with dabrafenib plus trametinib and continued on treatment with dabrafenib plus trametinib or were treated with IgG1-AXL-107-vcMMAE alone showed significantly shorther survival compared to treatment with the triple combination of dabrafenib, trametinib, and IgG1-AXL-107-vcMMAE (p=0.0042 and p=0.0403, respectively; Figure 28C). Furthermore, mice treated with IgG1-AXL-107-vcMMAE after initial treatment with dabrafenib plus trametinib also showed significantly longer survival compared to mice that continued treatment with dabrafenib plus trametinib (p=0.0462; Figure 28C).

*Example 20 - Anti-tumor activity of IgG1-AXL-107-vcMMAE in combination with BRAFi/MEKi in a resistant BRAF mutant melanoma model (M009R) in vivo*

*BRAF* mutant M009R xenograft model derived from a malignant melanoma patient

The anti-tumor activity of IgG1-AXL-107-vcMMAE was evaluated in the subcutaneous melanoma model M009R in NMRI nude mice. The M009R PDX-derived cell culture was obtained as described in Example 18 and was derived from tumor material of a melanoma patient with a BRAF V600E-mutation who initially showed a response to vemurafenib, but acquired resistance to vemurafenib. Mice were subcutaneously injected in the left flank with $2.5x10^5$ M009R melanoma cells, which were resuspended 1:1 in matrigel in a total volume of 100 μL. Tumors were measured two times weekly with a caliper, and when tumors were 100 $mm^3$ on day 62 after tumor cell inoculation, the animals were randomized over the following 4 treatment groups (7 or 8 mice per group): IgG1-b12-vcMMAE ('control ADC'; 4 mg/kg, i.v.), IgG1-AXL-107-vcMMAE (4 mg/kg, i.v.), the BRAF-inhibitor dabrafenib (30 mg/kg, oral gavage) plus the MEK-inhibitor trametinib (0.1 mg/kg, oral gavage) plus IgG1-b12-vcMMAE ('control ADC'; 4 mg/kg, i.v.), and the dabrafenib (30 mg/kg, oral gavage) plus trametinib (0.1 mg/kg, oral gavage) plus IgG1-AXL-107-vcMMAE (4 mg/kg, i.v.).

On day 0 and day 7 after randomization the ADCs were injected into the tail vein of the animals in a total volume of 100 μL. Starting on the day of randomization, dabrafenib and trametinib were given orally on a daily basis until the end of the study. Animals were sacrificed when the size of the tumor exceeded 1000 $mm^3$.

Results

M009R cells show heterogeneous Axl expression (Example 18), consistent with clinical resistance of the patient from which this model was derived. Furthermore, IgG1-AXL-107-vcMMAE did not induce killing of M009R cells *in vitro* at a concentration of 1 μg/mL.

M009R cells were transplanted subcutaneously in nude mice and the antitumor efficacy of IgG1-AXL-107-vcMMAE alone or in combination with dabrafenib plus trametinib was evaluated. Treatment with control ADC (IgG1-b12-vcMMAE, 4 mg/kg) or IgG1-AXL-107-vcMMAE (4 mg/kg) did not result in significant tumor growth inhibition in this model (Figure 29A). Treatment with the control ADC in combination with dabrafenib plus trametinib (in combination) induced tumor growth inhibition, while IgG1-AXL-107-vcMMAE in combination with dabrafenib plus trametinib induced partial tumor regression (Figure 29A). Using a Mann-Whitney test performed on day 14, it was shown that the average tumor size in mice treated with the combination of IgG1-AXL-107-vcMMAE with dabrafenib plus trametinib was significantly smaller than in mice treated with control ADC (p=0.003), IgG1-AXL-107-vcMMAE (p=0.0002), control ADC in combination with dabrafenib plus trametinib (p=0.0034; Figure 29B).

*Example 21 - IgG1-AXL-107-vcMMAE induces cytotoxicity in NRAS mutant, MEKi resistant tumor cell lines*

Cell culture

SKMEL2, FM6 and BLM cell lines, all of which harbor a mutation in NRAS codon 61 (Table 19), were obtained from Thermo Fischer or ATCC. Melanoma cell lines were cultured in DMEM supplemented with 10% fetal bovine serum (Sigma), 100 U/ml penicillin and 0.1 mg/ml streptomycin (all Gibco). The cell lines were maintained at 37°C in a 5% (vol/vol) $CO_2$ humidified incubator.

Generation of trametinib resistant cell line

The MEK inhibitor sensitive cell line SKMEL2 was cultured for 2 to 3 months in the presence of increasing

concentration of the MEK inhibitor trametinib (Selleck Chemicals; Cat no: S2673) at concentrations up to 0.1 μM to establish the corresponding trametinib resistant SKMEL2R cell line.

Quantification of Axl expression on the plasma membrane of melanoma cell lines

Axl expression on the plasma membrane of human tumor cell lines was quantified by indirect immunofluorescence using QIFIKIT analysis (DAKO, Cat nr K0078). Axl was detected using the mouse monoclonal antibody ab89224 (Abcam, Cambridge, UK). Adherent cells were trypsinized and passed through a cell strainer to obtain single cell suspensions. Cells were pelleted by centrifugation for 5 minutes at 1,200 rpm, washed with PBS and resuspended at a concentration of $1\times10^6$ cells/mL. The next steps were performed on ice. 100 μL of the single cell suspensions (100,000 cells per well) were seeded in polystyrene 96-well round-bottom plates (Greiner Bio-One, Cat nr 650101). Cells were pelleted by centrifugation for 3 minutes at 300xg and resuspended in 50 μL antibody sample or mouse IgG1 isotype control sample (cat number QF2040741, lot number MA1-10406, Pierce) at a concentration of 10 μg/mL. After an incubation of 30 minutes at 4ºC, cells were pelleted and resuspended in 150 μL FACS buffer (PBS containing 0.1 % BSA). Set-up and calibration beads were added to the plate according to the manufacturer's instructions. Cells and beads in parallel were washed two more times with 150 μL FACS buffer and resuspended in 50 μL FITC-conjugated goat-antimouse IgG (1/50; DAKO, cat.no. K0078). Secondary antibody was incubated for 30 minutes at 4ºC in the dark. Cells and beads were washed twice with 150 μL FACS buffer and resuspended in 100 μL FACS buffer. Immunofluorescence was measured on a FACS Calibur (BD Biosciences) by recording 10,000 events within the gate of viable cells. The mean fluorescence intensity of the calibration beads was used to calculate the calibration curve using GraphPad Prism software (GraphPad Software, San Diego, CA, USA). For each cell line, the antibody binding capacity (ABC), an estimate for the number of Axl molecules expressed on the plasma membrane, was calculated using the mean fluorescence intensity of the Axl antibody-stained cells, based on the equation of the calibration curve (interpolation of unknowns from the standard curve, using GraphPad Software).

*In vitro* cytotoxicity

Cells were cultured to near confluency, after which cells were trypsinized, resuspended in culture medium and passed through a cell strainer (BD Falcon, cat.no. 352340) to obtain single cell suspensions. Cells were plated in a 96-well format at 2000 cells/well and IgG1-AXL-107-vcMMAE was added 4 hours after seeding. Serial dilutions (10-fold; final concentrations ranging from 0.0001 to 10 μg/mL) of IgG1-AXL-107-vcMMAE were prepared in culture medium and added to the plates. After 5 days of incubation at 37ºC, 5% $CO_2$, CellTiter-Glo Reagent (Promega; cat.no. G7571) was added to the wells and the Luminescent Cell Viability Assay (Promega, Madison, WI) was performed according to the manufacturer's protocol. Luminescence was measured by the Infinite M200 microplate reader (Tecan) and viability was calculated as follows: % viability = (luminescence sample of interest - luminescence PAO)/(average luminescence of control vehicle treated - luminescence PAO), with PAO representing treatment with 5 μM phenyl arsine oxide for 100% cell killing.

Results

Axl expression, as determined by Western blotting or flow cytometry, was noted in 2 out of 3 *NRAS* mutant cell lines (Table 19). Of interest, a fourth cell line (SKMEL2R) was derived from the SKMEL2 cell line by continuous exposure to the MEK inhibitor trametinib *in vitro.* The SKMEL2R cell line, which acquired resistance to trametinib, showed strong Axl expression, while the parental SKMEL2 cells, which were sensitive to trametinib, did not express detectable levels of Axl on the cell surface (Table 19). Figure 30 shows that IgG1-AXL-107-vcMMAE induced specific killing of melanoma cell lines with high Axl expression, SKMEL2R, FM6, and BLM, whereas SKMEL2 cells, which lacks Axl expression, were insensitive to treatment with IgG1-AXL-107-vcMMAE.

Thus, Axl expression was observed in *NRAS* mutant, malignant melanoma cell lines, including a cell line that acquired resistance to the MEK inhibitor trametinib. Furthermore, IgG1-AXL-107-vcMMAE induced cytotoxicity in Axl-expressing, NRAS mutant melanoma cell lines, demonstrating that Axl expression levels in these cells were sufficient to allow induction of cytotoxicity with IgG1-AXL-107-vcMMAE *in vitro.*

*Table 19. Characteristics of NRAS mutant melanoma cell lines*

| Cell line | Axl expressio n (western blot) | Axl expressio n (FACS) Receptor number (ABC) | BRA F | NRA S | PLX4720 sensitivity [a] | trametinib sensitivity [a] | IgG1-AXL-107-vcMMAE Sensitivity [b] |
|---|---|---|---|---|---|---|---|
| SKMEL2 | - | BLQ | wt | Q61R | insensitive | sensitive | insensitive |
| SKMEL2 R | + | 70222 | wt | Q61R | insensitive | insensitive | sensitive |
| FM6 | + | 22361 | wt | Q61K | insensitive | n.a. | sensitive |
| BLM | + | 10792 | wt | Q61R | insensitive | n.a. | sensitive |

Abbreviations used: FACS, fluorescence-activated cell sorting; ABC, antibody binding capacity; BLQ, Below Limit of Quantitation (<3300, lowest ABC value of calibration beads); wt, wild-type; n.a., not assessed
[a]Insensitive cell lines show no significant cell death at a concentration of 3 μM PLX4720 or 0.1 μM trametinib
[b]Insensitive cell lines show no significant cell death or cell death comparable to IgG1-b12-vcMMAE at a concentration of 1 μg/mL IgG1-AXL-107-vcMMAE.

*Example 22 -IHC analysis of Axl expression in advanced malignant melanoma tissues*

Immunohistochemistry

Expression of Axl was evaluated in freshly cut paraffin embedded and formalin fixated (FFPE) whole tissues sections obtained from patients with advanced malignant melanoma containing NRAS mutations (n=10). Staining was performed manually in Sequenza Slide Racks (Ted Pella Inc., Redding, CA, USA; cat. no. 36105).

Prior to staining, FFPE tissue slides were deparaffinized in 100% xylene (Sigma-Aldrich, cat. no. 16446; three times, 5 min.) and dehydrated in 96% ethanol (Sigma Aldrich, cat. no. 32294; two times, 5 min.) at RT. Thereafter, antigen retrieval was performed. IHC slides were incubated in citrate buffer (pH6; DAKO; cat. no. S2369) for 5 min. and blocked for endogenous peroxidase in citrate/phosphate buffer (0.43 M citric acid, 0.35 M Na2HPO4.2H2O; pH5.8) at RT for 15 min. Slides were incubated in 10% normal human serum (CLB/Sanquin, cat. no. K1146) in PBS, prior to incubation with primary antibodies. Axl expression was determined by incubation with 3 μg/mL rabbit polyclonal anti-human Axl antibody H-124 in PBS supplemented with 2% normal human serum at RT for 60 min. Slides were washed in PBS supplemented with 0.1% Tween-20 (twice, 3 min.) and binding of rabbit antibodies specific for Axl were detected with undiluted Bright Vision poly-HRP-anti-rabbit IgG. HRP was visualized with 3-amino-9-ethylcarbazole (AEC) chromophore (red color; Sigma, cat. no. A6926-100TAB); nuclei were counterstained with hematoxylin (DAKO, cat. no. S3309). Slides were analyzed in blinded fashion by a certified pathologist, who scored the percentage of Axl-positive tumor cells and staining intensity (1+, 2+, 3+) of Axl-positive tumor cells in each sample. For each tissue the H-score was calculated according to the following equation:

$$\text{H-score} = (\%\text{ of }1+\text{ cells x }1) + (\%\text{ of }2+\text{ cells x }2) + (\%\text{ of }3+\text{ cells x }3)$$

Results

Axl expression was detected in at least a subset of the tumor cells in 9/10 of the advanced, NRAS mutant melanoma tissues (Table 20; Figure 31). Staining intensity and percentage of Axl-positive tumor cells differed between patients.

*Table 20. Axl expression in a panel of advanced, NRAS mutant melanoma tissue samples*

| Sample | % Axl 1+ | % Axl 2+ | % Axl 3+ | Axl H-score |
|---|---|---|---|---|
| **B2** | 70 | 10 | 10 | 120 |
| **B3** | 50 | 10 | 0 | 70 |
| **B4** | 0 | 0 | 0 | 0 |
| **B6** | 60 | 10 | 10 | 110 |
| **B7** | 30 | 0 | 10 | 60 |

(continued)

| Sample | % Axl 1+ | % Axl 2+ | % Axl 3+ | Axl H-score |
|---|---|---|---|---|
| **B8** | 80 | 20 | 0 | 120 |
| **B9** | 60 | 0 | 0 | 60 |
| **B12** | 10 | 10 | 0 | 30 |
| **B15** | 60 | 10 | 10 | 110 |
| **B16** | 50 | 0 | 0 | 50 |

LIST OF REFERENCES

Bahadoran et al., J Clin Oncol; 2013 Jul 1; 31(19): e324-e326
Bansal et al., Oncotarget. 2015 Jun 20;6(17):15321-31
Blakely et al., Cancer Discov. 2012 Oct;2(10):872-5
Bleeker et al., J Immunol. 2004 Oct 1;173(7):4699-707.
Bollag et al., Nat Rev Drug Discov 2012 Nov;11(11):873-86
Brand et al., Clin Cancer Res. 2015 Jun 1;21(11):2601-12
Colombino et al., J Clin Oncol 2012;30(20):2522-9
Dahlman et al., Cancer Discov. 2012 Sep;2(9):791-7. Epub 2012 Jul 13.
Debruyne et al., Oncogene. 2015 Nov 30. doi: 10.1038/onc.2015.434
Dufies et al., Oncotarget. 2011 Nov;2(11):874-85
Elkabets et al., Cancer Cell. 2015 Apr 13;27(4):533-46
Greig et al., Drugs, 2016 Feb;76(2):263-73.
Herbst et al., Expert Opin Investig Drugs. 2007 Feb;16(2):239-49
Hilger et al., Int J Clin Pharmacol Ther. 2002 Dec;40(12):567-8.
Hong et al., Cancer Lett. 2008 Sep 18;268(2):314-24.
Hong et al., Cancer Res. 2013 Jan 1;73(1):331-40.
Hong et al., Clin Cancer Res. 2012 Apr 15;18(8):2326-35.
Huang et al., Cancer Res. 2010 Sep 15;70(18):7221-31. doi: 10.1158/0008-5472.CAN-10-0391
Kim et al., Curr Opin Mol Ther. 2004 Feb;6(1):96-103.
Kim et al., Mol Oncol. 2013 Dec;7(6):1093-102.
Konieczkowski et al., 2014, Cancer Discov 4: 816-827.
Li et al., Oncogene. 2008 Aug 7;27(34):4702-11.
Li et al., Cancer Lett. 2016 Jan 28;370(2):332-44.
Liu et al., Cancer Res. 2009 Sep 1;69(17):6871-8
Mahadevan et al., Oncotarget. 2015 Feb 10;6(4):1954-66
Mordant et al., Mol Cancer Ther. 2010 Feb;9(2):358-68
Müller et al., Nat Commun. 2014 Dec 15;5:5712
Park et al., Leukemia. 2015 Dec;29(12):2382-9
Pettazzoni et al., Cancer Research 2015;75: 1091-1101.
Pollack et al., J Pharmacol Exp Ther. 1999 Nov;291(2):739-48
Prewett et al., J Immunother Emphasis Tumor Immunol. 1996 Nov;19(6):419-27.
Sirotnak et al., Clin Cancer Res. 2000 Dec;6(12):4885-92.
Talavera et al., Cancer Res. 2009 Jul 15;69(14):5851-9
Tan et al., Lung Cancer. 2012 May;76(2):177-82.
Wilson et al., Cancer Res. 2014 Oct 15;74(20):5878-90
Wong et al., J Pharmacol Exp Ther. 2009 Apr;329(1):360-7.
Xia et al., Oncogene. 2002 Sep 12;21(41):6255-63.
Yang et al., Crit Rev Oncol Hematol. 2001 Apr;38(1):17-23.
Zhang et al., Nat Genet. 2012 Jul 1;44(8):852-60
Zhou et al., Oncogene. 2016 May;35(21):2687-97
WO 2014/174111; Pierre Fabré Medicament and Spirogen Sarl
WO 09/062690; U3 Pharma
WO 2010/130751; U3 Pharma

WO 2014/093707; Stanford University
EP 2 228 392 A1; Chugai
Yang et al., EORTC meeting 2013, Poster 493 (2013a)
Yang et al., Int. J. Cancer: 132, E74-E84 (2013b)
Paccez et al., Int. J. Cancer: 134, 1024-1033 (2014) (Epub 2013 Jun 4)
Leconet et al., Oncogene, 1-10 (2013)
Linger et al., Expert Opin. Ther. Targets, 14(10):1073-1090 (2010)
Li et al., Oncogene, 28, 3442-3455 (2009)
Ye et al., Oncogene, 1-11 (2010)
Alley et al., Current Opinion in Chem. Bio., 4, 529-537 (2010)
Iida et al., Anticancer Research, 34:1821-1828 (2014)
Tschuch et al., AACR-EORTC meeting 2015, Poster A10 (2015)
King et al., Cancer Res. 2006 Dec 1;66(23):11100-5.
Montagut et al., J.Cancer Res. 2008 Jun 15;68(12):4853-61.
Sequist et al., N Engl J Med. 2015 Aug 6;373(6):578-9
Li et al., Structure. 2008 Feb;16(2):216-27
Pedersen et al., Cancer Res. 2010 Jan 15;70(2):588-97.
Mishima et al., Cancer Res. 2001 Jul 15;61(14):5349-54
Lynette et al., J Chem Biol (2009) 2:131-151)
Uitdehaag et al., Br J Pharmacol. 2012 Jun; 166(3): 858-876.
Tsai et al. (Proc Natl Acad Sci U S A. 2008 Feb 26; 105(8): 3041-3046)
Sullivan, Curr Opin Oncol 2016 Mar;28(2):185-91
Bollag et al. Nature, 2010, 467(7315), 596-599.
Laquerre et al., 2009, EORTC International Conference. Abst B88.
Stuart et al., Cancer Res, 2012, 72(8 Supplement): 3790
Wilhelm et al., Cancer Res, 2004, 64(19), 7099-7109
Yamaguchi et al., Int J Oncol, 2011, 39(1), 23-31.
Hoeflich KP, et al. Cancer Res. 2012, 72(1), 210-219.
Huynh H, et al, Mol Cancer Therapy, 2007, 6 (1), 138-146
J Pheneger, et al. 2006, ACR Annual Scientific Meeting. Abst 794.
Iverson et al, Cancer Res, 2009, 69(17), 6839-6847.
Kim K, et al. Br J Haematol, 2010, 149(4), 537-549.
Sebolt-Leopold et al., Nat Med, 1999, 5(7), 810-816.
Tatake RJ, et al. Biochem Biophys Res Commun, 2008, 377(1), 120-125.
Aronov et al., J Med Chem 2009, 52(20), 6362-6368.
Ward et al., J Med Chem 2015;58(11):4790-4801.
WO 2012/175691; INSERM
WO 2012/175692; INSERM
WO 2013/064685; PF Medicament
WO 2013/090776; INSERM
WO 2009/063965; Chugai Pharmaceuticals
WO 2010/131733
Hfizi et al. et al., 2006, FEBS Journal, 273; 5231-5244
WO 2007/059782; Genmab A/S
Ward et al., Nature 341, 544-546 (1989)
Holt et al.; Trends Biotechnol. 2003 Nov;21(11):484-90
Revets et al.; Expert Opin Biol Ther. 2005 Jan;5(1):111-24
Bird et al., Science 242, 423-426 (1988)
Huston et al., PNAS USA 85, 5879-5883 (1988)
Fundamental Immunology Ch. 7 (Paul, W., ed., 2nd ed. Raven Press, N.Y. (1989)
Lefranc MP. et al., Nucleic Acids Research, 27 et al., 209-212, 1999
Brochet X. Nucl. Acids Res. 36, W503-508 (2008)
Korshunov et al, Clin Sci (Lond). 2012 Apr;122(8):361-8.
Sambrook et al, Molecular Cloning: A laboratory Manual, New York: Cold Spring Harbor Laboratory Press, 1989, Ch. 15
Kabat, E.A. et al., Sequences of proteins of immunological interest. 5th Edition - US Department of Health and Human Services, NIH publication No. 91-3242, pp 662,680,689 (1991)
WO 2004/010957; Seattle Genetics, Inc.

US 7,659,241; Seattle Genetics, Inc.
Wu *et al.,* Generation and Characterization of a Dual Variable Domain Immunoglobulin (DVD-Ig™) Molecule, In: Antibody Engineering, Springer Berlin Heidelberg (2010)
WO 2011/131746; Genmab A/S
WO/2002/020039; Trion Pharma/Fresenius Biotech
WO9850431; Genetech
WO2011117329; Roche
EP1870459; Amgen
WO2009089004; Amgen
US 2010/00155133; Chugai
WO 2010/129304; Oncomed
WO2007/110205; EMD Serono
WO 2010/015792; Regeneron
WO 11/143545; Pfizer/Rinat
WO 2012/058768: Zymeworks/Merck
WO 2011/028952; Xencor
WO 2009/080254; Roche
WO 2008/003116; F-Star
US 7,262,028; Crucell/Merus
US 7,612,181; Abbott
WO 2010/0226923; Unilever, Sanofi Aventis
US 7,951,918; Biogen Idec
CN 102250246; Changzhou Adam Biotech Inc
WO 2012/025525; Roche
WO 2012/025530; Roche
WO 2008/157379; Macrogenics
WO 2010/080538; Macrogenics
Goodman et al., Goodman and Gilman's The Pharmacological Basis Of Therapeutics, 8th Ed., Macmillan Publishing Co., 1990
Vitetta, Immunol. Today 14, 252 (1993)
US 5,194,594
US 2005/0238649
WO 2013/173391; Concortis Biosystems, Corp.
Junghans et al., in Cancer Chemotherapy and Biotherapy 655-686 (2d edition, Chafner and Longo, eds., Lippincott Raven (1996))
US 4,681,581
US 4,735,210
US 5,101,827
US 5,102,990
US 5,648,471
US 5,697,902
US 4,766,106
US 4,179,337
US 4,495,285
US 4,609,546
Hunter et al., Nature 144, 945 (1962), David et al., Biochemistry 13, 1014 (1974)
Pain et al., J. Immunol. Meth. 40, 219 (1981)
Nygren, J. Histochem. and Cytochem. 30, 407 (1982)
Antibody Engineering Handbook, edited by Osamu Kanemitsu, published by Chijin Shokan (1994)
WO 2002/083180; Syngenta BV
WO 2004/043493; Syngenta BV
WO 2007/018431; Syngenta BV
WO 2007/089149; Syngenta BV
WO 2009/017394; Syngenta BV
WO 2010/62171; Syngenta BV
US 6,989,452; Medarex
Remington: The Science and Practice of Pharmacy, 19th Edition, Gennaro, Ed., Mack Publishing Co., Easton, PA, 1995

Sustained and Controlled Release Drug Delivery Systems, J.R. Robinson, ed., Marcel Dekker, Inc., New York, 1978
Sykes and Johnston, Nat Biotech 17, 355-59 (1997)
US 6,077, 835
WO 00/70087
Schakowski et al., Mol Ther 3, 793-800 (2001)
WO 00/46147
Benvenisty and Reshef, PNAS USA 83, 9551-55 (1986)
Wigler et al., Cell 14, 725 (1978)
Coraro and Pearson, Somatic Cell Genetics 7, 603 (1981)
US 5,589,466
US 5,973,972
Van Heeke & Schuster, J Biol Chem 264, 5503-5509 (1989)
Ausubel et al., ed. Current Protocols in Molecular Biology, Greene Publishing and Wiley InterScience New York (1987)
Grant et al., Methods in Enzymol 153, 516-544 (1987)
Lonberg, N. et al., Nature 368, 856 859 (1994a)
Lonberg, N. Handbook of Experimental Pharmacology 113, 49 101 (1994b)
Lonberg, N. and Huszar, D., Intern. Rev. Immunol. Vol. 13 65 93 (1995)
Harding, F. and Lonberg, N. Ann. N.Y. Acad. Sci 764 536 546 (1995)
Taylor, L. et al., Nucleic Acids Research 20, 6287 6295 (1992)
Chen, J. et al., International Immunology 5, 647 656 (1993)
Tuaillon et al., J. Immunol. 152, 2912 2920 (1994)
Taylor, L. et al., International Immunology 6, 579 591 (1994)
Fishwild, D. et al., Nature Biotechnology 14, 845 851 (1996)
US 5,545,806
US 5,569,825
US 5,625,126
US 5,633,425
US 5,789,650
US 5,877,397
US 5,661,016
US 5,814,318
US 5,874,299
US 5,770,429
US 5,545,807
WO 98/024884
WO 94/025585
WO 93/001227
WO 92/022645
WO 92/003918
WO 01/009187
Shieh, Neoplasia 2005
Koorstra, Cancer Biol Ther 2009
Hector, Cancer Biol Ther 2010
Sun, Ann Oncol 2003
Srivastava (ed.), Radiolabeled Monoclonal Antibodies For Imaging And Therapy (Plenum Press 1988), Chase "Medical Applications of Radioisotopes," in Remington's Pharmaceutical Sciences, 18th Edition, Gennaro et al., (eds.), pp. 624-652 (Mack Publishing Co., 1990)
Brown, "Clinical Use of Monoclonal Antibodies," in Biotechnology And Pharmacy 227-49, Pezzuto et al., (eds.) (Chapman & Hall 1993)
US 5,057,313
US 6,331,175
US 5,635,483
US 5,780,588
Woyke et al (2001) Antimicrob. Agents and Chemother. 45(12): 3580-3584
US5663149
Pettit et al., (1998) Antimicrob. Agents and Chemother. 42:2961-2965
Senter et al., Proceedings of the American Association for Cancer Research. Volume 45, abstract number 623, presented March 28, 2004

US 2005/0238649
US 7,498,298; Seattle Genetics, Inc.
US 7,994,135; Seattle Genetics, Inc.
WO 2005/081711; Seattle Genetics, Inc.
Kozak et al. (1999) Gene 234: 187-208
EP 2 220 131; U3 Pharma
WO 2011/159980; Genentech
Barbas, CF. J Mol Biol. 1993 Apr 5;230(3):812-23
US 7,829,531; Seattle Genetics, Inc.
US 7,851,437; Seattle Genetics, Inc.
WO 2013/173392; Concortis Biosystems, Corp.
WO 2013/173393; Concortis Biosystems, Corp.
Sun et al. (2005) Bioconjugate Chem. 16: 1282-1290
McDonagh et al., (2006) Protein Eng. Design Sel. 19: 299-307
Alley et al., (2008) Bioconjugate Chem. 19: 759-765

## Claims

1. An antibody-drug conjugate (ADC) comprising an antibody binding to human AXL and a cytotoxic agent, for use in treating melanoma in a subject in combination with one or more inhibitors of the MAP kinase (MAPK) pathway, selected from a B-RAF (BRAF) inhibitor, a MEK inhibitor and an ERK inhibitor.

2. The ADC for the use according to claim 1, wherein the one or more inhibitors of the MAPK pathway comprise or consist of a BRAF inhibitor.

3. The ADC for the use according to claim 2, wherein the BRAF-inhibitor is selected from vemurafenib, dabrafenib, encorafenib, sorafenib, PLX4720, GDC-0879, RAF265, SB590885, AZ628, AB-024, TAK-580, BAL-3833, BGB-283, optionally wherein the melanoma exhibits a mutation in BRAF providing for inhibition of the kinase activity of the mutant BRAF by the BRAF inhibitor.

4. The ADC for the use according to claim 3, wherein the melanoma exhibits a mutation in BRAF.

5. The ADC for the use according to claim 4, wherein the mutation is in a BRAF residue selected from V600, L597 and K601, such as V600.

6. The ADC for the use according to claim 5, wherein the mutation in BRAF is selected from V600E, V600K, V600D, L597R and K601E, such as V600E.

7. The ADC for the use according to any one of claims 3 to 6, wherein the melanoma does not exhibit a mutation in NRAS selected from residue Q61, G12 and G13.

8. The ADC for the use according to claim 7, wherein the melanoma does not exhibit an activating mutation in NRAS.

9. The ADC for the use according to claim 1, wherein the one or more inhibitors of the MAPK pathway comprise or consist of a MEK inhibitor.

10. The ADC for the use according to claim 9, wherein the MEK-inhibitor is selected from trametinib, cobimetinib, binimetinib, selumetinib, refametinib, pimasertib, U0126-EtOH, PD184352, BIX 02189.

11. The ADC for the use according to any one of claims 9 to 10, wherein the melanoma exhibits a mutation in NRAS, such as in an NRAS residue selected from Q61, G12 and G13, such as Q61.

12. The ADC for the use according to claim 11, such as a mutation in NRAS selected from Q61R, Q61K, Q61L, G12D, G12S, G12C, G12V, G13D and G13R.

13. The ADC for the use according to claim 1, wherein the one or more inhibitors of the MAPK pathway comprise or consist of an ERK-inhibitor.

**14.** The ADC for the use according to claim 13, wherein the ERK inhibitor is selected from LTT-462, ulixertinib, SCH772984 and VTX11E.

**15.** The ADC for the use according to any one of the preceding claims, in combination with a BRAF-inhibitor and a MEK inhibitor.

**16.** The ADC for the use according to claim 15, wherein

(a) the BRAF-inhibitor is selected from vemurafenib, dabrafenib, encorafenib, sorafenib, GDC-0879, RAF265, SB590885, AZ628, AB-024, TAK-580, BAL-3833, BGB-283; and/or
(b) the MEK-inhibitor is selected from trametinib, cobimetinib, binimetinib, selumetinib, refametinib, pimasertib, U0126-EtOH, PD184352, BIX 02189.

**17.** The ADC for the use according to any one of claims 15 and 16, in combination with

(a) vemurafenib and trametinib;
(b) vemurafenib and cobimetinib;
(c) vemurafenib and binimetinib;
(d) vemurafenib and selumetinib;
(e) dabrafenib and trametinib;
(f) dabrafenib and cobimetinib;
(g) dabrafenib and binimetinib;
(h) dabrafenib and selumetinib;
(i) encorafenib and trametinib;
(j) encorafenib and cobimetinib;
(k) encorafenib and binimetinib;
(l) encorafenib and selumetinib;
(m) sorafenib and trametinib
(n) sorafenib and cobimetinib;
(o) sorafenib and binimetinib; or
(p) sorafenib and selumetinib,

optionally wherein the melanoma exhibits a BRAF mutation providing for inhibition of the kinase activity of the mutant BRAF by the BRAF inhibitor.

**18.** The ADC for the use according to claim 17, in combination with vemurafenib and trametinib.

**19.** The ADC for the use according to claim 17, in combination with dabrafenib and trametinib.

**20.** The ADC for the use according to any one of claims 16 to 18, wherein the BRAF mutation is in a BRAF residue selected from V600, L597 and K601, such as in V600.

**21.** The ADC for the use according to claim 20, wherein the BRAF mutation is selected from V600E, V600K, V600D, L597R and K601E, such as V600E.

**22.** The ADC for the use according to any one of claims 16 to 18, wherein the melanoma does not exhibit an NRAS mutation in a residue selected from Q61, G12 and G13.

**23.** The ADC for the use according to claim 22, wherein the melanoma does not exhibit an activating NRAS mutation.

**24.** The ADC for the use according to any one of the preceding claims, wherein the ADC and the one or more inhibitors of the MAPK pathway are administered simultaneously, separately or sequentially.

**25.** The ADC for the use according to any of the preceding claims, wherein the melanoma has intrinsic or acquired resistance to one or more inhibitors of the MAPK pathway.

**26.** The ADC for the use of any one of claims 1 to 24, wherein the melanoma is not resistant to the one or more inhibitors.

**27.** The ADC for the use according to any one of the preceding claims, wherein the ADC is administered every 1 week, every 2 weeks, every 3 weeks or three times over 4 weeks.

**28.** The ADC for the use according to any one of the preceding claims, wherein the ADC is administered at a dose of 0.02-30 mg/kg, such as about 0.05-10 mg/kg.

**29.** The ADC for the use according to any one of the preceding claims, wherein the cytotoxic agent is linked to the ADC with a linker.

**30.** The ADC for the use according to claim 29, wherein the linker is mc-vc-PAB and the cytotoxic agent is MMAE.

**31.** The ADC for the use according to any one of the preceding claims, wherein maximal antibody binding to human AXL in the presence of Gas6 is at least 90%, such as at least 95%, such as at least 97%, such as at least 99%, such as 100%, of binding in the absence of Gas6 as determined by a competition assay, wherein competition between said antibody binding to human AXL and said Gas6 is determined on A431 cells pre-incubated with Gas6 and without Gas6.

**32.** The ADC for the use according to any one of the preceding claims, wherein the antibody comprises at least one binding region comprising a VH region and a VL region selected from the group consisting of:

(a) a VH region comprising the CDR1, CDR2, and CDR3 sequences of SEQ ID Nos.: 36, 37, and 38, respectively; and a VL region comprising the CDR1, CDR2, and CDR3 sequences of SEQ ID Nos.: 39, GAS, and 40, respectively;
(b) a VH region comprising the CDR1, CDR2, and CDR3 sequences of SEQ ID Nos.: 46, 47, and 48, respectively; and a VL region comprising the CDR1, CDR2, and CDR3 sequences of SEQ ID Nos.: 49, AAS, and 50, respectively;
(c) a VH region comprising the CDR1, CDR2, and CDR3 sequences of SEQ ID Nos.: 114, 115, and 116, respectively, and a VL region comprising the CDR1, CDR2, and CDR3 sequences of SEQ ID Nos.: 117, DAS, and 118, respectively;
(d) a VH region comprising the CDR1, CDR2, and CDR3 sequences of SEQ ID Nos.: 51, 52, and 53, respectively; and a VL region comprising the CDR1, CDR2, and CDR3 sequences of SEQ ID Nos.: 55, GAS, and 56, respectively;
(e) a VH region comprising the CDR1, CDR2, and CDR3 sequences of SEQ ID Nos.: 51, 52, and 54, respectively; and a VL region comprising the CDR1, CDR2, and CDR3 sequences of SEQ ID Nos.: 55, GAS, and 56, respectively;
(f) a VH region comprising the CDR1, CDR2, and CDR3 sequences of SEQ ID Nos.: 57, 58, and 59, respectively; and a VL region comprising the CDR1, CDR2, and CDR3 sequences of SEQ ID Nos.: 60, GAS, and 61, respectively;
(g) a VH region comprising the CDR1, CDR2, and CDR3 sequences of SEQ ID Nos.: 62, 63, and 64, respectively; and a VL region comprising the CDR1, CDR2, and CDR3 sequences of SEQ ID Nos.: 65, GAS, and 66, respectively;
(h) a VH region comprising the CDR1, CDR2, and CDR3 sequences of SEQ ID Nos.: 67, 68, and 69, respectively; and a VL region comprising the CDR1, CDR2, and CDR3 sequences of SEQ ID Nos.: 70, GAS, and 71, respectively;
(i) a VH region comprising the CDR1, CDR2, and CDR3 sequences of SEQ ID Nos.: 72, 73, and 75, respectively; and a VL region comprising the CDR1, CDR2, and CDR3 sequences of SEQ ID Nos.: 76, ATS, and 77, respectively;
(j) a VH region comprising the CDR1, CDR2, and CDR3 sequences of SEQ ID Nos.: 72, 74, and 75, respectively; and a VL region comprising the CDR1, CDR2, and CDR3 sequences of SEQ ID Nos.: 76, ATS, and 77, respectively;
(k) a VH region comprising the CDR1, CDR2, and CDR3 sequences of SEQ ID Nos.: 78, 79, and 80, respectively; and a VL region comprising the CDR1, CDR2, and CDR3 sequences of SEQ ID Nos.: 81, AAS, and 82, respectively;
(l) a VH region comprising the CDR1, CDR2, and CDR3 sequences of SEQ ID Nos.: 83, 84, and 85, respectively; and a VL region comprising the CDR1, CDR2, and CDR3 sequences of SEQ ID Nos.: 86, GAS, and 87, respectively;
(m) a VH region comprising the CDR1, CDR2, and CDR3 sequences of SEQ ID Nos.: 88, 89, and 90, respectively; and a VL region comprising the CDR1, CDR2, and CDR3 sequences of SEQ ID Nos.: 91, GAS, and 92, respectively;

(n) a VH region comprising the CDR1, CDR2, and CDR3 sequences of SEQ ID Nos.: 93, 94, and 95, respectively; and a VL region comprising the CDR1, CDR2, and CDR3 sequences of SEQ ID Nos.: 96, GAS, and 97, respectively;
(o) a VH region comprising the CDR1, CDR2, and CDR3 sequences of SEQ ID Nos.: 98, 99, and 100, respectively; and a VL region comprising the CDR1, CDR2, and CDR3 sequences of SEQ ID Nos.: 101, DAS, and 102, respectively;
(p) a VH region comprising the CDR1, CDR2, and CDR3 sequences of SEQ ID Nos.: 103, 104, and 105, respectively; and a VL region comprising the CDR1, CDR2, and CDR3 sequences of SEQ ID Nos.: 106, GAS, and 107, respectively;
(q) a VH region comprising the CDR1, CDR2, and CDR3 sequences of SEQ ID Nos.: 108, 109, and 110, respectively; and a VL region comprising the CDR1, CDR2, and CDR3 sequences of SEQ ID Nos.: 112, AAS, and 113, respectively;
(r) a VH region comprising the CDR1, CDR2, and CDR3 sequences of SEQ ID Nos.: 108, 109, and 111, respectively; and a VL region comprising the CDR1, CDR2, and CDR3 sequences of SEQ ID Nos.: 112, AAS, and 113, respectively;
(s) a VH region comprising the CDR1, CDR2, and CDR3 sequences of SEQ ID Nos.: 41, 42, and 43, respectively; and a VL region comprising the CDR1, CDR2, and CDR3 sequences of SEQ ID Nos.: 44, AAS, and 45, respectively;
(t) a VH region comprising the CDR1, CDR2, and CDR3 sequences of SEQ ID Nos.: 93, 94, and 95, respectively, and a VL region comprising the CDR1, CDR2, and CDR3 sequences of SEQ ID Nos.: 128, XAS, wherein X is D or G, and 129, respectively;
(u) a VH region comprising the CDR1, CDR2, and CDR3 sequences of SEQ ID Nos.: 46, 119, and 120, respectively; and a VL region comprising CDR1, CDR2, and CDR3 sequences of SEQ ID Nos.: 49, AAS, and 50, respectively;
(v) a VH region comprising the CDR1, CDR2, and CDR3 sequences of SEQ ID Nos.: 123, 124, and 125, respectively; and a VL region comprising CDR1, CDR2, and CDR3 sequences of SEQ ID Nos.: 60, GAS, and 61, respectively;
(w) a VH region comprising the CDR1, CDR2, and CDR3 sequences of SEQ ID Nos.: 121, 109, and 122, respectively; and a VL region comprising the CDR1, CDR2, and CDR3 sequences of SEQ ID Nos.: 112, AAS, and 113, respectively; and
(x) a VH region comprising the CDR1, CDR2, and CDR3 sequences of SEQ ID Nos.: 93, 126, and 127, respectively; and a VL region comprising the CDR1, CDR2, and CDR3 sequences of SEQ ID Nos.: 96, GAS, and 97, respectively.

**33.** The ADC for the use according to any one of the preceding claims, wherein the antibody comprises at least one binding region comprising

(a) a VH region comprising the CDR1, CDR2, and CDR3 sequences of SEQ ID Nos.: 36, 37, and 38, respectively, and
(b) a VL region comprising the CDR1, CDR2, and CDR3 sequences of SEQ ID Nos.: 39, GAS, and 40, respectively.

**34.** The ADC for the use according to any one of the preceding claims, wherein the antibody comprises at least one binding region comprising a VH region and a VL region selected from the group consisting of:

(a) a VH region at least 90%, such as at least 95%, such as at least 97%, such as at least 99% identical to SEQ ID No: 1 and a VL region at least 90%, such as at least 95%, such as at least 97%, such as at least 99% identical to SEQ ID No: 2;
(b) a VH region at least 90%, such as at least 95%, such as at least 97%, such as at least 99% identical to SEQ ID No: 5 and a VL region at least 90%, such as at least 95%, such as at least 97%, such as at least 99% identical to SEQ ID No: 6;
(c) a VH region at least 90%, such as at least 95%, such as at least 97%, such as at least 99% identical to SEQ ID No: 34 and a VL region at least 90%, such as at least 95%, such as at least 97%, such as at least 99% identical to SEQ ID No: 35;
(d) a VH region at least 90%, such as at least 95%, such as at least 97%, such as at least 99% identical to SEQ ID No: 7 and a VL region at least 90%, such as at least 95%, such as at least 97%, such as at least 99% identical to SEQ ID No: 9;
(e) a VH region at least 90%, such as at least 95%, such as at least 97%, such as at least 99% identical to SEQ ID

No: 10 and a VL region at least 90%, such as at least 95%, such as at least 97%, such as at least 99% identical to SEQ ID No: 11;

(f) a VH region at least 90%, such as at least 95%, such as at least 97%, such as at least 99% identical to SEQ ID No: 16 and a VL region at least 90%, such as at least 95%, such as at least 97%, such as at least 99% identical to SEQ ID No: 18;

(g) a VH region at least 90%, such as at least 95%, such as at least 97%, such as at least 99% identical to SEQ ID No: 25 and a VL region at least 90%, such as at least 95%, such as at least 97%, such as at least 99% identical to SEQ ID No: 26;

(h) a VH region at least 90%, such as at least 95%, such as at least 97%, such as at least 99% identical to SEQ ID No: 31 and a VL region at least 90%, such as at least 95%, such as at least 97%, such as at least 99% identical to SEQ ID No: 33;

(i) a VH region at least 90%, such as at least 95%, such as at least 97%, such as at least 99% identical to SEQ ID No: 3 and a VL region at least 90%, such as at least 95%, such as at least 97%, such as at least 99% identical to SEQ ID No: 4;

(j) a VH region at least 90%, such as at least 95%, such as at least 97%, such as at least 99% identical to SEQ ID No:8 and a VL region at least 90%, such as at least 95%, such as at least 97%, such as at least 99% identical to SEQ ID No: 9;

(k) a VH region at least 90%, such as at least 95%, such as at least 97%, such as at least 99% identical to SEQ ID No:12 and a VL region at least 90%, such as at least 95%, such as at least 97%, such as at least 99% identical to SEQ ID No: 13;

(l) a VH region at least 90%, such as at least 95%, such as at least 97%, such as at least 99% identical to SEQ ID No:14 and a VL region at least 90%, such as at least 95%, such as at least 97%, such as at least 99% identical to SEQ ID No: 15;

(m) a VH region at least 90%, such as at least 95%, such as at least 97%, such as at least 99% identical to SEQ ID No:17 and a VL region at least 90%, such as at least 95%, such as at least 97%, such as at least 99% identical to SEQ ID No: 18;

(n) a VH region at least 90%, such as at least 95%, such as at least 97%, such as at least 99% identical to SEQ ID No:19 and a VL region at least 90%, such as at least 95%, such as at least 97%, such as at least 99% identical to SEQ ID No: 20;

(o) a VH region at least 90%, such as at least 95%, such as at least 97%, such as at least 99% identical to SEQ ID No:21 and a VL region at least 90%, such as at least 95%, such as at least 97%, such as at least 99% identical to SEQ ID No: 22;

(p) a VH region at least 90%, such as at least 95%, such as at least 97%, such as at least 99% identical to SEQ ID No:23 and a VL region at least 90%, such as at least 95%, such as at least 97%, such as at least 99% identical to SEQ ID No: 24;

(q) a VH region at least 90%, such as at least 95%, such as at least 97%, such as at least 99% identical to SEQ ID No:27 and a VL region at least 90%, such as at least 95%, such as at least 97%, such as at least 99% identical to SEQ ID No: 28;

(r) a VH region at least 90%, such as at least 95%, such as at least 97%, such as at least 99% identical to SEQ ID No:29 and a VL region at least 90%, such as at least 95%, such as at least 97%, such as at least 99% identical to SEQ ID No: 30; and

(s) a VH region at least 90%, such as at least 95%, such as at least 97%, such as at least 99% identical to SEQ ID No:32 and a VL region at least 90%, such as at least 95%, such as at least 97%, such as at least 99% identical to SEQ ID No: 33.

**35.** The ADC for the use according to any one of the preceding claims, wherein the antibody comprises at least one binding region comprising a VH region and a VL region selected from the group consisting of;

(a) a VH region comprising SEQ ID No: 1 and a VL region comprising SEQ ID No: 2;
(b) a VH region comprising SEQ ID No: 5 and a VL region comprising SEQ ID No: 6;
(c) a VH region comprising SEQ ID No: 34 and a VL region comprising SEQ ID No: 35;
(d) a VH region comprising SEQ ID No: 7 and a VL region comprising SEQ ID No: 9;
(e) a VH region comprising SEQ ID No: 10 and a VL region comprising SEQ ID No: 11;
(f) a VH region comprising SEQ ID No: 16 and a VL region comprising SEQ ID No: 18;
(g) a VH region comprising SEQ ID No: 25 and a VL region comprising SEQ ID No: 26;
(h) a VH region comprising SEQ ID No: 31 and a VL region comprising SEQ ID No: 33;
(i) a VH region comprising SEQ ID No: 3 and a VL region comprising SEQ ID No: 4;
(j) a VH region comprising SEQ ID No:8 and a VL region comprising SEQ ID No: 9;

(k) a VH region comprising SEQ ID No:12 and a VL region comprising SEQ ID No: 13;
(l) a VH region comprising SEQ ID No:14 and a VL region comprising SEQ ID No: 15;
(m) a VH region comprising SEQ ID No:17 and a VL region comprising SEQ ID No: 18;
(n) a VH region comprising SEQ ID No:19 and a VL region comprising SEQ ID No: 20;
(o) a VH region comprising SEQ ID No:21 and a VL region comprising SEQ ID No: 22;
(p) a VH region comprising SEQ ID No:23 and a VL region comprising SEQ ID No: 24;
(q) a VH region comprising SEQ ID No:27 and a VL region comprising SEQ ID No: 28;
(r) a VH region comprising SEQ ID No:29 and a VL region comprising SEQ ID No: 30; and
(s) a VH region comprising SEQ ID No:32 and a VL region comprising SEQ ID No: 33.

**36.** The ADC for the use according to any one of the preceding claims, wherein the antibody comprises at least one binding region comprising a VH region comprising SEQ ID No: 1 and a VL region comprising SEQ ID No: 2.

**37.** The ADC for the use according to any one of the preceding claims, wherein

the antibody comprises at least one binding region comprising a VH region comprising the CDR1, CDR2, and CDR3 sequences of SEQ ID Nos.: 36, 37, and 38, respectively; and a VL region comprising the CDR1, CDR2, and CDR3 sequences of SEQ ID Nos.: 39, GAS, and 40, respectively,
the linker is mc-vc-PAB, and
the cytotoxic agent is MMAE.

**38.** The ADC for the use according to any one of the preceding claims, wherein the antibody binds to an epitope on AXL wherein the epitope is recognized by any of the antibodies defined in claim 35.

**39.** The ADC for the use according to any one of the preceding claims, wherein the antibody binds to an epitope within the Ig1 domain of AXL, the epitope comprising or requiring one or more amino acids corresponding to positions L121 to Q129 or T112 to Q124 of human AXL.

**40.** The ADC for the use according to any one of claims 1 to 35, wherein the antibody binds to an epitope within the Ig2 domain of AXL, the epitope comprising or requiring the amino acids corresponding to position D170 or the combination of D179 and one or more amino acids corresponding to positions T182 to R190 of human AXL.

**41.** The ADC for the use according to any one of claims 1 to 35, wherein the antibody binds to an epitope within the FN1 domain of human AXL, the epitope comprises or requires one or more amino acids corresponding to positions Q272 to A287 and G297 to P301 of human AXL.

**42.** The ADC for the use according to any one of claims 1 to 35, wherein the antibody binds to an epitope within the FN2 domain of human AXL, the epitope comprises or requires the amino acids corresponding to positions A359, R386, and one or more amino acids corresponding to positions Q436 to K439 of human AXL.

**43.** The ADC for the use according to any one of the preceding claims, wherein the antibody comprises a heavy chain of an isotype selected from the group consisting of IgG1, IgG2, IgG3, and IgG4.

**44.** The ADC for the use of claim 43, wherein the isotype is IgG1, optionally allotype IgG1m(f).

**45.** The ADC for the use according to any one of the preceding claims, wherein the antibody is a full-length monoclonal antibody, such as a full-length monoclonal IgG1,κ antibody.

**46.** The ADC for the use according to any one of the preceding claims, wherein the ADC is comprised in a pharmaceutical composition comprising a pharmaceutical acceptable carrier.

**Patentansprüche**

**1.** Antikörper-Wirkstoff-Konjugat (ADC), umfassend einen Antikörper, der an humanes AXL bindet, und ein zytotoxisches Mittel, zur Verwendung bei der Behandlung von Melanom in einem Subjekt in Kombination mit einem oder mehreren Inhibitoren des MAP-Kinase (MAPK)-Wegs, ausgewählt aus einem B-RAF (BRAF)-Inhibitor, einem MEK-Inhibitor und einem ERK-Inhibitor.

2. ADC zur Verwendung nach Anspruch 1, wobei der eine oder die mehreren Inhibitoren des MAPK-Wegs einen BRAF-Inhibitor umfassen oder daraus bestehen.

3. ADC zur Verwendung nach Anspruch 2, wobei der BRAF-Inhibitor ausgewählt ist aus Vemurafenib, Dabrafenib, Encorafenib, Sorafenib, PLX4720, GDC-0879, RAF265, SB590885, AZ628, AB-024, TAK-580, BAL-3833, BGB-283, optional wobei das Melanom eine Mutation in BRAF aufweist, die eine Hemmung der Kinaseaktivität des mutierten BRAF durch den BRAF-Inhibitor bereitstellt.

4. ADC zur Verwendung nach Anspruch 3, wobei das Melanom eine Mutation in BRAF aufweist.

5. ADC zur Verwendung nach Anspruch 4, wobei die Mutation in einem BRAF-Rest vorliegt, der ausgewählt ist aus V600, L597 und K601, wie etwa V600.

6. ADC zur Verwendung nach Anspruch 5, wobei die Mutation in BRAF ausgewählt ist aus V600E, V600K, V600D, L597R und K601E, wie etwa V600E.

7. ADC zur Verwendung nach einem der Ansprüche 3 bis 6, wobei das Melanom keine Mutation in NRAS aufweist, die ausgewählt ist aus Rest Q61, G12 und G13.

8. ADC zur Verwendung nach Anspruch 7, wobei das Melanom keine aktivierende Mutation in NRAS aufweist.

9. ADC zur Verwendung nach Anspruch 1, wobei der eine oder die mehreren Inhibitoren des MAPK-Wegs einen MEK-Inhibitor umfassen oder daraus bestehen.

10. ADC zur Verwendung nach Anspruch 9, wobei der MEK-Inhibitor aus Trametinib, Cobimetinib, Binimetinib, Selumetinib, Refametinib, Pimasertib, U0126-EtOH, PD184352, BIX 02189 ausgewählt ist.

11. ADC zur Verwendung nach einem der Ansprüche 9 bis 10, wobei das Melanom eine Mutation in NRAS aufweist, wie etwa in einem NRAS-Rest, der ausgewählt ist aus Q61, G12 und G13, wie etwa Q61.

12. ADC zur Verwendung nach Anspruch 11, wie etwa eine Mutation in NRAS, die ausgewählt ist aus Q61R, Q61K, Q61L, G12D, G12S, G12C, G12V, G13D und G13R.

13. ADC zur Verwendung nach Anspruch 1, wobei der eine oder die mehreren Inhibitoren des MAPK-Wegs einen ERK-Inhibitor umfassen oder daraus bestehen.

14. ADC zur Verwendung nach Anspruch 13, wobei der ERK-Inhibitor ausgewählt ist aus LTT-462, Ulixertinib, SCH772984 und VTX11E.

15. ADC zur Verwendung nach einem der vorhergehenden Ansprüche, in Kombination mit einem BRAF-Inhibitor und einem MEK-Inhibitor.

16. ADC zur Verwendung nach Anspruch 15, wobei

(a) der BRAF-Inhibitor ausgewählt ist aus Vemurafenib, Dabrafenib, Encorafenib, Sorafenib, GDC-0879, RAF265, SB590885, AZ628, AB-024, TAK-580, BAL-3833, BGB-283; und/oder
(b) der MEK-Inhibitor ausgewählt ist aus Trametinib, Cobimetinib, Binimetinib, Selumetinib, Refametinib, Pimasertib, U0126-EtOH, PD184352, BIX 02189.

17. ADC zur Verwendung nach einem der Ansprüche 15 und 16, in Kombination mit

(a) Vemurafenib und Trametinib;
(b) Vemurafenib und Cobimetinib;
(c) Vemurafenib und Binimetinib;
(d) Vemurafenib und Selumetinib;
(e) Dabrafenib und Trametinib;
(f) Dabrafenib und Cobimetinib;
(g) Dabrafenib und Binimetinib;

(h) Dabrafenib und Selumetinib;
(i) Encorafenib und Trametinib;
(j) Encorafenib und Cobimetinib;
(k) Encorafenib und Binimetinib;
(1) Encorafenib und Selumetinib;
(m) Sorafenib und Trametinib;
(n) Sorafenib und Cobimetinib;
(o) Sorafenib und Binimetinib; oder
(p) Sorafenib und Selumetinib,

optional wobei das Melanom eine BRAF-Mutation aufweist, die eine Hemmung der Kinaseaktivität des mutierten BRAF durch den BRAF-Inhibitor bereitstellt.

**18.** ADC zur Verwendung nach Anspruch 17, in Kombination mit Vemurafenib und Trametinib.

**19.** ADC zur Verwendung nach Anspruch 17, in Kombination mit Dabrafenib und Trametinib.

**20.** ADC zur Verwendung nach einem der Ansprüche 16 bis 18, wobei die BRAF-Mutation in einem BRAF-Rest vorliegt, der ausgewählt ist aus V600, L597 und K601, wie etwa in V600.

**21.** ADC zur Verwendung nach Anspruch 20, wobei die BRAF-Mutation ausgewählt ist aus V600E, V600K, V600D, L597R und K601E, wie etwa V600E.

**22.** ADC zur Verwendung nach einem der Ansprüche 16 bis 18, wobei das Melanom keine NRAS-Mutation in einem Rest aufweist, der ausgewählt ist aus Q61, G12 und G13.

**23.** ADC zur Verwendung nach Anspruch 22, wobei das Melanom keine aktivierende NRAS-Mutation aufweist.

**24.** ADC zur Verwendung nach einem der vorhergehenden Ansprüche, wobei das ADC und der eine oder die mehreren Inhibitoren des MAPK-Wegs gleichzeitig, getrennt oder sequentiell verabreicht werden.

**25.** ADC zur Verwendung nach einem der vorhergehenden Ansprüche, wobei das Melanom eine intrinsische oder erworbene Resistenz gegenüber einem oder mehreren Inhibitoren des MAPK-Wegs aufweist.

**26.** ADC zur Verwendung nach einem der Ansprüche 1 bis 24, wobei das Melanom gegenüber dem einen oder den mehreren Inhibitoren nicht resistent ist.

**27.** ADC zur Verwendung nach einem der vorhergehenden Ansprüche, wobei das ADC alle 1 Woche, alle 2 Wochen, alle 3 Wochen oder dreimal über 4 Wochen verabreicht wird.

**28.** ADC zur Verwendung nach einem der vorhergehenden Ansprüche, wobei das ADC in einer Dosis von 0,02-30 mg/kg, wie etwa 0,05-10 mg/kg, verabreicht wird.

**29.** ADC zur Verwendung nach einem der vorhergehenden Ansprüche, wobei das zytotoxische Mittel mit einem Linker an das ADC gebunden ist.

**30.** ADC zur Verwendung nach Anspruch 29, wobei der Linker mc-vc-PAB ist und das zytotoxische Mittel MMAE ist.

**31.** ADC zur Verwendung nach einem der vorhergehenden Ansprüche, wobei die maximale Antikörperbindung an humanes AXL in Gegenwart von Gas6 mindestens 90%, wie mindestens 95%, wie mindestens 97%, wie mindestens 99%, wie 100%, der Bindung in Abwesenheit von Gas6 beträgt, wie durch einen Kompetitionstest bestimmt, wobei die Kompetition zwischen dem Antikörper, der an humanes AXL bindet, und dem Gas6 auf A431-Zellen bestimmt wird, die mit Gas6 und ohne Gas6 vorinkubiert wurden.

**32.** ADC zur Verwendung nach einem der vorhergehenden Ansprüche, wobei der Antikörper mindestens eine Bindungsregion umfasst, die eine VH-Region und eine VL-Region umfasst, ausgewählt aus der Gruppe bestehend aus:

(a) einer VH-Region, die die CDR1-, CDR2- und CDR3-Sequenzen von SEQ ID Nos.: 36, 37 bzw. 38 umfasst; und einer VL-Region, die die CDR1-, CDR2- und CDR3-Sequenzen von SEQ ID Nos.: 39, GAS bzw. 40 umfasst;

(b) einer VH-Region, die die CDR1-, CDR2- und CDR3-Sequenzen von SEQ ID Nos.: 46, 47 bzw. 48 umfasst; und einer VL-Region, die die CDR1-, CDR2- und CDR3-Sequenzen von SEQ ID Nos.: 49, AAS bzw. 50 umfasst;
(c) einer VH-Region, die die CDR1-, CDR2- und CDR3-Sequenzen von SEQ ID Nos.: 114, 115 bzw. 116 umfasst, und einer VL-Region, die die CDR1-, CDR2- und CDR3-Sequenzen von SEQ ID Nos.: 117, DAS bzw. 118 umfasst;
(d) einer VH-Region, die die CDR1-, CDR2- und CDR3-Sequenzen von SEQ ID Nos.: 51, 52 bzw. 53 umfasst; und einer VL-Region, die die CDR1-, CDR2- und CDR3-Sequenzen von SEQ ID Nos.: 55, GAS bzw. 56 umfasst;
(e) einer VH-Region, die die CDR1-, CDR2- und CDR3-Sequenzen von SEQ ID Nos.: 51, 52 bzw. 54 umfasst; und einer VL-Region, die die CDR1-, CDR2- und CDR3-Sequenzen von SEQ ID Nos.: 55, GAS bzw. 56 umfasst;
(f) einer VH-Region, die die CDR1-, CDR2- und CDR3-Sequenzen von SEQ ID Nos.: 57, 58 bzw. 59 umfasst; und einer VL-Region, die die CDR1-, CDR2- und CDR3-Sequenzen von SEQ ID Nos.: 60, GAS bzw. 61 umfasst;
(g) einer VH-Region, die die CDR1-, CDR2- und CDR3-Sequenzen von SEQ ID Nos.: 62, 63 bzw. 64 umfasst; und einer VL-Region, die die CDR1-, CDR2- und CDR3-Sequenzen von SEQ ID Nos.: 65, GAS bzw. 66 umfasst;
(h) einer VH-Region, die die CDR1-, CDR2- und CDR3-Sequenzen von SEQ ID Nos.: 67, 68 bzw. 69 umfasst; und einer VL-Region, die die CDR1-, CDR2- und CDR3-Sequenzen von SEQ ID Nos.: 70, GAS bzw. 71 umfasst;
(i) einer VH-Region, die die CDR1-, CDR2- und CDR3-Sequenzen von SEQ ID Nos.: 72, 73 bzw. 75 umfasst; und einer VL-Region, die die CDR1-, CDR2- und CDR3-Sequenzen von SEQ ID Nos.: 76, ATS bzw. 77 umfasst;
(j) einer VH-Region, die die CDR1-, CDR2- und CDR3-Sequenzen von SEQ ID Nos.: 72, 74 bzw. 75 umfasst; und einer VL-Region, die die CDR1-, CDR2- und CDR3-Sequenzen von SEQ ID Nos.: 76, ATS bzw. 77 umfasst;
(k) einer VH-Region, die die CDR1-, CDR2- und CDR3-Sequenzen von SEQ ID Nos.: 78, 79 bzw. 80 umfasst; und einer VL-Region, die die CDR1-, CDR2- und CDR3-Sequenzen von SEQ ID Nos.: 81, AAS bzw. 82 umfasst;
(1) einer VH-Region, die die CDR1-, CDR2- und CDR3-Sequenzen von SEQ ID Nos.: 83, 84 bzw. 85 umfasst; und einer VL-Region, die die CDR1-, CDR2- und CDR3-Sequenzen von SEQ ID Nos.: 86, GAS bzw. 87 umfasst;
(m) einer VH-Region, die die CDR1-, CDR2- und CDR3-Sequenzen von SEQ ID Nos.: 88, 89 bzw. 90 umfasst; und einer VL-Region, die die CDR1-, CDR2- und CDR3-Sequenzen von SEQ ID Nos.: 91, GAS bzw. 92 umfasst;
(n) einer VH-Region, die die CDR1-, CDR2- und CDR3-Sequenzen von SEQ ID Nos.: 93, 94 bzw. 95 umfasst; und einer VL-Region, die die CDR1-, CDR2- und CDR3-Sequenzen von SEQ ID Nos.: 96, GAS bzw. 97 umfasst;
(o) einer VH-Region, die die CDR1-, CDR2- und CDR3-Sequenzen von SEQ ID Nos.: 98, 99 bzw. 100 umfasst; und einer VL-Region, die die CDR1-, CDR2- und CDR3-Sequenzen von SEQ ID Nos.: 101, DAS bzw. 102 umfasst;
(p) einer VH-Region, die die CDR1-, CDR2- und CDR3-Sequenzen von SEQ ID Nos.: 103, 104 bzw. 105 umfasst; und einer VL-Region, die die CDR1-, CDR2- und CDR3-Sequenzen von SEQ ID Nos.: 106, GAS bzw. 107 umfasst;
(q) einer VH-Region, die die CDR1-, CDR2- und CDR3-Sequenzen von SEQ ID Nos.: 108, 109 bzw. 110 umfasst; und einer VL-Region, die die CDR1-, CDR2- und CDR3-Sequenzen von SEQ ID Nos.: 112, AAS bzw. 113 umfasst;
(r) einer VH-Region, die die CDR1-, CDR2- und CDR3-Sequenzen von SEQ ID Nos.: 108, 109 bzw. 111 umfasst; und einer VL-Region, die die CDR1-, CDR2- und CDR3-Sequenzen von SEQ ID Nos.: 112, AAS bzw. 113 umfasst;
(s) einer VH-Region, die die CDR1-, CDR2- und CDR3-Sequenzen von SEQ ID Nos.: 41, 42 bzw. 43 umfasst; und einer VL-Region, die die CDR1-, CDR2- und CDR3-Sequenzen von SEQ ID Nos.: 44, AAS bzw. 45 umfasst;
(t) einer VH-Region, die die CDR1-, CDR2- und CDR3-Sequenzen von SEQ ID Nos.: 93, 94 bzw. 95 umfasst, und einer VL-Region, die die CDR1-, CDR2- und CDR3-Sequenzen von SEQ ID Nos.: 128, XAS, wobei X D oder G ist, bzw. 129 umfasst;
(u) einer VH-Region, die die CDR1-, CDR2- und CDR3-Sequenzen von SEQ ID Nos.: 46, 119 bzw. 120 umfasst; und einer VL-Region, die die CDR1-, CDR2- und CDR3-Sequenzen von SEQ ID Nos.: 49, AAS bzw. 50 umfasst;
(v) einer VH-Region, die die CDR1-, CDR2- und CDR3-Sequenzen von SEQ ID Nos.: 123, 124 bzw. 125 umfasst; und einer VL-Region, die die CDR1-, CDR2- und CDR3-Sequenzen von SEQ ID Nos.: 60, GAS bzw. 61 umfasst;
(w) einer VH-Region, die die CDR1-, CDR2- und CDR3-Sequenzen von SEQ ID Nos.: 121, 109 bzw. 122 umfasst; und einer VL-Region, die die CDR1-, CDR2- und CDR3-Sequenzen von SEQ ID Nos.: 112, AAS bzw. 113 umfasst; und
(x) einer VH-Region, die die CDR1-, CDR2- und CDR3-Sequenzen von SEQ ID Nos.: 93, 126 bzw. 127 umfasst; und einer VL-Region, die die CDR1-, CDR2- und CDR3-Sequenzen von SEQ ID Nos.: 96, GAS bzw. 97 umfasst.

**33.** ADC zur Verwendung nach einem der vorhergehenden Ansprüche, wobei der Antikörper mindestens eine Bindungsregion umfasst, die Folgendes umfasst:

(a) eine VH-Region, die die CDR1-, CDR2- und CDR3-Sequenzen von SEQ ID Nos.: 36, 37 bzw. 38 umfasst, und

(b) eine VL-Region, die die CDR1-, CDR2- und CDR3-Sequenzen von SEQ ID Nos.: 39, GAS bzw. 40 umfasst.

**34.** ADC zur Verwendung nach einem der vorhergehenden Ansprüche, wobei der Antikörper mindestens eine Bindungsregion umfasst, die eine VH-Region und eine VL-Region umfasst, ausgewählt aus der Gruppe bestehend aus:

(a) einer VH-Region, die zu mindestens 90%, wie z. B. mindestens 95%, wie z. B. mindestens 97%, wie z. B. mindestens 99% mit SEQ ID No: 1 identisch ist, und einer VL-Region, die zu mindestens 90%, wie z. B. mindestens 95%, wie z. B. mindestens 97%, wie z. B. mindestens 99% mit SEQ ID No: 2 identisch ist;
(b) einer VH-Region, die zu mindestens 90%, wie z. B. mindestens 95%, wie z. B. mindestens 97%, wie z. B. mindestens 99% mit SEQ ID No: 5 identisch ist, und einer VL-Region, die zu mindestens 90%, wie z. B. mindestens 95%, wie z. B. mindestens 97%, wie z. B. mindestens 99% mit SEQ ID No: 6 identisch ist;
(c) einer VH-Region, die zu mindestens 90%, wie z. B. mindestens 95%, wie z. B. mindestens 97%, wie z. B. mindestens 99% mit SEQ ID No: 34 identisch ist, und einer VL-Region, die zu mindestens 90%, wie z. B. mindestens 95%, wie z. B. mindestens 97%, wie z. B. mindestens 99% mit SEQ ID No: 35 identisch ist;
(d) einer VH-Region, die zu mindestens 90%, wie z. B. mindestens 95%, wie z. B. mindestens 97%, wie z. B. mindestens 99% mit SEQ ID No: 7 identisch ist, und einer VL-Region, die zu mindestens 90%, wie z. B. mindestens 95%, wie z. B. mindestens 97%, wie z. B. mindestens 99% mit SEQ ID No: 9 identisch ist;
(e) einer VH-Region, die zu mindestens 90%, wie z. B. mindestens 95%, wie z. B. mindestens 97%, wie z. B. mindestens 99% mit SEQ ID No: 10 identisch ist, und einer VL-Region, die zu mindestens 90%, wie z. B. mindestens 95%, wie z. B. mindestens 97%, wie z. B. mindestens 99% mit SEQ ID No: 11 identisch ist;
(f) einer VH-Region, die zu mindestens 90%, wie z. B. mindestens 95%, wie z. B. mindestens 97%, wie z. B. mindestens 99% mit SEQ ID No: 16 identisch ist, und einer VL-Region, die zu mindestens 90%, wie z. B. mindestens 95%, wie z. B. mindestens 97%, wie z. B. mindestens 99% mit SEQ ID No: 18 identisch ist;
(g) einer VH-Region, die zu mindestens 90%, wie z. B. mindestens 95%, wie z. B. mindestens 97%, wie z. B. mindestens 99% mit SEQ ID No: 25 identisch ist, und einer VL-Region, die zu mindestens 90%, wie z. B. mindestens 95%, wie z. B. mindestens 97%, wie z. B. mindestens 99% mit SEQ ID No: 26 identisch ist;
(h) einer VH-Region, die zu mindestens 90%, wie z. B. mindestens 95%, wie z. B. mindestens 97%, wie z. B. mindestens 99% mit SEQ ID No: 31 identisch ist, und einer VL-Region, die zu mindestens 90%, wie z. B. mindestens 95%, wie z. B. mindestens 97%, wie z. B. mindestens 99% mit SEQ ID No: 33 identisch ist;
(i) einer VH-Region, die zu mindestens 90%, wie z. B. mindestens 95%, wie z. B. mindestens 97%, wie z. B. mindestens 99% mit SEQ ID No: 3 identisch ist, und einer VL-Region, die zu mindestens 90%, wie z. B. mindestens 95%, wie z. B. mindestens 97%, wie z. B. mindestens 99% mit SEQ ID No: 4 identisch ist;
(j) einer VH-Region, die zu mindestens 90%, wie z. B. mindestens 95%, wie z. B. mindestens 97%, wie z. B. mindestens 99% mit SEQ ID No: 8 identisch ist, und einer VL-Region, die zu mindestens 90%, wie z. B. mindestens 95%, wie z. B. mindestens 97%, wie z. B. mindestens 99% mit SEQ ID No: 9 identisch ist;
(k) einer VH-Region, die zu mindestens 90%, wie z. B. mindestens 95%, wie z. B. mindestens 97%, wie z. B. mindestens 99% mit SEQ ID No: 12 identisch ist, und einer VL-Region, die zu mindestens 90%, wie z. B. mindestens 95%, wie z. B. mindestens 97%, wie z. B. mindestens 99% mit SEQ ID No: 13 identisch ist;
(1) einer VH-Region, die zu mindestens 90%, wie z. B. mindestens 95%, wie z. B. mindestens 97%, wie z. B. mindestens 99% mit SEQ ID No: 14 identisch ist, und einer VL-Region, die zu mindestens 90%, wie z. B. mindestens 95%, wie z. B. mindestens 97%, wie z. B. mindestens 99% mit SEQ ID No: 15 identisch ist;
(m) einer VH-Region, die zu mindestens 90%, wie z. B. mindestens 95%, wie z. B. mindestens 97%, wie z. B. mindestens 99% mit SEQ ID No: 17 identisch ist, und einer VL-Region, die zu mindestens 90%, wie z. B. mindestens 95%, wie z. B. mindestens 97%, wie z. B. mindestens 99% mit SEQ ID No: 18 identisch ist;
(n) einer VH-Region, die zu mindestens 90%, wie z. B. mindestens 95%, wie z. B. mindestens 97%, wie z. B. mindestens 99% mit SEQ ID No: 19 identisch ist, und einer VL-Region, die zu mindestens 90%, wie z. B. mindestens 95%, wie z. B. mindestens 97%, wie z. B. mindestens 99% mit SEQ ID No: 20 identisch ist;
(o) einer VH-Region, die zu mindestens 90%, wie z. B. mindestens 95%, wie z. B. mindestens 97%, wie z. B. mindestens 99% mit SEQ ID No: 21 identisch ist, und einer VL-Region, die zu mindestens 90%, wie z. B. mindestens 95%, wie z. B. mindestens 97%, wie z. B. mindestens 99% mit SEQ ID No: 22 identisch ist;
(p) einer VH-Region, die zu mindestens 90%, wie z. B. mindestens 95%, wie z. B. mindestens 97%, wie z. B. mindestens 99% mit SEQ ID No: 23 identisch ist, und einer VL-Region, die zu mindestens 90%, wie z. B. mindestens 95%, wie z. B. mindestens 97%, wie z. B. mindestens 99% mit SEQ ID No: 24 identisch ist;
(q) einer VH-Region, die zu mindestens 90%, wie z. B. mindestens 95%, wie z. B. mindestens 97%, wie z. B. mindestens 99% mit SEQ ID No: 27 identisch ist, und einer VL-Region, die zu mindestens 90%, wie z. B. mindestens 95%, wie z. B. mindestens 97%, wie z. B. mindestens 99% mit SEQ ID No: 28 identisch ist;
(r) einer VH-Region, die zu mindestens 90%, wie z. B. mindestens 95%, wie z. B. mindestens 97%, wie z. B. mindestens 99% mit SEQ ID No: 29 identisch ist, und einer VL-Region, die zu mindestens 90%, wie z. B.

mindestens 95%, wie z. B. mindestens 97%, wie z. B. mindestens 99% mit SEQ ID No: 30 identisch ist; und
(s) einer VH-Region, die zu mindestens 90%, wie z. B. mindestens 95%, wie z. B. mindestens 97%, wie z. B. mindestens 99% mit SEQ ID No: 32 identisch ist, und
einer VL-Region, die zu mindestens 90%, wie z. B. mindestens 95%, wie z. B. mindestens 97%, wie z. B. mindestens 99% mit SEQ ID No: 33 identisch ist.

**35.** ADC zur Verwendung nach einem der vorhergehenden Ansprüche, wobei der Antikörper mindestens eine Bindungsregion umfasst, die eine VH-Region und eine VL-Region umfasst, ausgewählt aus der Gruppe bestehend aus:

(a) einer VH-Region, die SEQ ID No: 1 umfasst, und einer VL-Region, die SEQ ID No: 2 umfasst;
(b) einer VH-Region, die SEQ ID No: 5 umfasst, und einer VL-Region, die SEQ ID No: 6 umfasst;
(c) einer VH-Region, die SEQ ID No: 34 umfasst, und einer VL-Region, die SEQ ID No: 35 umfasst;
(d) einer VH-Region, die SEQ ID No: 7 umfasst, und einer VL-Region, die SEQ ID No: 9 umfasst;
(e) einer VH-Region, die SEQ ID No: 10 umfasst, und einer VL-Region, die SEQ ID No: 11 umfasst;
(f) einer VH-Region, die SEQ ID No: 16 umfasst, und einer VL-Region, die SEQ ID No: 18 umfasst;
(g) einer VH-Region, die SEQ ID No: 25 umfasst, und einer VL-Region, die SEQ ID No: 26 umfasst;
(h) einer VH-Region, die SEQ ID No: 31 umfasst, und einer VL-Region, die SEQ ID No: 33 umfasst;
(i) einer VH-Region, die SEQ ID No: 3 umfasst, und einer VL-Region, die SEQ ID No: 4 umfasst;
(j) einer VH-Region, die SEQ ID No: 8 umfasst, und einer VL-Region, die SEQ ID No: 9 umfasst;
(k) einer VH-Region, die SEQ ID No: 12 umfasst, und einer VL-Region, die SEQ ID No: 13 umfasst;
(1) einer VH-Region, die SEQ ID No: 14 umfasst, und einer VL-Region, die SEQ ID No: 15 umfasst;
(m) einer VH-Region, die SEQ ID No: 17 umfasst, und einer VL-Region, die SEQ ID No: 18 umfasst;
(n) einer VH-Region, die SEQ ID No: 19 umfasst, und einer VL-Region, die SEQ ID No: 20 umfasst;
(o) einer VH-Region, die SEQ ID No: 21 umfasst, und einer VL-Region, die SEQ ID No: 22 umfasst;
(p) einer VH-Region, die SEQ ID No: 23 umfasst, und einer VL-Region, die SEQ ID No: 24 umfasst;
(q) einer VH-Region, die SEQ ID No: 27 umfasst, und einer VL-Region, die SEQ ID No: 28 umfasst;
(r) einer VH-Region, die SEQ ID No: 29 umfasst, und einer VL-Region, die SEQ ID No: 30 umfasst; und
(s) einer VH-Region, die SEQ ID No: 32 umfasst, und einer VL-Region, die SEQ ID No: 33 umfasst.

**36.** ADC zur Verwendung nach einem der vorhergehenden Ansprüche, wobei der Antikörper mindestens eine Bindungsregion umfasst, die eine VH-Region, die SEQ ID No: 1 umfasst, und eine VL-Region, die SEQ ID No: 2 umfasst, umfasst.

**37.** ADC zur Verwendung nach einem der vorhergehenden Ansprüche, wobei

der Antikörper mindestens eine Bindungsregion umfasst, die eine VH-Region, die die CDR1-, CDR2- und CDR3-Sequenzen von SEQ ID Nos.: 36, 37 bzw. 38 umfasst, und eine VL-Region, die die CDR1-, CDR2- und CDR3-Sequenzen von SEQ ID Nos.: 39, GAS bzw. 40 umfasst, umfasst,
der Linker mc-vc-PAB ist und
das zytotoxische Mittel MMAE ist.

**38.** ADC zur Verwendung nach einem der vorhergehenden Ansprüche, wobei der Antikörper an ein Epitop auf AXL bindet, wobei das Epitop von einem der in Anspruch 35 definierten Antikörper erkannt wird.

**39.** ADC zur Verwendung nach einem der vorhergehenden Ansprüche, wobei der Antikörper an ein Epitop innerhalb der Ig1-Domäne von AXL bindet, wobei das Epitop eine oder mehrere Aminosäuren umfasst oder erfordert, die den Positionen L121 bis Q129 oder T112 bis Q124 von humanem AXL entsprechen.

**40.** ADC zur Verwendung nach einem der Ansprüche 1 bis 35, wobei der Antikörper an ein Epitop innerhalb der Ig2-Domäne von AXL bindet, wobei das Epitop die Aminosäuren umfasst oder erfordert, die der Position D170 oder der Kombination von D179 und einer oder mehreren Aminosäuren, die den Positionen T182 bis R190 von humanem AXL entsprechen, entsprechen.

**41.** ADC zur Verwendung nach einem der Ansprüche 1 bis 35, wobei der Antikörper an ein Epitop innerhalb der FN1-Domäne von humanem AXL bindet, wobei das Epitop eine oder mehrere Aminosäuren umfasst oder erfordert, die den Positionen Q272 bis A287 und G297 bis P301 von humanem AXL entsprechen.

**42.** ADC zur Verwendung nach einem der Ansprüche 1 bis 35, wobei der Antikörper an ein Epitop innerhalb der FN2-Domäne von humanem AXL bindet, wobei das Epitop die Aminosäuren umfasst oder erfordert, die den Positionen A359, R386 und einer oder mehreren Aminosäuren, die den Positionen Q436 bis K439 von humanem AXL entsprechen, entsprechen.

**43.** ADC zur Verwendung nach einem der vorhergehenden Ansprüche, wobei der Antikörper eine schwere Kette eines Isotyps umfasst, ausgewählt aus der Gruppe bestehend aus IgG1, IgG2, IgG3 und IgG4.

**44.** ADC zur Verwendung nach Anspruch 43, wobei der Isotyp IgG1, optional Allotyp IgG1m(f) ist.

**45.** ADC zur Verwendung nach einem der vorhergehenden Ansprüche, wobei der Antikörper ein monoklonaler Antikörper voller Länge ist, wie etwa ein monoklonaler IgG1,K-Antikörper voller Länge.

**46.** ADC zur Verwendung nach einem der vorhergehenden Ansprüche, wobei das ADC in einer pharmazeutischen Zusammensetzung enthalten ist, die einen pharmazeutisch annehmbaren Träger umfasst.

## Revendications

**1.** Conjugué anticorps-médicament (CAM) comprenant un anticorps qui se lie à la protéine AXL humaine et un agent cytotoxique, pour utilisation dans le traitement d'un mélanome chez un sujet, en combinaison avec un ou plusieurs inhibiteur(s) de la voie MAP kinase (MAPK), choisi(s) parmi un inhibiteur de B-RAF (BRAF), un inhibiteur de MEK et un inhibiteur de ERK.

**2.** CAM pour utilisation conforme à la revendication 1, dans lequel le ou les inhibiteur(s) de la voie MAPK comprennent un inhibiteur de BRAF ou consistent en un tel inhibiteur.

**3.** CAM pour utilisation conforme à la revendication 2, dans lequel l'inhibiteur de BRAF est choisi parmi les vémurafénib, dabrafénib, encorafénib, sorafénib, PLX4720, GDC-0879, RAF265, SB590885, AZ628, AB-024, TAK-580, BAL-3833, BGB-283, étant entendu, en option, que le mélanome présente une mutation de BRAF qui provoque l'inhibition de l'activité de kinase du BRAF mutant par l'inhibiteur de BRAF.

**4.** CAM pour utilisation conforme à la revendication 3, étant entendu que le mélanome présente une mutation de BRAF.

**5.** CAM pour utilisation conforme à la revendication 4, étant entendu que la mutation se trouve au niveau d'un résidu de BRAF choisi parmi les V600, L597, et K601, comme V600.

**6.** CAM pour utilisation conforme à la revendication 5, étant entendu que la mutation de BRAF est choisie parmi les V600E, V600K, V600D, L597R et K601E, comme V600E.

**7.** CAM pour utilisation conforme à l'une des revendications 3 à 6, étant entendu que le mélanome ne présente pas une mutation de NRAS affectant un résidu choisi parmi Q61, G12 et G13.

**8.** CAM pour utilisation conforme à la revendication 7, étant entendu que le mélanome ne présente pas une mutation activante de NRAS.

**9.** CAM pour utilisation conforme à la revendication 1, dans lequel le ou les inhibiteur(s) de la voie MAPK comprennent un inhibiteur de MEK ou consistent en un tel inhibiteur.

**10.** CAM pour utilisation conforme à la revendication 9, dans lequel l'inhibiteur de MEK est choisi parmi les tramétinib, cobimétinib, binimétinib, sélumétinib, réfamétinib, pimasertib, U0126-EtOH, PD184352, BIX 02189.

**11.** CAM pour utilisation conforme à l'une des revendications 9 et 10, étant entendu que le mélanome présente une mutation de NRAS, comme au niveau d'un résidu de NRAS choisi parmi les Q61, G12 et G13, comme Q61.

**12.** CAM pour utilisation conforme à la revendication 11, étant entendu que la mutation de NRAS est choisie parmi les Q61R, Q61K, Q61L, G12D, G12S, G12C, G12V, G13D et G13R.

**13.** CAM pour utilisation conforme à la revendication 1, dans lequel le ou les inhibiteur(s) de la voie MAPK comprennent un

inhibiteur d'ERK ou consistent en un tel inhibiteur

**14.** CAM pour utilisation conforme à la revendication 13, dans lequel l'inhibiteur d'ERK est choisi parmi les LTT-462, ulixertinib, SCH772984 et VTX11E.

**15.** CAM pour utilisation conforme à l'une des revendications précédentes, en combinaison avec un inhibiteur de BRAF et un inhibiteur de MEK.

**16.** CAM pour utilisation conforme à la revendication 15, dans lequel

a) l'inhibiteur de BRAF est choisi parmi les vémurafénib, dabrafénib, encorafénib, sorafénib, GDC-0879, RAF265, SB590885, AZ628, AB-024, TAK-580, BAL-3833, BGB-283,
b) et/ou l'inhibiteur de MEK est choisi parmi les tramétinib, cobimétinib, binimétinib, sélumétinib, réfamétinib, pimasertib, U0126-EtOH, PD184352, BIX 02189.

**17.** CAM pour utilisation conforme à l'une des revendications 15 et 16, en combinaison avec

a) vémurafénib et tramétinib,
b) vémurafénib et cobimétinib,
c) vémurafénib et binimétinib,
d) vémurafénib et sélumétinib,
e) dabrafénib et tramétinib,
f) dabrafénib et cobimétinib,
g) dabrafénib et binimétinib,
h) dabrafénib et sélumétinib,
i) encorafénib et tramétinib,
j) encorafénib et cobimétinib,
k) encorafénib et binimétinib,
l) encorafénib et sélumétinib,
m) sorafénib et tramétinib,
n) sorafénib et cobimétinib,
o) sorafénib et binimétinib,
p) ou sorafénib et sélumétinib,

étant entendu que, en option, le mélanome présente une mutation de BRAF qui provoque l'inhibition de l'activité de kinase du BRAF mutant par l'inhibiteur de BRAF.

**18.** CAM pour utilisation conforme à la revendication 17, en combinaison avec vémurafénib et tramétinib.

**19.** CAM pour utilisation conforme à la revendication 17, en combinaison avec dabrafénib et tramétinib.

**20.** CAM pour utilisation conforme à l'une des revendications 16 à 18, étant entendu que la mutation de BRAF se trouve au niveau d'un résidu de BRAF choisi parmi les V600, L597, et K601, comme V600.

**21.** CAM pour utilisation conforme à la revendication 20, étant entendu que la mutation de BRAF est choisie parmi les V600E, V600K, V600D, L597R et K601E, comme V600E.

**22.** CAM pour utilisation conforme à l'une des revendications 16 à 18, étant entendu que le mélanome ne présente pas une mutation de NRAS affectant un résidu choisi parmi Q61, G12 et G13.

**23.** CAM pour utilisation conforme à la revendication 22, étant entendu que le mélanome ne présente pas une mutation activante de NRAS.

**24.** CAM pour utilisation conforme à l'une des revendications précédentes, étant entendu que le CAM et le ou les inhibiteurs de la voie MAPK sont administrés simultanément, séparément, ou séquentiellement.

**25.** CAM pour utilisation conforme à l'une des revendications précédentes, étant entendu que le mélanome présente une résistance intrinsèque ou acquise à un ou plusieurs inhibiteur(s) de la voie MAPK.

**26.** CAM pour utilisation conforme à l'une des revendications 1 à 24, étant entendu que le mélanome n'est pas résistant à l'inhibiteur ou aux inhibiteurs.

**27.** CAM pour utilisation conforme à l'une des revendications précédentes, lequel CAM est administré chaque semaine, toutes les deux semaines, toutes les trois semaines, ou trois fois en quatre semaines.

**28.** CAM pour utilisation conforme à l'une des revendications précédentes, lequel CAM est administré en une dose de 0,02 à 30 mg/kg, comme d'environ 0,05 à 10 mg/kg.

**29.** CAM pour utilisation conforme à l'une des revendications précédentes, dans lequel l'agent cytotoxique est lié au sein du CAM au moyen d'un raccord.

**30.** CAM pour utilisation conforme à la revendication 29, dans lequel le raccord est mc-vc-PAB et l'agent cytotoxique est MMAE.

**31.** CAM pour utilisation conforme à l'une des revendications précédentes, dans lequel la liaison maximale de l'anticorps à l'AXL humaine en présence de facteur Gas6 représente au moins 90 %, comme au moins 95 %, comme au moins 97 %, comme au moins 99 %, comme 100 %, de cette liaison en l'absence de Gas6, déterminée dans un test par compétition, étant entendu que la compétition entre ledit anticorps se liant à l'AXL humaine et ledit Gas6 est évaluée sur des cellules A431 qu'on a fait incuber au préalable avec Gas6 et sans Gas6.

**32.** CAM pour utilisation conforme à l'une des revendications précédentes, dans lequel l'anticorps comprend au moins un domaine de liaison comportant une région VH et une région VL choisies dans l'ensemble constitué par les suivantes :

a) une région VH comprenant pour séquences CDR1, CDR2 et CDR3, respectivement, les Séquences N° 36, N° 37 et N° 38, et une région VL comprenant pour séquences CDR1, CDR2 et CDR3, respectivement, les Séquences N° 39, GAS et N° 40 ;
b) une région VH comprenant pour séquences CDR1, CDR2 et CDR3, respectivement, les Séquences N° 46, N° 47 et N° 48, et une région VL comprenant pour séquences CDR1, CDR2 et CDR3, respectivement, les Séquences N° 49, AAS et N° 50 ;
c) une région VH comprenant pour séquences CDR1, CDR2 et CDR3, respectivement, les Séquences N° 114, N° 115 et N° 116, et une région VL comprenant pour séquences CDR1, CDR2 et CDR3, respectivement, les Séquences N° 117, DAS et N° 118 ;
d) une région VH comprenant pour séquences CDR1, CDR2 et CDR3, respectivement, les Séquences N° 51, N° 52 et N° 53, et une région VL comprenant pour séquences CDR1, CDR2 et CDR3, respectivement, les Séquences N° 55, GAS et N° 56 ;
e) une région VH comprenant pour séquences CDR1, CDR2 et CDR3, respectivement, les Séquences N° 51, N° 52 et N° 54, et une région VL comprenant pour séquences CDR1, CDR2 et CDR3, respectivement, les Séquences N° 55, GAS et N° 56 ;
f) une région VH comprenant pour séquences CDR1, CDR2 et CDR3, respectivement, les Séquences N° 57, N° 58 et N° 59, et une région VL comprenant pour séquences CDR1, CDR2 et CDR3, respectivement, les Séquences N° 60, GAS et N° 61 ;
g) une région VH comprenant pour séquences CDR1, CDR2 et CDR3, respectivement, les Séquences N° 62, N° 63 et N° 64, et une région VL comprenant pour séquences CDR1, CDR2 et CDR3, respectivement, les Séquences N° 65, GAS et N° 66 ;
h) une région VH comprenant pour séquences CDR1, CDR2 et CDR3, respectivement, les Séquences N° 67, N° 68 et N° 69, et une région VL comprenant pour séquences CDR1, CDR2 et CDR3, respectivement, les Séquences N° 70, GAS et N° 71 ;
i) une région VH comprenant pour séquences CDR1, CDR2 et CDR3, respectivement, les Séquences N° 72, N° 73 et N° 75, et une région VL comprenant pour séquences CDR1, CDR2 et CDR3, respectivement, les Séquences N° 76, ATS et N° 77 ;
j) une région VH comprenant pour séquences CDR1, CDR2 et CDR3, respectivement, les Séquences N° 72, N° 74 et N° 75, et une région VL comprenant pour séquences CDR1, CDR2 et CDR3, respectivement, les Séquences N° 76, ATS et N° 77 ;
k) une région VH comprenant pour séquences CDR1, CDR2 et CDR3, respectivement, les Séquences N° 78, N° 79 et N° 80, et une région VL comprenant pour séquences CDR1, CDR2 et CDR3, respectivement, les Séquences N° 81, AAS et N° 82 ;
l) une région VH comprenant pour séquences CDR1, CDR2 et CDR3, respectivement, les Séquences N° 83, N°

84 et N° 85, et une région VL comprenant pour séquences CDR1, CDR2 et CDR3, respectivement, les Séquences N° 86, GAS et N° 87 ;
m) une région VH comprenant pour séquences CDR1, CDR2 et CDR3, respectivement, les Séquences N° 88, N° 89 et N° 90, et une région VL comprenant pour séquences CDR1, CDR2 et CDR3, respectivement, les Séquences N° 91, GAS et N° 92 ;
n) une région VH comprenant pour séquences CDR1, CDR2 et CDR3, respectivement, les Séquences N° 93, N° 94 et N° 95, et une région VL comprenant pour séquences CDR1, CDR2 et CDR3, respectivement, les Séquences N° 96, GAS et N° 97 ;
o) une région VH comprenant pour séquences CDR1, CDR2 et CDR3, respectivement, les Séquences N° 98, N° 99 et N° 100, et une région VL comprenant pour séquences CDR1, CDR2 et CDR3, respectivement, les Séquences N° 101, DAS et N° 102 ;
p) une région VH comprenant pour séquences CDR1, CDR2 et CDR3, respectivement, les Séquences N° 103, N° 104 et N° 105, et une région VL comprenant pour séquences CDR1, CDR2 et CDR3, respectivement, les Séquences N° 106, GAS et N° 107 ;
q) une région VH comprenant pour séquences CDR1, CDR2 et CDR3, respectivement, les Séquences N° 108, N° 109 et N° 110, et une région VL comprenant pour séquences CDR1, CDR2 et CDR3, respectivement, les Séquences N° 112, AAS et N° 113 ;
r) une région VH comprenant pour séquences CDR1, CDR2 et CDR3, respectivement, les Séquences N° 108, N° 109 et N° 111, et une région VL comprenant pour séquences CDR1, CDR2 et CDR3, respectivement, les Séquences N° 112, AAS et N° 113 ;
s) une région VH comprenant pour séquences CDR1, CDR2 et CDR3, respectivement, les Séquences N° 41, N° 42 et N° 43, et une région VL comprenant pour séquences CDR1, CDR2 et CDR3, respectivement, les Séquences N° 44, AAS et N° 45 ;
t) une région VH comprenant pour séquences CDR1, CDR2 et CDR3, respectivement, les Séquences N° 93, N° 94 et N° 95, et une région VL comprenant pour séquences CDR1, CDR2 et CDR3, respectivement, les Séquences N° 128, XAS, où X représente D ou G, et N° 129 ;
u) une région VH comprenant pour séquences CDR1, CDR2 et CDR3, respectivement, les Séquences N° 46, N° 119 et N° 120, et une région VL comprenant pour séquences CDR1, CDR2 et CDR3, respectivement, les Séquences N° 49, AAS et N° 50 ;
v) une région VH comprenant pour séquences CDR1, CDR2 et CDR3, respectivement, les Séquences N° 123, N° 124 et N° 125, et une région VL comprenant pour séquences CDR1, CDR2 et CDR3, respectivement, les Séquences N° 60, GAS et N° 61 ;
w) une région VH comprenant pour séquences CDR1, CDR2 et CDR3, respectivement, les Séquences N° 121, N ° 109 et N° 122, et une région VL comprenant pour séquences CDR1, CDR2 et CDR3, respectivement, les Séquences N° 112, AAS et N° 113 ;
x) une région VH comprenant pour séquences CDR1, CDR2 et CDR3, respectivement, les Séquences N° 93, N° 126 et N° 127, et une région VL comprenant pour séquences CDR1, CDR2 et CDR3, respectivement, les Séquences N° 96, GAS et N° 97.

**33.** CAM pour utilisation conforme à l'une des revendications précédentes, dans lequel l'anticorps comprend au moins un domaine de liaison comportant :

a) une région VH comprenant pour séquences CDR1, CDR2 et CDR3, respectivement, les Séquences N° 36, N° 37 et N° 38,
b) et une région VL comprenant pour séquences CDR1, CDR2 et CDR3, respectivement, les Séquences N° 39, GAS et N° 40.

**34.** CAM pour utilisation conforme à l'une des revendications précédentes, dans lequel l'anticorps comprend au moins un domaine de liaison comportant une région VH et une région VL choisies dans l'ensemble constitué par les suivantes :

a) une région VH identique, pour au moins 90 %, comme au moins 95 %, comme au moins 97 %, comme au moins 99 %, à la Séquence N° 1, et une région VL identique, pour au moins 90 %, comme au moins 95 %, comme au moins 97 %, comme au moins 99 %, à la Séquence N° 2 ;
b) une région VH identique, pour au moins 90 %, comme au moins 95 %, comme au moins 97 %, comme au moins 99 %, à la Séquence N° 5, et une région VL identique, pour au moins 90 %, comme au moins 95 %, comme au moins 97 %, comme au moins 99 %, à la Séquence N° 6 ;
c) une région VH identique, pour au moins 90 %, comme au moins 95 %, comme au moins 97 %, comme au moins 99 %, à la Séquence N° 34, et une région VL identique, pour au moins 90 %, comme au moins 95 %, comme au

moins 97 %, comme au moins 99 %, à la Séquence N° 35 ;

d) une région VH identique, pour au moins 90 %, comme au moins 95 %, comme au moins 97 %, comme au moins 99 %, à la Séquence N° 7, et une région VL identique, pour au moins 90 %, comme au moins 95 %, comme au moins 97 %, comme au moins 99 %, à la Séquence N° 9 ;

e) une région VH identique, pour au moins 90 %, comme au moins 95 %, comme au moins 97 %, comme au moins 99 %, à la Séquence N° 10, et une région VL identique, pour au moins 90 %, comme au moins 95 %, comme au moins 97 %, comme au moins 99 %, à la Séquence N° 11 ;

f) une région VH identique, pour au moins 90 %, comme au moins 95 %, comme au moins 97 %, comme au moins 99 %, à la Séquence N° 16, et une région VL identique, pour au moins 90 %, comme au moins 95 %, comme au moins 97 %, comme au moins 99 %, à la Séquence N° 18 ;

g) une région VH identique, pour au moins 90 %, comme au moins 95 %, comme au moins 97 %, comme au moins 99 %, à la Séquence N° 25, et une région VL identique, pour au moins 90 %, comme au moins 95 %, comme au moins 97 %, comme au moins 99 %, à la Séquence N° 26 ;

h) une région VH identique, pour au moins 90 %, comme au moins 95 %, comme au moins 97 %, comme au moins 99 %, à la Séquence N° 31, et une région VL identique, pour au moins 90 %, comme au moins 95 %, comme au moins 97 %, comme au moins 99 %, à la Séquence N° 33 ;

i) une région VH identique, pour au moins 90 %, comme au moins 95 %, comme au moins 97 %, comme au moins 99 %, à la Séquence N° 3, et une région VL identique, pour au moins 90 %, comme au moins 95 %, comme au moins 97 %, comme au moins 99 %, à la Séquence N° 4 ;

j) une région VH identique, pour au moins 90 %, comme au moins 95 %, comme au moins 97 %, comme au moins 99 %, à la Séquence N° 8, et une région VL identique, pour au moins 90 %, comme au moins 95 %, comme au moins 97 %, comme au moins 99 %, à la Séquence N° 9 ;

k) une région VH identique, pour au moins 90 %, comme au moins 95 %, comme au moins 97 %, comme au moins 99 %, à la Séquence N° 12, et une région VL identique, pour au moins 90 %, comme au moins 95 %, comme au moins 97 %, comme au moins 99 %, à la Séquence N° 13 ;

l) une région VH identique, pour au moins 90 %, comme au moins 95 %, comme au moins 97 %, comme au moins 99 %, à la Séquence N° 14, et une région VL identique, pour au moins 90 %, comme au moins 95 %, comme au moins 97 %, comme au moins 99 %, à la Séquence N° 15 ;

m) une région VH identique, pour au moins 90 %, comme au moins 95 %, comme au moins 97 %, comme au moins 99 %, à la Séquence N° 17, et une région VL identique, pour au moins 90 %, comme au moins 95 %, comme au moins 97 %, comme au moins 99 %, à la Séquence N° 18 ;

n) une région VH identique, pour au moins 90 %, comme au moins 95 %, comme au moins 97 %, comme au moins 99 %, à la Séquence N° 19, et une région VL identique, pour au moins 90 %, comme au moins 95 %, comme au moins 97 %, comme au moins 99 %, à la Séquence N° 20 ;

o) une région VH identique, pour au moins 90 %, comme au moins 95 %, comme au moins 97 %, comme au moins 99 %, à la Séquence N° 21, et une région VL identique, pour au moins 90 %, comme au moins 95 %, comme au moins 97 %, comme au moins 99 %, à la Séquence N° 22 ;

p) une région VH identique, pour au moins 90 %, comme au moins 95 %, comme au moins 97 %, comme au moins 99 %, à la Séquence N° 23, et une région VL identique, pour au moins 90 %, comme au moins 95 %, comme au moins 97 %, comme au moins 99 %, à la Séquence N° 24 ;

q) une région VH identique, pour au moins 90 %, comme au moins 95 %, comme au moins 97 %, comme au moins 99 %, à la Séquence N° 27, et une région VL identique, pour au moins 90 %, comme au moins 95 %, comme au moins 97 %, comme au moins 99 %, à la Séquence N° 28 ;

r) une région VH identique, pour au moins 90 %, comme au moins 95 %, comme au moins 97 %, comme au moins 99 %, à la Séquence N° 29, et une région VL identique, pour au moins 90 %, comme au moins 95 %, comme au moins 97 %, comme au moins 99 %, à la Séquence N° 30 ;

s) une région VH identique, pour au moins 90 %, comme au moins 95 %, comme au moins 97 %, comme au moins 99 %, à la Séquence N° 32, et une région VL identique, pour au moins 90 %, comme au moins 95 %, comme au moins 97 %, comme au moins 99 %, à la Séquence N° 33.

**35.** CAM pour utilisation conforme à l'une des revendications précédentes, dans lequel l'anticorps comprend au moins un domaine de liaison comportant une région VH et une région VL choisies dans l'ensemble constitué par les suivantes :

a) une région VH comprenant la Séquence N° 1, et une région VL comprenant la Séquence N° 2 ;

b) une région VH comprenant la Séquence N° 5, et une région VL comprenant la Séquence N° 6 ;

c) une région VH comprenant la Séquence N° 34, et une région VL comprenant la Séquence N° 35 ;

d) une région VH comprenant la Séquence N° 7, et une région VL comprenant la Séquence N° 9 ;

e) une région VH comprenant la Séquence N° 10, et une région VL comprenant la Séquence N° 11 ;

f) une région VH comprenant la Séquence N° 16, et une région VL comprenant la Séquence N° 18 ;
g) une région VH comprenant la Séquence N° 25, et une région VL comprenant la Séquence N° 26 ;
h) une région VH comprenant la Séquence N° 31, et une région VL comprenant la Séquence N° 33 ;
i) une région VH comprenant la Séquence N° 3, et une région VL comprenant la Séquence N° 4 ;
j) une région VH comprenant la Séquence N° 8, et une région VL comprenant la Séquence N° 9 ;
k) une région VH comprenant la Séquence N° 12, et une région VL comprenant la Séquence N° 13 ;
l) une région VH comprenant la Séquence N° 14, et une région VL comprenant la Séquence N° 15 ;
m) une région VH comprenant la Séquence N° 17, et une région VL comprenant la Séquence N° 18 ;
n) une région VH comprenant la Séquence N° 19, et une région VL comprenant la Séquence N° 20 ;
o) une région VH comprenant la Séquence N° 21, et une région VL comprenant la Séquence N° 22 ;
p) une région VH comprenant la Séquence N° 23, et une région VL comprenant la Séquence N° 24 ;
q) une région VH comprenant la Séquence N° 27, et une région VL comprenant la Séquence N° 28 ;
r) une région VH comprenant la Séquence N° 29, et une région VL comprenant la Séquence N° 30 ;
s) une région VH comprenant la Séquence N° 32, et une région VL comprenant la Séquence N° 33.

**36.** CAM pour utilisation conforme à l'une des revendications précédentes, dans lequel l'anticorps comprend au moins un domaine de liaison comportant une région VH comprenant la Séquence N° 1, et une région VL comprenant la Séquence N° 2.

**37.** CAM pour utilisation conforme à l'une des revendications précédentes, dans lequel

- l'anticorps comprend au moins un domaine de liaison comportant une région VH comprenant pour séquences CDR1, CDR2 et CDR3, respectivement, les Séquences N° 36, N° 37 et N° 38, et une région VL comprenant pour séquences CDR1, CDR2 et CDR3, respectivement, les Séquences N° 39, GAS et N° 40,
- le raccord est mc-vc-PAB,
- et l'agent cytotoxique est MMAE.

**38.** CAM pour utilisation conforme à l'une des revendications précédentes, étant entendu que l'anticorps se lie à un épitope de l'AXL, lequel épitope est reconnu par n'importe lequel des anticorps définis dans la revendication 35.

**39.** CAM pour utilisation conforme à l'une des revendications précédentes, étant entendu que l'anticorps se lie à un épitope situé dans le domaine Ig1 de l'AXL, lequel épitope comprend ou requiert un ou plusieurs acide(s) aminé(s) correspondant aux positions L121 à Q129 ou T112 à Q124 de l'AXL humaine.

**40.** CAM pour utilisation conforme à l'une des revendications 1 à 35, étant entendu que l'anticorps se lie à un épitope situé dans le domaine Ig2 de l'AXL, lequel épitope comprend ou requiert les acides aminés correspondant à la position D170 ou la combinaison de D179 et d'un ou plusieurs acide(s) aminé(s) correspondant aux positions T182 à R190 de l'AXL humaine.

**41.** CAM pour utilisation conforme à l'une des revendications 1 à 35, étant entendu que l'anticorps se lie à un épitope situé dans le domaine FN1 de l'AXL humaine, lequel épitope comprend ou requiert un ou plusieurs acide(s) aminé(s) correspondant aux positions Q272 à A287 et G297 à P301 de l'AXL humaine.

**42.** CAM pour utilisation conforme à l'une des revendications 1 à 35, étant entendu que l'anticorps se lie à un épitope situé dans le domaine FN2 de l'AXL humaine, lequel épitope comprend ou requiert les acides aminés correspondant aux positions A359, R386, et un ou plusieurs acide(s) aminé(s) correspondant aux positions Q436 à K439 de l'AXL humaine.

**43.** CAM pour utilisation conforme à l'une des revendications précédentes, dans lequel l'anticorps comprend une chaîne lourde d'un isotype choisi dans l'ensemble constitué par les IgG1, IgG2, IgG3 et IgG4.

**44.** CAM pour utilisation conforme à la revendication 43, dans lequel l'isotype est IgG1, et en option, l'allotype IgG1m(f).

**45.** CAM pour utilisation conforme à l'une des revendications précédentes, dans lequel l'anticorps est un anticorps monoclonal entier, comme un anticorps IgG1,κ monoclonal entier.

**46.** CAM pour utilisation conforme à l'une des revendications précédentes, lequel CAM est compris dans une composition pharmaceutique comprenant un véhicule pharmacologiquement admissible.

**Figure 1**

**A**

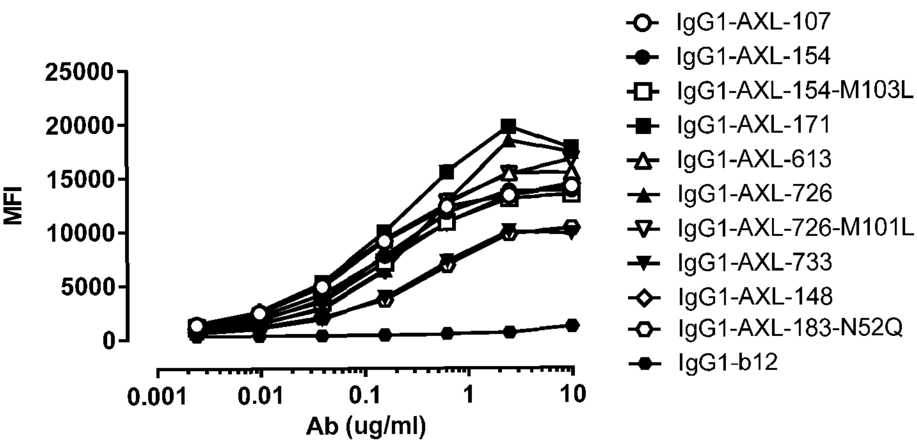
MFI
25000
20000
15000
10000
5000
0
0.001
0.01
0.1
1
10
Ab (ug/ml)
IgG1-AXL-107
IgG1-AXL-154
IgG1-AXL-154-M103L
IgG1-AXL-171
IgG1-AXL-613
IgG1-AXL-726
IgG1-AXL-726-M101L
IgG1-AXL-733
IgG1-AXL-148
IgG1-AXL-183-N52Q
IgG1-b12

**B**

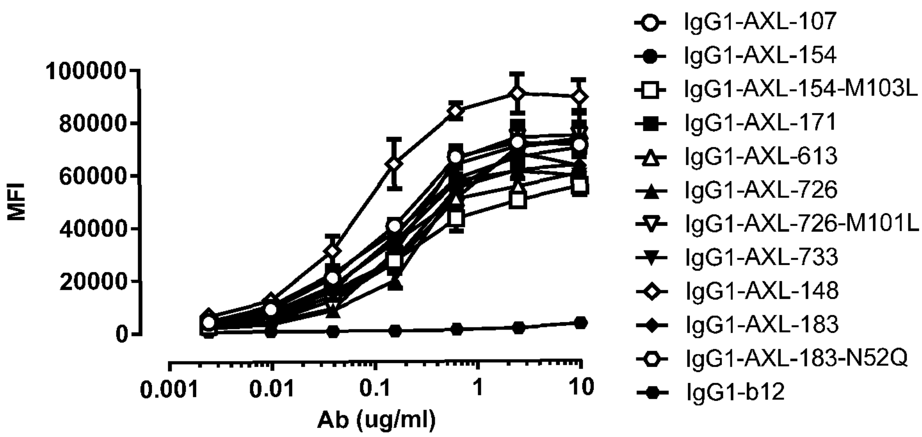
MFI
100000
80000
60000
40000
20000
0
0.001
0.01
0.1
1
10
Ab (ug/ml)
IgG1-AXL-107
IgG1-AXL-154
IgG1-AXL-154-M103L
IgG1-AXL-171
IgG1-AXL-613
IgG1-AXL-726
IgG1-AXL-726-M101L
IgG1-AXL-733
IgG1-AXL-148
IgG1-AXL-183
IgG1-AXL-183-N52Q
IgG1-b12

**C**

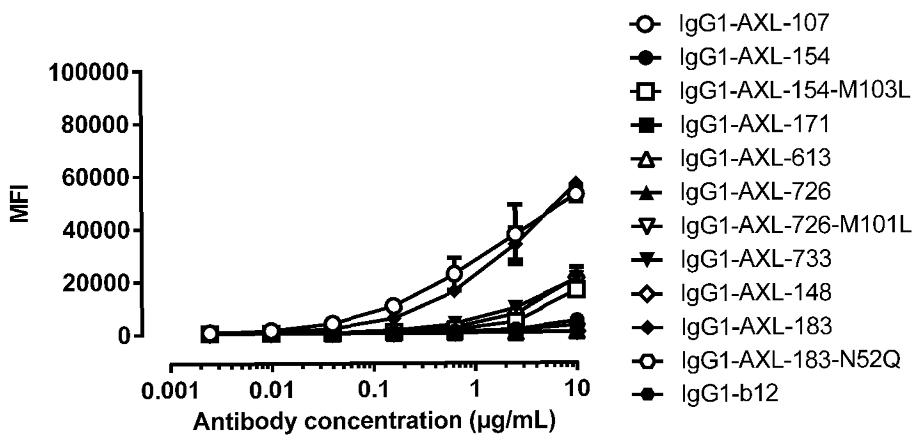
MFI
100000
80000
60000
40000
20000
0
0.001
0.01
0.1
1
10
Antibody concentration (µg/mL)
IgG1-AXL-107
IgG1-AXL-154
IgG1-AXL-154-M103L
IgG1-AXL-171
IgG1-AXL-613
IgG1-AXL-726
IgG1-AXL-726-M101L
IgG1-AXL-733
IgG1-AXL-148
IgG1-AXL-183
IgG1-AXL-183-N52Q
IgG1-b12

Figure 2

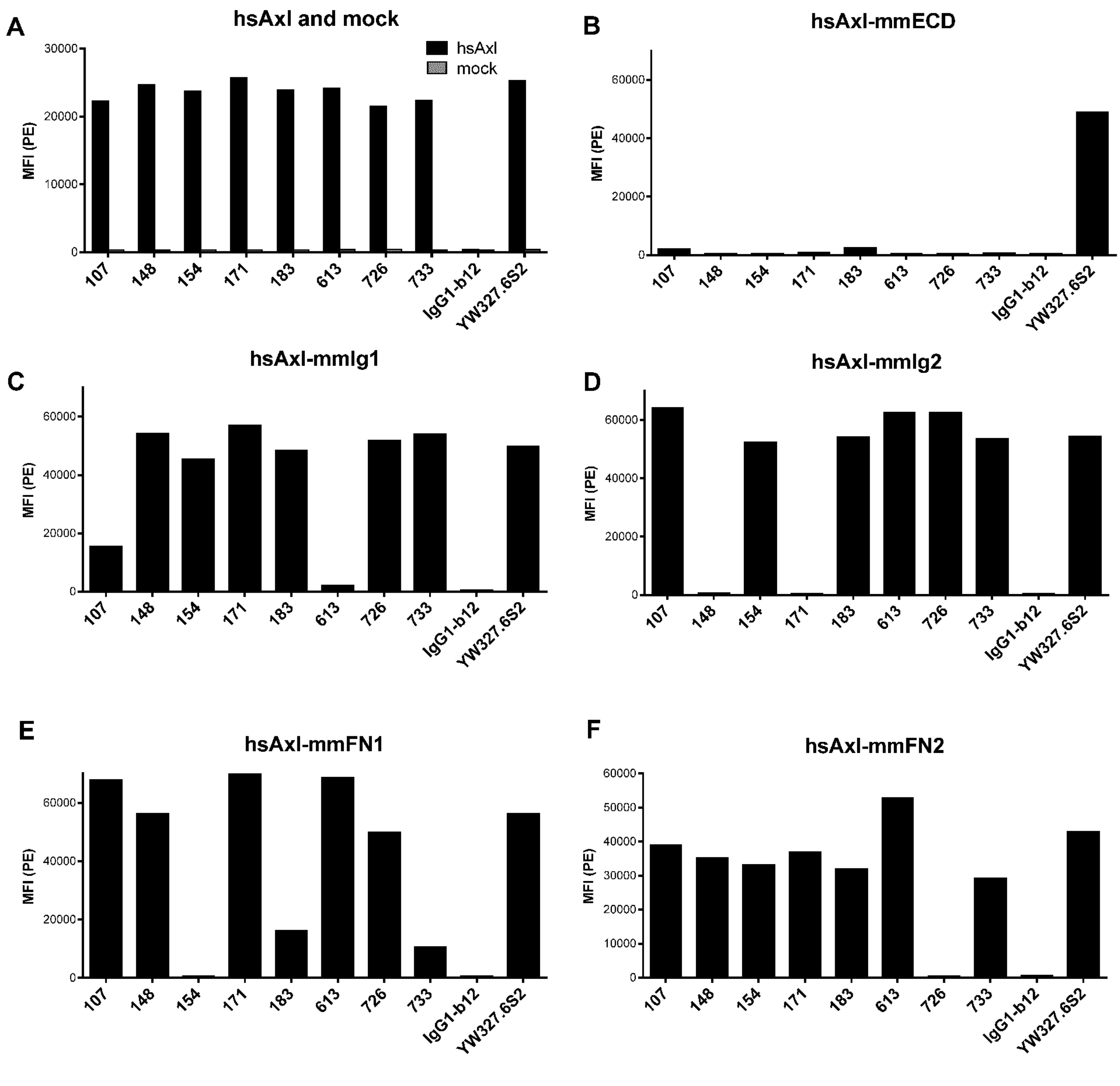
A
hsAxl and mock
hsAxl
mock
MFI (PE)
30000
20000
10000
0
107
148
154
171
183
613
726
733
IgG1-b12
YW327.6S2
B
hsAxl-mmECD
MFI (PE)
60000
40000
20000
0
C
hsAxl-mmIg1
MFI (PE)
60000
40000
20000
0
D
hsAxl-mmIg2
MFI (PE)
60000
40000
20000
0
E
hsAxl-mmFN1
MFI (PE)
60000
40000
20000
0
F
hsAxl-mmFN2
MFI (PE)
60000
50000
40000
30000
20000
10000
0

Figure 3

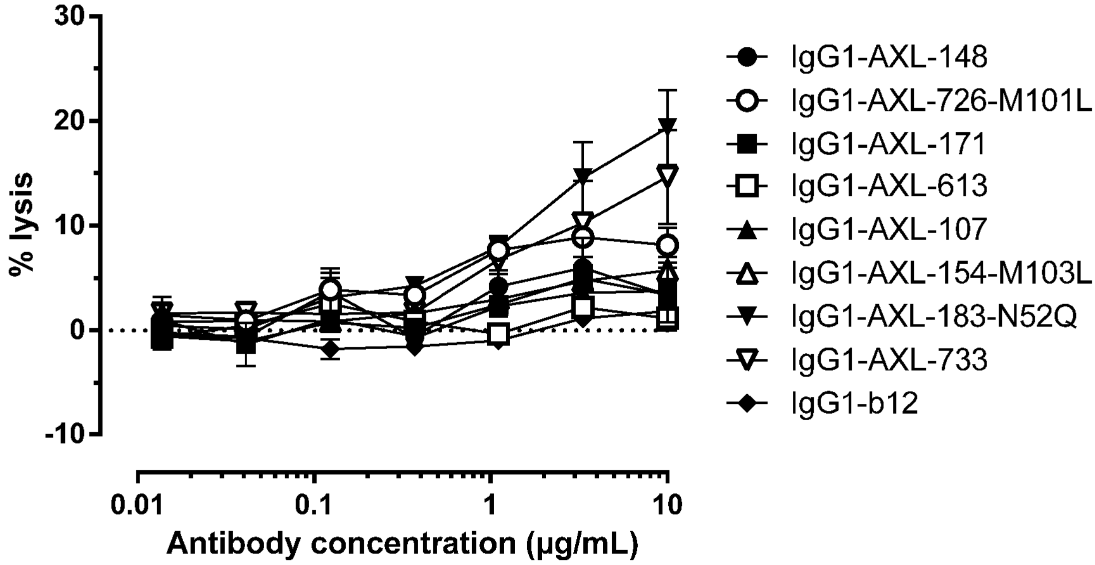


Figure 4

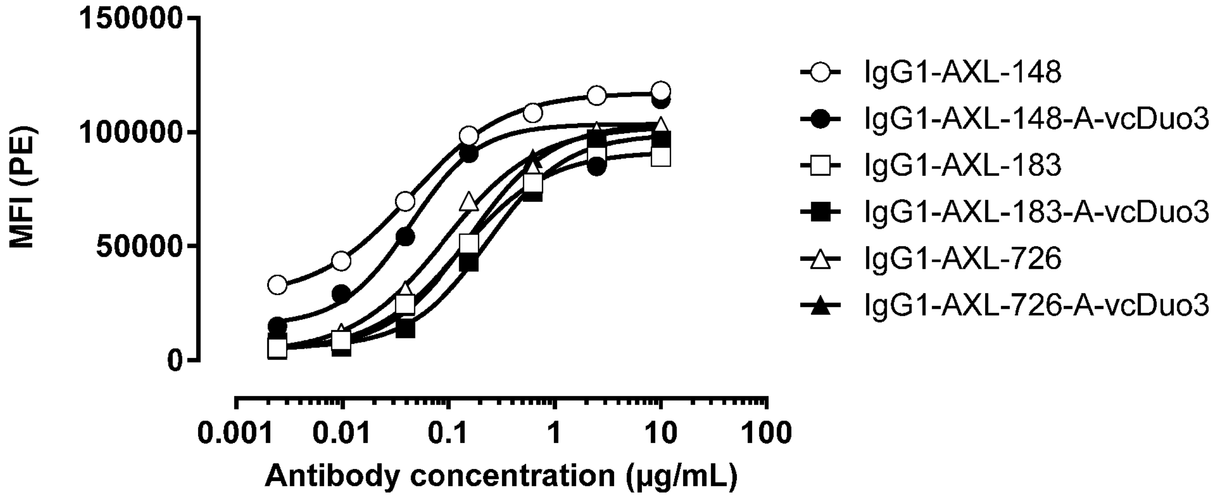

Figure 5

A

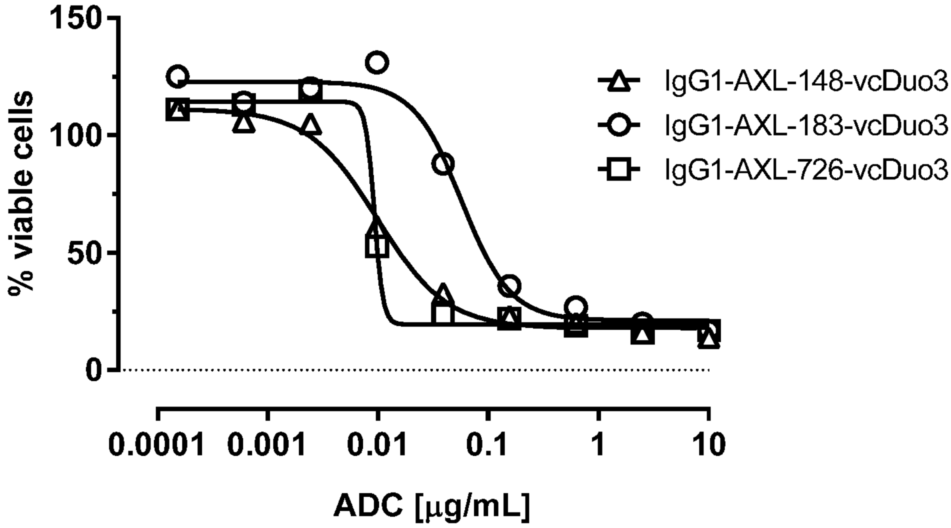
150
100
50
0
% viable cells
0.0001
0.001
0.01
0.1
1
10
ADC [μg/mL]
IgG1-AXL-148-vcDuo3
IgG1-AXL-183-vcDuo3
IgG1-AXL-726-vcDuo3

B

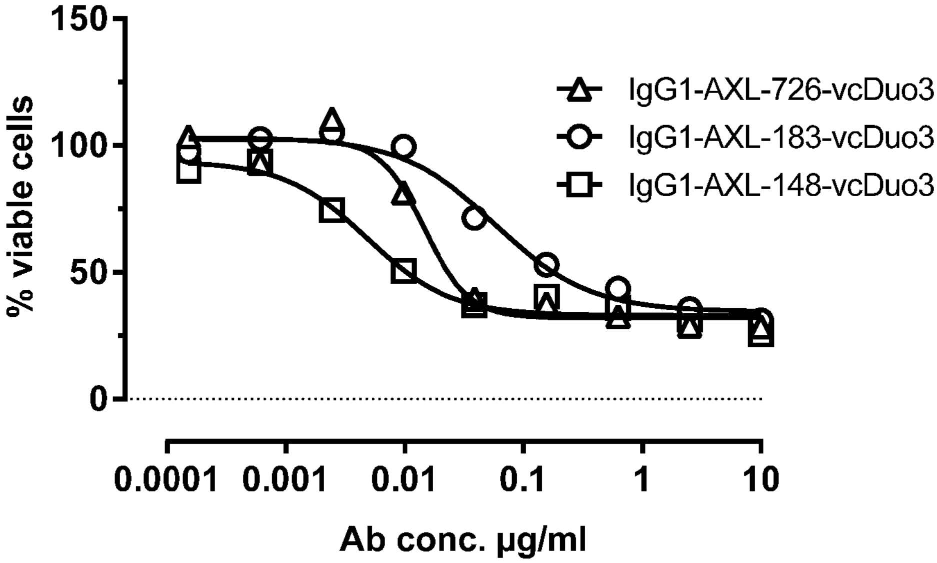
150
100
50
0
% viable cells
0.0001
0.001
0.01
0.1
1
10
Ab conc. μg/ml
IgG1-AXL-726-vcDuo3
IgG1-AXL-183-vcDuo3
IgG1-AXL-148-vcDuo3

# Figure 6

**A**

```
HC-IgG1-AXL-140  EVQLLESGGGLVQPGGSLRLSCAASGFTFSSYAMTWVRQAPGKGLEWVSAISISGASTFYADSVKGRFTISRDNSKNTLSLQMNSLRAEDTAVYFCRGYSGYVYDAFDIWGQGTMVTVSS
HC-IgG1-AXL-148  EVQLLESGGGLVQPGGSLRLSCAASGFTFSSYAMTWVRQAPGKGLEWVSAISISGGSTFYADSVKGRFTISRDNSKNTLYLQMNSLRAEDTAVYYCRGYSGYVYDAFDFWGQGTMVTVSS
```

CDR1 CDR2 CDR3

**B**

```
HC-IgG1-AXL-187  QVPLQQWGAGLLKPSETLSLTCAVYGGSFSGYHWSWIRQPPGKGLEWIGEISHSGRTNYNPSLKSRVTISIDTSKNQFSLKLSSVTAADTAVYYCASFITMIRGTIITHFDYWGQGTLVTVSS
HC-IgG1-AXL-726  QVQLQQWGAGLLKPSETLSLTCAIDGGSFSGYYWSWIRQPPGKGLEWIGEISHSGRTNYNPSLKSRVTISIDTSKNQFSLKLSSVAAADTAVYYCARFITMIRGAIITHFDYWGQGALVTVSS
```

CDR1 CDR2 CDR3

**C**

```
HC-IgG1-AXL-171  EVQLLESGGGLVQPGGSLRLSCAASGFTFSSYAMSWVRQAPGKGLEWVSDISVSGGSTYYADSVKGRFTISRDNSKNTLYLQMNSLRAEDTAVYYCAKEGYIWFGESLSYAFDIWGQGTMVTVSS
HC-IgG1-AXL-172  EVQLLESGGGLVQPGGSLRLSCAASGFTFSNYAMSWVRQAPGKGLEWVSDISVSGGSTYYADSVKGRFTISRDNSKNTLYLQMNSLRAEDTAVYYCAKEGYIWFGESLSYAFDIWGQGTMVTVSS
HC-IgG1-AXL-181  EVQLLESGGGLVQPGGSLRLSCAASGFTFSSYAMSWVRQAPGKGLEWVSDISVSGGSTYYADSVKGRFTISRDNSKNTLYLHMNSLRAEDTAVYYCAKEGYIWFGESLSYAFDIWGQGTMVTVSS
```

CDR1 CDR2 CDR3

**D**

```
HC-IgG1-AXL-608-01  QVQLVQSGAEVKKPGSSVKVSCKASGGTFSSYAISWVRQAPGQGLEWMGRIIPIFGIANYVQKFQGRVTITADKSTSTAYMELSSLRAEDTAVYYCARRGDYYGSGSPDVFDIWGQGTMVTVSS
HC-IgG1-AXL-610-01  QVQLVQSGAEVKKPGSSVKVSCKASGGTFSSYAISWVRQAPGQGLEWMGRIIPIFGIANYVQKFQGRVTITADKSTSTAYMELSSLRAEDTAVYYCARRGNYYGSGSPDVFDIWGQGTMVTVSS
HC-IgG1-AXL-613     QVQLVQSGAEVKKPGSSVKVSCKASGGTFSSYAINWMRQAPGQGLEWMGRIIPIFGIVNYAQKFQGRVTLTADKSTSTAYMELSSLRSEDTAVYYCARRGNYYGSGSPDVFDIWGQGTMVTVSS
HC-IgG1-AXL-620-06  QVQLVQSGAEVKKPGSSVKVSCKASGGTFSSYAISWVRQAPGQGLEWMGRIIPIFGIANYAQKFQGRVTITADKSTSTAYMELSSLRSEDTAVYYCARRGNYYGSGSPDVFDIWGQGTMVTVSS
```

CDR1 CDR2 CDR3

**E**

```
LC-IgG1-AXL-613-08  EIVLTQSPATLSLSPGERATLSCRASQSV-SSYLAWYQQKPGQAPRLLIYDASNRATGIPARFSGSGSGTDFTLTISSLEPEDFAVYYCQQRSNWLTFGGGTKVEIK
LC-IgG1-AXL-613     EIVLTQSPGTLSLSPGERATLSCRASQSVSSSYLAWYQQKPGQAPRLLIYGASSRATGIPDRFSGSGSGTDFTLTISRLEPEDFAVYYCQQYGSSYTFGQGTKLEIK
```

CDR1 CDR2 CDR3

Figure 7

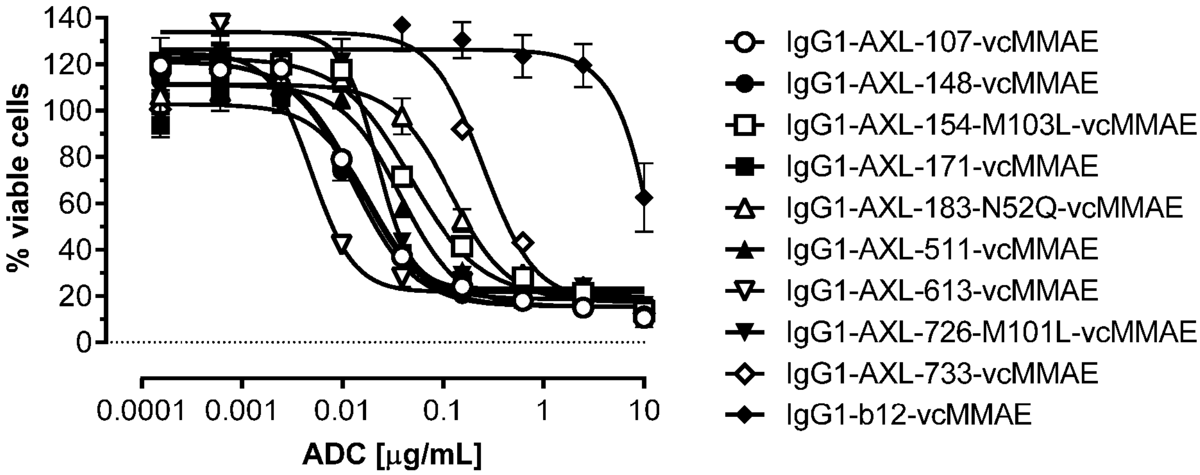


Figure 8

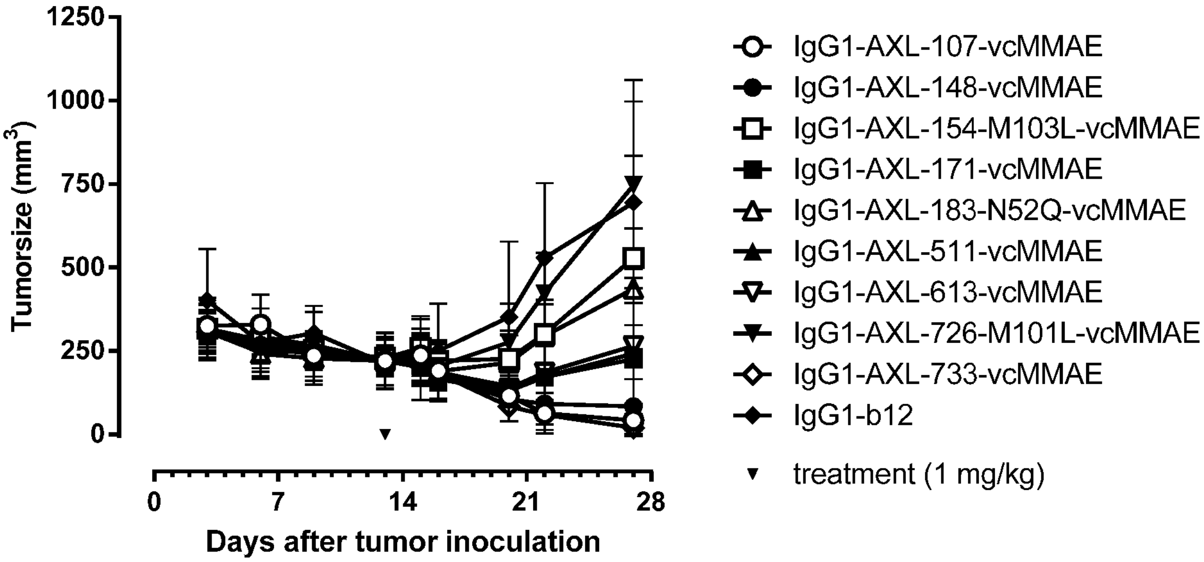

Figure 9

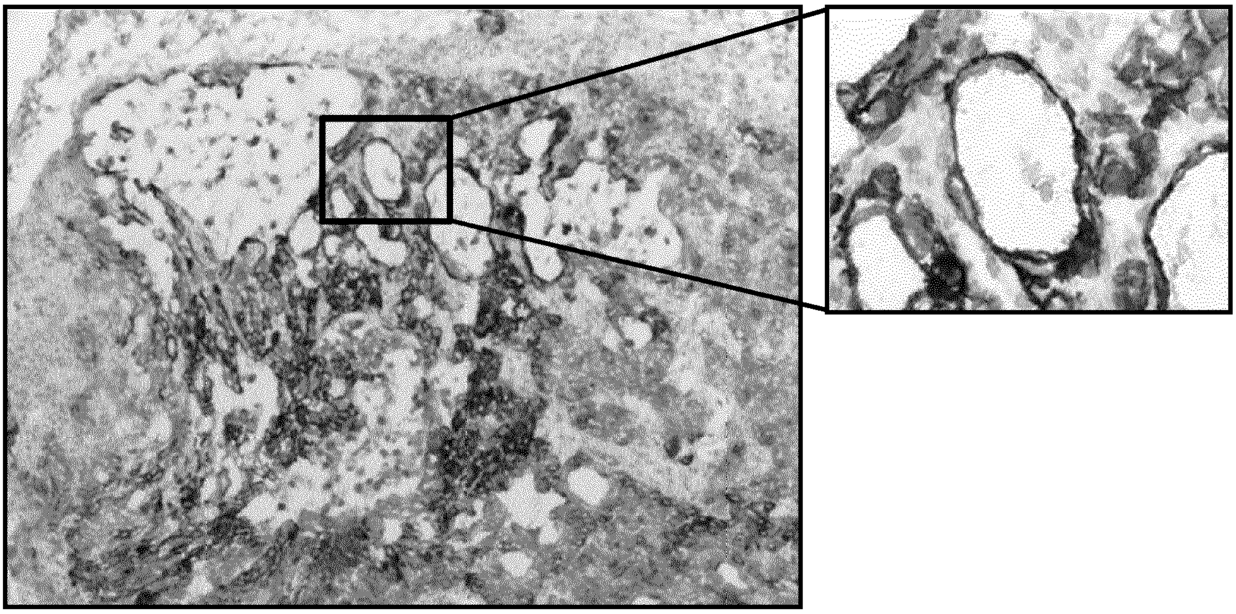

Figure 10

A

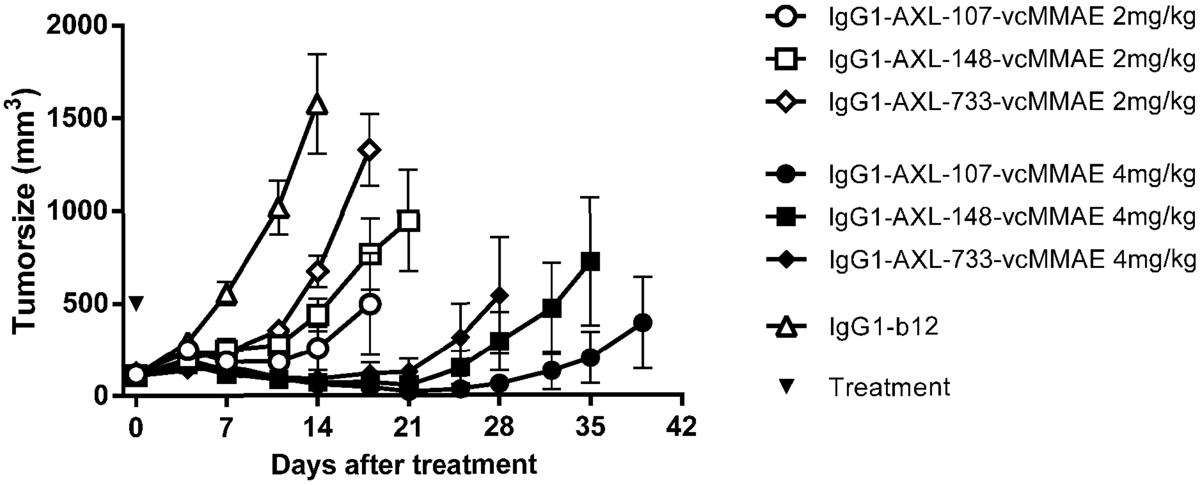
2000
1500
1000
500
0
Tumorsize (mm3)
0
7
14
21
28
35
42
Days after treatment
IgG1-AXL-107-vcMMAE 2mg/kg
IgG1-AXL-148-vcMMAE 2mg/kg
IgG1-AXL-733-vcMMAE 2mg/kg
IgG1-AXL-107-vcMMAE 4mg/kg
IgG1-AXL-148-vcMMAE 4mg/kg
IgG1-AXL-733-vcMMAE 4mg/kg
IgG1-b12
Treatment

B

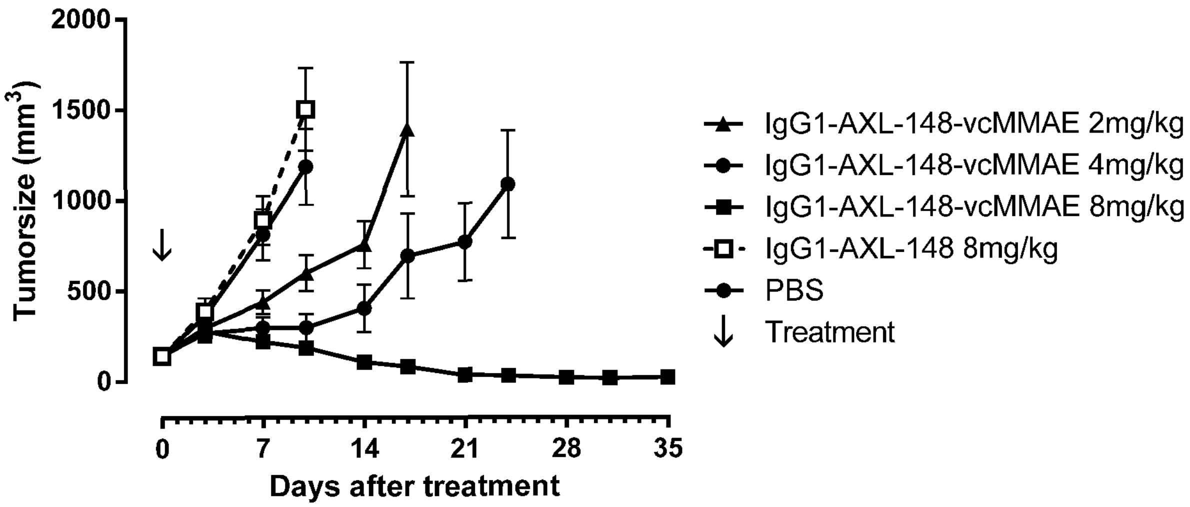
2000
1500
1000
500
0
Tumorsize (mm3)
0
7
14
21
28
35
Days after treatment
IgG1-AXL-148-vcMMAE 2mg/kg
IgG1-AXL-148-vcMMAE 4mg/kg
IgG1-AXL-148-vcMMAE 8mg/kg
IgG1-AXL-148 8mg/kg
PBS
Treatment

**Figure 10 (continued)**

C

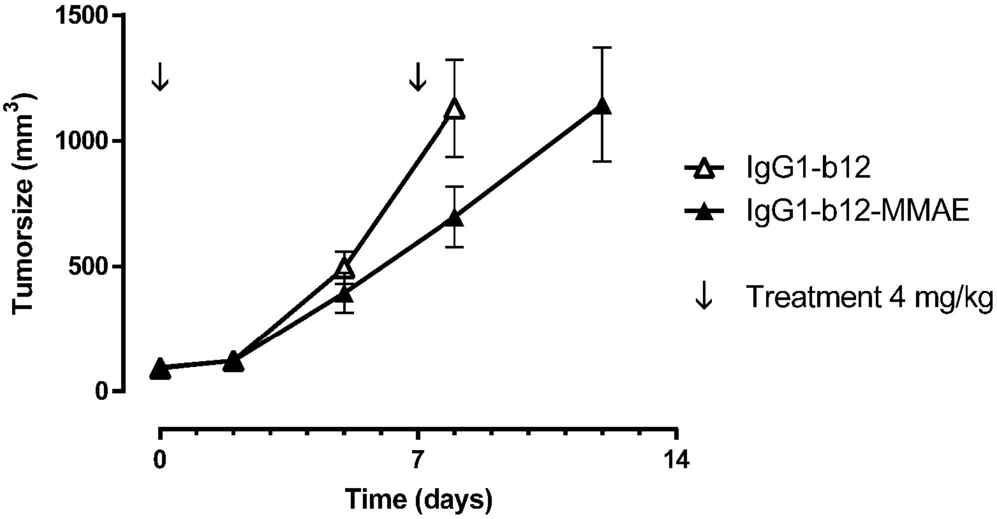

Figure 11

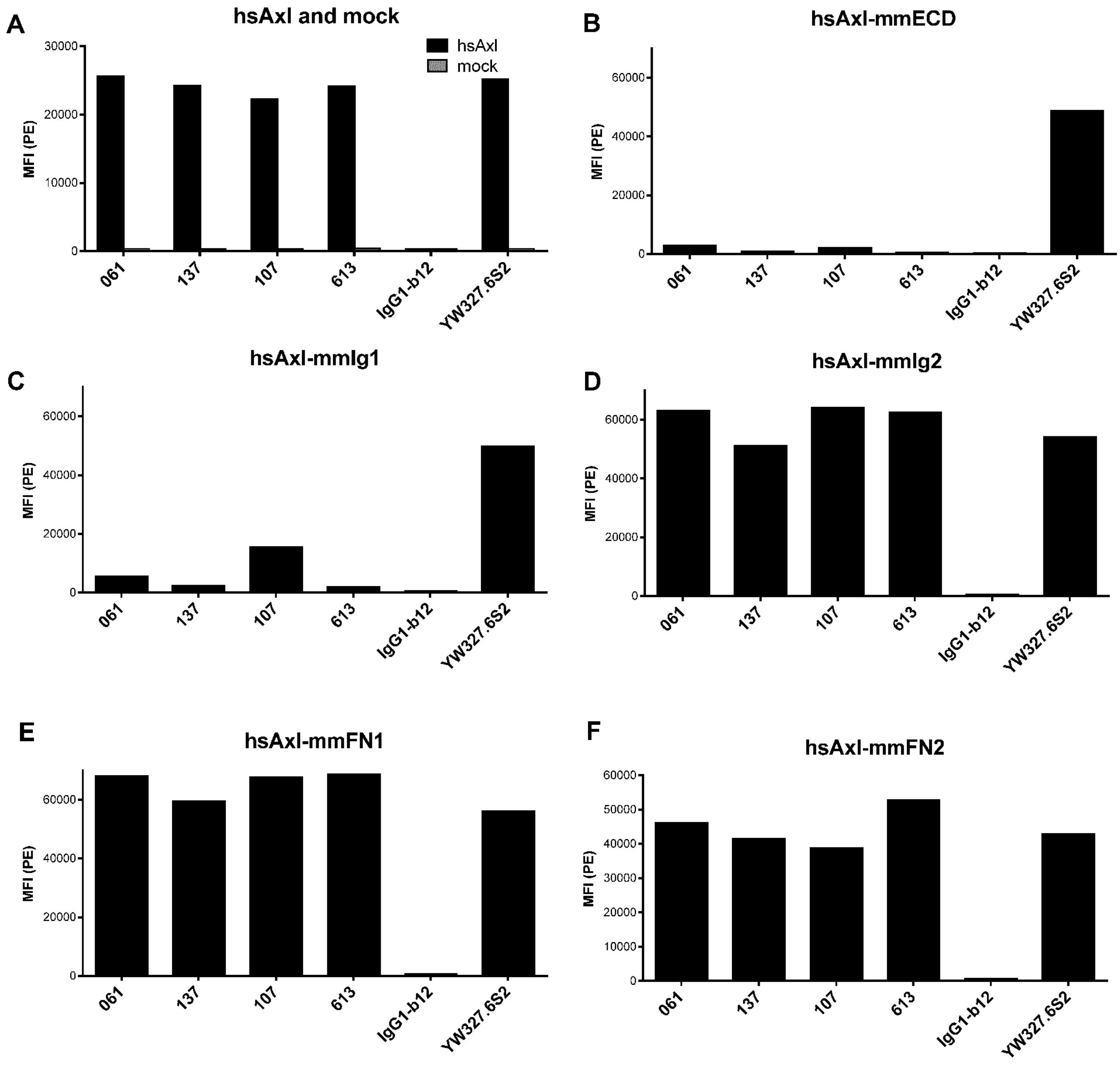
A
hsAxl and mock
hsAxl
mock
MFI (PE)
30000
20000
10000
0
061
137
107
613
IgG1-b12
YW327.6S2
B
hsAxl-mmECD
MFI (PE)
60000
40000
20000
0
061
137
107
613
IgG1-b12
YW327.6S2
C
hsAxl-mmIg1
MFI (PE)
60000
40000
20000
0
061
137
107
613
IgG1-b12
YW327.6S2
D
hsAxl-mmIg2
MFI (PE)
60000
40000
20000
0
061
137
107
613
IgG1-b12
YW327.6S2
E
hsAxl-mmFN1
MFI (PE)
60000
40000
20000
0
061
137
107
613
IgG1-b12
YW327.6S2
F
hsAxl-mmFN2
MFI (PE)
60000
50000
40000
30000
20000
10000
0
061
137
107
613
IgG1-b12
YW327.6S2

Figure 12

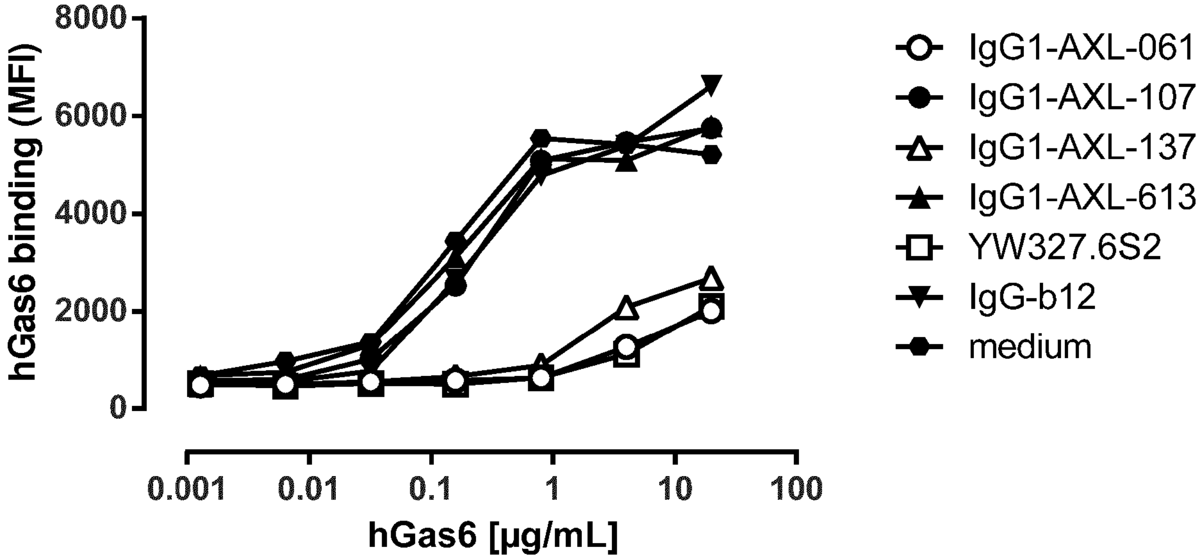
8000
6000
4000
2000
0
hGas6 binding (MFI)
0.001
0.01
0.1
1
10
100
hGas6 [µg/mL]
IgG1-AXL-061
IgG1-AXL-107
IgG1-AXL-137
IgG1-AXL-613
YW327.6S2
IgG-b12
medium

Figure 13

A

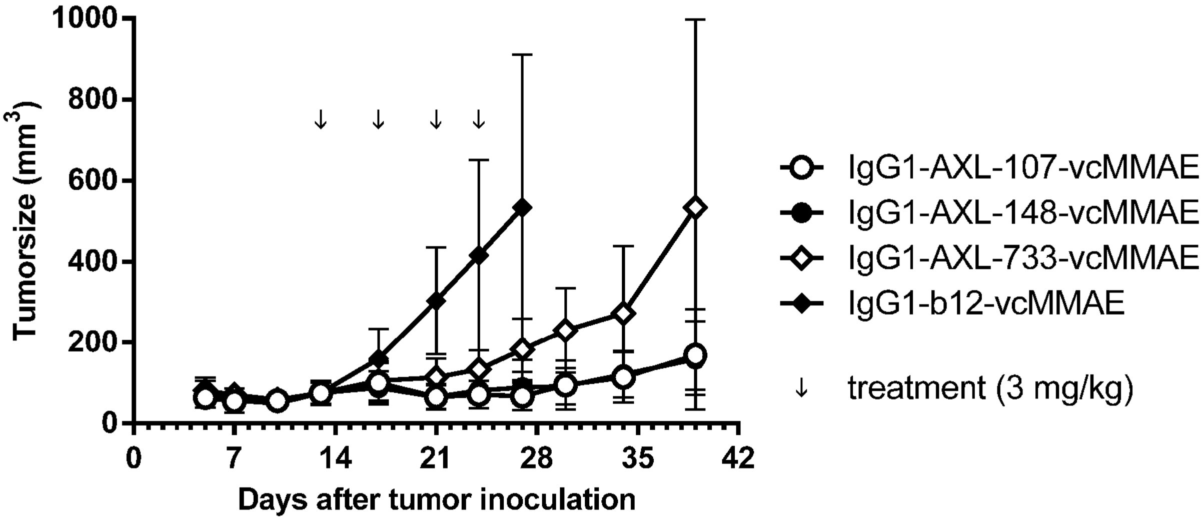
Tumorsize (mm$^3$)
1000
800
600
400
200
0
0
7
14
21
28
35
42
Days after tumor inoculation
IgG1-AXL-107-vcMMAE
IgG1-AXL-148-vcMMAE
IgG1-AXL-733-vcMMAE
IgG1-b12-vcMMAE
↓ treatment (3 mg/kg)

B

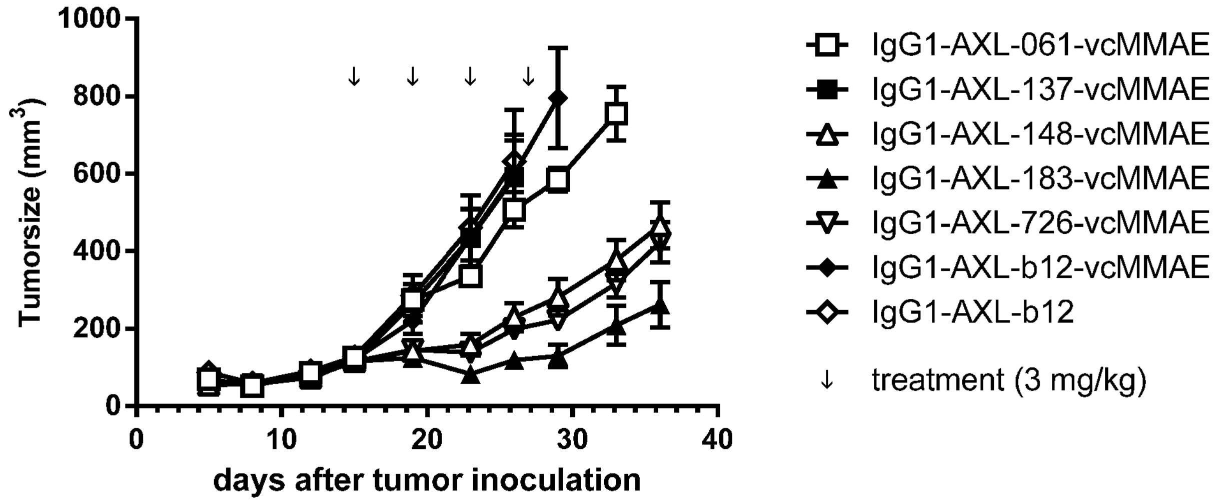
Tumorsize (mm$^3$)
1000
800
600
400
200
0
0
10
20
30
40
days after tumor inoculation
IgG1-AXL-061-vcMMAE
IgG1-AXL-137-vcMMAE
IgG1-AXL-148-vcMMAE
IgG1-AXL-183-vcMMAE
IgG1-AXL-726-vcMMAE
IgG1-AXL-b12-vcMMAE
IgG1-AXL-b12
↓ treatment (3 mg/kg)

Figure 14

A

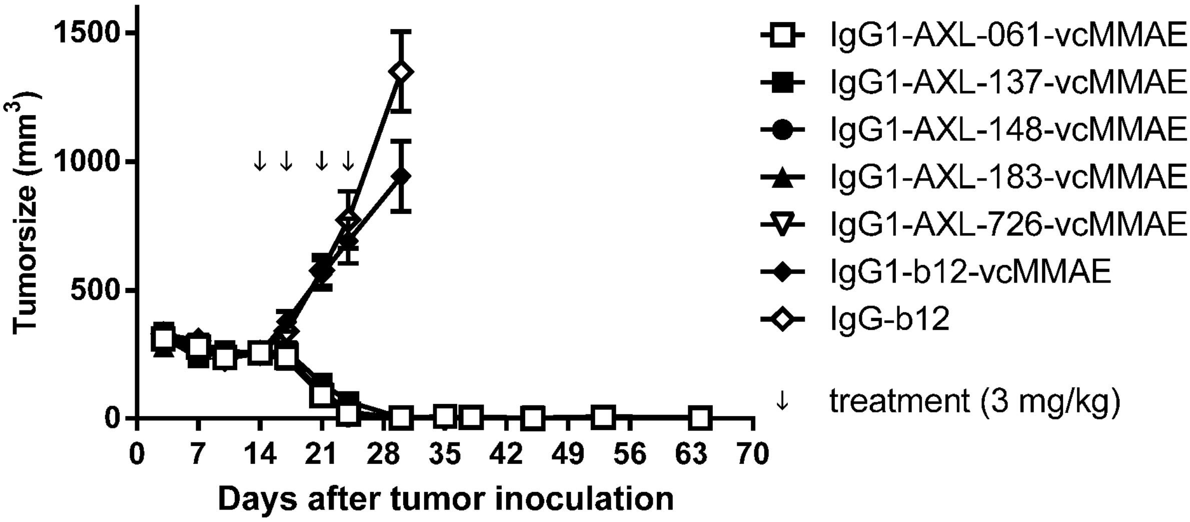
1500
1000
500
0
Tumorsize (mm³)
0 7 14 21 28 35 42 49 56 63 70
Days after tumor inoculation
IgG1-AXL-061-vcMMAE
IgG1-AXL-137-vcMMAE
IgG1-AXL-148-vcMMAE
IgG1-AXL-183-vcMMAE
IgG1-AXL-726-vcMMAE
IgG1-b12-vcMMAE
IgG-b12
↓ treatment (3 mg/kg)

B

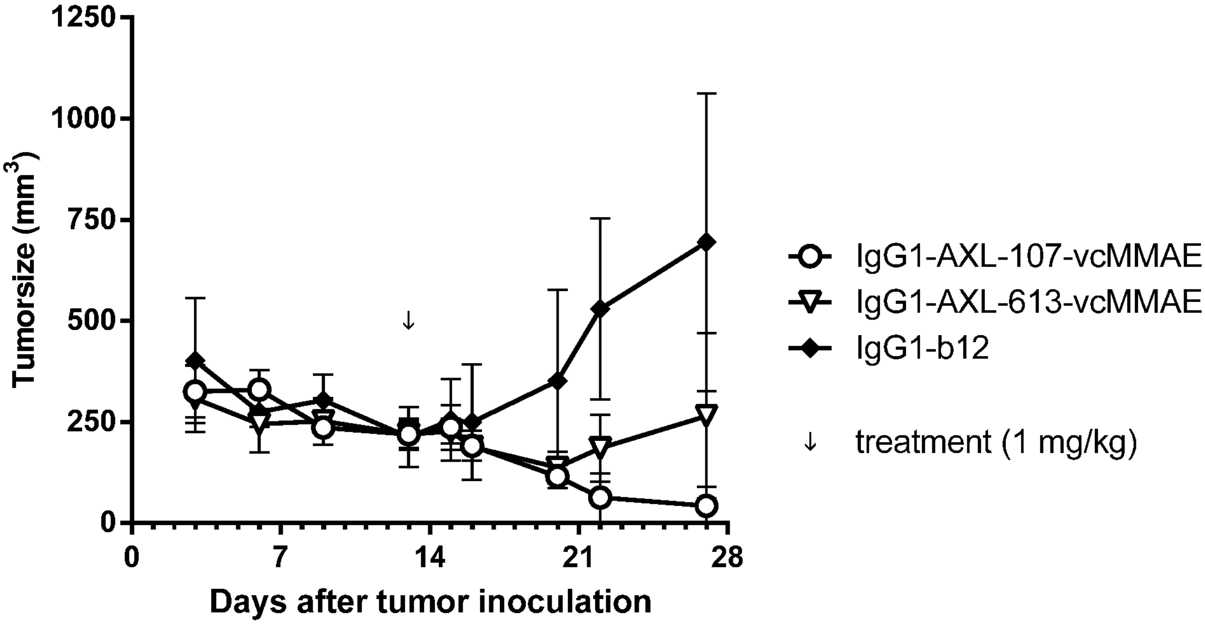
1250
1000
750
500
250
0
Tumorsize (mm³)
0 7 14 21 28
Days after tumor inoculation
IgG1-AXL-107-vcMMAE
IgG1-AXL-613-vcMMAE
IgG1-b12
↓ treatment (1 mg/kg)

Figure 15

A

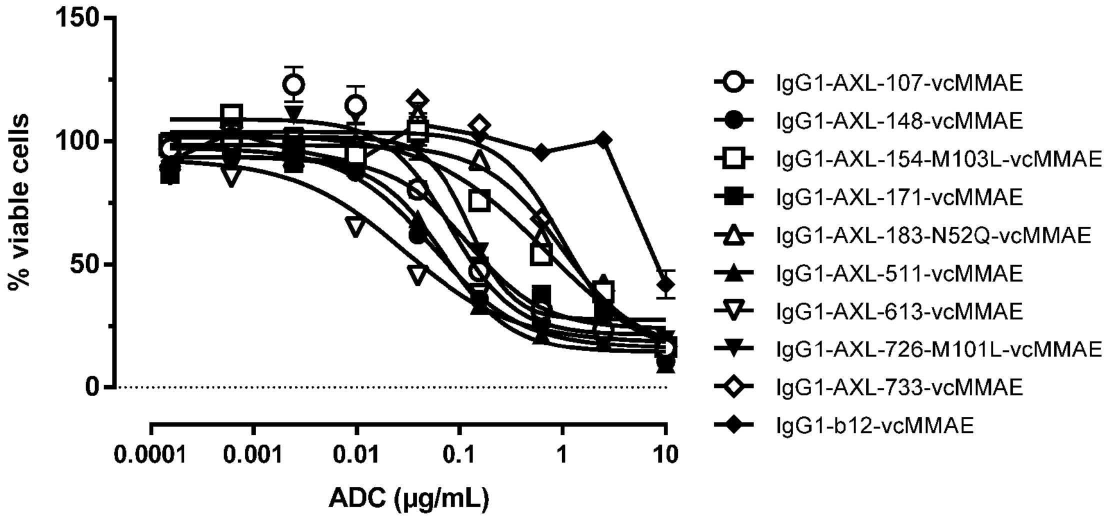
150
100
50
0
% viable cells
0.0001
0.001
0.01
0.1
1
10
ADC (µg/mL)
IgG1-AXL-107-vcMMAE
IgG1-AXL-148-vcMMAE
IgG1-AXL-154-M103L-vcMMAE
IgG1-AXL-171-vcMMAE
IgG1-AXL-183-N52Q-vcMMAE
IgG1-AXL-511-vcMMAE
IgG1-AXL-613-vcMMAE
IgG1-AXL-726-M101L-vcMMAE
IgG1-AXL-733-vcMMAE
IgG1-b12-vcMMAE

B

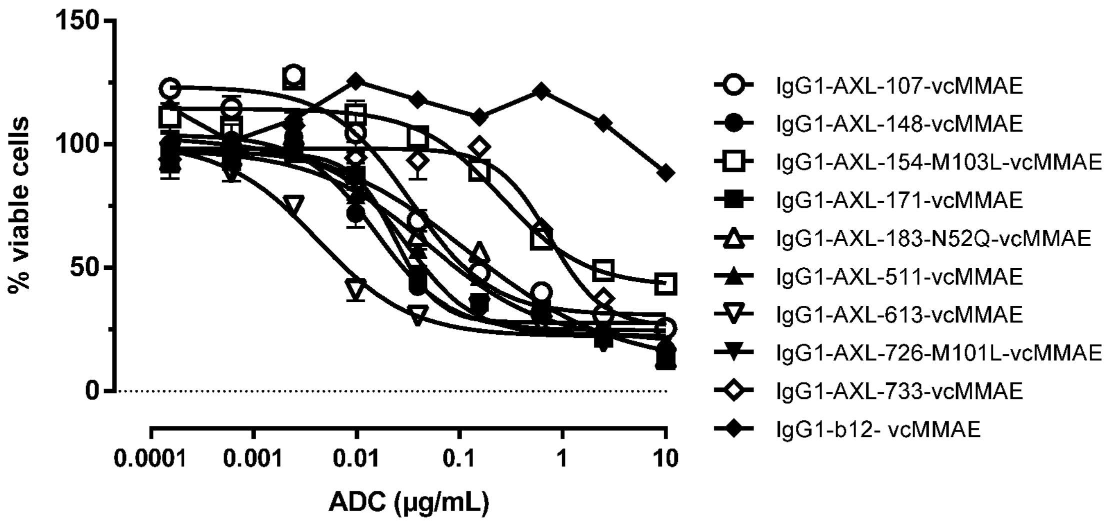
150
100
50
0
% viable cells
0.0001
0.001
0.01
0.1
1
10
ADC (µg/mL)
IgG1-AXL-107-vcMMAE
IgG1-AXL-148-vcMMAE
IgG1-AXL-154-M103L-vcMMAE
IgG1-AXL-171-vcMMAE
IgG1-AXL-183-N52Q-vcMMAE
IgG1-AXL-511-vcMMAE
IgG1-AXL-613-vcMMAE
IgG1-AXL-726-M101L-vcMMAE
IgG1-AXL-733-vcMMAE
IgG1-b12- vcMMAE

Figure 16

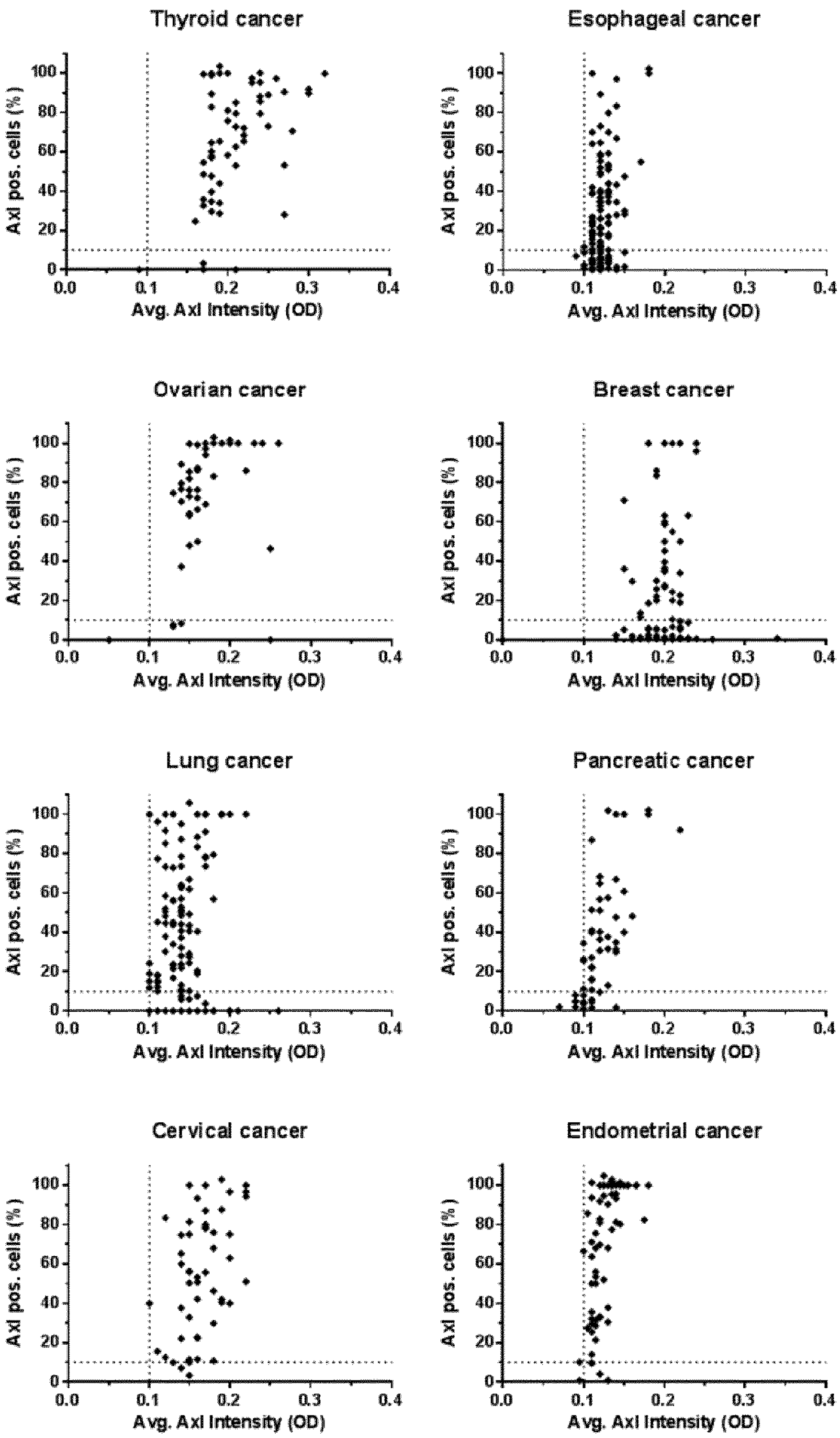
Thyroid cancer
Esophageal cancer
Ovarian cancer
Breast cancer
Lung cancer
Pancreatic cancer
Cervical cancer
Endometrial cancer
Axl pos. cells (%)
Avg. Axl Intensity (OD)
0
20
40
60
80
100
0.0
0.1
0.2
0.3
0.4

Figure 17

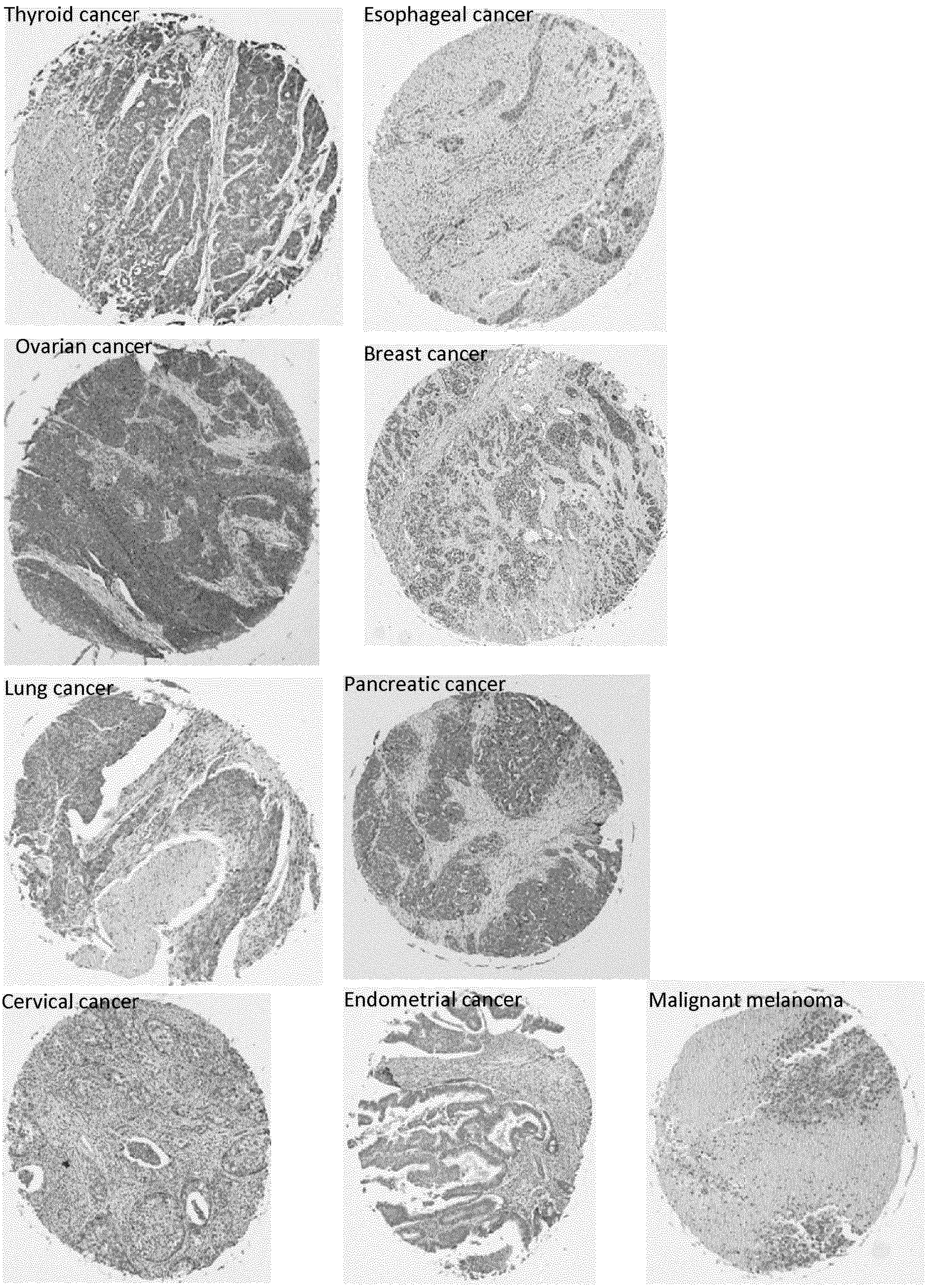
Thyroid cancer
Esophageal cancer
Ovarian cancer
Breast cancer
Lung cancer
Pancreatic cancer
Cervical cancer
Endometrial cancer
Malignant melanoma

Figure 18

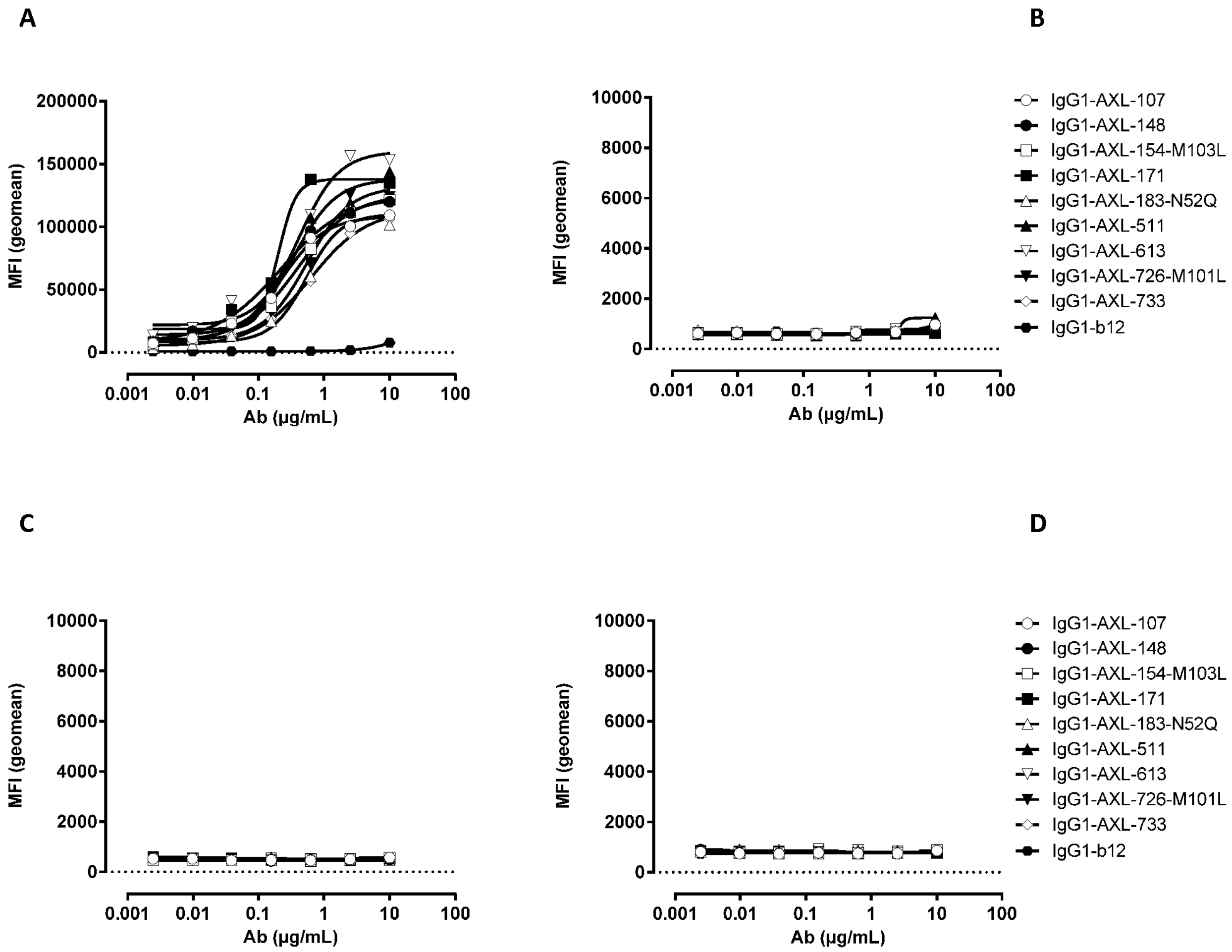
A
B
C
D
200000
150000
100000
50000
0
10000
8000
6000
4000
2000
MFI (geomean)
0.001
0.01
0.1
1
10
100
Ab (µg/mL)
IgG1-AXL-107
IgG1-AXL-148
IgG1-AXL-154-M103L
IgG1-AXL-171
IgG1-AXL-183-N52Q
IgG1-AXL-511
IgG1-AXL-613
IgG1-AXL-726-M101L
IgG1-AXL-733
IgG1-b12

Figure 18 (continued)

E

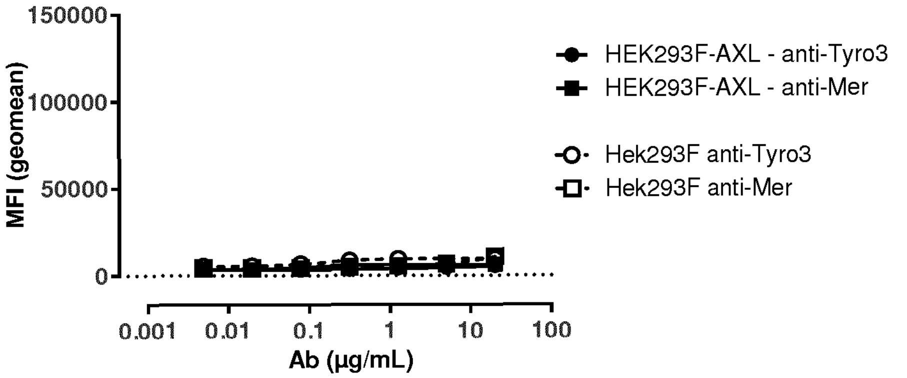


F

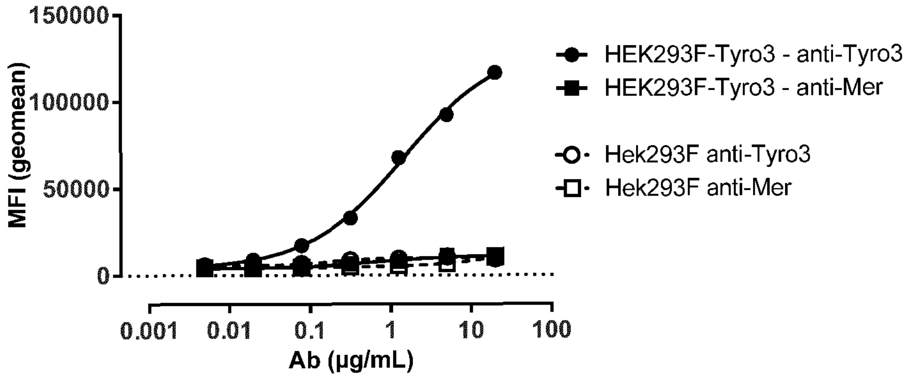


G

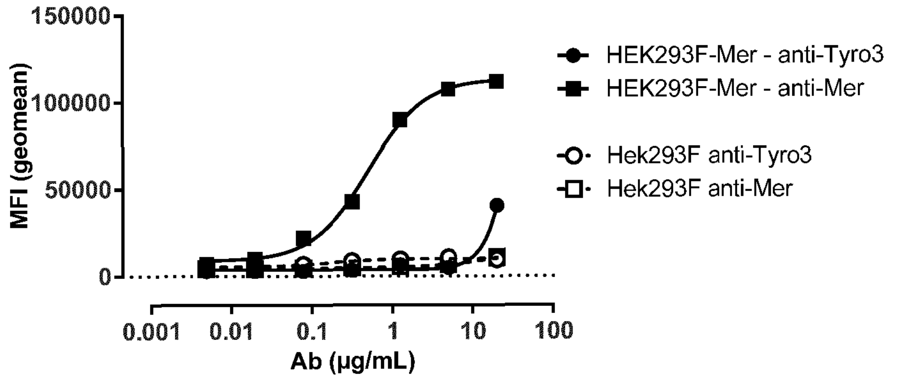

**Figure 19**

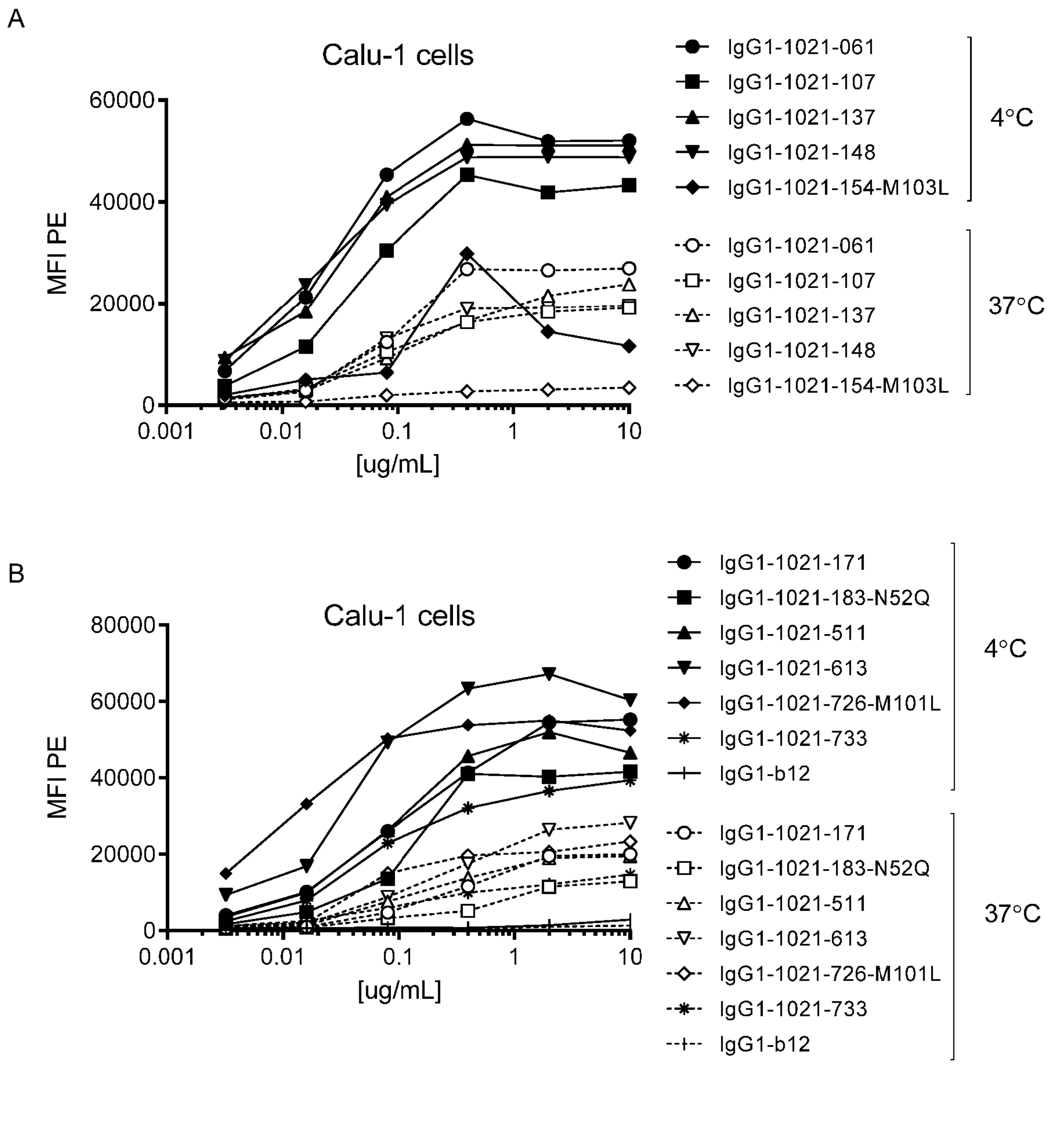
A
Calu-1 cells
MFI PE
60000
40000
20000
0
0.001
0.01
0.1
1
10
[ug/mL]
IgG1-1021-061
IgG1-1021-107
IgG1-1021-137
IgG1-1021-148
IgG1-1021-154-M103L
4°C
IgG1-1021-061
IgG1-1021-107
IgG1-1021-137
IgG1-1021-148
IgG1-1021-154-M103L
37°C
B
Calu-1 cells
MFI PE
80000
60000
40000
20000
0
0.001
0.01
0.1
1
10
[ug/mL]
IgG1-1021-171
IgG1-1021-183-N52Q
IgG1-1021-511
IgG1-1021-613
IgG1-1021-726-M101L
IgG1-1021-733
IgG1-b12
4°C
IgG1-1021-171
IgG1-1021-183-N52Q
IgG1-1021-511
IgG1-1021-613
IgG1-1021-726-M101L
IgG1-1021-733
IgG1-b12
37°C

**Figure 19 (continued)**

C

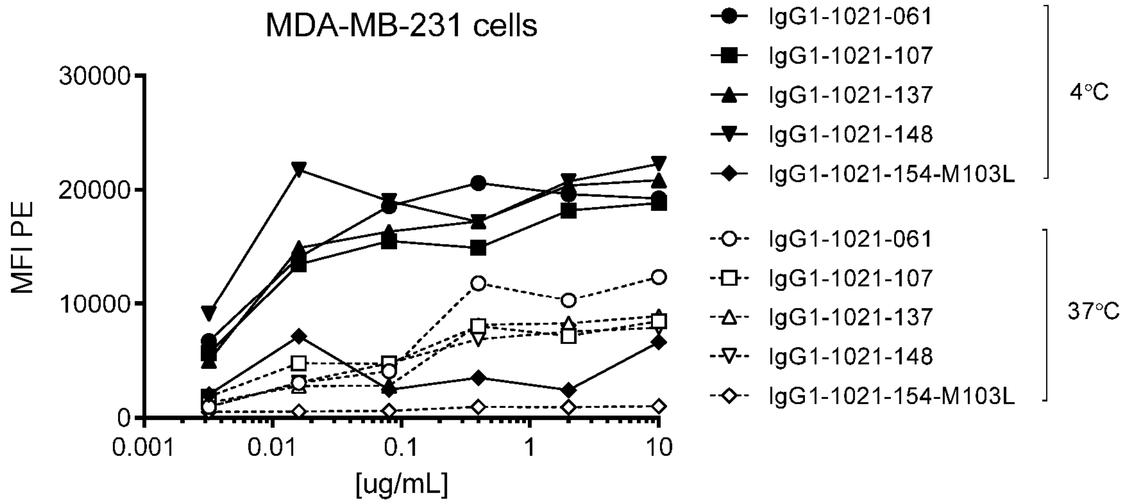


D

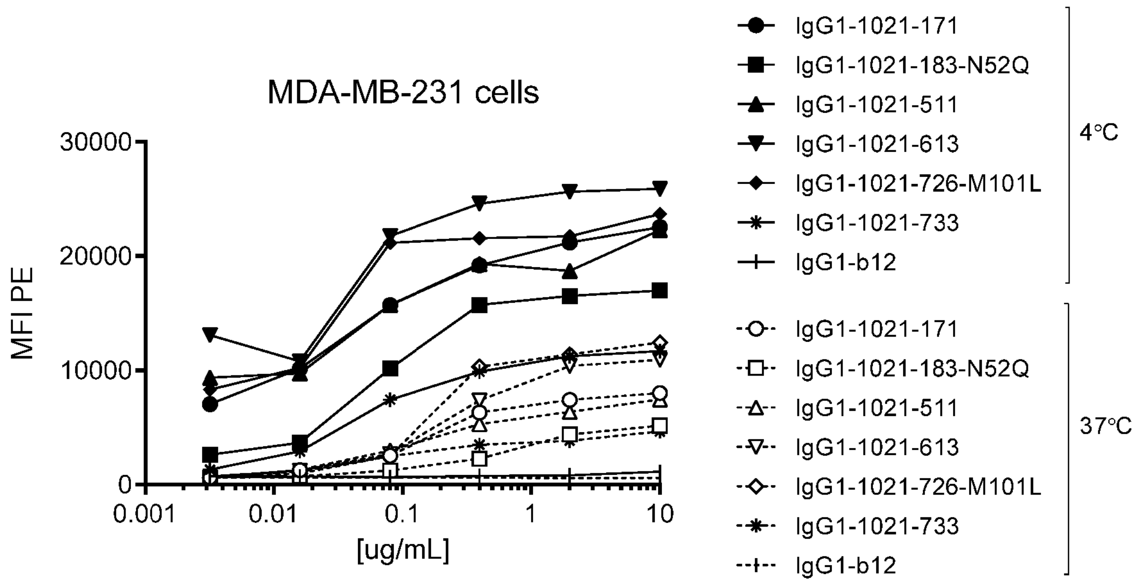

Figure 20

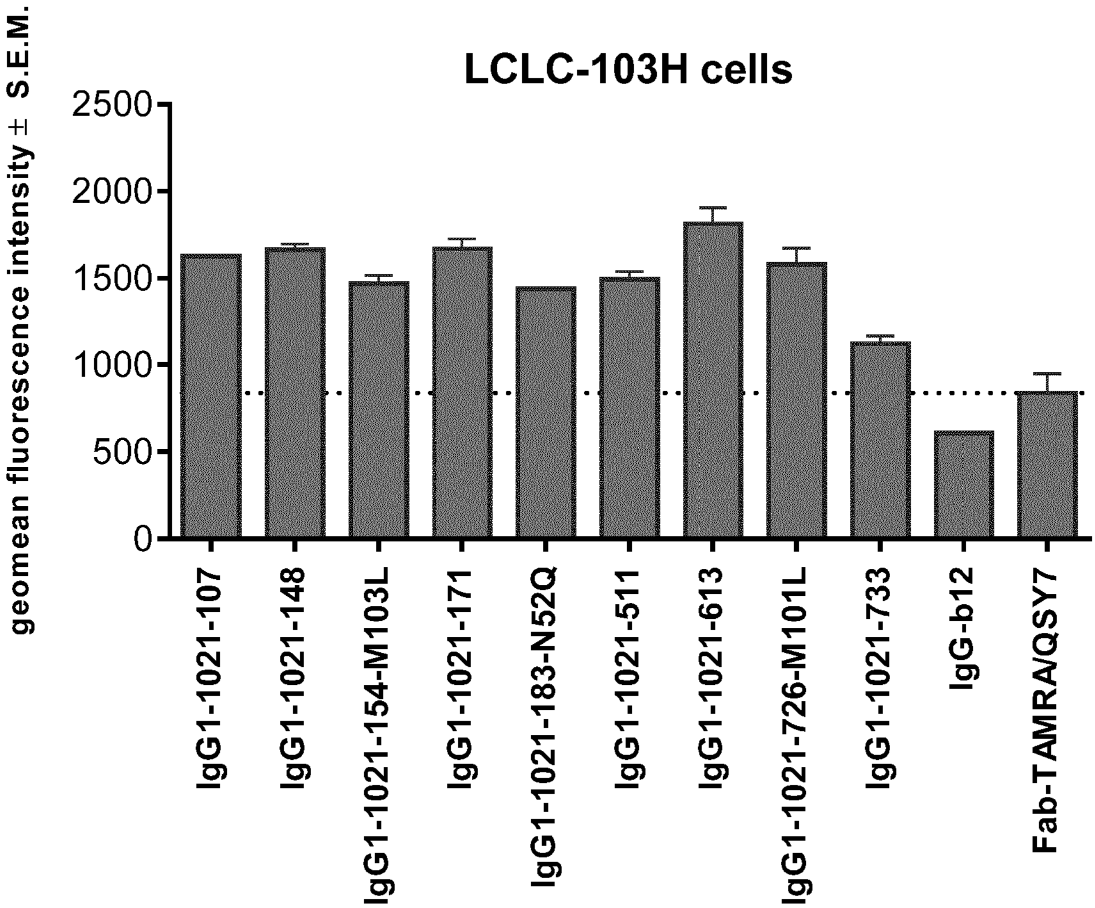
LCLC-103H cells
geomean fluorescence intensity ± S.E.M.
2500
2000
1500
1000
500
0
IgG1-1021-107
IgG1-1021-148
IgG1-1021-154-M103L
IgG1-1021-171
IgG1-1021-183-N52Q
IgG1-1021-511
IgG1-1021-613
IgG1-1021-726-M101L
IgG1-1021-733
IgG-b12
Fab-TAMRA/QSY7

Figure 21

A

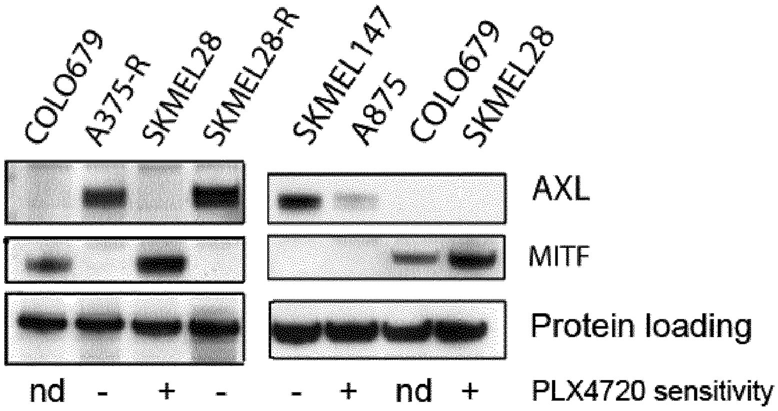
COLO679
A375-R
SKMEL28
SKMEL28-R
SKMEL147
A875
COLO679
SKMEL28
AXL
MITF
Protein loading
nd - + - - + nd +
PLX4720 sensitivity

B

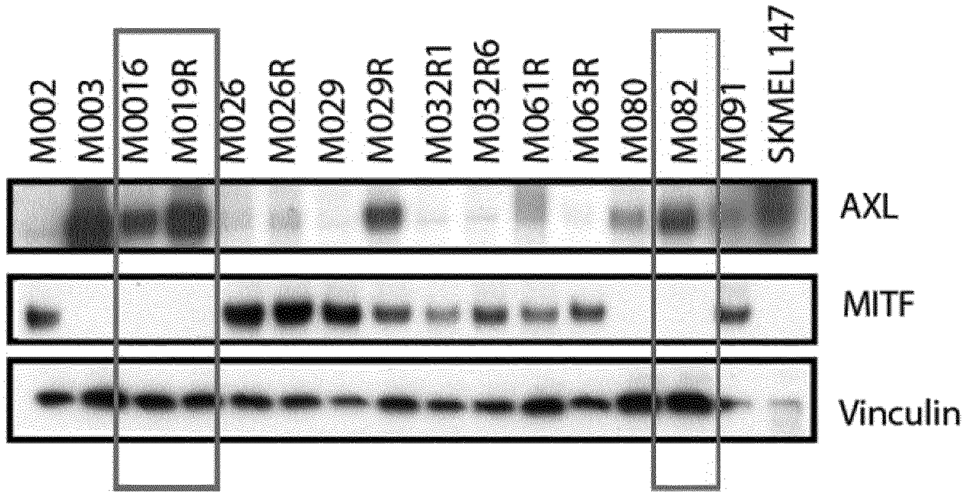
M002
M003
M0016
M019R
M026
M026R
M029
M029R
M032R1
M032R6
M061R
M063R
M080
M082
M091
SKMEL147
AXL
MITF
Vinculin

Figure 22

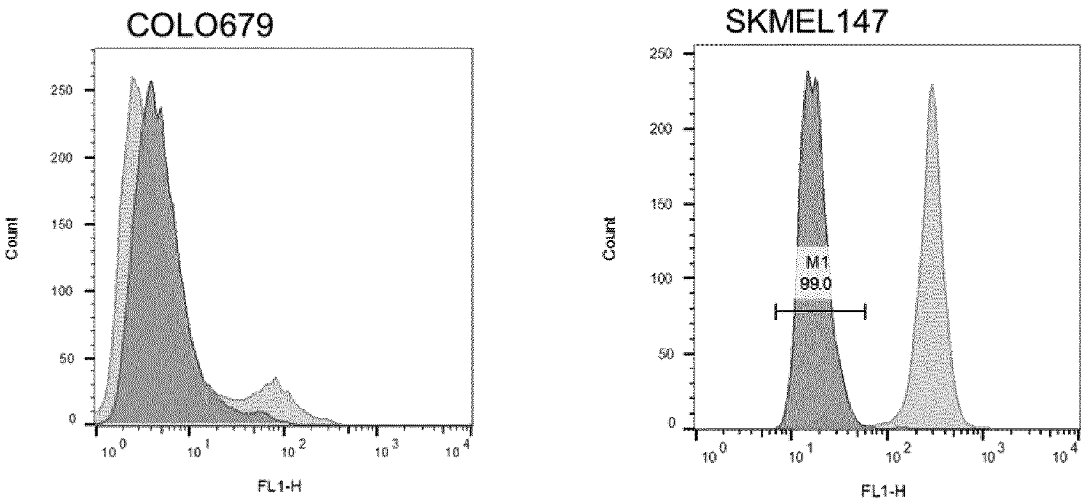

Figure 23

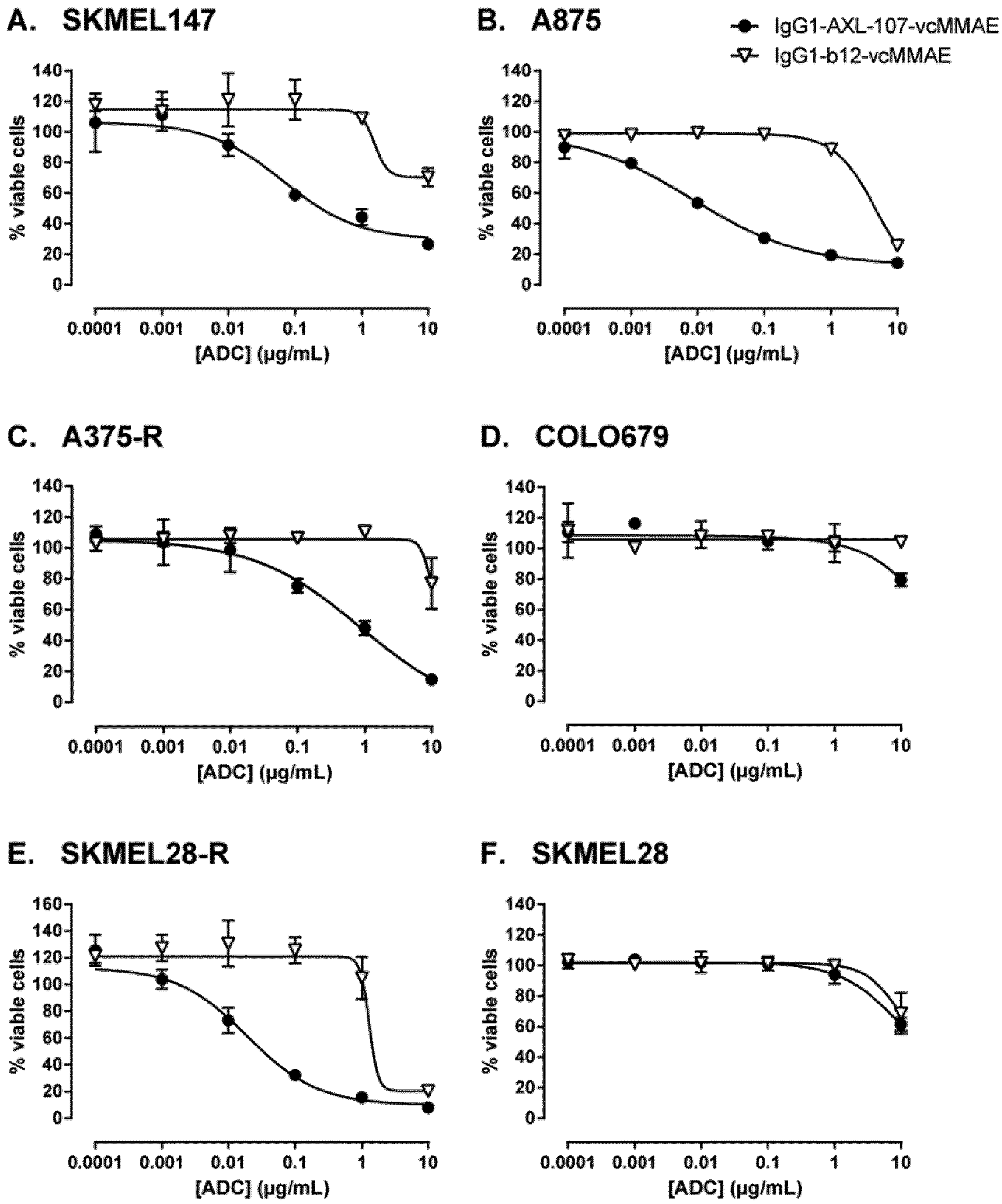
A. SKMEL147
B. A875
C. A375-R
D. COLO679
E. SKMEL28-R
F. SKMEL28
IgG1-AXL-107-vcMMAE
IgG1-b12-vcMMAE
% viable cells
[ADC] (µg/mL)
0.0001
0.001
0.01
0.1
1
10

Figure 24

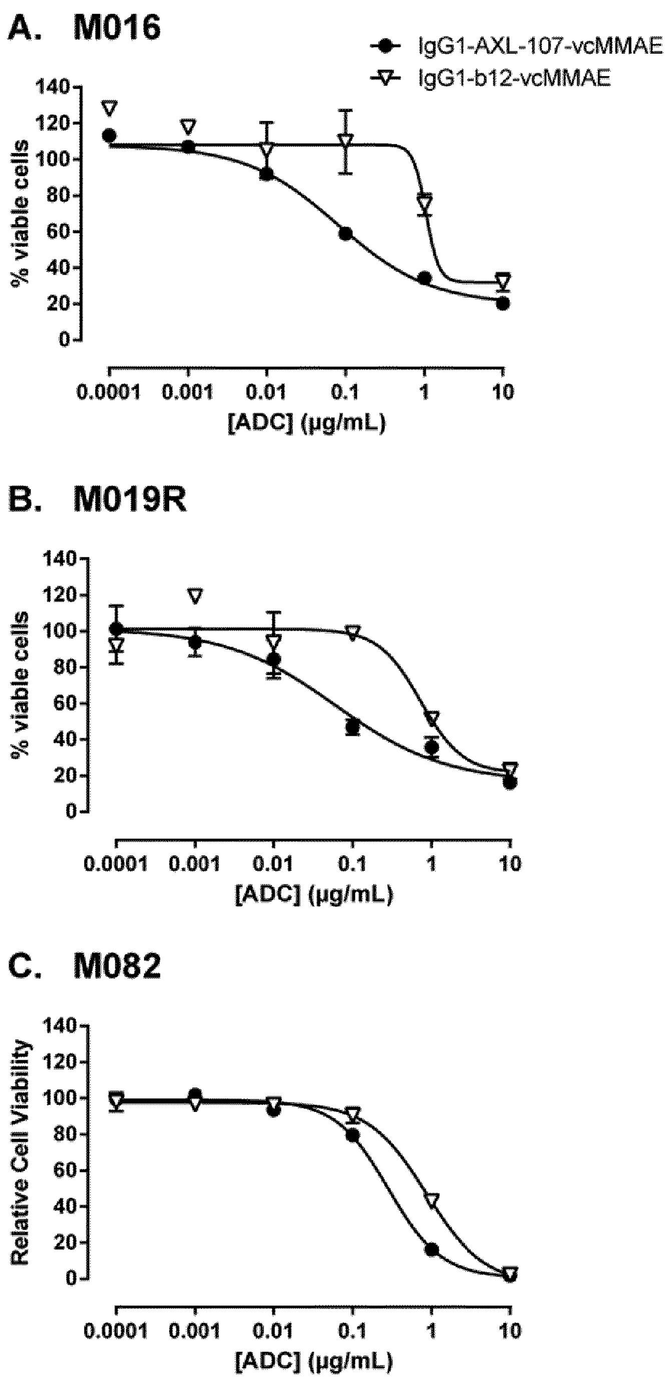
A. M016
IgG1-AXL-107-vcMMAE
IgG1-b12-vcMMAE
% viable cells
140
120
100
80
60
40
20
0
0.0001
0.001
0.01
0.1
1
10
[ADC] (µg/mL)
B. M019R
% viable cells
[ADC] (µg/mL)
C. M082
Relative Cell Viability
[ADC] (µg/mL)

Figure 25

A

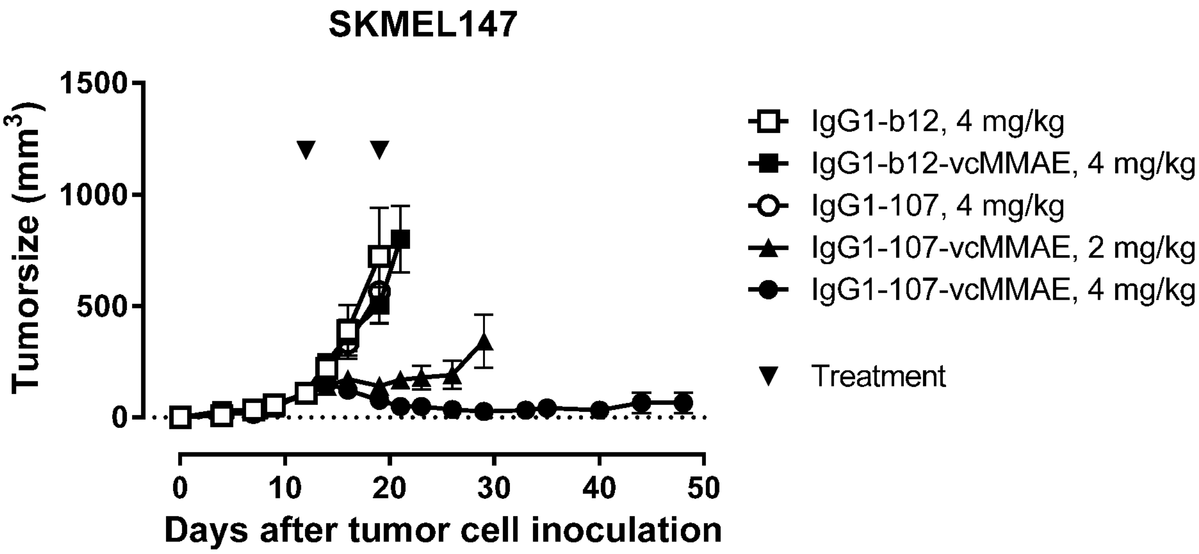
SKMEL147
Tumorsize (mm³)
1500
1000
500
0
0
10
20
30
40
50
Days after tumor cell inoculation
IgG1-b12, 4 mg/kg
IgG1-b12-vcMMAE, 4 mg/kg
IgG1-107, 4 mg/kg
IgG1-107-vcMMAE, 2 mg/kg
IgG1-107-vcMMAE, 4 mg/kg
Treatment

B

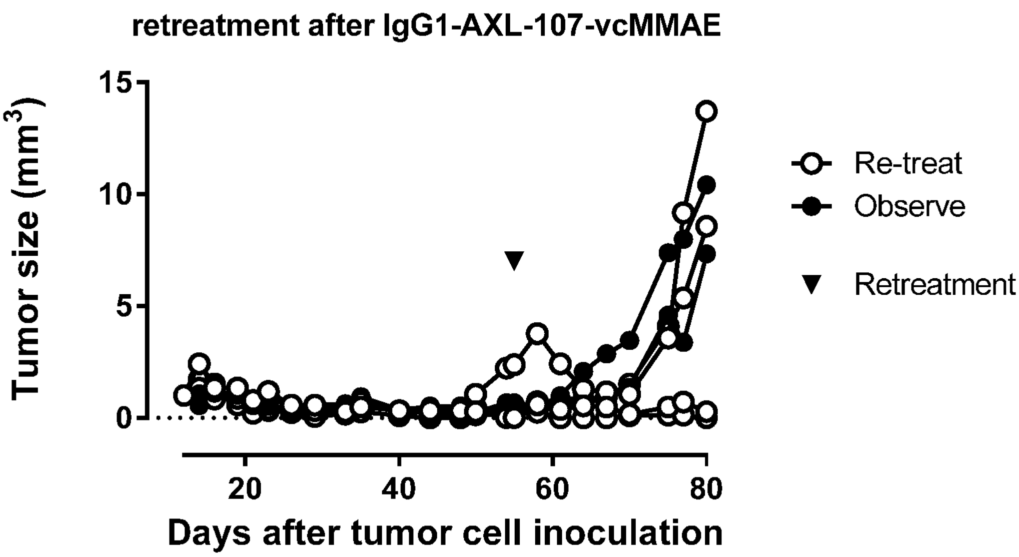
retreatment after IgG1-AXL-107-vcMMAE
Tumor size (mm³)
15
10
5
0
20
40
60
80
Days after tumor cell inoculation
Re-treat
Observe
Retreatment

Figure 26

A

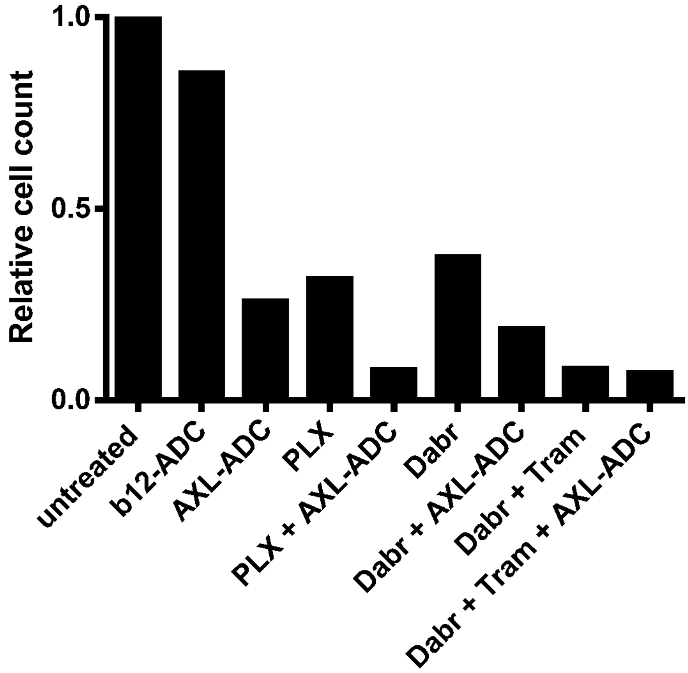
1.0
0.5
0.0
Relative cell count
untreated
b12-ADC
AXL-ADC
PLX
PLX + AXL-ADC
Dabr
Dabr + AXL-ADC
Dabr + Tram
Dabr + Tram + AXL-ADC

B

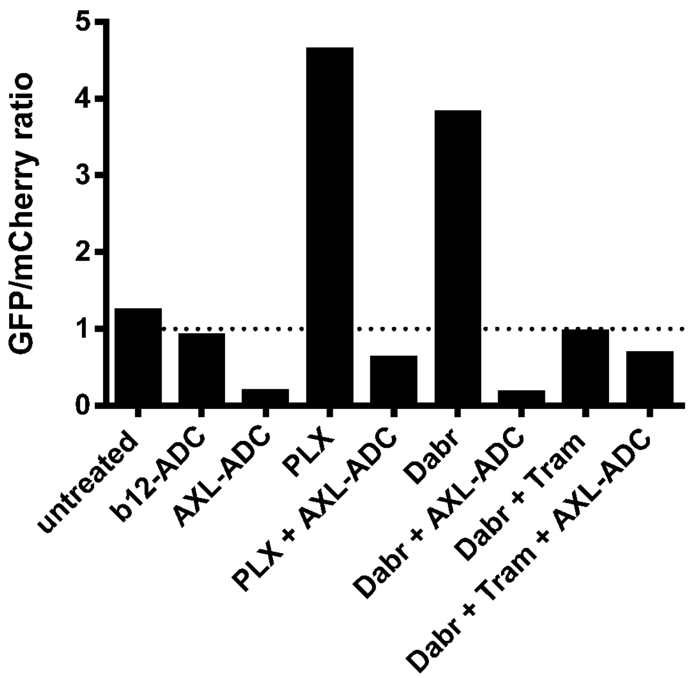
5
4
3
2
1
0
GFP/mCherry ratio
untreated
b12-ADC
AXL-ADC
PLX
PLX + AXL-ADC
Dabr
Dabr + AXL-ADC
Dabr + Tram
Dabr + Tram + AXL-ADC

Figure 27

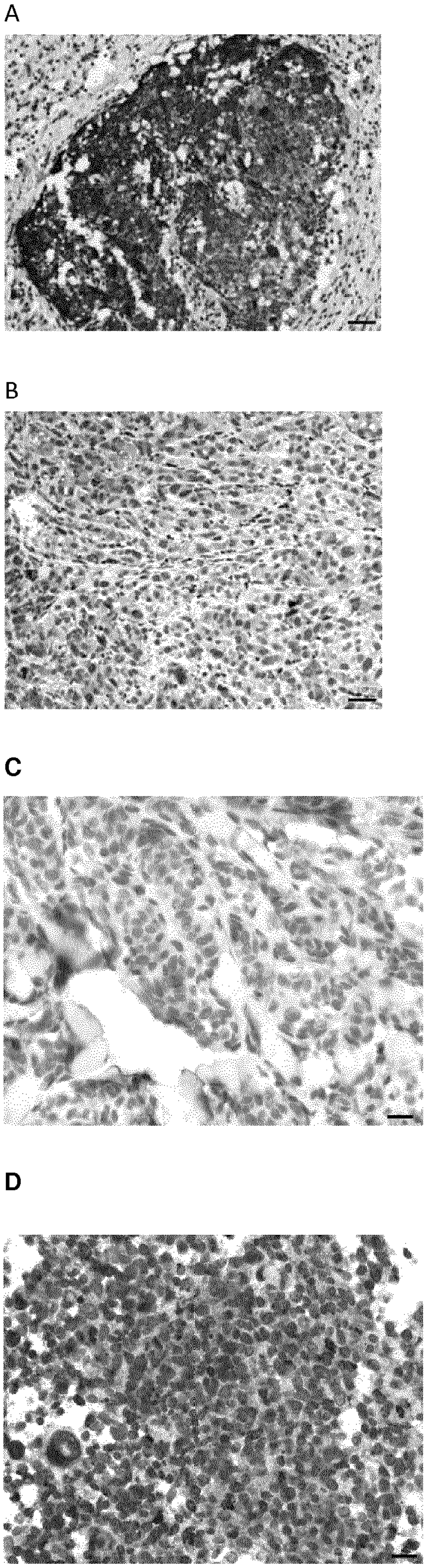

Figure 28

A

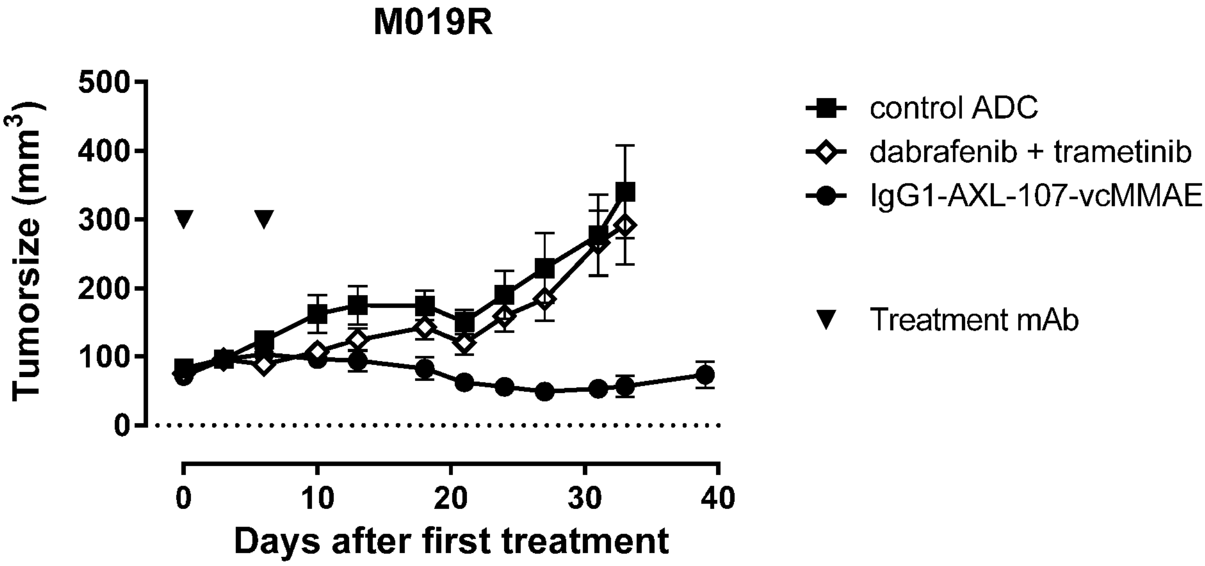
M019R
Tumorsize (mm³)
500
400
300
200
100
0
0
10
20
30
40
Days after first treatment
control ADC
dabrafenib + trametinib
IgG1-AXL-107-vcMMAE
Treatment mAb

B

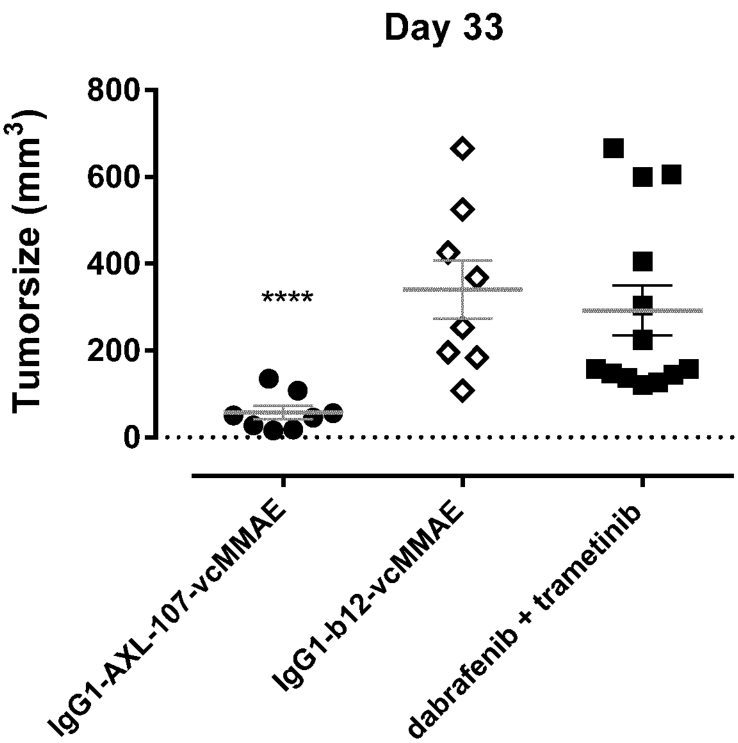
Day 33
Tumorsize (mm³)
800
600
400
200
0
****
IgG1-AXL-107-vcMMAE
IgG1-b12-vcMMAE
dabrafenib + trametinib

**Figure 28 (continued)**

C

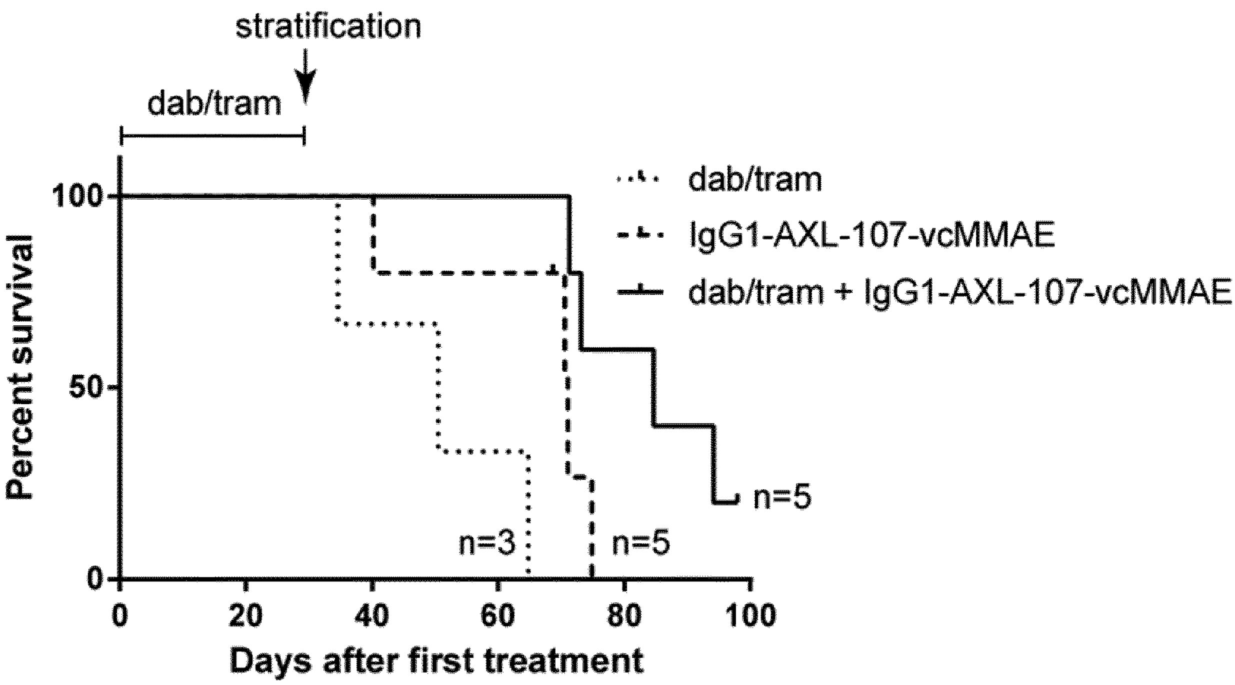

Figure 29

A

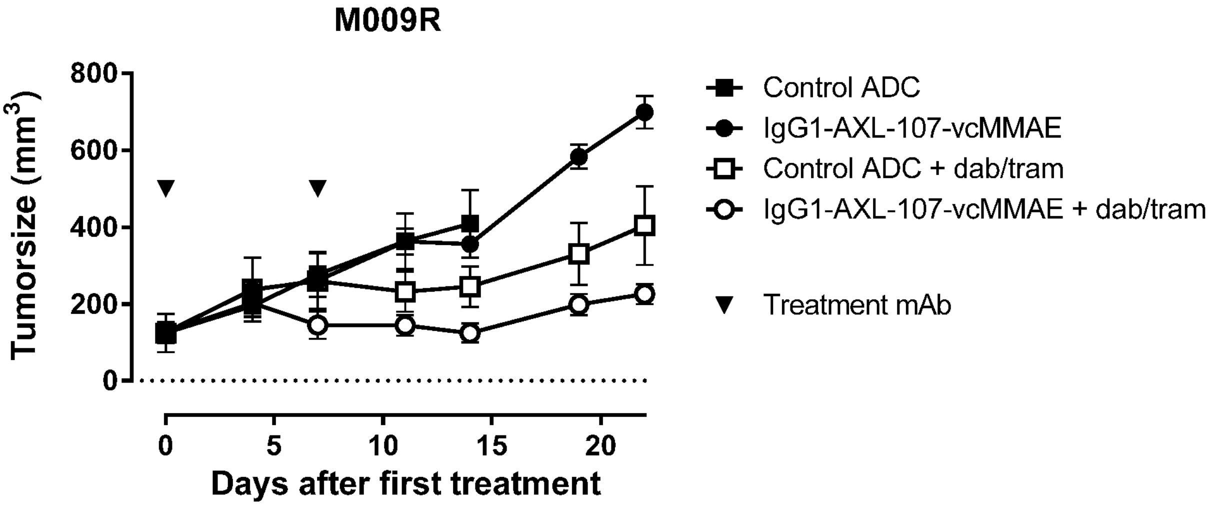
M009R
Tumorsize (mm3)
800
600
400
200
0
0
5
10
15
20
Days after first treatment
Control ADC
IgG1-AXL-107-vcMMAE
Control ADC + dab/tram
IgG1-AXL-107-vcMMAE + dab/tram
Treatment mAb

B

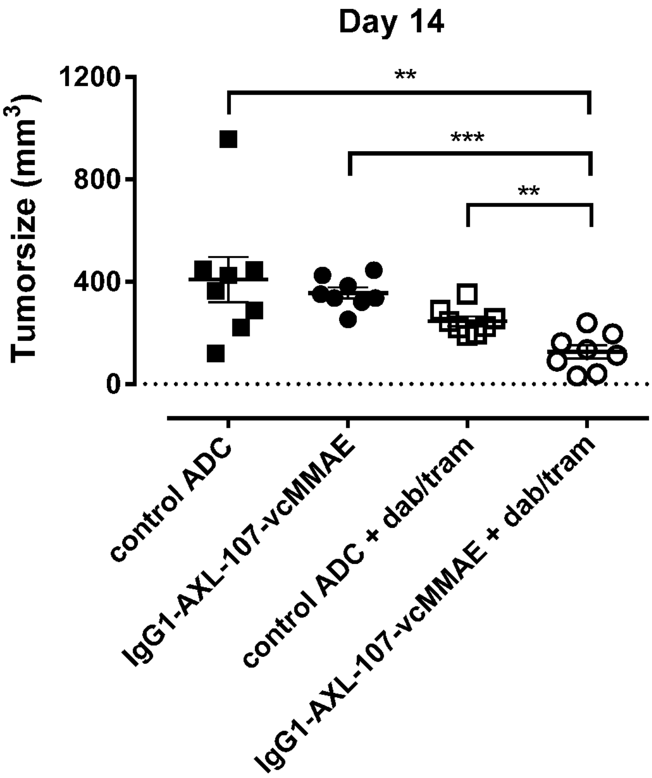
Day 14
Tumorsize (mm3)
1200
800
400
0
**
***
**
control ADC
IgG1-AXL-107-vcMMAE
control ADC + dab/tram
IgG1-AXL-107-vcMMAE + dab/tram

Figure 30

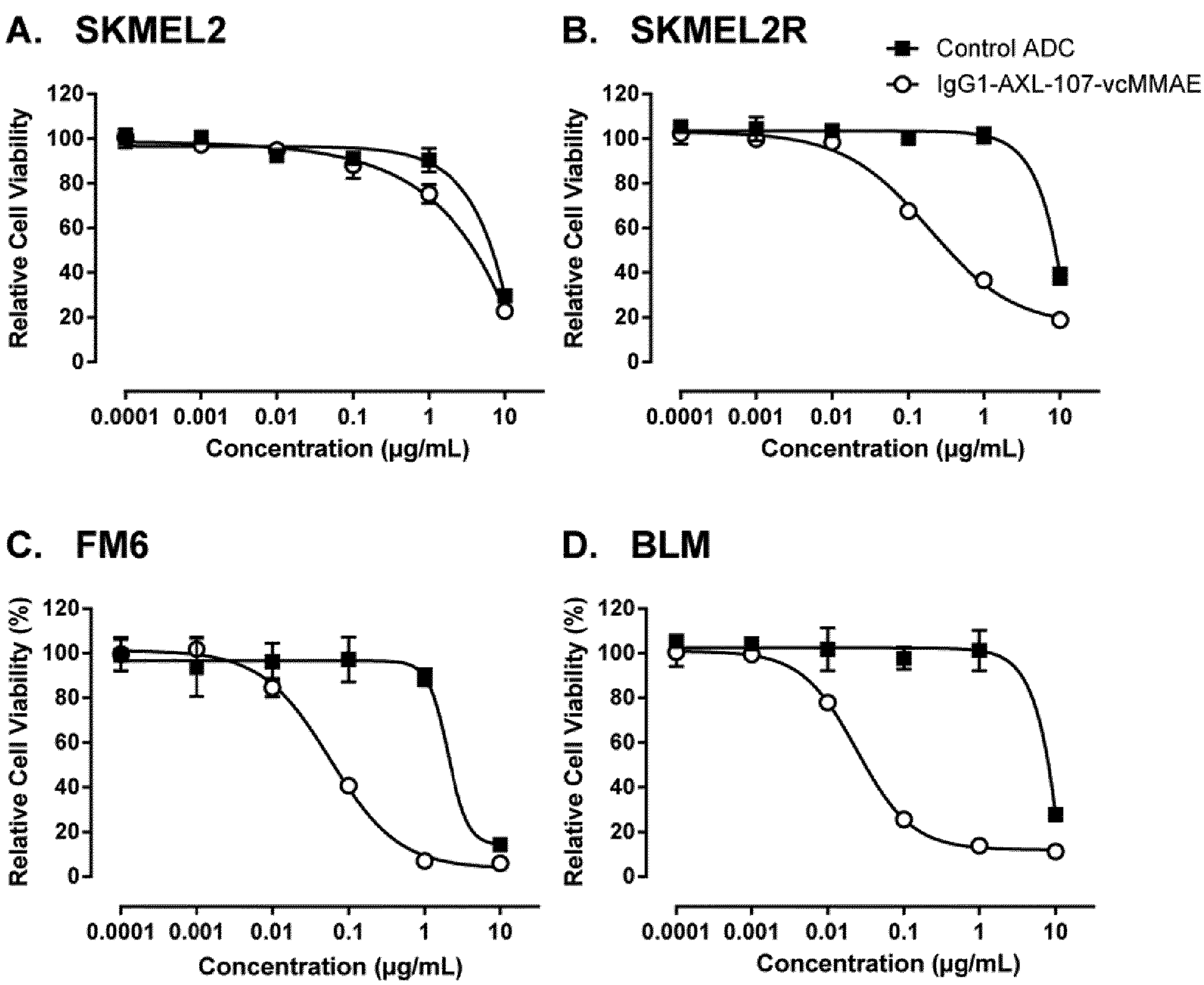
A. SKMEL2
B. SKMEL2R
C. FM6
D. BLM
Control ADC
IgG1-AXL-107-vcMMAE
Relative Cell Viability
Relative Cell Viability (%)
Concentration (µg/mL)
0.0001 0.001 0.01 0.1 1 10
0 20 40 60 80 100 120

Figure 31

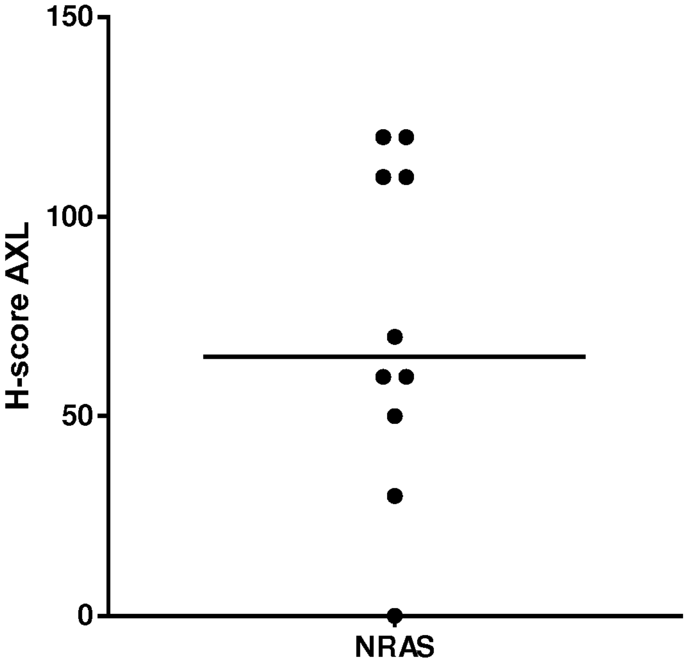

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011159980 A
- WO 2012175691 A
- WO 2012175692 A
- WO 2013064685 A
- WO 2013090776 A
- WO 2009063965 A
- WO 2010131733 A
- WO 2016005593 A
- WO 2014174111 A
- WO 2014068139 A
- WO 2015193430 A
- WO 2007059782 A
- WO 09062690 A
- WO 2010130751 A
- WO 2014093707 A
- EP 2228392 A1
- WO 2011131746 A
- WO 2002020039 A
- WO 9850431 A
- WO 2009080251 A
- WO 2009080252 A
- WO 2009080253 A
- EP 1870459 A
- WO 2009089004 A
- US 201000155133 A
- WO 2010129304 A
- WO 2007110205 A
- WO 11143545 A
- WO 2012058768 A
- WO 2011028952 A
- WO 2009080254 A
- US 20140348839 A
- WO 2013157953 A
- WO 2012023053 A
- WO 2010151792 A
- WO 2008003116 A
- US 761218 A
- WO 20100226923 A
- US 007951918 B
- CN 102250246
- WO 2012025525 A
- WO 2012025530 A
- WO 2008157379 A
- WO 2010080538 A
- US 7262028 B
- US 7612181 B
- US 7951918 B
- US 5194594 A
- US 6214345 B
- US 20050238649 A
- US 5635483 A
- US 5780588 A
- US 5663149 A
- WO 2004010957 A
- US 7659241 B
- US 7829531 B
- US 7851437 B
- US 7498298 B
- US 7994135 B
- WO 2005081711 A
- WO 2013173391 A
- WO 2013173392 A
- WO 2013173393 A
- US 4681581 A
- US 4735210 A
- US 5101827 A
- US 5102990 A
- US 5648471 A
- US 5697902 A
- US 4766106 A
- US 4179337 A
- US 4495285 A
- US 4609546 A
- WO 2002083180 A
- WO 2004043493 A
- WO 2007018431 A
- WO 2007089149 A
- WO 2009017394 A
- WO 201062171 A
- US 6989452 B
- US 6077835 A
- WO 0070087 A
- WO 0046147 A
- US 5589466 A
- US 5973972 A
- US 5545806 A
- US 5569825 A
- US 5625126 A
- US 5633425 A
- US 5789650 A
- US 5877397 A
- US 5661016 A
- US 5814318 A
- US 5874299 A
- US 5770429 A
- US 5545807 A
- WO 98024884 A
- WO 94025585 A

- WO 93001227 A
- WO 92022645 A
- WO 92003918 A
- WO 01009187 A
- WO 2016005593 A1
- EP 2220131 A
- WO 2011117329 A
- WO 2010015792 A
- US 5057313 A
- US 6331175 B

**Non-patent literature cited in the description**


- **BREIJ et al.** *American Association of cancer research*, 2015, 1-2
- **BREIJ et al.** *Journal of clinical oncology*, 2015, vol. 33, 1-2
- **TSAI et al.** *Proc Natl Acad Sci U S A.*, 26 February 2008, vol. 105 (8), 3041-3046
- Fundamental Immunology. Raven Press, 1989
- **NEEDLEMAN** ; **WUNSCH**. *J. Mol. Biol.*, 1970, vol. 48, 443-453
- **RICE et al.** EMBOSS: The European Molecular Biology Open Software Suite. *Trends Genet.*, 2000, vol. 16, 276-277
- **PASTAN et al.** *Cell*, 1986, vol. 47, 641
- **GOLDENBERG**. *Calif. A Cancer Journal for Clinicians*, 1994, vol. 44, 43
- **GOODMAN et al.** *Additional techniques relevant to the preparation of antibody immunotoxins are provided in for instance*, 1990
- **DUBOWCHIK** ; **WALKER**. *Pharm. Therapeutics*, 1999, vol. 83, 67-123
- Remington: The Science and Practice of Pharmacy. 1995
- **ROBINBSON**. *Sustained and Controlled Release Drug Delivery Systems*, 1978
- **ASLANIDIS, C.** ; **P.J. DE JONG**. *Nucleic Acids Res*, 1990, vol. 18 (20), 6069-74
- **KOZAK et al.** *Gene*, 1999, vol. 234, 187-208
- **VINK, T. et al.** A simple, robust and highly efficient transient expression system for producing antibodies. *Methods*, 2014, vol. 65 (1), 5-10
- **OGAWA et al.** *Mol Pharm*, 2009, vol. 6 (2), 386-395
- **BAHADORAN et al.** *J Clin Oncol*, 01 July 2013, vol. 31 (19), e324-e326
- **BANSAL et al.** *Oncotarget.*, 20 June 2015, vol. 6 (17), 15321-31
- **BLAKELY et al.** *Cancer Discov.*, October 2012, vol. 2 (10), 872-5
- **BLEEKER et al.** *J Immunol.*, 01 October 2004, vol. 173 (7), 4699-707
- **BOLLAG et al.** *Nat Rev Drug Discov*, November 2012, vol. 11 (11), 873-86
- **BRAND et al.** *Clin Cancer Res.*, 01 June 2015, vol. 21 (11), 2601-12
- **COLOMBINO et al.** *J Clin Oncol*, 2012, vol. 30 (20), 2522-9
- **DAHLMAN et al.** *Cancer Discov.*, 13 July 2012, vol. 2 (9), 791-7
- **DEBRUYNE et al.** *Oncogene*, 30 November 2015
- **DUFIES et al.** *Oncotarget.*, November 2011, vol. 2 (11), 874-85
- **ELKABETS et al.** *Cancer Cell*, 13 April 2015, vol. 27 (4), 533-46
- **GREIG et al.** *Drugs*, February 2016, vol. 76 (2), 263-73
- **HERBST et al.** *Expert Opin Investig Drugs.*, February 2007, vol. 16 (2), 239-49
- **HILGER et al.** *Int J Clin Pharmacol Ther.*, December 2002, vol. 40 (12), 567-8
- **HONG et al.** *Cancer Lett.*, 18 September 2008, vol. 268 (2), 314-24
- **HONG et al.** *Cancer Res.*, 01 January 2013, vol. 73 (1), 331-40
- **HONG et al.** *Clin Cancer Res.*, 15 April 2012, vol. 18 (8), 2326-35
- **HUANG et al.** *Cancer Res.*, 15 September 2010, vol. 70 (18), 7221-31
- **KIM et al.** *Curr Opin Mol Ther.*, February 2004, vol. 6 (1), 96-103
- **KIM et al.** *Mol Oncol.*, December 2013, vol. 7 (6), 1093-102
- **KONIECZKOWSKI et al.** *Cancer Discov*, 2014, vol. 4, 816-827
- **LI et al.** *Oncogene*, 07 August 2008, vol. 27 (34), 4702-11
- **LI et al.** *Cancer Lett.*, 28 January 2016, vol. 370 (2), 332-44
- **LIU et al.** *Cancer Res.*, 01 September 2009, vol. 69 (17), 6871-8
- **MAHADEVAN et al.** *Oncotarget*, 10 February 2015, vol. 6 (4), 1954-66
- **MORDANT et al.** *Mol Cancer Ther.*, February 2010, vol. 9 (2), 358-68
- **MÜLLER et al.** *Nat Commun.*, 15 December 2014, vol. 5, 5712
- **PARK et al.** *Leukemia.*, December 2015, vol. 29 (12), 2382-9
- **PETTAZZONI et al.** *Cancer Research*, 2015, vol. 75, 1091-1101
- **POLLACK et al.** *J Pharmacol Exp Ther.*, November 1999, vol. 291 (2), 739-48
- **PREWETT et al.** *J Immunother Emphasis Tumor Immunol.*, November 1996, vol. 19 (6), 419-27

- **SIROTNAK et al.** *Clin Cancer Res.*, December 2000, vol. 6 (12), 4885-92
- **TALAVERA et al.** *Cancer Res.*, 15 July 2009, vol. 69 (14), 5851-9
- **TAN et al.** *Lung Cancer.*, May 2012, vol. 76 (2), 177-82
- **WILSON et al.** *Cancer Res.*, 15 October 2014, vol. 74 (20), 5878-90
- **WONG et al.** *J Pharmacol Exp Ther.*, April 2009, vol. 329 (1), 360-7
- **XIA et al.** *Oncogene*, 12 September 2002, vol. 21 (41), 6255-63
- **YANG et al.** *Crit Rev Oncol Hematol.*, April 2001, vol. 38 (1), 17-23
- **ZHANG et al.** *Nat Genet.*, 01 July 2012, vol. 44 (8), 852-60
- **ZHOU et al.** *Oncogene*, May 2016, vol. 35 (21), 2687-97
- **YANG et al.** *EORTC meeting 2013, Poster 493*, 2013
- **YANG et al.** *Int. J. Cancer*, 2013, vol. 132, E74-E84
- **PACCEZ et al.** *Int. J. Cancer*, 04 June 2013, vol. 134, 1024-1033
- **LECONET et al.** *Oncogene*, 2013, vol. 1-10
- **LINGER et al.** *Expert Opin. Ther. Targets*, 2010, vol. 14 (10), 1073-1090
- **LI et al.** *Oncogene*, 2009, vol. 28, 3442-3455
- **YE et al.** *Oncogene*, 2010, vol. 1-11
- **ALLEY et al.** *Current Opinion in Chem. Bio.*, 2010, vol. 4, 529-537
- **LIDA et al.** *Anticancer Research*, 2014, vol. 34, 1821-1828
- **TSCHUCH et al.** *AACR-EORTC meeting 2015, Poster A10*, 2015
- **KING et al.** *Cancer Res.*, 01 December 2006, vol. 66 (23), 11100-5
- **MONTAGUT et al.** *J.Cancer Res.*, 15 June 2008, vol. 68 (12), 4853-61
- **SEQUIST et al.** *N Engl J Med.*, 06 August 2015, vol. 373 (6), 578-9
- **LI et al.** *Structure.*, February 2008, vol. 16 (2), 216-27
- **PEDERSEN et al.** *Cancer Res.*, 15 January 2010, vol. 70 (2), 588-97
- **MISHIMA et al.** *Cancer Res.*, 15 July 2001, vol. 61 (14), 5349-54
- **LYNETTE et al.** *J Chem Biol*, 2009, vol. 2, 131-151
- **UITDEHAAG et al.** *Br J Pharmacol.*, June 2012, vol. 166 (3), 858-876
- **SULLIVAN**. *Curr Opin Oncol*, March 2016, vol. 28 (2), 185-91
- **BOLLAG et al.** *Nature*, 2010, vol. 467 (7315), 596-599
- **LAQUERRE et al.** *EORTC International Conference. Abst B88*, 2009
- **STUART et al.** *Cancer Res*, 2012, vol. 72 (8), 3790
- **WILHELM et al.** *Cancer Res*, 2004, vol. 64 (19), 7099-7109
- **YAMAGUCHI et al.** *Int J Oncol*, 2011, vol. 39 (1), 23-31
- **HOEFLICH KP et al.** *Cancer Res.*, 2012, vol. 72 (1), 210-219
- **HUYNH H et al.** *Mol Cancer Therapy*, 2007, vol. 6 (1), 138-146
- **J PHENEGER et al.** *ACR Annual Scientific Meeting. Abst 794*, 2006
- **IVERSON et al.** *Cancer Res*, 2009, vol. 69 (17), 6839-6847
- **KIM K et al.** *Br J Haematol*, 2010, vol. 149 (4), 537-549
- **SEBOLT-LEOPOLD et al.** *Nat Med*, 1999, vol. 5 (7), 810-816
- **TATAKE RJ et al.** *Biochem Biophys Res Commun*, 2008, vol. 377 (1), 120-125
- **ARONOV et al.** *J Med Chem*, 2009, vol. 52 (20), 6362-6368
- **WARD et al.** *J Med Chem*, 2015, vol. 58 (11), 4790-4801
- **HFIZI et al.** *FEBS Journal*, 2006, vol. 273, 5231-5244
- **WARD et al.** *Nature*, 1989, vol. 341, 544-546
- **HOLT et al.** *Trends Biotechnol.*, November 2003, vol. 21 (11), 484-90
- **REVETS et al.** *Expert Opin Biol Ther.*, January 2005, vol. 5 (1), 111-24
- **BIRD et al.** *Science*, 1988, vol. 242, 423-426
- **HUSTON et al.** *PNAS USA*, 1988, vol. 85, 5879-5883
- **LEFRANC MP. et al.** *Nucleic Acids Research*, 1999, vol. 27, 209-212
- **BROCHET X**. *Nucl. Acids Res.*, 2008, vol. 36, W503-508
- **KORSHUNOV et al.** *Clin Sci (Lond).*, April 2012, vol. 122 (8), 361-8
- **SAMBROOK et al.** Molecular Cloning: A laboratory Manual, New York. Cold Spring Harbor Laboratory Press, 1989
- **KABAT, E.A. et al.** Sequences of proteins of immunological interest. US Department of Health and Human Services, NIH, 1991, 662, 680, 689
- Generation and Characterization of a Dual Variable Domain Immunoglobulin (DVD-Ig™) Molecule. **WU**. Antibody Engineering. Springer Berlin Heidelberg, 2010
- **GOODMAN et al.** Goodman and Gilman’s The Pharmacological Basis Of Therapeutics. Macmillan Publishing Co., 1990
- **VITETTA**. *Immunol. Today*, 1993, vol. 14, 252
- **JUNGHANS et al.** Cancer Chemotherapy and Biotherapy. Lippincott Raven, 1996, 655-686
- **HUNTER et al.** *Nature*, 1962, vol. 144, 945

- **DAVID et al.** *Biochemistry*, 1974, vol. 13, 1014
- **PAIN et al.** *J. Immunol. Meth.*, 1981, vol. 40, 219
- **NYGREN**. *J. Histochem. and Cytochem.*, 1982, vol. 30, 407
- Antibody Engineering Handbook. Chijin Shokan, 1994
- Remington: The Science and Practice of Pharmacy. Mack Publishing Co., 1995
- Sustained and Controlled Release Drug Delivery Systems. Marcel Dekker, Inc., 1978
- **SYKES** ; **JOHNSTON**. *Nat Biotech*, 1997, vol. 17, 355-59
- **SCHAKOWSKI et al.** *Mol Ther*, 2001, vol. 3, 793-800
- **BENVENISTY** ; **RESHEF**. *PNAS USA*, 1986, vol. 83, 9551-55
- **WIGLER et al.** *Cell*, 1978, vol. 14, 725
- **CORARO** ; **PEARSON**. *Somatic Cell Genetics*, 1981, vol. 7, 603
- **VAN HEEKE** ; **SCHUSTER**. *J Biol Chem*, 1989, vol. 264, 5503-5509
- Current Protocols in Molecular Biology. Greene Publishing and Wiley InterScience, 1987
- **GRANT et al.** *Methods in Enzymol*, 1987, vol. 153, 516-544
- **LONBERG, N. et al.** *Nature*, 1994, vol. 368, 856-859
- **LONBERG, N.** Handbook of Experimental Pharmacology. 1994, vol. 113, 49-101
- **LONBERG, N.** ; **HUSZAR, D.** *Intern. Rev. Immunol.*, 1995, vol. 13, 65-93
- **HARDING, F.** ; **LONBERG, N.** *Ann. N.Y. Acad. Sci*, 1995, vol. 764, 536-546
- **TAYLOR, L. et al.** *Nucleic Acids Research*, 1992, vol. 20, 6287-6295
- **CHEN, J. et al.** *International Immunology*, 1993, vol. 5, 647-656
- **TUAILLON et al.** *J. Immunol.*, 1994, vol. 152, 2912-2920
- **TAYLOR, L. et al.** *International Immunology*, 1994, vol. 6, 579-591
- **FISHWILD, D. et al.** *Nature Biotechnology*, 1996, vol. 14, 845-851
- **SHIEH**. *Neoplasia*, 2005
- **KOORSTRA**. *Cancer Biol Ther*, 2009
- **HECTOR**. *Cancer Biol Ther*, 2010
- **SUN**. *Ann Oncol*, 2003
- Radiolabeled Monoclonal Antibodies For Imaging And Therapy. Plenum Press, 1988
- Medical Applications of Radioisotopes. **CHASE**. Remington's Pharmaceutical Sciences. Mack Publishing Co., 1990, 624-652
- Clinical Use of Monoclonal Antibodies. **BROWN et al.** Biotechnology And Pharmacy. Chapman & Hall, 1993, 227-49
- **WOYKE et al.** *Antimicrob. Agents and Chemother.*, 2001, vol. 45 (12), 3580-3584
- **PETTIT et al.** *Antimicrob. Agents and Chemother.*, 1998, vol. 42, 2961-2965
- **SENTER et al.** *Proceedings of the American Association for Cancer Research*, 28 March 2004, vol. 45
- **BARBAS, CF.** *J Mol Biol.*, 05 April 1993, vol. 230 (3), 812-23
- **SUN et al.** *Bioconjugate Chem.*, 2005, vol. 16, 1282-1290
- **MCDONAGH et al.** *Protein Eng. Design Sel.*, 2006, vol. 19, 299-307
- **ALLEY et al.** *Bioconjugate Chem.*, 2008, vol. 19, 759-765